# EUROPEAN PATENT SPECIFICATION

(11) **EP 3 835 299 B1**
(45) Date of publication and mention of the grant of the patent: **08.03.2023**
(21) Application number: 19862593.1
(22) Date of filing: 20.09.2019
(51) Int. Cl.: C07D 409/14, C07D 487/04, C07D 498/04, C07D 513/04, H01L 51/00, H01L 51/50, C07D 405/14, C07D 491/048, C07D 495/04

(54) **NOVEL HETEROCYCLIC COMPOUND AND ORGANIC LIGHT EMITTING DEVICE USING SAME**
NEUARTIGE HETEROCYCLISCHE VERBINDUNG UND ORGANISCHE LICHTEMITTIERENDE VORRICHTUNG MIT VERWENDUNG DAVON
NOUVEAU COMPOSÉ HÉTÉROCYCLIQUE ET DISPOSITIF ÉLECTROLUMINESCENT ORGANIQUE L'UTILISANT

(30) Priority: 21.09.2018 KR 20180114411; 19.09.2019 KR 20190115650
(43) Date of publication of application: 16.06.2021
(73) Proprietor: LG Chem, Ltd., Seoul 07336, (KR)
(72) Inventor: HAN, Su Jin, Daejeon 34122 (KR); LEE, Dong Hoon, Daejeon 34122 (KR); JANG, Boon Jae, Daejeon 34122 (KR); HONG, Wanpyo, Daejeon 34122 (KR); JUNG, Min Woo, Daejeon 34122 (KR); LEE, Jungha, Daejeon 34122 (KR); PARK, Seulchan, Daejeon 34122 (KR); HWANG, Sunghyun, Daejeon 34122 (KR)
(74) Representative: Hoffmann Eitle
(86) International application number: PCT/KR2019/012236
(87) International publication number: WO 2020/060286

(56) References cited:
- EP-A1- 3 257 850
- WO-A1-2015/165563
- WO-A1-2019/093666
- JP-A- 2017 092 276
- KR-A- 20170 053 590
- KR-A- 20170 067 671
- KR-A- 20180 051 355
- KR-A- 20180 068 882
- US-A1- 2018 166 634

## Description

### BACKGROUND OF THE INVENTION

### (a) Field of the Invention

### CROSS-REFERENCE TO RELATED APPLICATION(S)

This application claims the benefit of Korean Patent Application No. 10-2018-0114411 filed on September 21, 2018 and Korean Patent Application No. 10-2019-0115650 filed on September 19, 2019 in the Korean Intellectual Property Office.

The present disclosure relates to a novel heterocyclic compound and to an organic light emitting device including the same.

### (b) Description of the Related Art

In general, an organic light emitting phenomenon refers to a phenomenon where electric energy is converted into light energy by using an organic material. The organic light emitting device using the organic light emitting phenomenon has characteristics such as a wide viewing angle, an excellent contrast, a fast response time, an excellent luminance, driving voltage and response speed, and thus many studies have proceeded.

The organic light emitting device generally has a structure which comprises an anode, a cathode, and an organic material layer interposed between the anode and the cathode. The organic material layer frequently has a multilayered structure that comprises different materials in order to enhance efficiency and stability of the organic light emitting device, and for example, the organic material layer may be formed of a hole injection layer, a hole transport layer, a light emitting layer, an electron transport layer, an electron injection layer and the like. In the structure of the organic light emitting device, if a voltage is applied between two electrodes, the holes are injected from an anode into the organic material layer and the electrons are injected from the cathode into the organic material layer, and when the injected holes and electrons meet each other, an exciton is formed, and light is emitted when the exciton falls to a ground state again.

US 2018/166634 A1, EP 3 257 850 A1 and WO 2015/165563 A1 describe compunds for use in organic light emitting devices having a structure related to the compounds of the present invention.

There is a continuing need for the development of new materials for the organic materials used in these organic light emitting devices.

### [Prior Art Literature]

### [Patent Literature]

(Patent Literature 0001) Korean Unexamined Patent Publication No. 10-2013-073537

### SUMMARY OF THE INVENTION

### [Technical Problem]

It is an object of the present disclosure to provide a novel heterocyclic compound and an organic light emitting device including the same.

### [Technical Solution]

In one aspect of the invention, there is provided a compound represented by the following Chemical Formula 1: 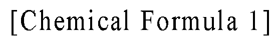 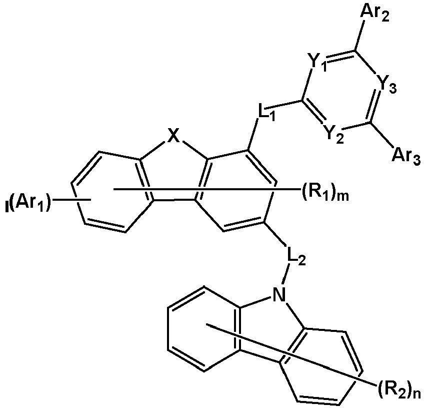 wherein, in Chemical Formula 1,
X is O or S,
Y₁, Y₂ and Y₃ are each N,
L₁ and L₂ are each independently a direct bond or any one selected from the group consisting of: 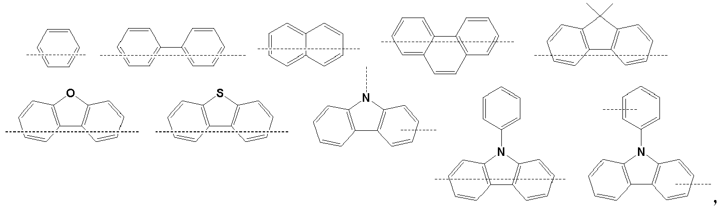
Ar₁ is a substituted or unsubstituted C₆₋₆₀ aryl,
Ar₂ and Ar₃ are each independently a substituted or unsubstituted C₆₋₆₀ aryl; or a substituted or unsubstituted C₅₋₆₀ heteroaryl containing at least one heteroatom selected from the group consisting of N, O and S,
provided that when all of Ar₁, Ar₂ and Ar₃ are a substituted or unsubstituted C₆₋₆₀ aryl, any one of Ar₁, Ar₂ and Ar₃ is substituted with 4 or more deuteriums, wherein all of Ar₁, Ar₂ and Ar₃ are a substituted or unsubstituted C₆₋₆₀ aryl, it means a case where they are the same or different substituted or unsubstituted C₆₋₆₀ aryl
each R₁ is independently hydrogen; deuterium; halogen, or a substituted or unsubstituted C₁₋₆₀ alkyl,
each R₂ is independently hydrogen; deuterium; halogen; cyano; a substituted or unsubstituted C₁₋₆₀ alkyl; a substituted or unsubstituted C₁₋₆₀ alkoxy; a substituted or unsubstituted C₃₋₆₀ cycloalkyl; a substituted or unsubstituted C₁₋₆₀ haloalkyl; a substituted or unsubstituted C₁₋₆₀ haloalkoxy; a substituted or unsubstituted C₆₋₆₀ arylamine; a substituted or unsubstituted C₁₋₆₀ alkylamine; a C₁₋₆₀ trifluoroalkyl; a C₁₋₆₀ trifluoroalkoxy; a substituted or unsubstituted C₆₋₆₀ aryl; or a substituted or unsubstituted C₅₋₆₀ heteroaryl containing at least one of N, O and S, which can bond with the carbon atom of carbazole to form a condensed ring,
m is an integer from 0 to 3,
n is an integer from 0 to 6, and
| is 1 or 2.

In another aspect of the invention, there is provided an organic light emitting device including: a first electrode; a second electrode that is provided opposite to the first electrode; and one or more organic material layers that are provided between the first electrode and the second electrode, wherein one or more layers of the organic material layers include the above-mentioned compound of the present disclosure.

### [ADVANTAGEOUS EFFECTS]

The above-mentioned compound represented by Chemical Formula 1 can be used as a material of an organic material layer of an organic light emitting device and may improve the efficiency, achieve low driving voltage and/or improve lifetime characteristics in the organic light emitting device. In particular, the compound represented by Chemical Formula 1 may be used as a hole injection material, hole transport material, hole injection and transport material, light emitting material, electron transport material, or electron injection material.

### BRIEF DESCRIPTION OF THE DRAWINGS

FIG. 1 shows an example of an organic light emitting device comprising a substrate 1, an anode 2, a light emitting layer 3, and a cathode 4.
FIG. 2 shows an example of an organic light emitting device comprising a substrate 1, an anode 2, a hole injection layer 5, a hole transport layer 6, a light emitting layer 7, an electron transport layer 8, and a cathode 4.

### DETAILED DESCRIPTION OF THE EMBODIMENTS

Hereinafter, embodiments of the present disclosure will be described in more detail to facilitate understanding of the invention.

One embodiment of the invention provides a compound represented by the following Chemical Formula 1: 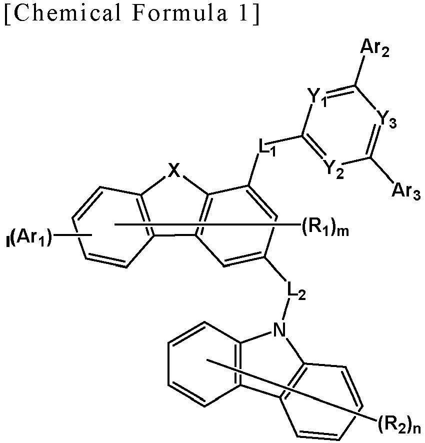 wherein, in Chemical Formula 1,
X is O or S,
Y₁, Y₂ and Y₃ are each independently N,
L₁ and L₂ are each independently a direct bond or any one selected from the group consisting of: 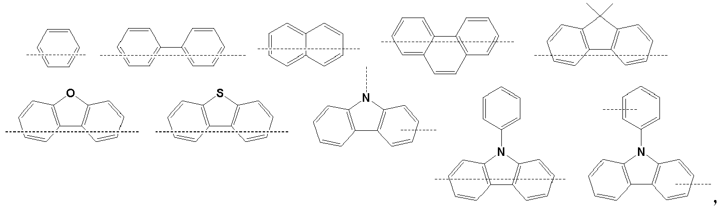
Ar₁ is a substituted or unsubstituted C₆₋₆₀ aryl,
Ar₂ and Ar₃ are each independently a substituted or unsubstituted C₆₋₆₀ aryl; or a substituted or unsubstituted C₅₋₆₀ heteroaryl containing at least one heteroatom selected from the group consisting of N, O and S,
provided that when all of Ar₁, Ar₂ and Ar₃ are a substituted or unsubstituted C₆₋₆₀ aryl, any one of Ar₁, Ar₂ and Ar₃ is substituted with 4 or more deuteriums, wherein all of Ar₁, Ar₂ and Ar₃ are a substituted or unsubstituted C₆₋₆₀ aryl, it means a case where they are the same or different substituted or unsubstituted C₆₋₆₀ aryl.
each R₁ is independently hydrogen; deuterium; halogen; or a substituted or unsubstituted C₁₋₆₀ alkyl,
each R₂ is independently hydrogen; deuterium; halogen; cyano; a substituted or unsubstituted C₁₋₆₀ alkyl; a substituted or unsubstituted C₁₋₆₀ alkoxy; a substituted or unsubstituted C₃₋₆₀ cycloalkyl; a substituted or unsubstituted C₁₋₆₀ haloalkyl; a substituted or unsubstituted C₁₋₆₀ haloalkoxy; a substituted or unsubstituted C₆₋₆₀ arylamine; a substituted or unsubstituted C₁₋₆₀ alkylamine; a C₁₋₆₀ trifluoroalkyl; a C₁₋₆₀ trifluoroalkoxy; a substituted or unsubstituted C₆₋₆₀ aryl; or a substituted or unsubstituted C₅₋₆₀ heteroaryl containing at least one of N, O and S, which can bond with the carbon atom of carbazole to form a condensed ring,
m is an integer from 0 to 3,
n is an integer from 0 to 6, and
| is 1 or 2.

In the above, when Ar₁, Ar₂ and Ar₃ are a substituted or unsubstituted C₆₋₆₀ aryl, it means a case where Ar₁, Ar₂ and Ar₃ are simultaneously the same or different substituted or unsubstituted C₆₋₆₀ aryl.

As used herein, the notation and 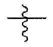 and 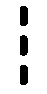 mean a bond linked to another substituent group.

As used herein, the term "substituted or unsubstituted" means being unsubstituted or substituted with one or more substituents selected from the group consisting of deuterium; a halogen group; a nitrile group; a nitro group; a hydroxy group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; or a heterocyclic group containing at least one of N, O and S atoms, or being unsubstituted or substituted with a substituent to which two or more substituents are linked among the substituents exemplified above. For example, "the substituent to which two or more substituents are linked" may be a biphenyl group. That is, the biphenyl group may also be an aryl group and may be interpreted as a substituent to which two phenyl groups are linked.

In the present specification, the number of carbon atoms of a carbonyl group is not particularly limited, but is preferably 1 to 40. Specifically, the carbonyl group may be a compound having the following structural formulae but is not limited thereto.

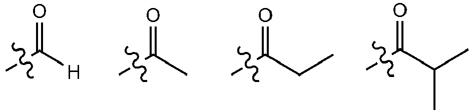

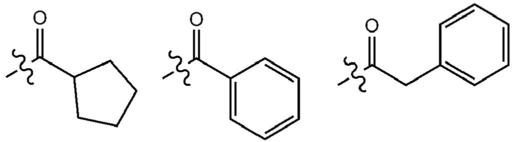

In the present specification, an ester group may have a structure in which oxygen of the ester group may be substituted with a straight-chain, branched-chain, or cyclic alkyl group having 1 to 25 carbon atoms, or an aryl group having 6 to 25 carbon atoms. Specifically, the ester group may be a compound having the following structural formulae, but is not limited thereto.

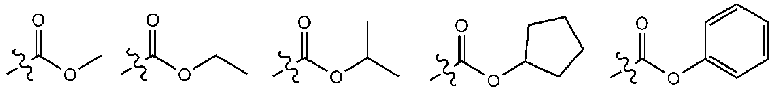

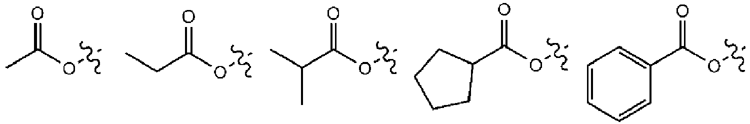

In the present specification, the number of carbon atoms of an imide group is not particularly limited, but is preferably 1 to 25. Specifically, the imide group may be a compound having the following structural formulae, but is not limited thereto.

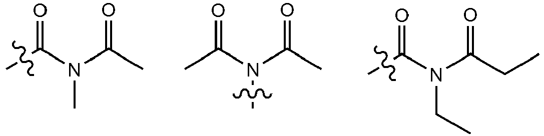

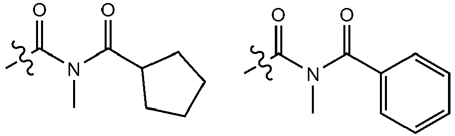

In the present specification, a silyl group specifically includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but is not limited thereto.

In the present specification, a boron group specifically includes a trimethylboron group, a triethylboron group, a t-butyldimethylboron group, a triphenylboron group, and a phenylboron group, but is not limited thereto.

In the present specification, examples of a halogen group include fluorine, chlorine, bromine, or iodine.

In the present specification, the alkyl group may be a straight-chain or branched chain, and the number of carbon atoms thereof is not particularly limited, but is preferably 1 to 40. According to one embodiment, the number of carbon atoms of the alkyl group is 1 to 20. According to another embodiment, the number of carbon atoms of the alkyl group is 1 to 10. According to another embodiment, the number of carbon atoms of the alkyl group is 1 to 6. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tertpentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 4-methylhexyl, 5-methylhexyl, and the like, but are not limited thereto.

In the present specification, the alkenyl group may be a straight chain or branched chain, and the number of carbon atoms thereof is not particularly limited, but is preferably 2 to 40. According to one embodiment, the number of carbon atoms of the alkenyl group is 2 to 20. According to another embodiment, the number of carbon atoms of the alkenyl group is 2 to 10. According to still another embodiment, the number of carbon atoms of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present specification, a cycloalkyl group is not particularly limited, but the number of carbon atoms thereof is preferably 3 to 60. According to one embodiment, the number of carbon atoms of the cycloalkyl group is 3 to 30. According to another embodiment, the number of carbon atoms of the cycloalkyl group is 3 to 20. According to still another embodiment, the number of carbon atoms of the cycloalkyl group is 3 to 6. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present specification, an aryl group is not particularly limited, but preferably has 6 to 60 carbon atoms, and may be a monocyclic aryl group or a polycyclic aryl group. According to one embodiment, the number of carbon atoms of the aryl group is 6 to 30. According to one embodiment, the number of carbon atoms of the aryl group is 6 to 20. The aryl group may be a phenyl group, a biphenyl group, a terphenyl group or the like as the monocyclic aryl group, but is not limited thereto. Examples of the polycyclic aryl group include a naphthyl group, an anthracenyl group, a phenanthryl group, a pyrenyl group, a perylenyl group, a chrysenyl group, a fluorenyl group or the like, but is not limited thereto.

In the present specification, a fluorenyl group may be substituted, and two substituent groups may be connected with each other to form a spiro structure. In the case where the fluorenyl group is substituted, 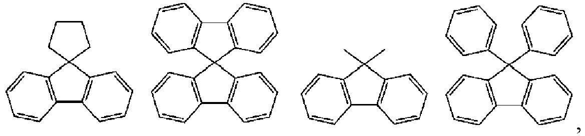 and the like can be formed. However, the structure is not limited thereto.

In the present specification, a heterocyclic group is a heterocyclic group including one or more of O, N, Si, and S as a heteroatom, and the number of carbon atoms thereof is not particularly limited, but is preferably 2 to 60. Examples of the heterocyclic group include a thiophene group, a furan group, a pyrrole group, an imidazole group, a thiazole group, an oxazol group, an oxadiazol group, a triazol group, a pyridyl group, a bipyridyl group, a pyrimidyl group, a triazine group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzimidazole group, a benzothiazol group, a benzocarbazole group, a benzothiophene group, a dibenzothiophene group, a benzofuranyl group, a phenanthroline group, an isoxazolyl group, an thiadiazolyl group, a phenothiazinyl group, a dibenzofuranyl group, and the like, but are not limited thereto.

In the present specification, the aryl group in the aralkyl group, the aralkenyl group, the alkylaryl group, and the arylamine group is the same as the aforementioned examples of the aryl group. In the present specification, the alkyl group in the aralkyl group, the alkylaryl group and the alkylamine group is the same as the aforementioned examples of the alkyl group. In the present specification, the heteroaryl in the heteroarylamine can be applied to the aforementioned description of the heteroaryl. In the present specification, the alkenyl group in the aralkenyl group is the same as the aforementioned examples of the alkenyl group. In the present specification, the aforementioned description of the aryl group may be applied except that the arylene is a divalent group. In the present specification, the aforementioned description of the heteroaryl can be applied except that the heteroarylene is a divalent group. In the present specification, the aforementioned description of the aryl group or cycloalkyl group can be applied except that the hydrocarbon ring is not a monovalent group but formed by combining two substituent groups. In the present specification, the aforementioned description of the heteroaryl can be applied, except that the heterocycle is not a monovalent group but formed by combining two substituent groups.

Preferably, the compound represented by Chemical Formula 1 may be any one selected from the group consisting of compounds represented by the following Chemical Formulae 2 to 8: 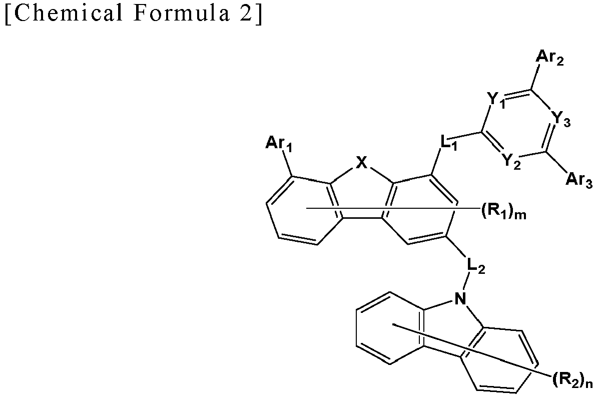 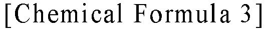 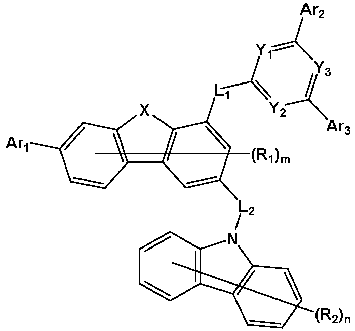 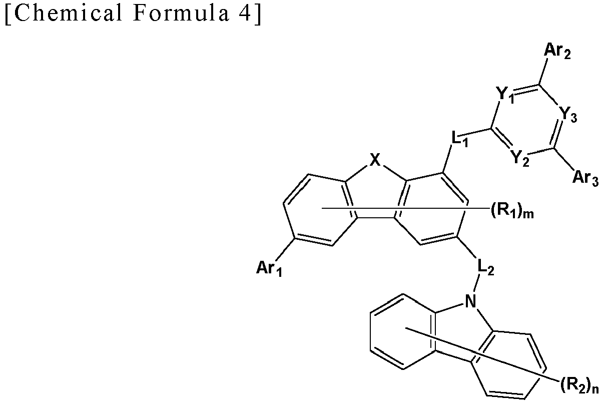 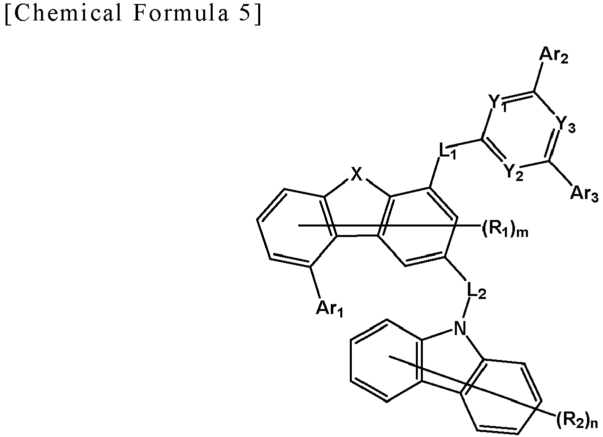 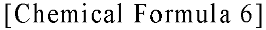 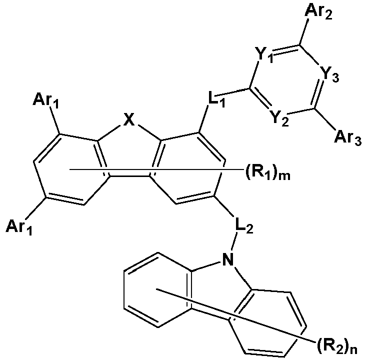 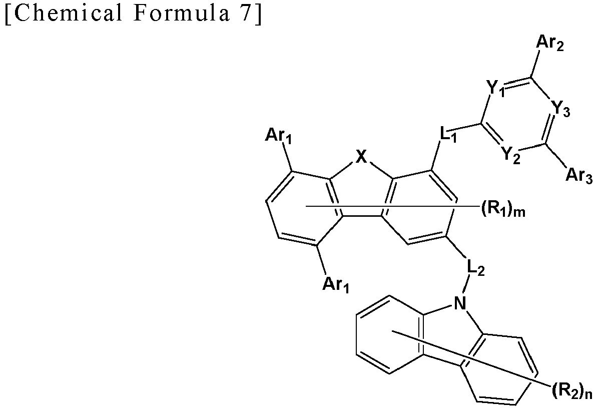 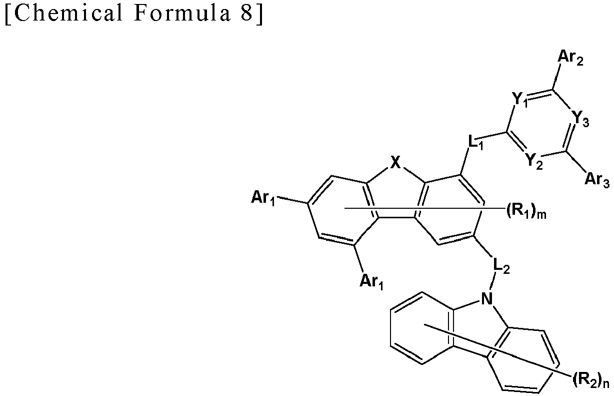 wherein, in Chemical Formulas 2 to 8,
X, Y₁, Y₂, Y₃, L₁, L₂, Ar₁, Ar₂, Ar₃, R₁, R₂, m and n are as previously defined.

Preferably, Ar₁ may be any one selected from the group consisting of:

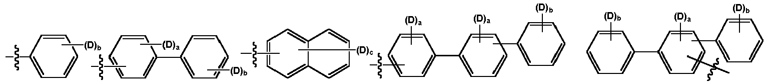

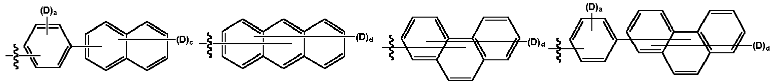

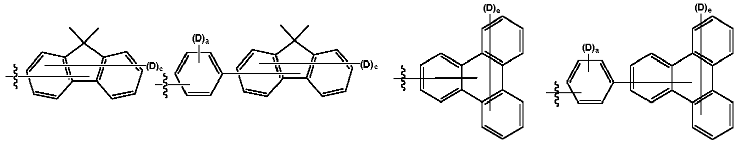

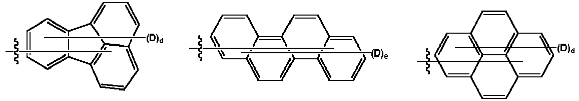

In the above formulas,
a is an integer from 0 to 4,
b is an integer from 0 to 5,
c is an integer from 0 to 7,
d is an integer from 0 to 9, and
e is an integer from 0 to 11.

Preferably, Ar₁ is each independently phenyl, biphenylyl, naphthyl, phenanthrenyl or triphenylenyl, which are unsubstituted or substituted with deuterium.

Preferably, Ar₂ and Ar₃ may be each independently any one selected from the group consisting of: 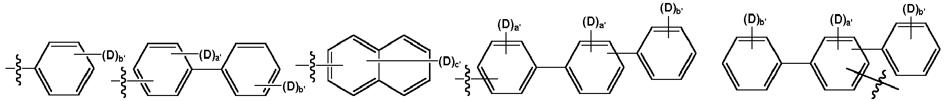 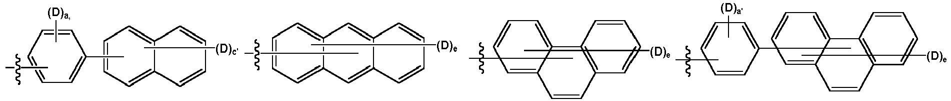 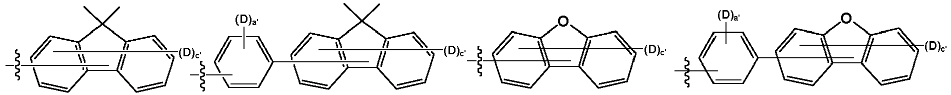 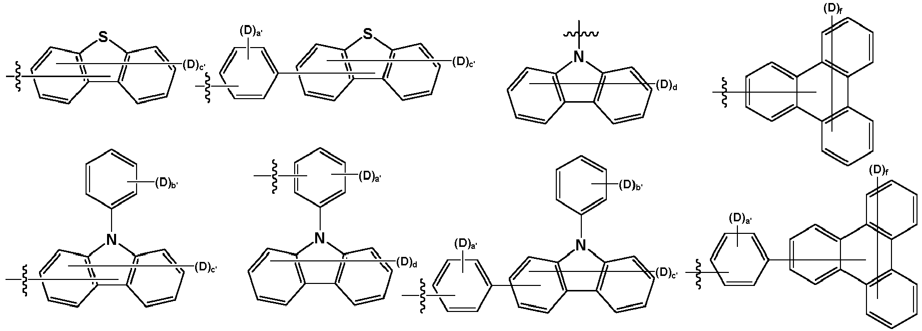 in the above formulas,
a' is an integer from 0 to 4,
b' is an integer from 0 to 5,
c' is an integer from 0 to 7,
d' is an integer from 0 to 8,
e' is an integer from 0 to 9, and
f' is an integer from 0 to 11.

Preferably, each R₁ may independently be hydrogen or deuterium, more preferably hydrogen.

Preferably, each R₂ is independently hydrogen; deuterium; halogen; cyano; methoxy; trifluoromethyl; trifluoromethoxy; phenyl; pyridinyl; isoquinolinyl; or any one selected from the group consisting of, 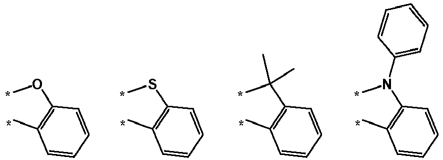
in the above formulas, two * are each connected to an adjacent carbon of the carbazole in Chemical Formula 1, and
the phenyl may be substituted with any one selected from the group consisting of halogen, cyano, methoxy, trifluoromethyl and trifluoromethoxy.

Preferably, in the compound represented by Chemical Formula 1, at least one of L₁, Ar₂ and Ar₃ may contain at least one heteroatom selected from the group consisting of N, O, and S.

Preferably, the compound represented by Chemical Formula 1 may be any one selected from the group consisting of:

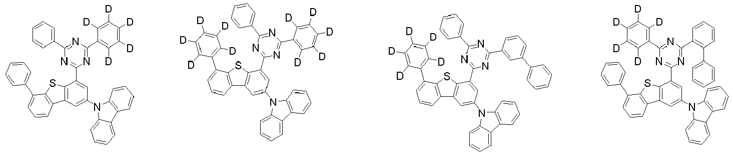

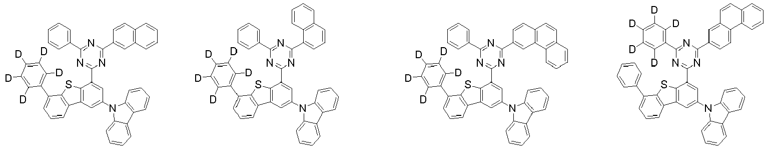

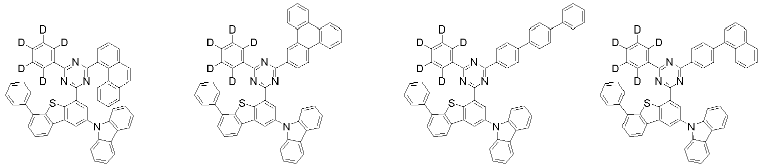

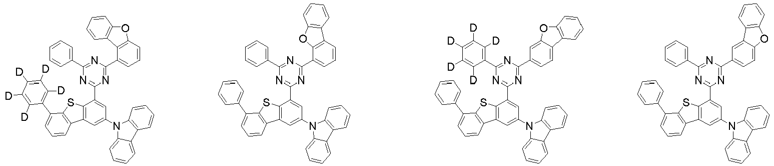

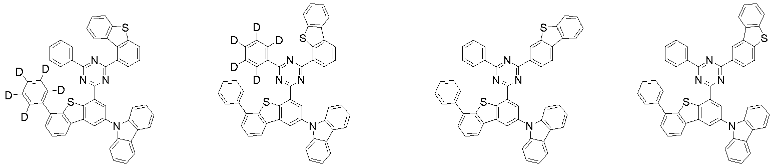

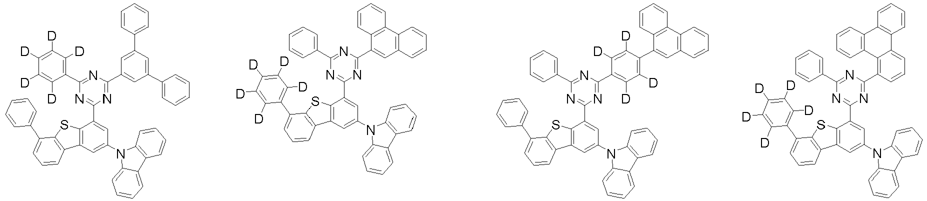

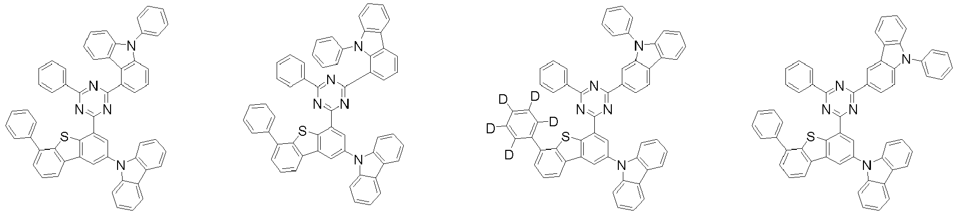

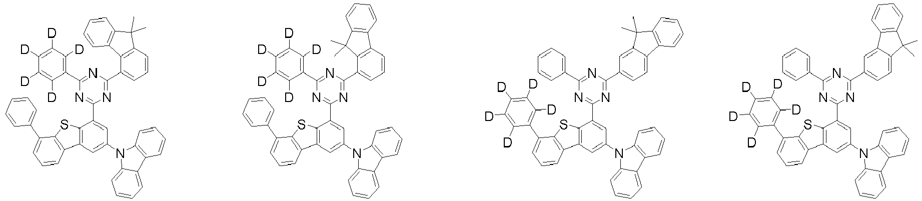

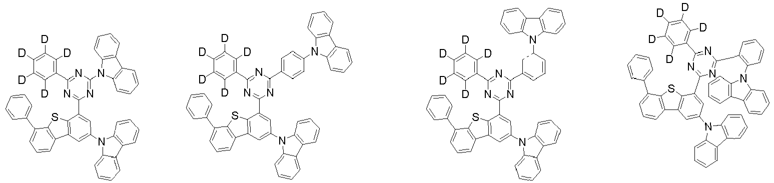

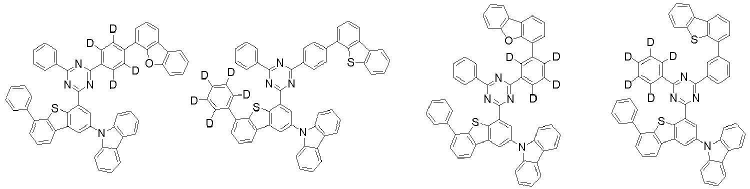

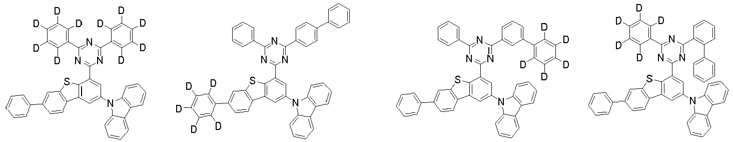

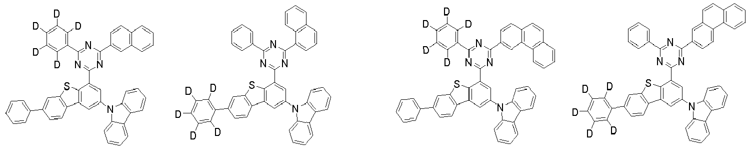

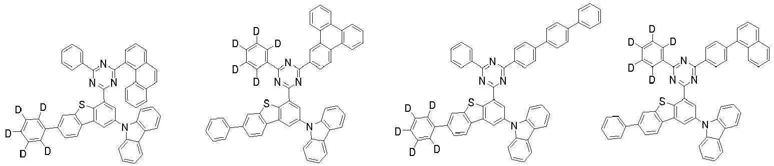

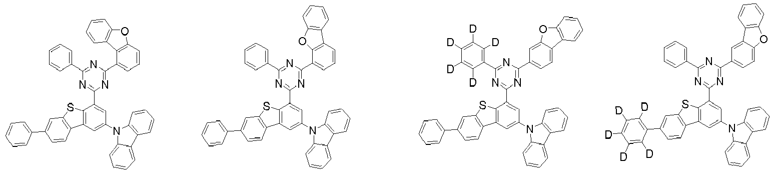

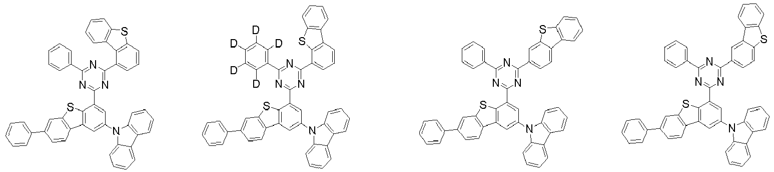

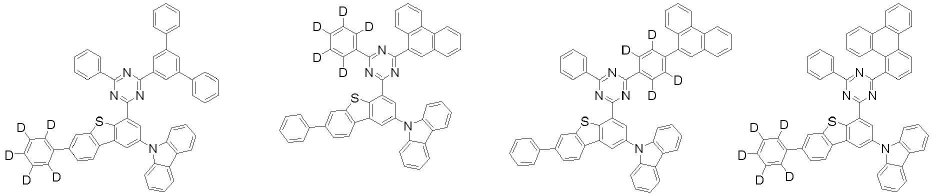

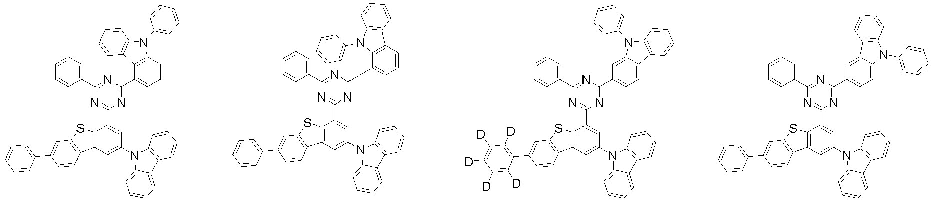

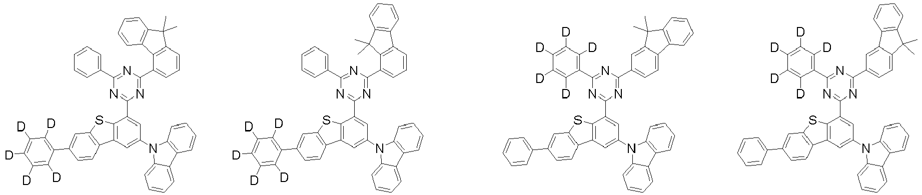

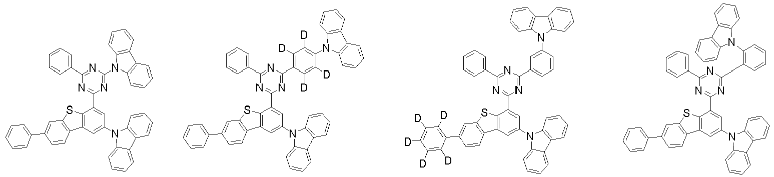

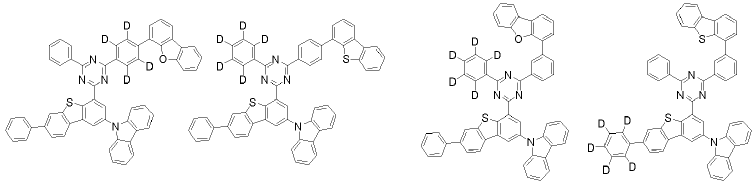

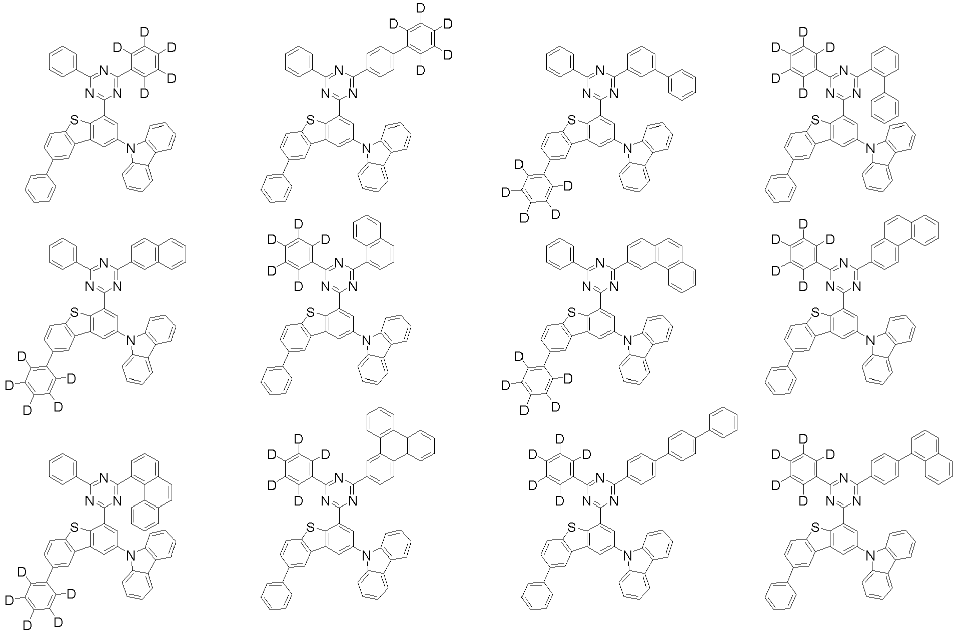

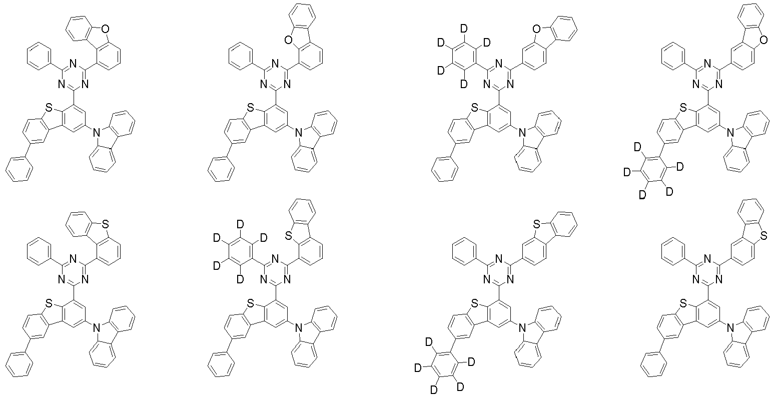

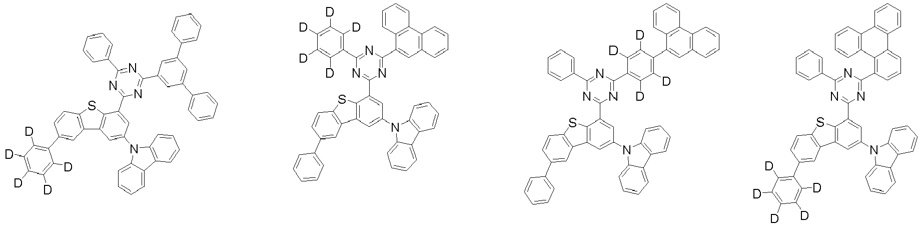

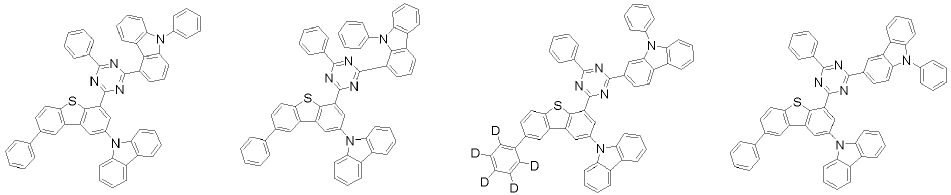

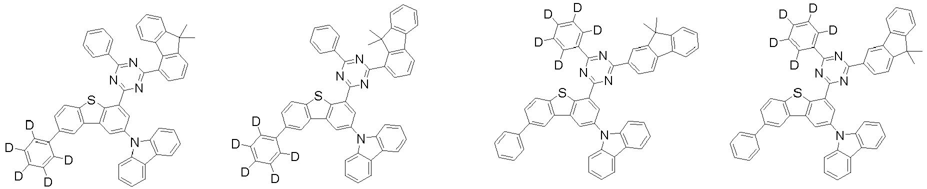

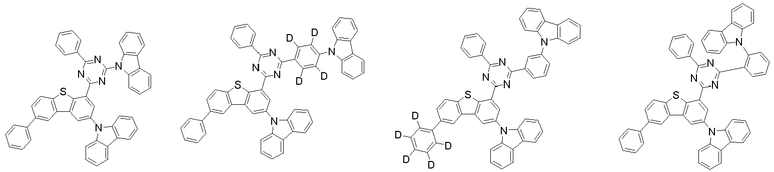

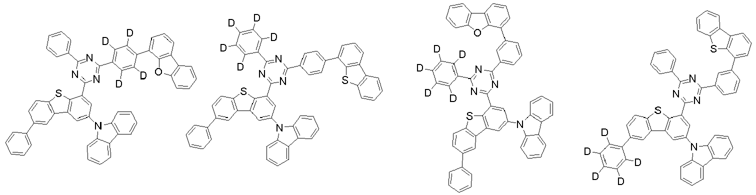

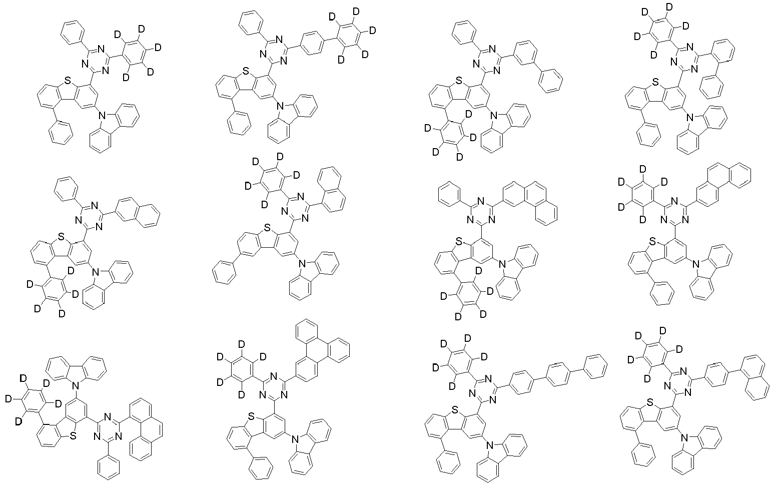

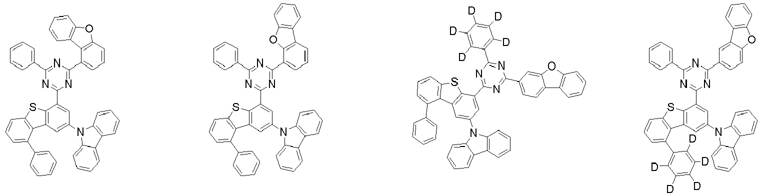

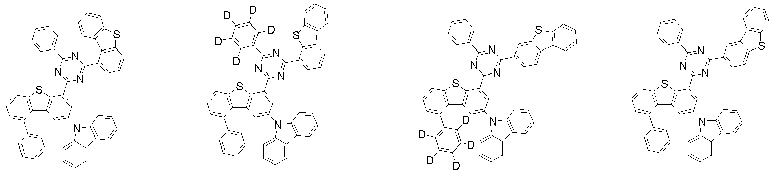

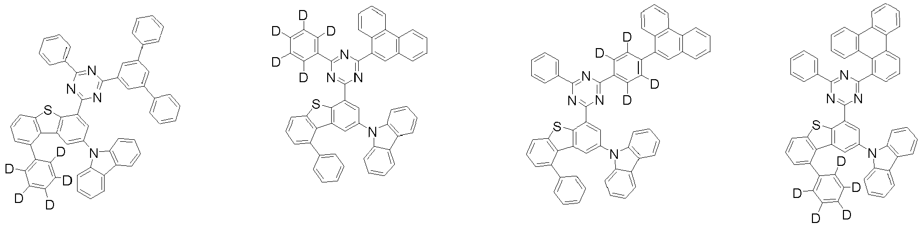

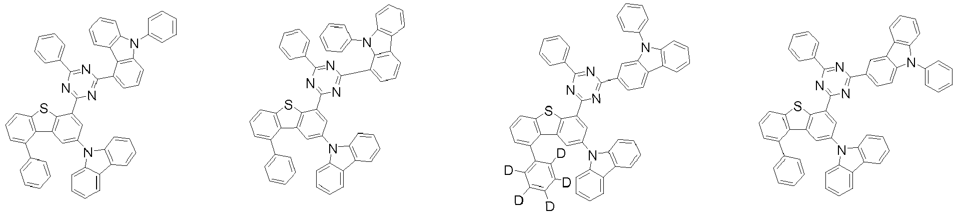

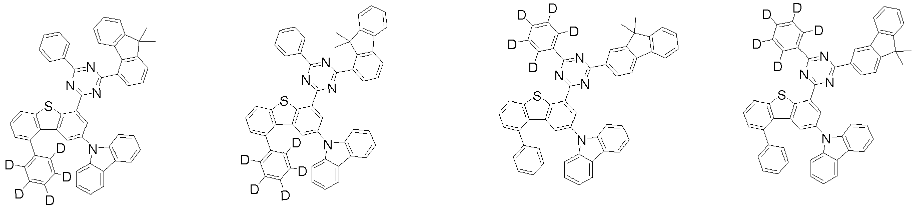

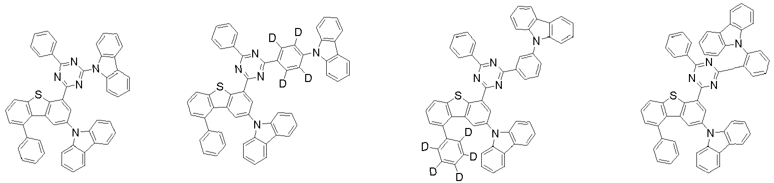

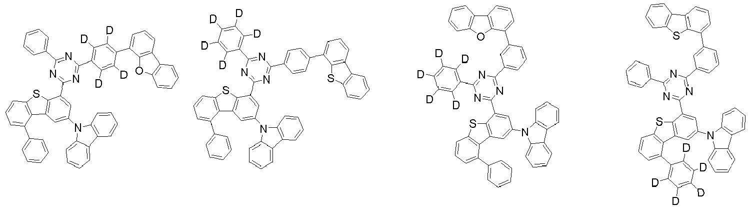

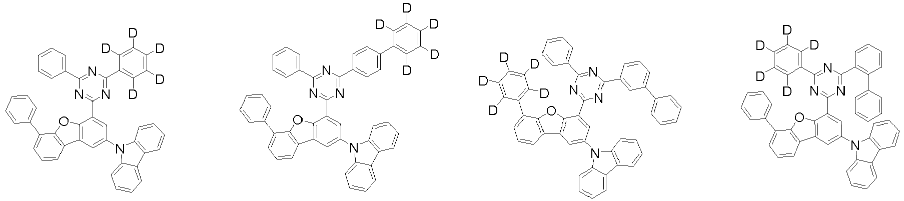

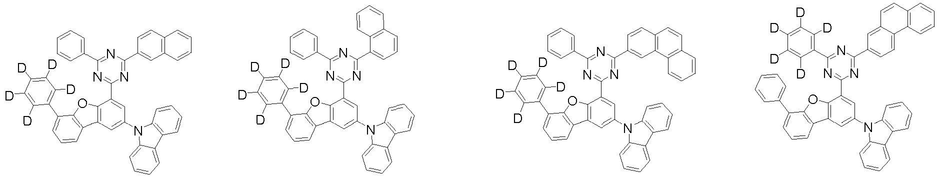

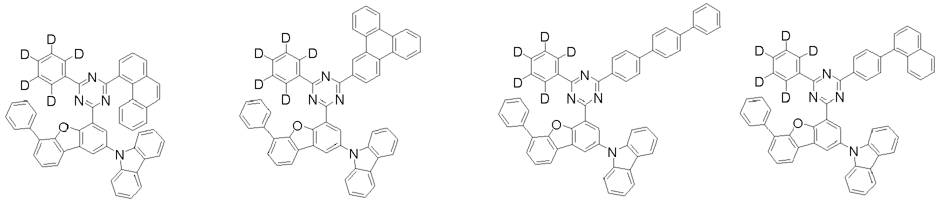

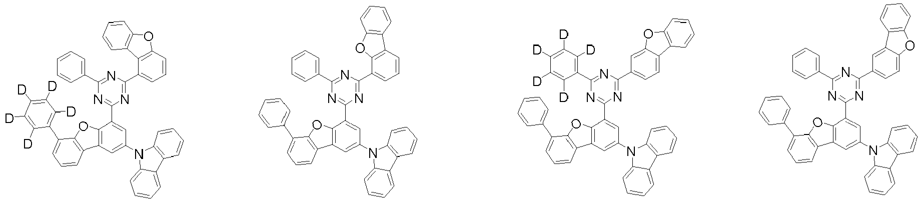

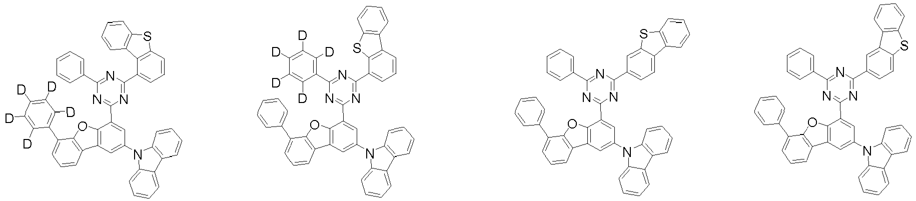

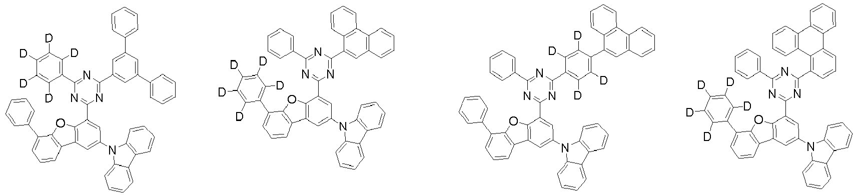

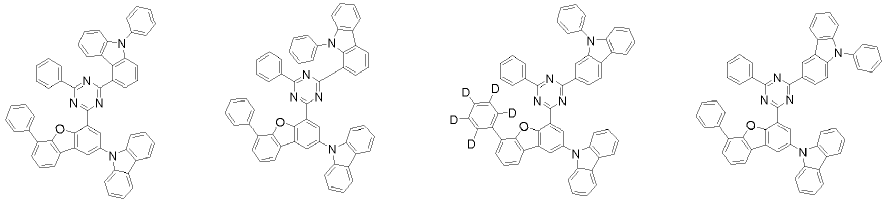

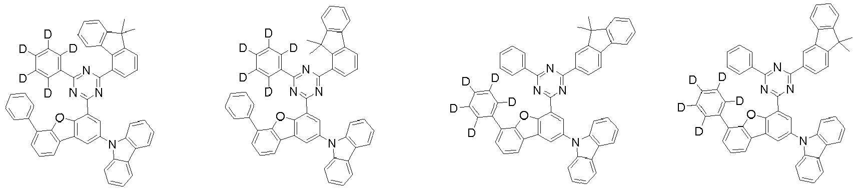

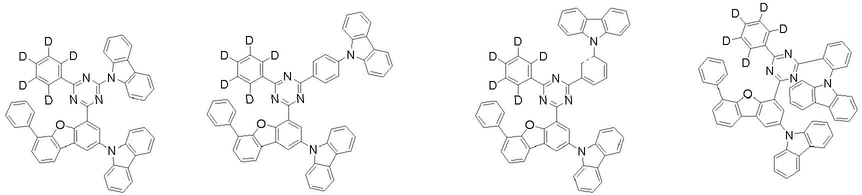

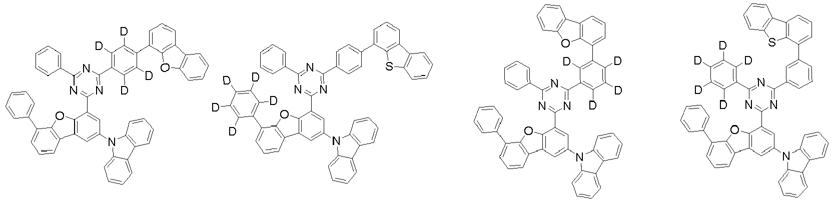

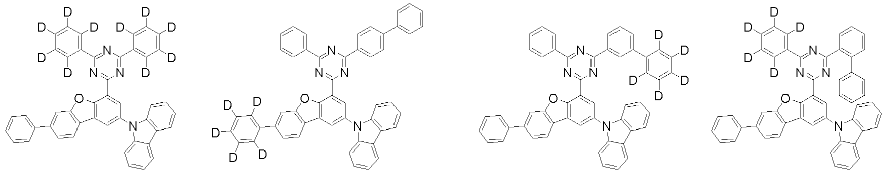

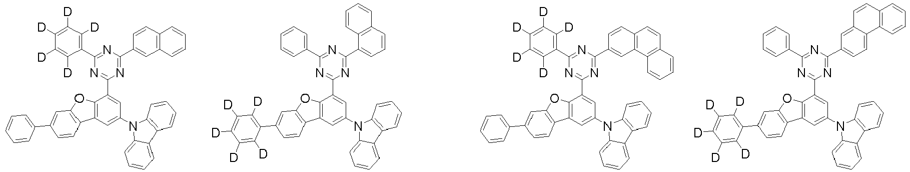

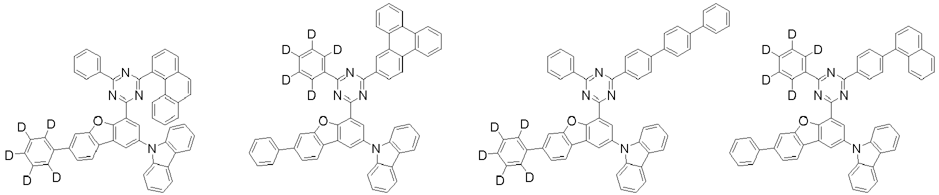

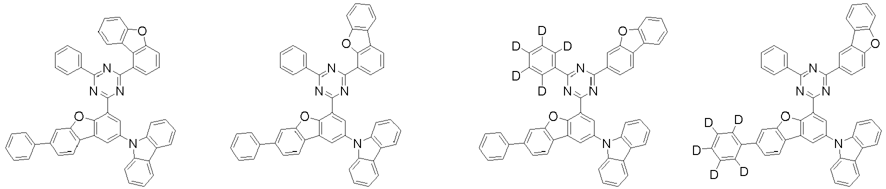

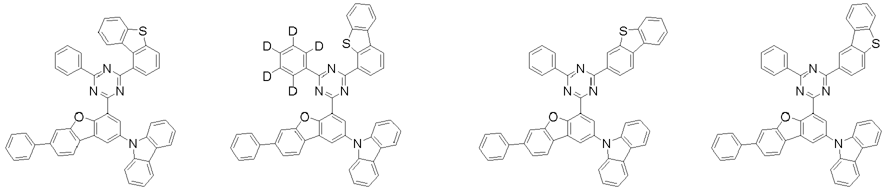

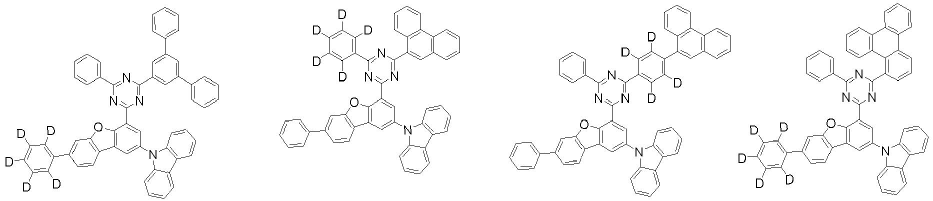

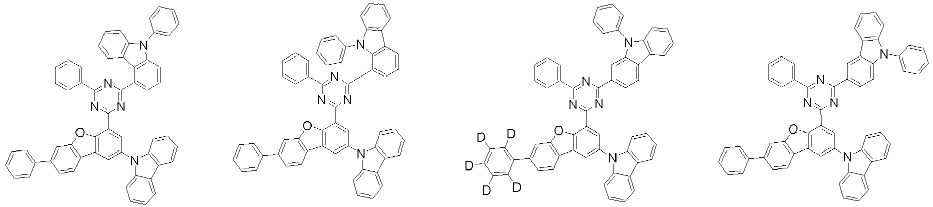

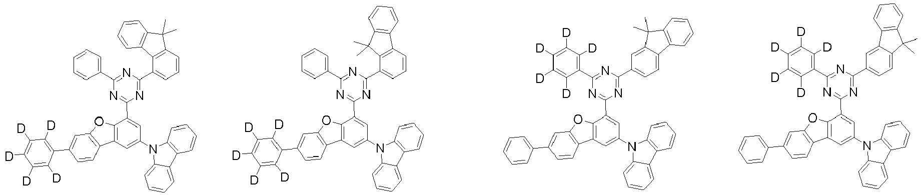

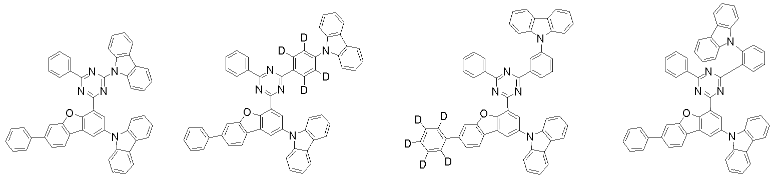

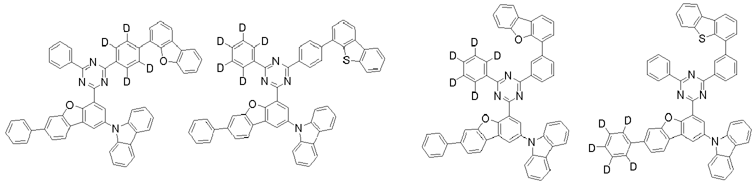

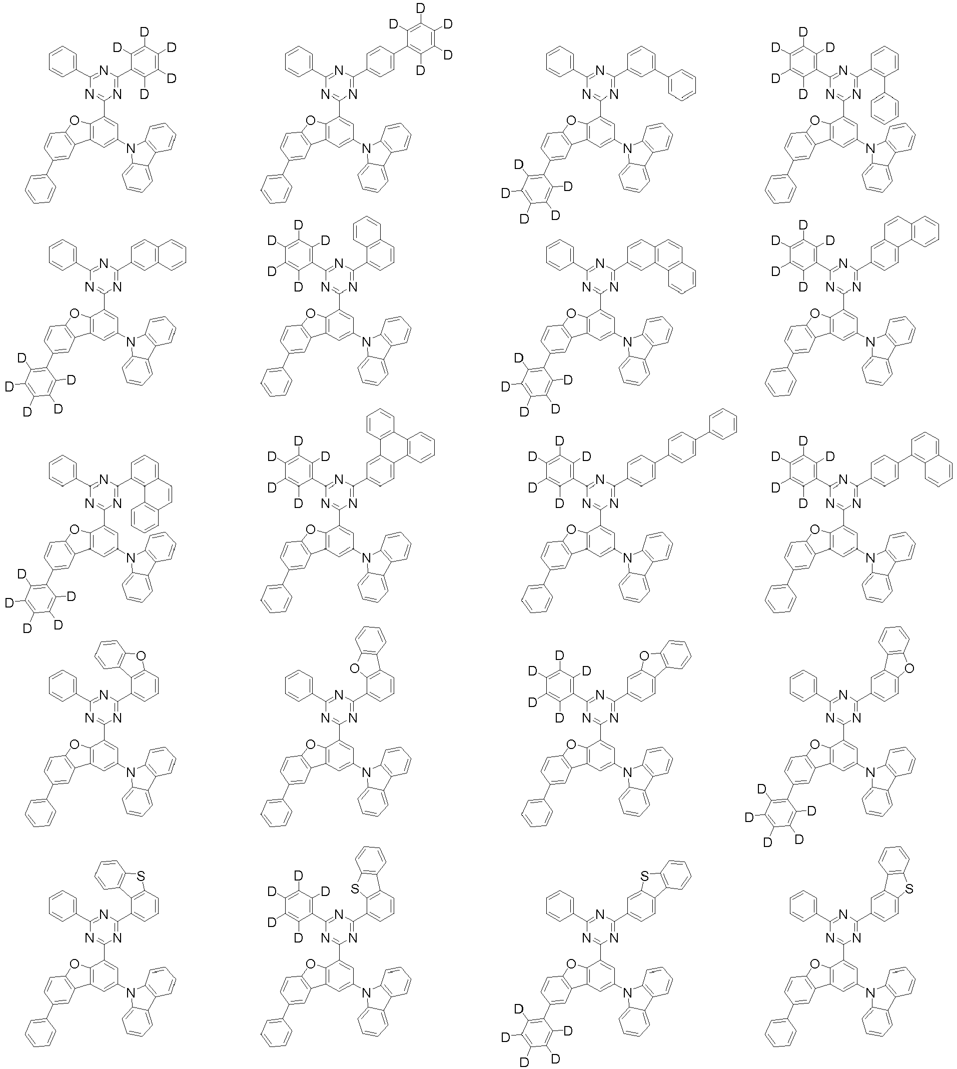

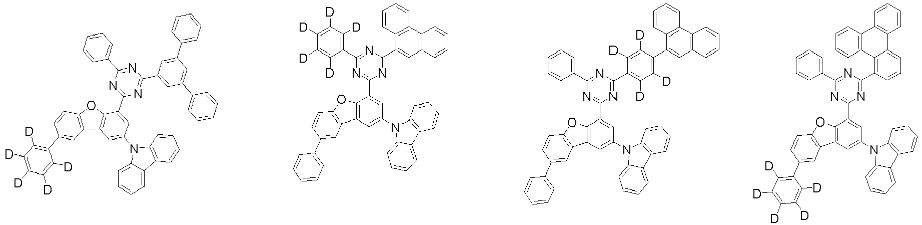

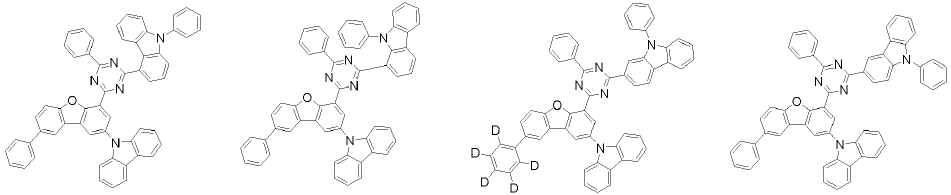

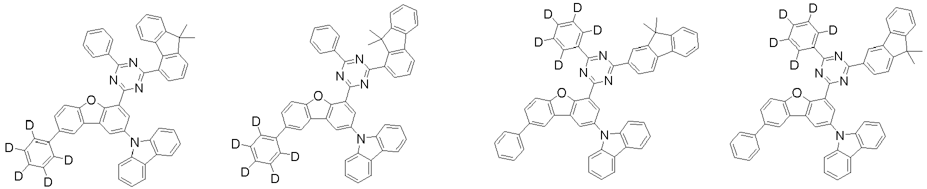

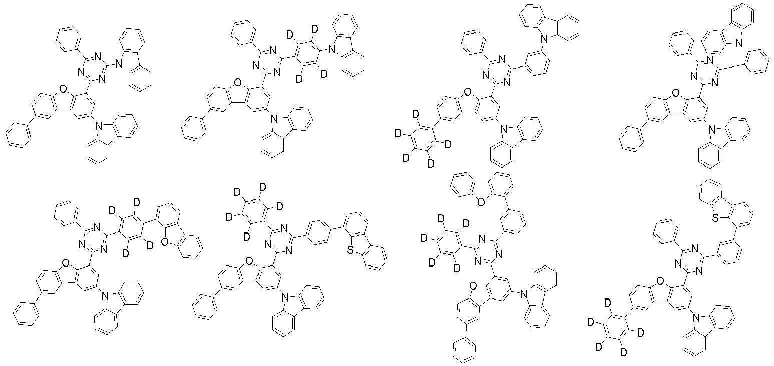

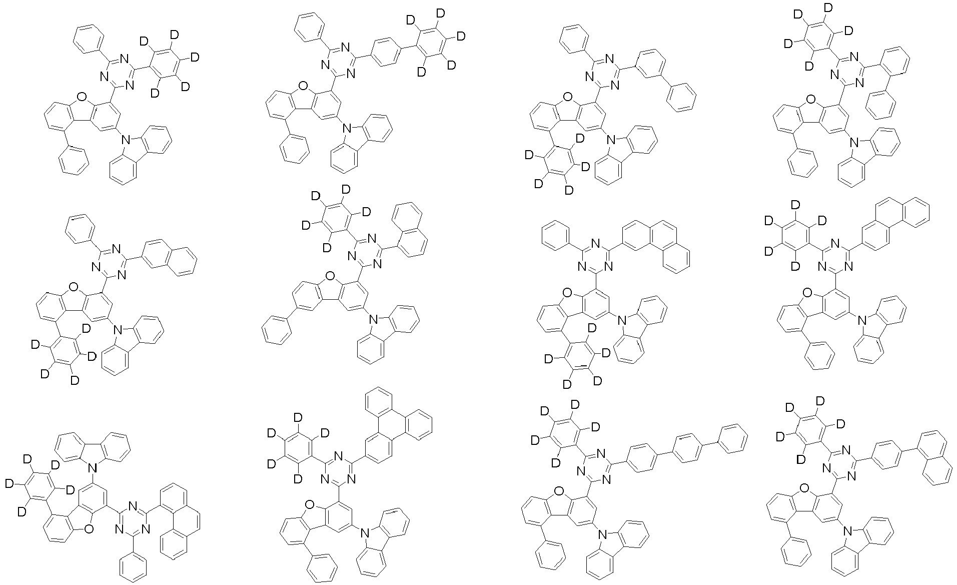

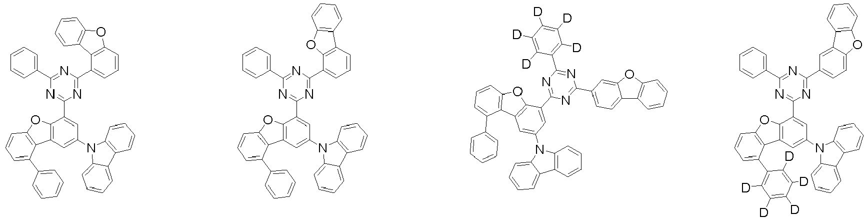

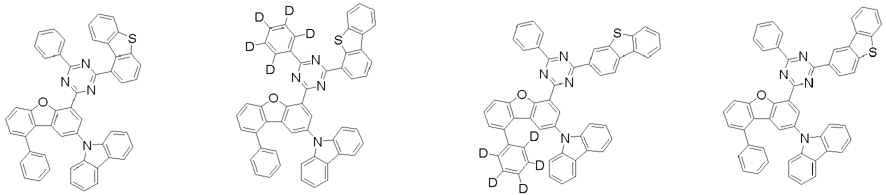

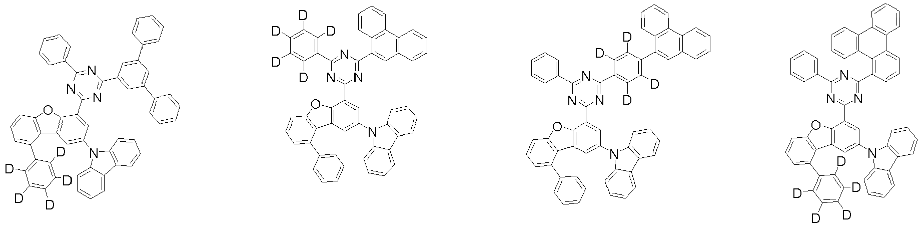

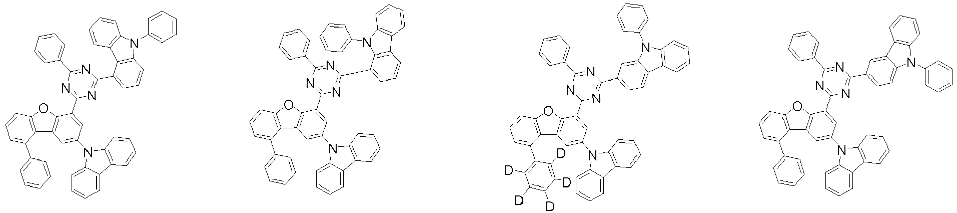

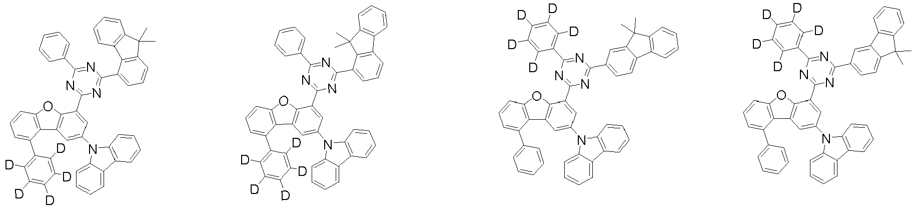

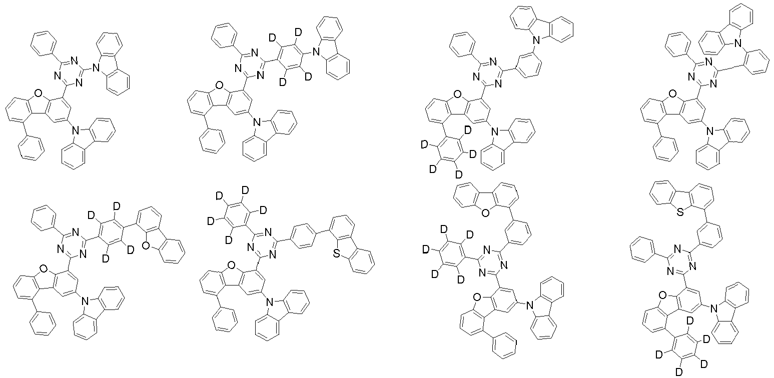

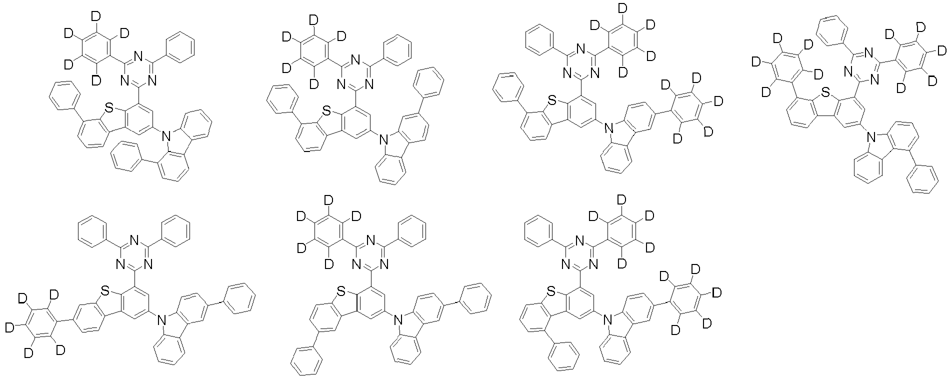

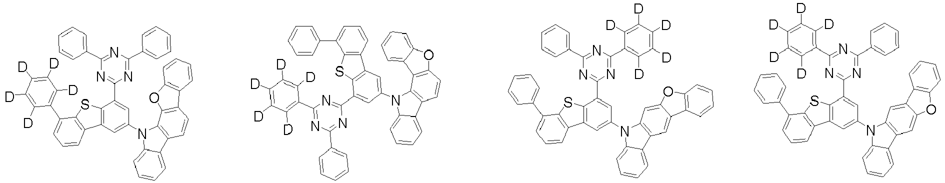

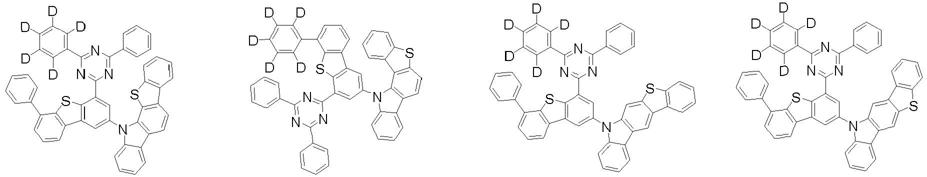

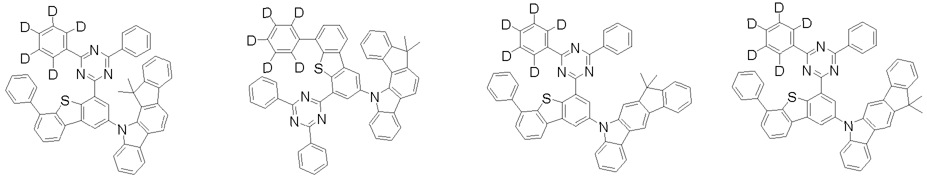

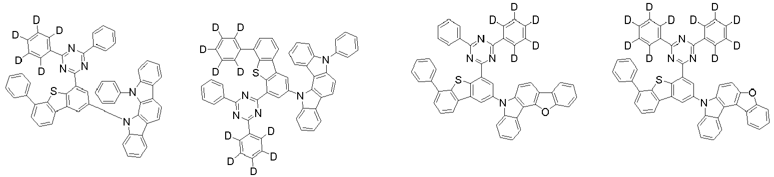

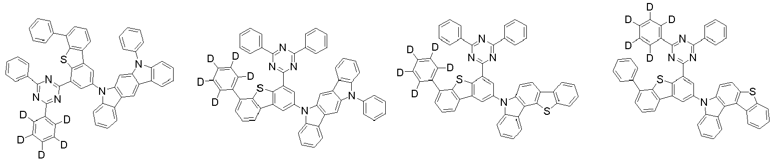

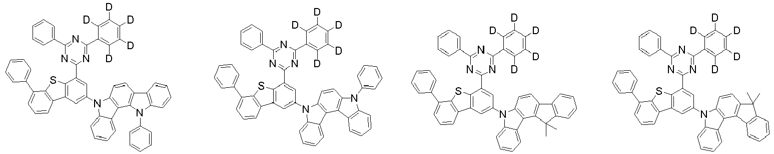

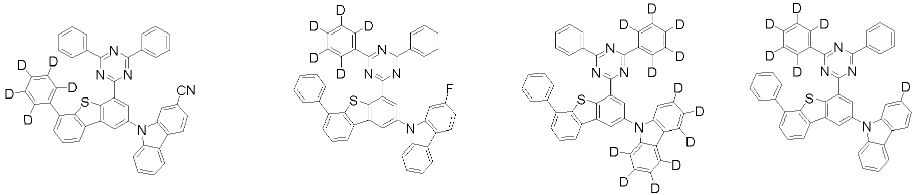

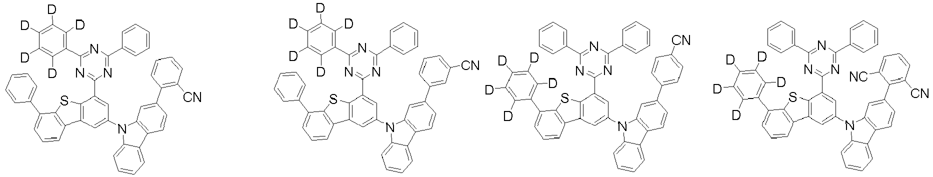

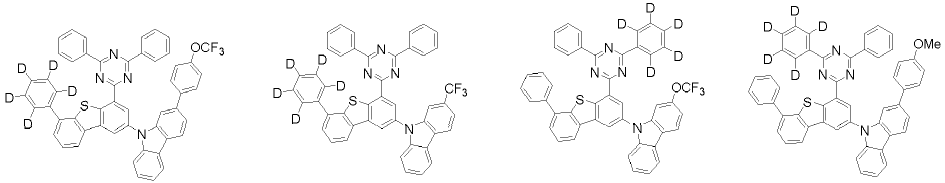

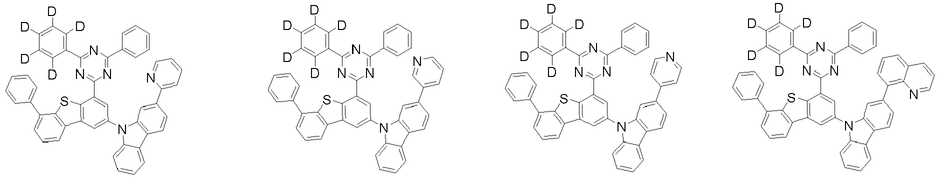

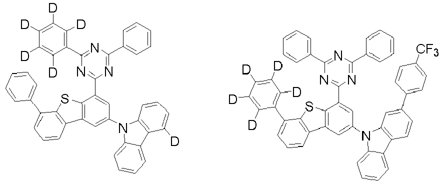

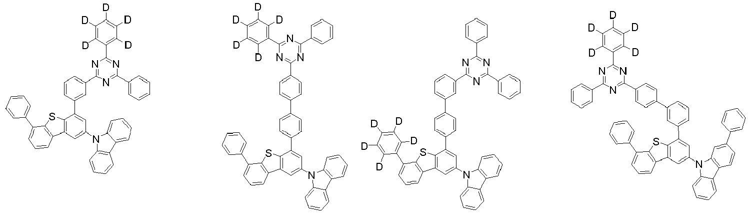

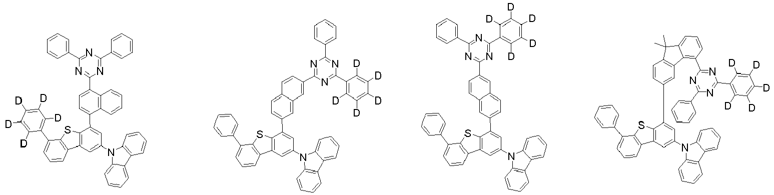

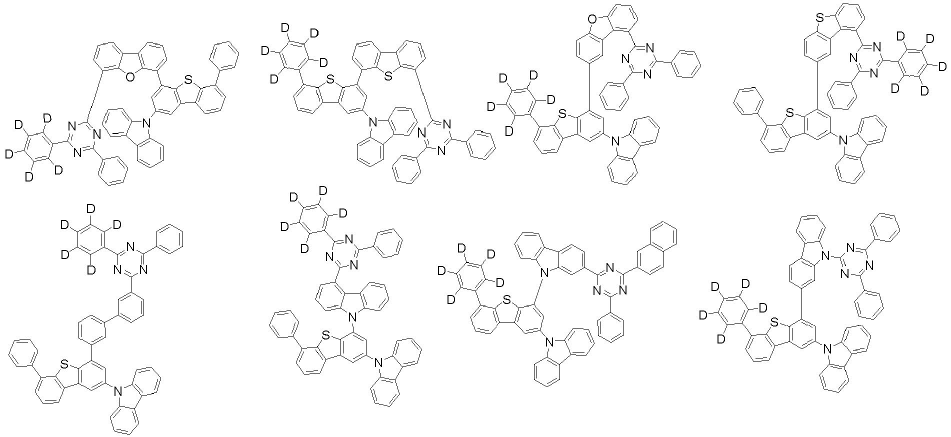

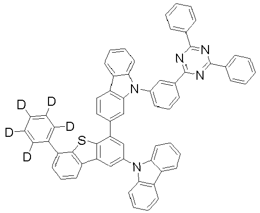

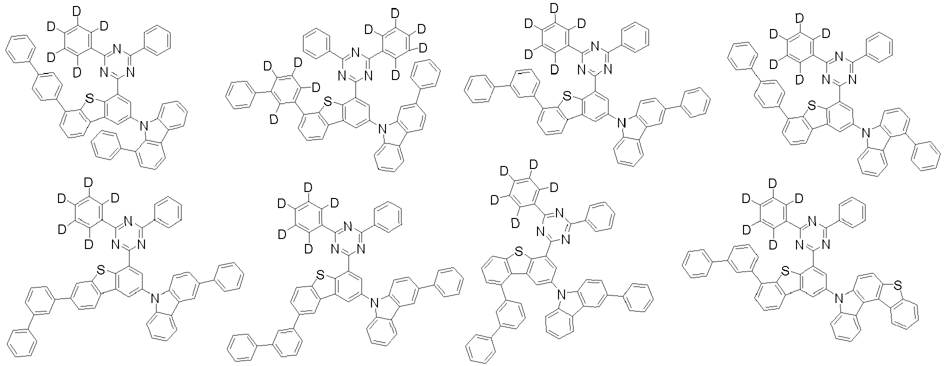

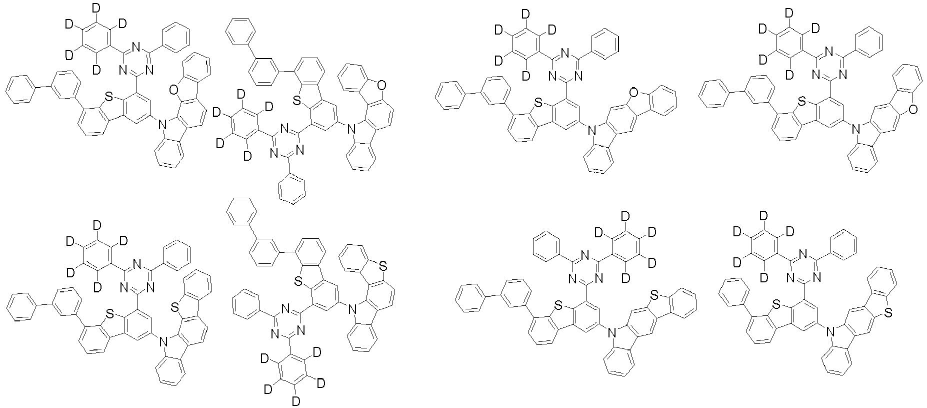

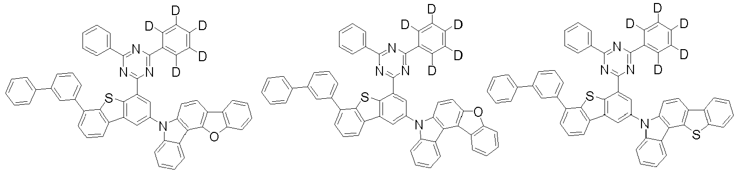

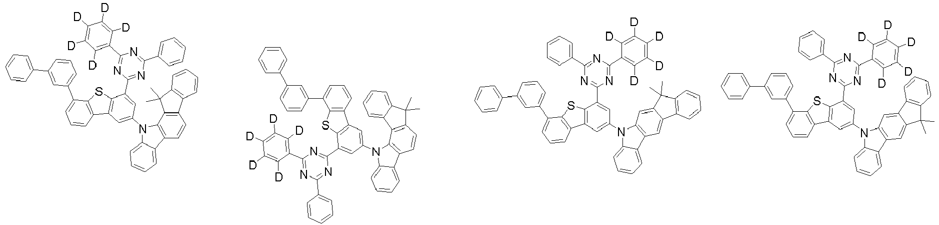

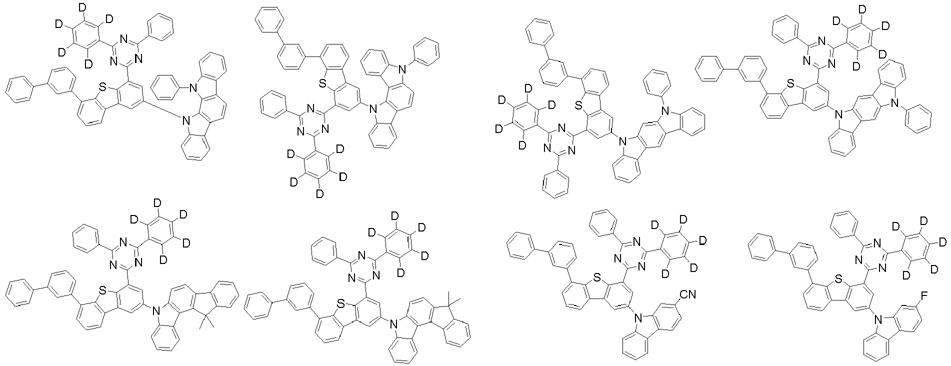

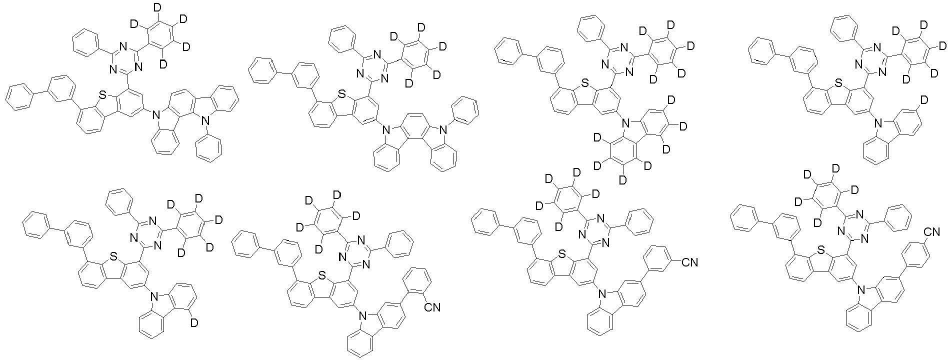

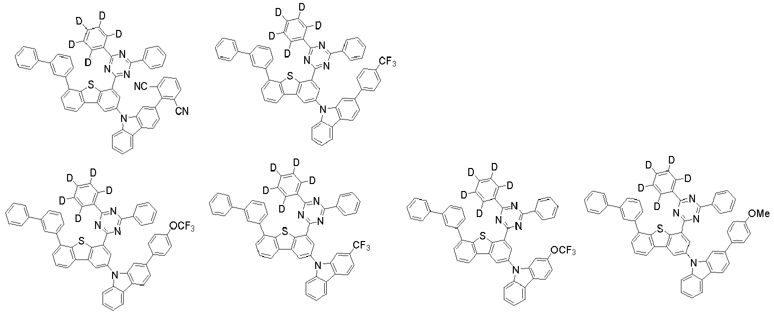

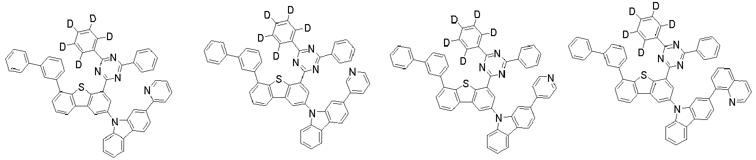

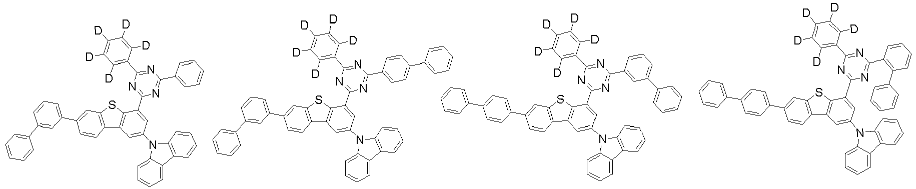

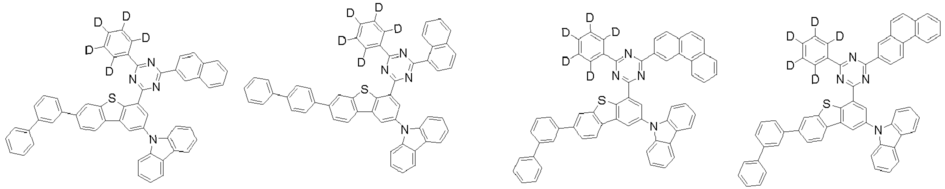

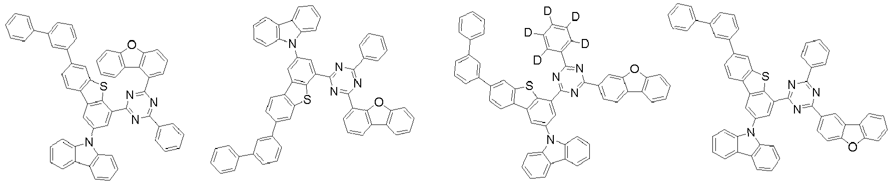

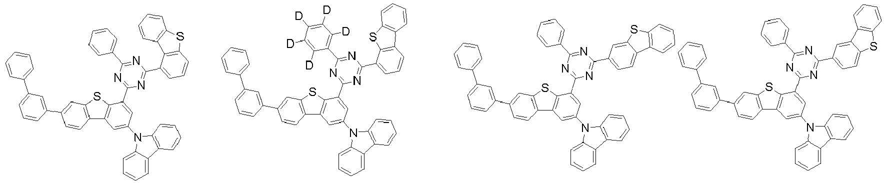

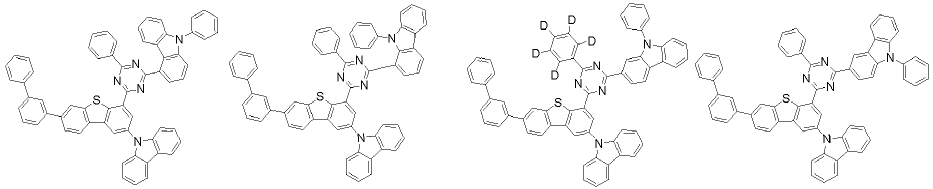

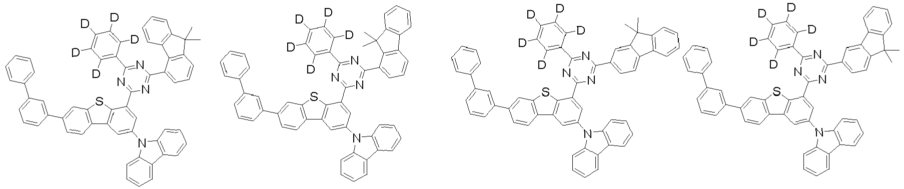

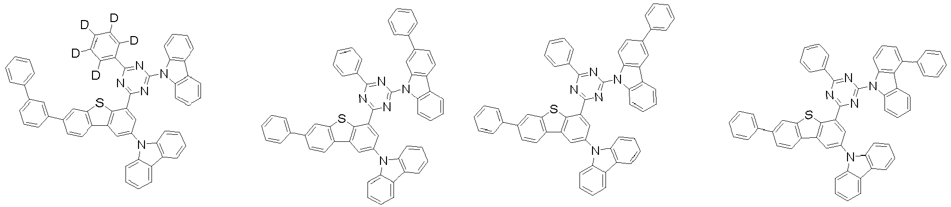

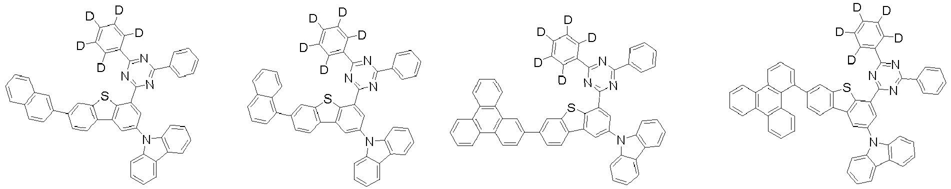

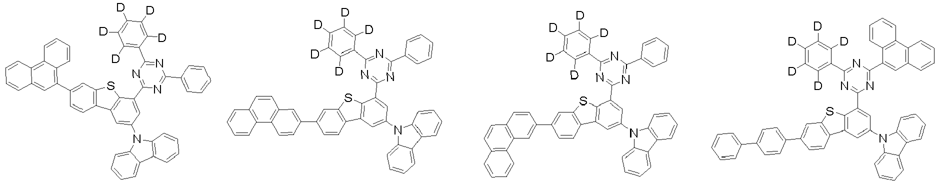

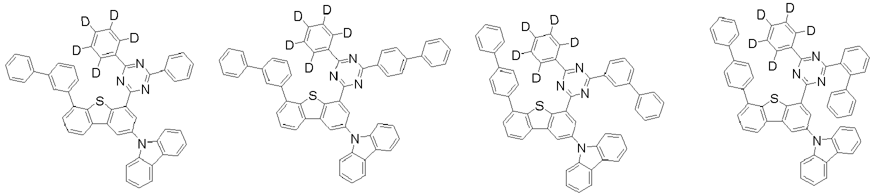

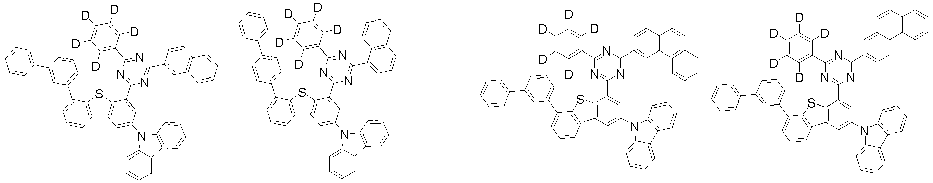

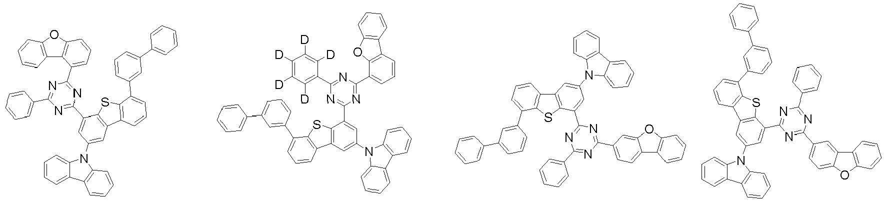

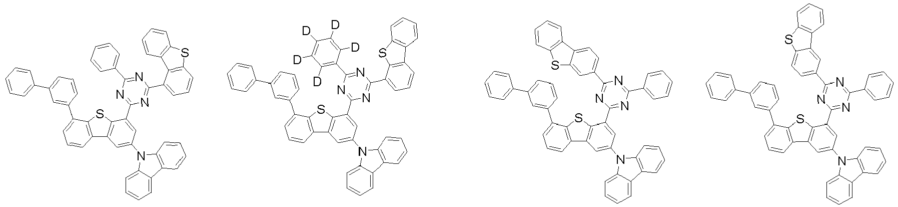

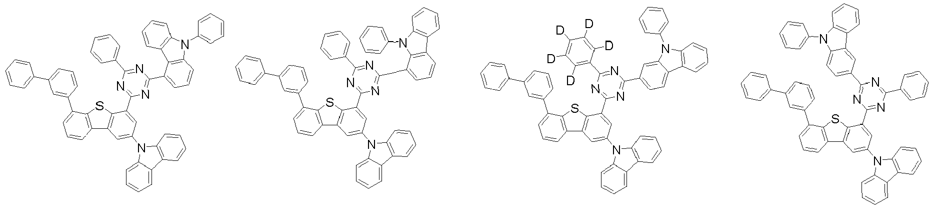

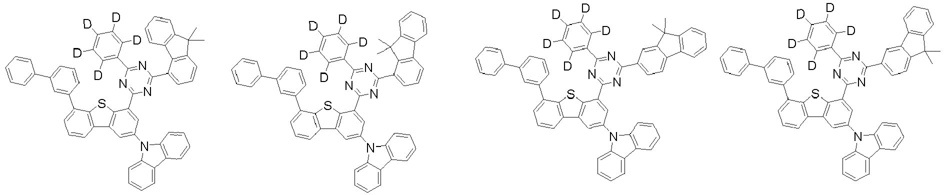

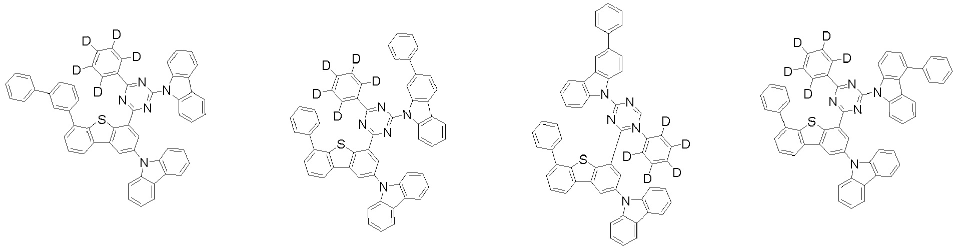

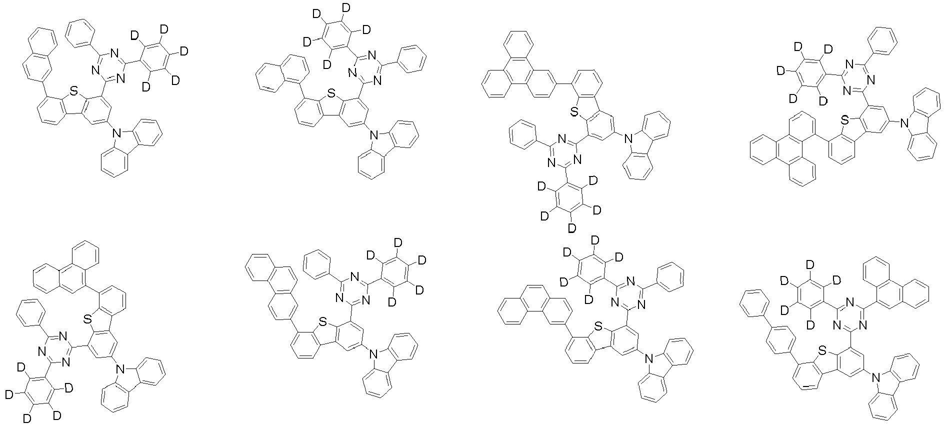

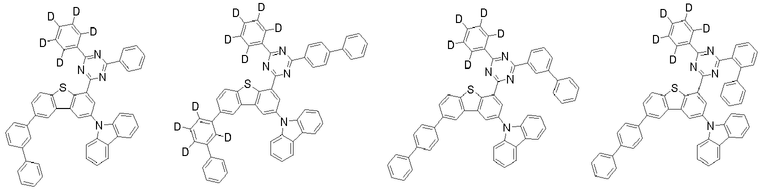

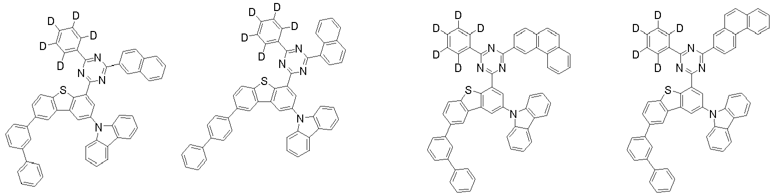

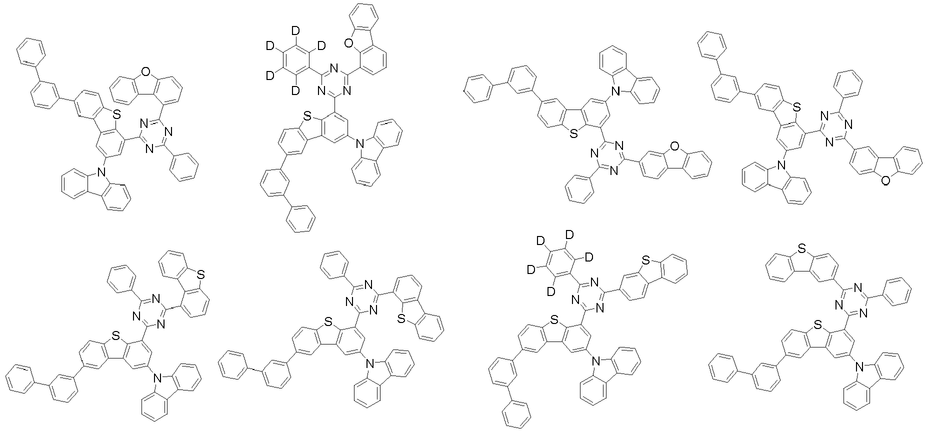

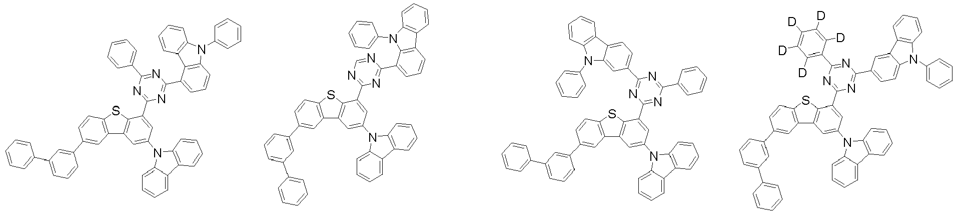

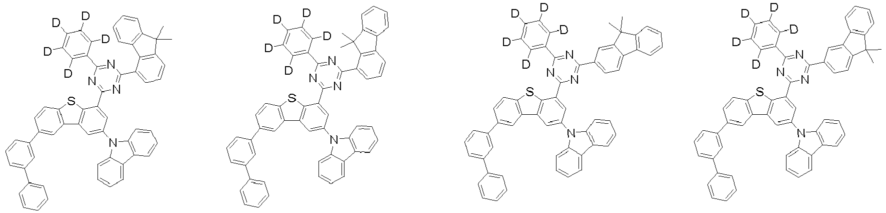

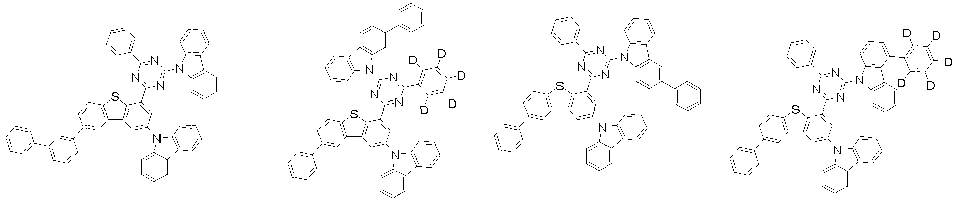

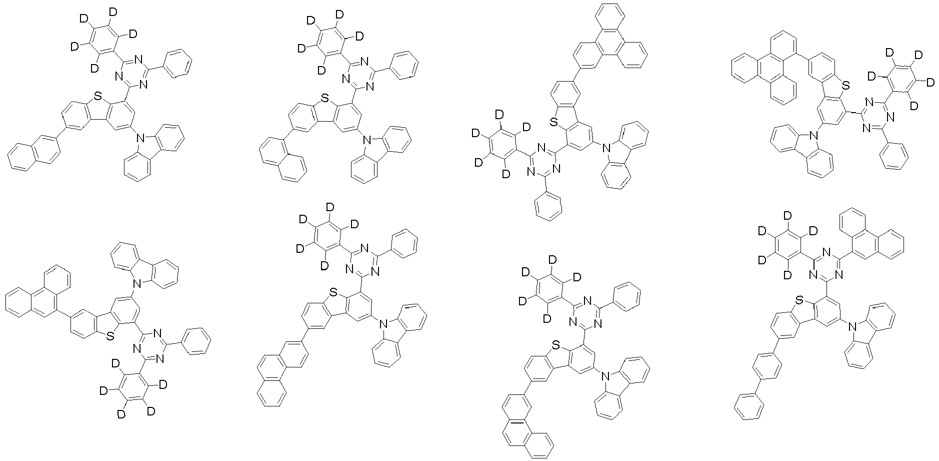

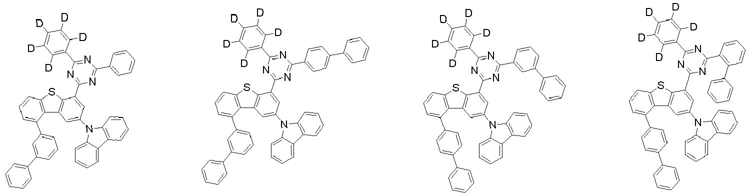

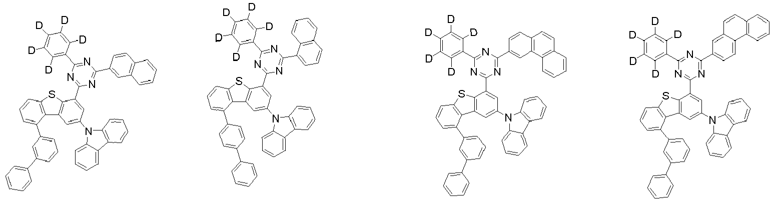

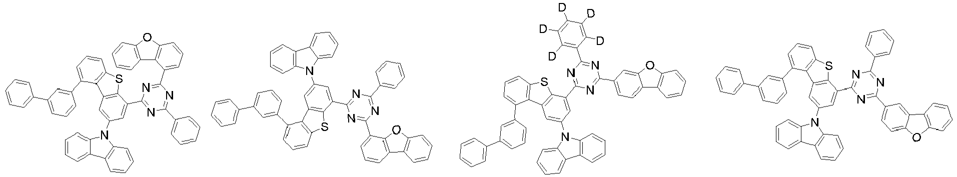

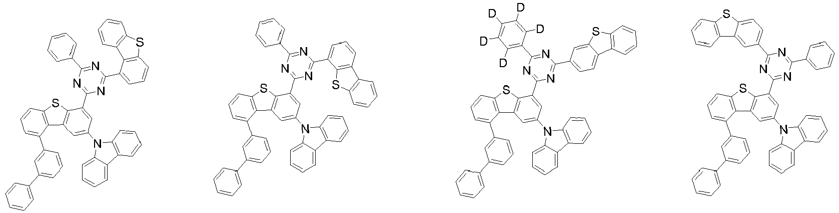

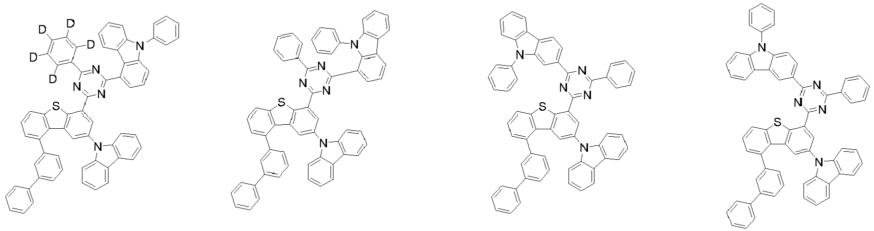

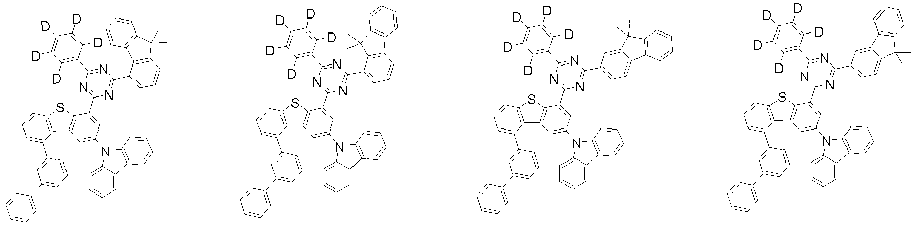

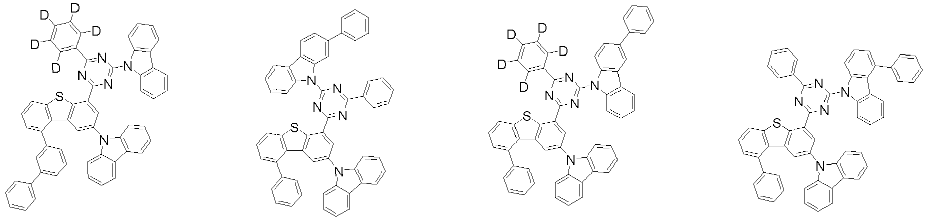

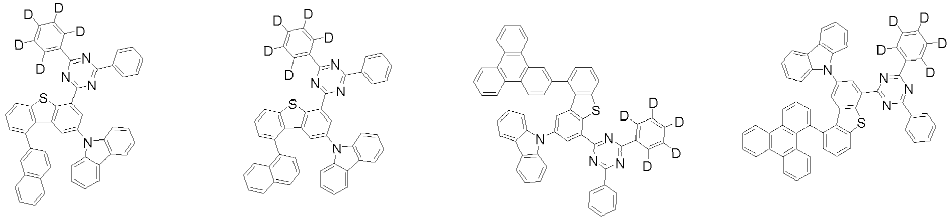

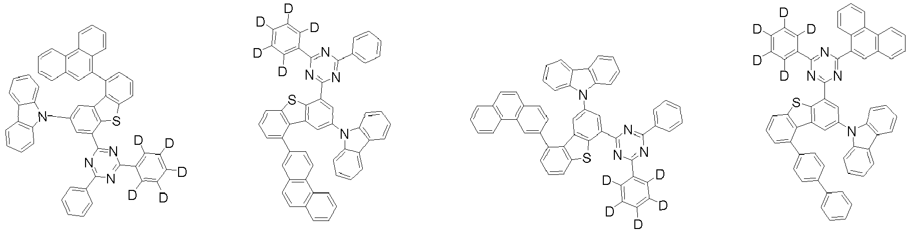

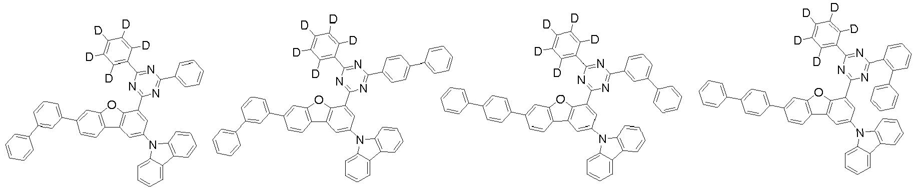

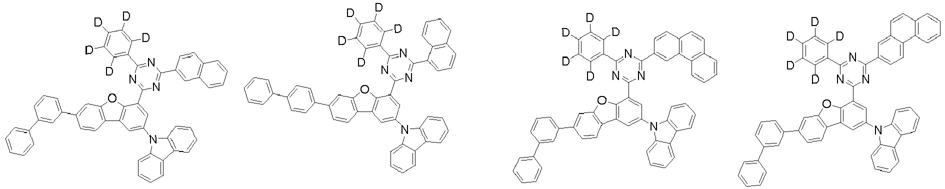

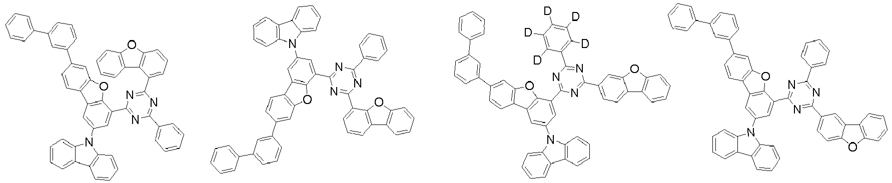

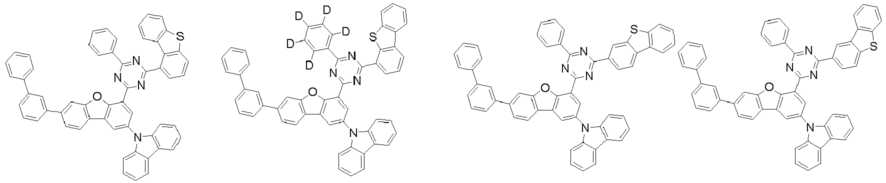

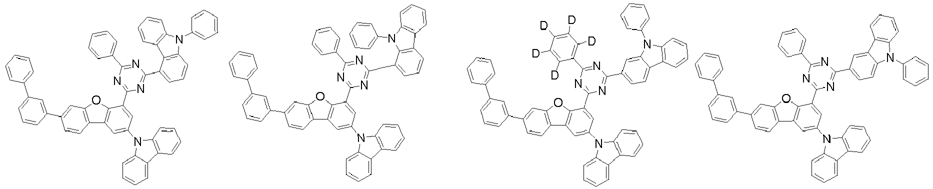

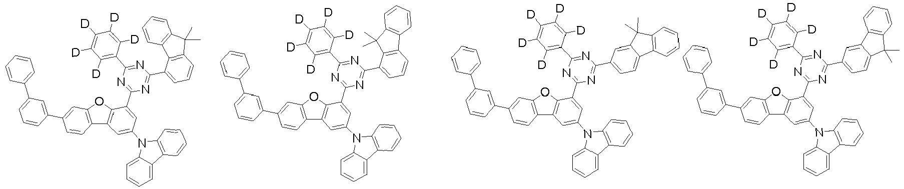

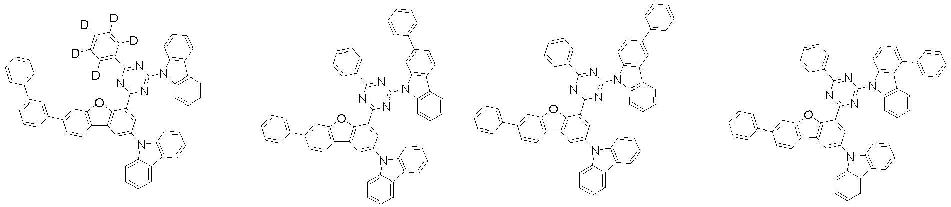

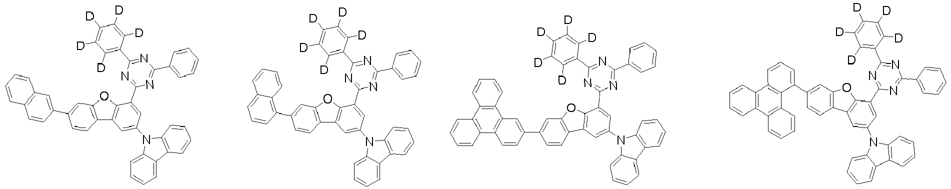

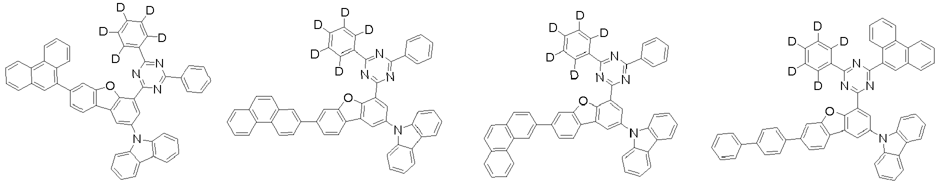

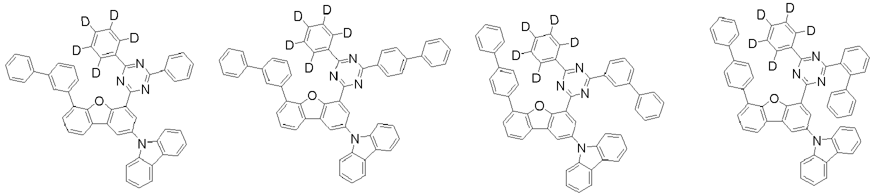

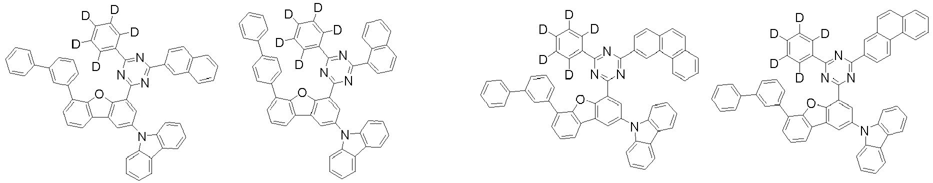

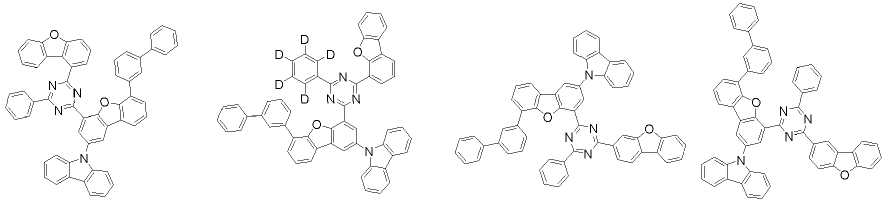

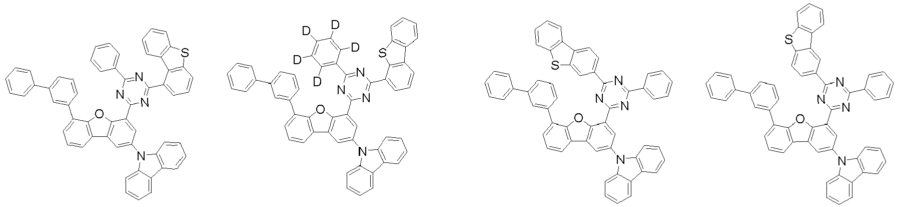

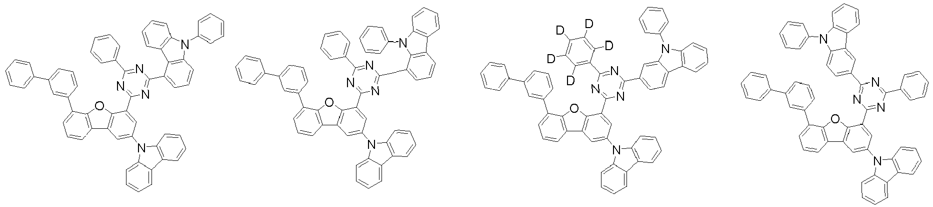

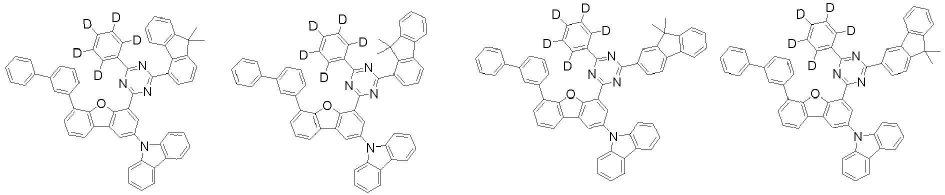

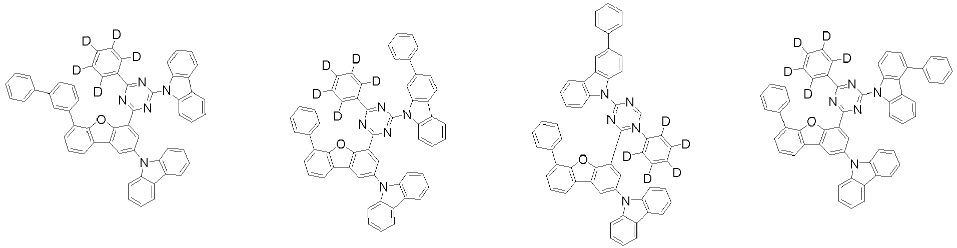

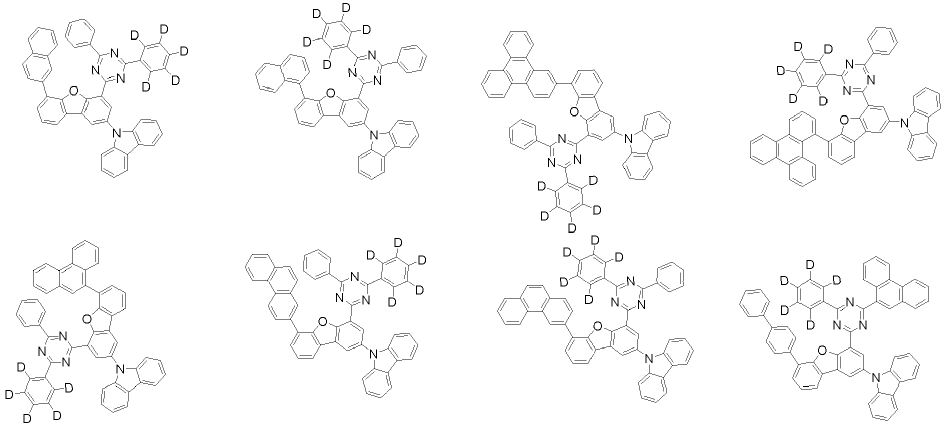

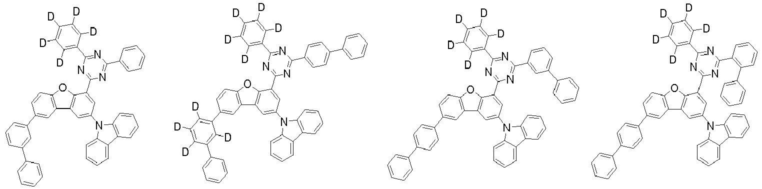

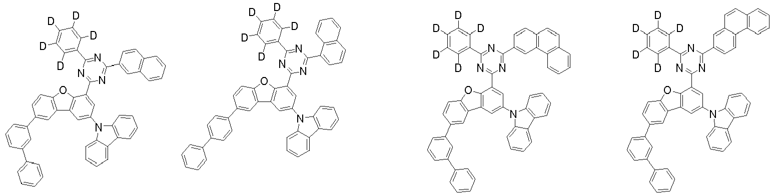

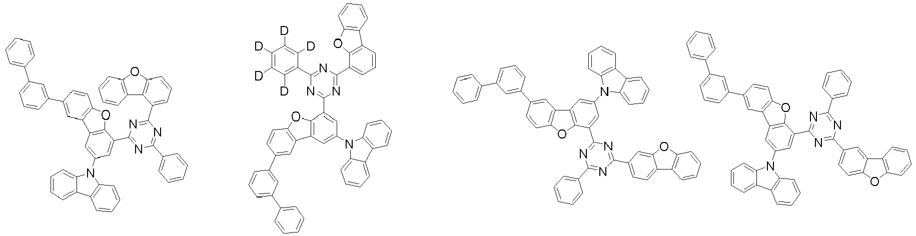

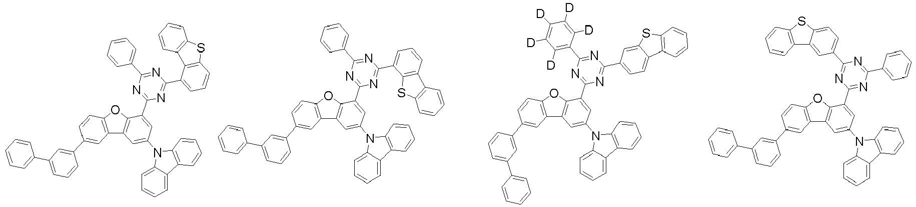

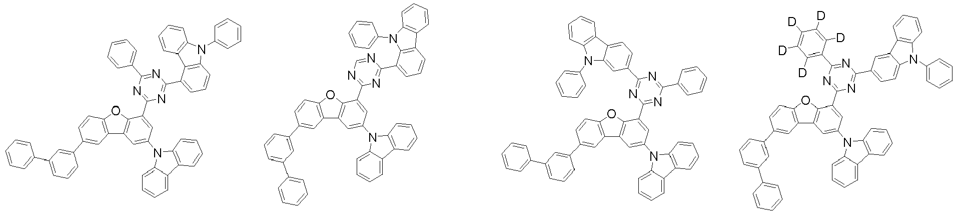

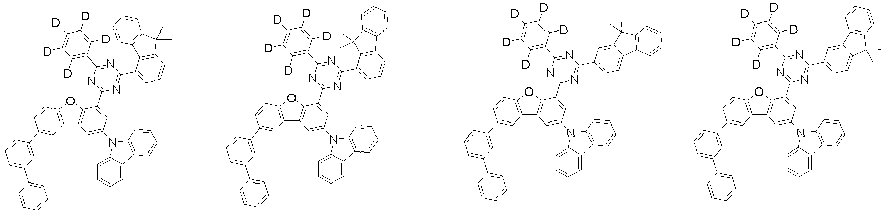

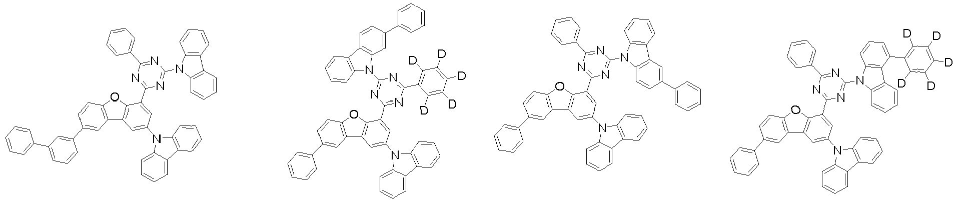

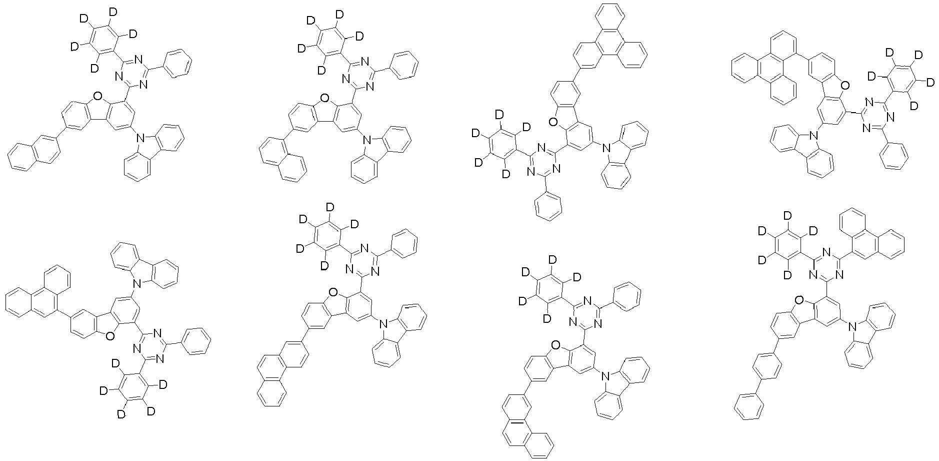

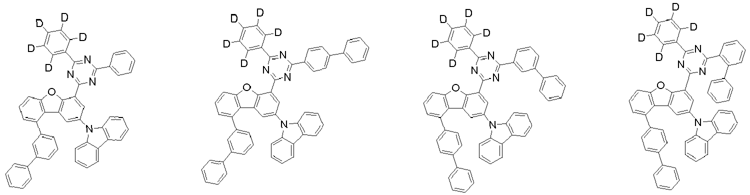

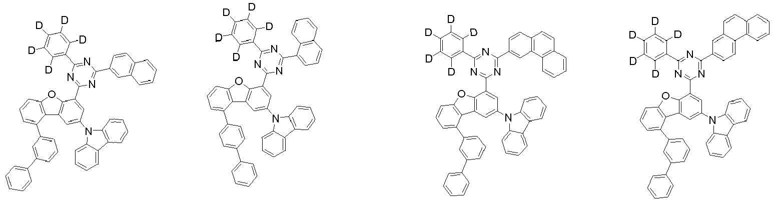

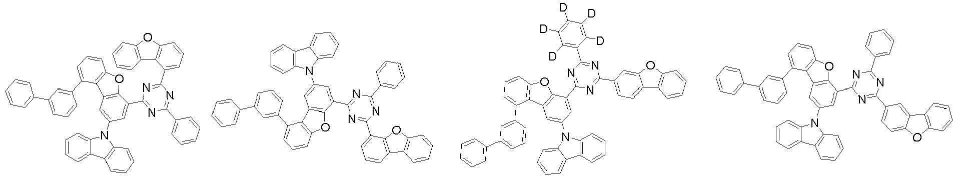

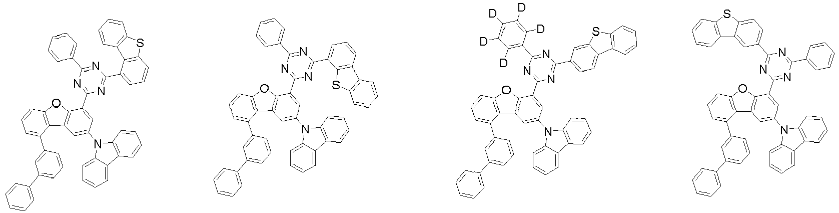

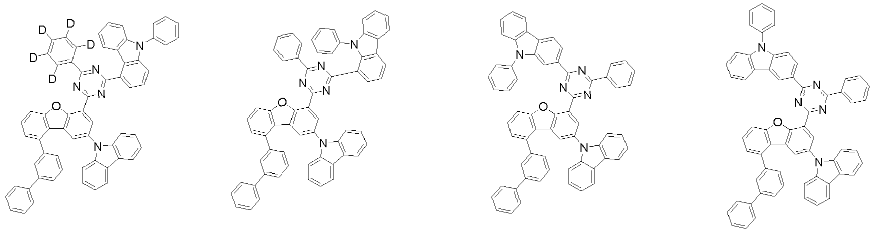

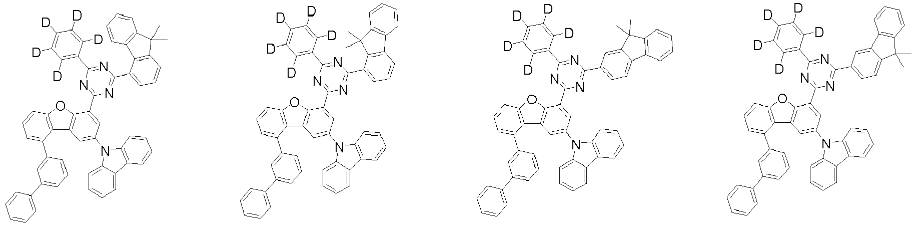

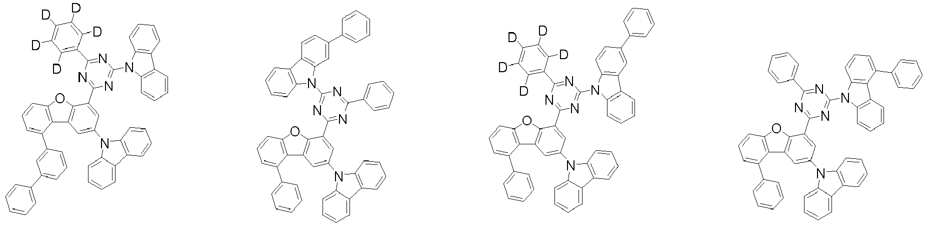

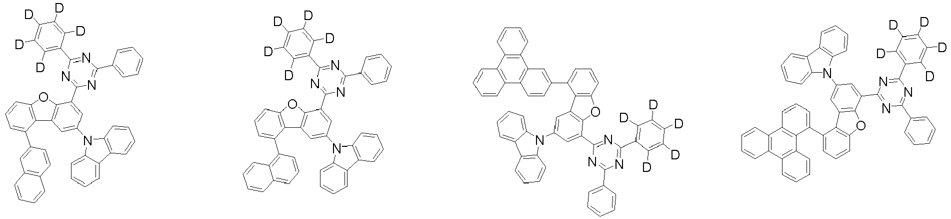

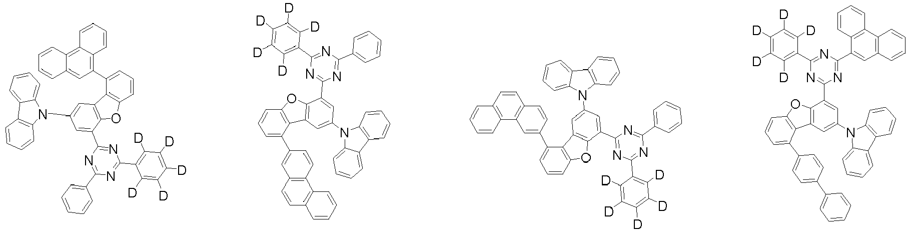

The compound represented by Chemical Formula 1 according to the present disclosure has structural properties that it simultaneously contains a carbazole-based substituent and a triazine-based substituent in one benzene ring of the core structure of dibenzofuran (dibenzothiophene), and contains an aryl substituent in the other benzene ring, and due to these properties, the compound may have improved thermal stability, and at the same time, can have high efficiency, low driving voltage, high luminance, and long lifetime. In particular, in the present disclosure, when Ar₁, Ar₂ and Ar₃ are the same or different substituted or unsubstituted C₆₋₆₀ aryl at the same time, any one of Ar₁, Ar₂ and Ar₃ is substituted with 4 or more deuteriums, which stabilizes LUMO. Thus, the present disclosure can have improved long lifetime properties as compared with an organic light-emitting device that employs a compound having a structure in which all of the terminal Ar₁, Ar₂ and Ar₃ are aryl, without being substituted with deuterium. In addition, as a heteroaryl group is substituted with a triazine having strong electronic properties, a balance between holes and electrons is appropriately aligned, which can be advantageous in terms of the efficiency and lifetime of a device.

The compound represented by Chemical Formula 1 can be prepared through the following Reaction Schemes 1-1 and 1-2.

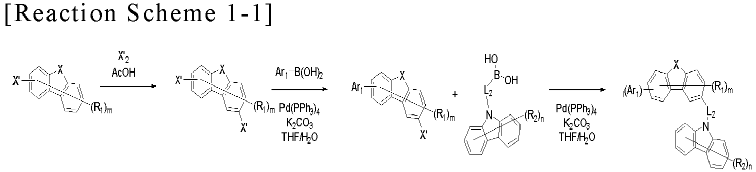

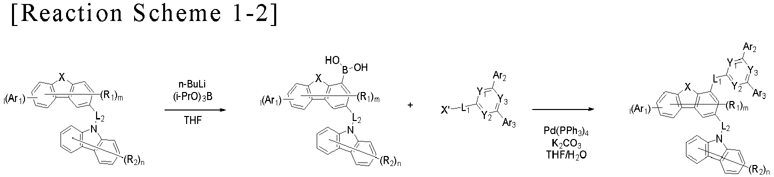

In Reaction Schemes 1-1 and 1-2, X' is halogen, preferably, bromo or chloro, and the definition of the remaining substituents is the same as defined above.

The above-mentioned reaction is a Suzuki coupling reaction, which is preferably carried out in the presence of a palladium catalyst and a base, and a reactant, catalyst and the like for the reaction can be modified as known in the art. The above preparation method may be further embodied in the Preparation Examples described hereinafter. In addition, when a plurality of substituents are introduced, it can be implemented by repeatedly performing the steps (for example, the introduction of a plurality of Ar₁ may be implemented by performing the first step several times in Reaction Scheme 1-1. In this case, a protecting group and a deprotecting group reaction known in the art can also be performed together to introduce the desired structure.)

Another embodiment of the invention provides an organic light emitting device including a compound represented by Chemical Formula 1 described above. As an example, there is provided an organic light emitting device including a first electrode; a second electrode that is provided opposite to the first electrode; and one or more organic material layers that are provided between the first electrode and the second electrode, wherein one or more layers of the organic material layers include the compound represented by Chemical Formula 1.

The organic material layer of the organic light emitting device of the present disclosure may have a single-layer structure, or it may have a multilayered structure in which two or more organic material layers are stacked. For example, the organic light emitting device of the present disclosure may have a structure comprising a hole injection layer, a hole transport layer, a light emitting layer, an electron transport layer, an electron injection layer and the like as the organic material layer. However, the structure of the organic light emitting device is not limited thereto, and it may include a smaller number of organic layers.

Further, the organic material layer may include a hole injection layer, a hole transport layer, or a layer for simultaneously performing hole injection and transport, wherein the hole injection layer, the hole transport layer, or the layer for simultaneously performing hole injection and transport include the compound represented by Chemical Formula 1.

Further, the organic material layer may include a light emitting layer, wherein the light emitting layer includes the compound represented by Chemical Formula 1.

Further, the organic material layer may include an electron transport layer, or an electron injection layer, wherein the electron transport layer, or the electron injection layer includes the compound represented by Chemical Formula 1.

Further, the electron transport layer, the electron injection layer, and the layer for simultaneously performing electron injection and electron transport includes the compound represented by Chemical Formula 1. In particular, the compound represented by Chemical Formula 1 according to the present disclosure has excellent thermal stability, a deep HOMO level of 6.0 eV or higher, high triplet energy (ET) and hole stability. In addition, when the compound represented by Chemical Formula 1 is used for the organic material layer capable of simultaneously performing electron injection and electron transport, an n-type dopant used in the art can be mixed and used.

Further, the organic material layer includes a light emitting layer and an electron transport layer, wherein the electron transport layer may include a compound represented by Chemical Formula 1.

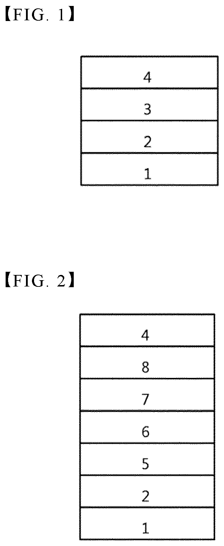

Further, the organic light emitting device according to the present disclosure may be a normal type organic light emitting device in which an anode, one or more organic material layers, and a cathode are sequentially stacked on a substrate. Further, the organic light emitting device according to the present disclosure may be an inverted type organic light emitting device in which a cathode, one or more organic material layers, and an anode are sequentially stacked on a substrate. For example, the structure of an organic light emitting device according to an embodiment of the present disclosure is illustrated in FIGS. 1 and 2.

FIG. 1 shows an example of an organic light emitting device comprising a substrate 1, an anode 2, a light emitting layer 3, and a cathode 4. In such a structure, the compound represented by Chemical Formula 1 may be included in the light emitting layer.

FIG. 2 shows an example of an organic light emitting device comprising a substrate 1, an anode 2, a hole injection layer 5, a hole transport layer 6, a light emitting layer 7, an electron transport layer 8, and a cathode 4. In such a structure, the compound represented by Chemical Formula 1 may be included in one or more layers of the hole injection layer, the hole transport layer, the light emitting layer, and the electron transport layer.

The organic light emitting device according to the present disclosure may be manufactured by materials and methods known in the art, except that one or more layers of the organic material layers include the compound represented by Chemical Formula 1. In addition, when the organic light emitting device includes a plurality of organic material layers, the organic material, the organic material layer can be formed of the same material or different materials.

For example, the organic light emitting device according to the present disclosure can be manufactured by sequentially stacking a first electrode, an organic material layer and a second electrode on a substrate. In this case, the organic light emitting device may be manufactured by depositing a metal, metal oxides having conductivity, or an alloy thereof on the substrate using a PVD (physical vapor deposition) method such as a sputtering method or an e-beam evaporation method to form an anode, forming organic material layers including the hole injection layer, the hole transport layer, the light emitting layer and the electron transport layer thereon, and then depositing a material that can be used as the cathode thereon. In addition to such a method, the organic light emitting device may be manufactured by sequentially depositing a cathode material, an organic material layer and an anode material on a substrate.

In addition, the compound represented by Chemical Formula 1 may be formed into an organic material layer by a solution coating method as well as a vacuum deposition method at the time of manufacturing an organic light emitting device. Herein, the solution coating method means a spin coating, a dip coating, a doctor blading, an inkjet printing, a screen printing, a spray method, a roll coating, or the like, but is not limited thereto.

In addition to such a method, the organic light emitting device may be manufactured by sequentially depositing a cathode material, an organic material layer and an anode material on a substrate (International Publication WO2003/012890). However, the manufacturing method is not limited thereto.

As an example, the first electrode is an anode, and the second electrode is a cathode, or alternatively the first electrode is a cathode and the second electrode is an anode.

As the anode material, generally, a material having a large work function is preferably used so that holes can be smoothly injected into the organic material layer. Specific examples of the anode material include metals such as vanadium, chrome, copper, zinc, and gold, or an alloy thereof; metal oxides such as zinc oxides, indium oxides, indium tin oxides (ITO), and indium zinc oxides (IZO); a combination of metals and oxides, such as ZnO:Al or SnO₂:Sb; conductive polymers such as poly(3-methylthiophene), poly[3,4-(ethylene-1,2-dioxy)thiophene](PEDOT), polypyrrole, and polyaniline, and the like, but are not limited thereto.

As the cathode material, generally, a material having a small work function is preferably used so that electrons can be easily injected into the organic material layer. Specific examples of the cathode material include metals such as magnesium, calcium, sodium, potassium, titanium, indium, yttrium, lithium, gadolinium, aluminum, silver, tin, and lead, or an alloy thereof; a multilayered structure material such as LiF/Al or LiO₂/Al, and the like, but are not limited thereto.

The hole injection material is a layer for injecting holes from the electrode, and the hole injection material is preferably a compound which has a capability of transporting the holes, thus has a hole injecting effect in the anode and an excellent hole injecting effect to the light emitting layer or the light emitting material, prevents excitons produced in the light emitting layer from moving to an electron injection layer or the electron injection material, and is excellent in the ability to form a thin film. It is preferable that a HOMO (highest occupied molecular orbital) of the hole injection material is between the work function of the anode material and a HOMO of a peripheral organic material layer. Specific examples of the hole injection material include metal porphyrine, oligothiophene, an arylamine-based organic material, a hexanitrilehexaazatriphenylene-based organic material, a quinacridone-based organic material, a perylene-based organic material, anthraquinone, polyaniline and polythiophene-based conductive polymer, and the like, but are not limited thereto.

The hole transport layer is a layer that receives holes from a hole injection layer and transports the holes to the light emitting layer, and it is suitably a material having large mobility to the holes, which may receive holes from the anode or the hole injection layer and transfer the holes to the light emitting layer. Specific examples thereof include an arylamine-based organic material, a conductive polymer, a block copolymer in which a conjugate portion and a non-conjugate portion are present together, and the like, but are not limited thereto.

The light emitting material is preferably a material which may receive holes and electrons transported from a hole transport layer and an electron transport layer, respectively, and combine the holes and the electrons to emit light in a visible ray region, and has good quantum efficiency to fluorescence or phosphorescence. Specific examples of the light emitting material include an 8-hydroxy-quinoline aluminum complex (Alq₃); a carbazole-based compound; a dimerized styryl compound; BAlq; a 10-hydroxybenzoquinoline-metal compound; a benzoxazole, benzthiazole and benzimidazole-based compound; a poly(p-phenylenevinylene)(PPV)-based polymer; a spiro compound; polyfluorene, lubrene, and the like, but are not limited thereto.

The light emitting layer may include a host material and a dopant material. The host material may be a fused aromatic ring derivative, a heterocycle-containing compound or the like. Specific examples of the fused aromatic ring derivatives include anthracene derivatives, pyrene derivatives, naphthalene derivatives, pentacene derivatives, phenanthrene compounds, fluoranthene compounds, and the like. Examples of the heterocyclic-containing compounds include carbazole derivatives, dibenzofuran derivatives, ladder-type furan compounds, pyrimidine derivatives, and the like, but are not limited thereto.

The dopant material includes an aromatic amine derivative, a styrylamine compound, a boron complex, a fluoranthene compound, a metal complex, and the like. Specifically, the aromatic amine derivative is a substituted or unsubstituted fused aromatic ring derivative having an arylamino group, and examples thereof include pyrene, anthracene, chrysene, periflanthene and the like, which have an arylamino group. The styrylamine compound is a compound where at least one arylvinyl group is substituted in substituted or unsubstituted arylamine, in which one or two or more substituent groups selected from the group consisting of an aryl group, a silyl group, an alkyl group, a cycloalkyl group, and an arylamino group are substituted or unsubstituted. Specific examples thereof include styrylamine, styryldiamine, styryltriamine, styryltetramine, and the like, but are not limited thereto. Further, the metal complex includes an iridium complex, a platinum complex, and the like, but is not limited thereto.

The electron transport layer is a layer which receives electrons from an electron injection layer and transports the electrons to a light emitting layer, and an electron transport material is suitably a material which may receive electrons well from a cathode and transfer the electrons to a light emitting layer, and has a large mobility for electrons. Specific examples of the electron transport material include: an Al complex of 8-hydroxyquinoline; a complex including Alq₃; an organic radical compound; a hydroxyflavonemetal complex, and the like, but are not limited thereto. The electron transport layer may be used with any desired cathode material, as used according to the related art. In particular, appropriate examples of the cathode material are a typical material which has a low work function, followed by an aluminum layer or a silver layer. Specific examples thereof include cesium, barium, calcium, ytterbium, and samarium, in each case followed by an aluminum layer or a silver layer.

The electron injection layer is a layer which injects electrons from an electrode, and is preferably a compound which has a capability of transporting electrons, has an effect of injecting electrons from a cathode and an excellent effect of injecting electrons into a light emitting layer or a light emitting material, prevents excitons produced from the light emitting layer from moving to a hole injection layer, and is also excellent in the ability to form a thin film. Specific examples of the electron injection layer include fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, a metal complex compound, a nitrogen-containing 5-membered ring derivative, and the like, but are not limited thereto.

The metal complex compound includes 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, bis(2-methyl-8-quinolinato)(2-naphtholato)gallium, and the like, but are not limited thereto.

The organic light emitting device according to the present disclosure may be a front side emission type, a back side emission type, or a double side emission type according to the used material.

In addition, the compound represented by Chemical Formula 1 may be included in an organic solar cell or an organic transistor in addition to an organic light emitting device.

The preparation of the compound represented by Chemical Formula 1 and the organic light emitting device containing the same will be described in detail in the following examples. However, these examples are presented for illustrative purposes only and are not intended to limit the scope of the present disclosure.

### <Preparation Example>

### Preparation Example 1-1: Preparation of Intermediate Compound B

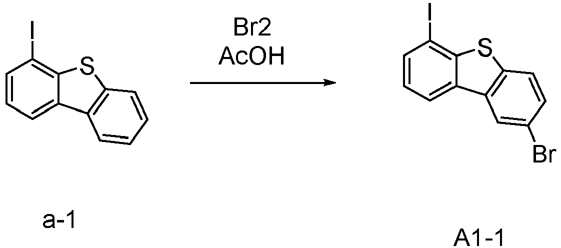

Compound a-1 (4-iododibenzothiophene) (20g, 96.73mmol) was added to a dried round flask under a nitrogen atmosphere and then 500ml of chloroform was added thereto. Then, 2.5eq of bromine (12.4ml, 24.1mmol) was added dropwise and reacted at room temperature for 12 hours. After completion of the reaction, the mixture was extracted with dichloromethane and an aqueous sodium thiosulfate solution, and the organic layer was dried over anhydrous magnesium sulfate and filtered. The filtered organic layer was concentrated under reduced pressure and then crystallized from ethyl acetate and hexane to give a white compound A1-1 (28g, 75%, MS:[M+H]⁺=388).

### Preparation Examples 1-2 to 1-8: Preparation of Intermediate Compound B

Intermediate Compound B was prepared in the same manner as in the method for preparing Compound A1-1, except that the reactant A shown in Table 1 below was used instead of Compound a-1 (4-iododibenzothiophene) as the reactant in Preparation Example 1-1.

**[Table 1]**

| Category | Reactant A | Intermediate Compound B | Color | Yield (%) | MS: [M+H]⁺ |
|---|---|---|---|---|---|
| Preparation Example 1-1 | 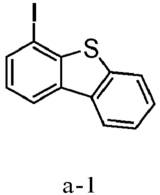 | 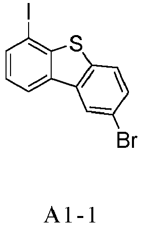 | white | 75% | 388 |
| Preparation Example 1-2 | 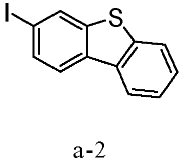 | 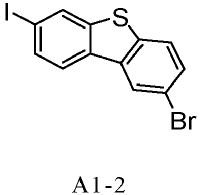 | white | 82% | 388 |
| Preparation Example 1-3 | 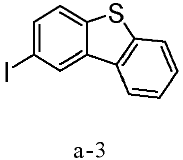 | 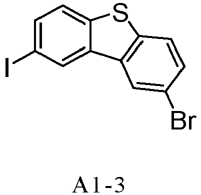 | white | 69% | 388 |
| Preparation Example 1-4 | 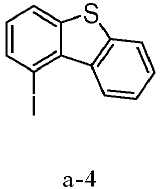 | 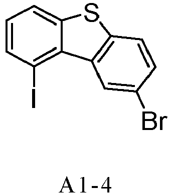 | white | 71% | 388 |
| Preparation Example 1-5 | 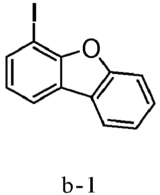 | 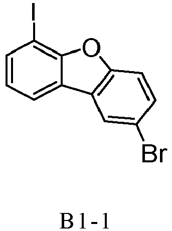 | white | 55% | 388 |
| Preparation Example 1-6 | 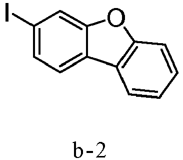 | 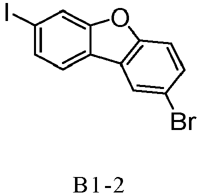 | white | 60% | 388 |
| Preparation Example 1-7 | 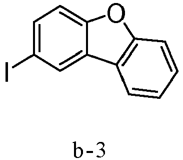 | 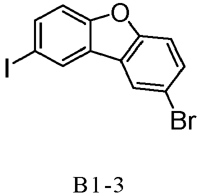 | white | 62% | 388 |
| Preparation Example 1-8 | 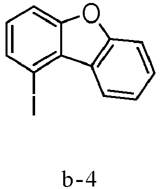 | 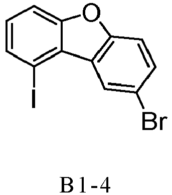 | white | 59% | 388 |

### Preparation Example 2-1: Preparation of Intermediate Compound D

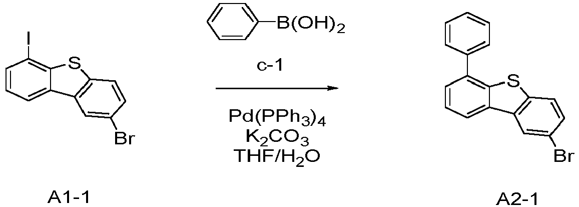

Compound A1-1 (20 g, 51.41 mmol) and Compound c-1 (phenylboronic acid) (6.27 g, 51.41 mmol) were dissolved in tetrahydrofuran (150 mL) under a nitrogen atmosphere, and then potassium carbonate (21.3 g, 154.2 mmol) was dissolved in water (50 mL) and added thereto, and tetrakis(triphenylphosphine)palladium (1.8 g, 1.54 mmol) was added, and the mixture was heated and stirred at 60°C for 9 hours. The temperature was lowered to room temperature, and the reaction mixture was filtered, washed with water and ethanol, and dried to prepare Compound A2-1 (15.2 g, yield: 87%, MS:[M+H]⁺=338).

### Preparation Examples 2-2 to 2-9: Preparation of Intermediate Compound D

Intermediate Compound D was prepared in the same manner as in Preparation Example 2-1, except that Intermediate Compound B and Intermediate Compound C shown in Table 2 below were used instead of Compound A1-1 and Compound c-1 (phenylboronic acid) in Preparation Example 2-1.

**[Table 2]**

| Category | Intermediat e Compound B | Intermediate Compound C | Intermediate Compound D | Colo r | Yiel d | MS : [M+H] ⁺ |
|---|---|---|---|---|---|---|
| Preparatio n Example 2-1 | 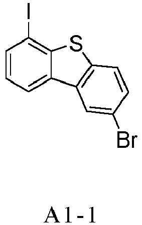 | 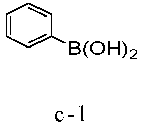 | 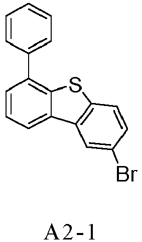 | whit e | 87% | 338 |
| Preparatio n Example 2-2 | 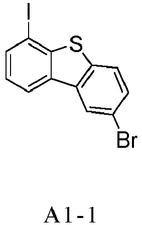 | 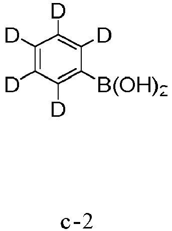 | 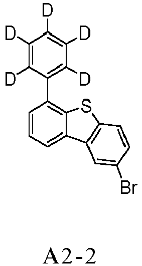 | whit e | 77% | 344 |
| Preparatio n Example 2-3 | 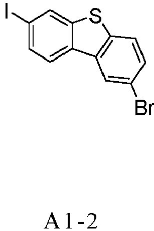 | 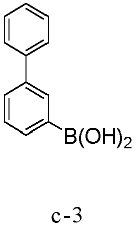 | 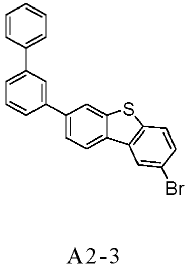 | whit e | 89% | 415 |
| Preparatio n Example 2-4 | 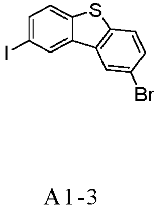 | 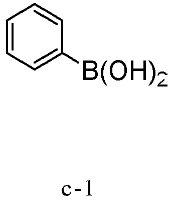 | 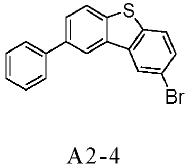 | whit e | 94% | 338 |
| Preparatio n Example 2-5 | 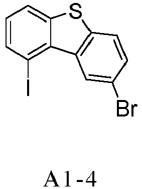 | 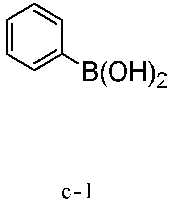 | 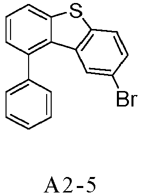 | whit e | 81% | 338 |
| Preparatio n Example 2-6 | 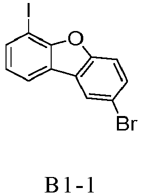 | 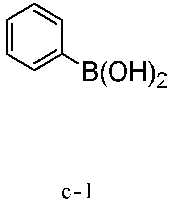 | 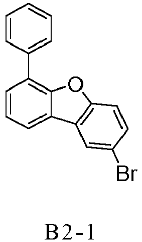 | whit e | 87% | 323 |
| Preparatio n Example 2-7 | 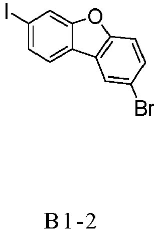 | 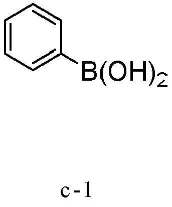 | 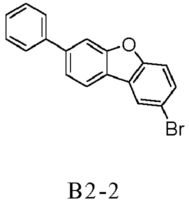 | whit e | 92% | 323 |
| Preparatio n Example 2-8 | 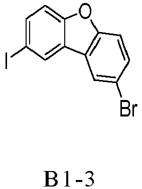 | 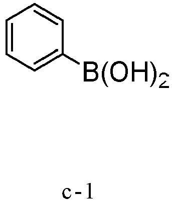 | 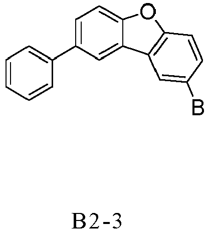 | whit e | 90% | 323 |
| Preparatio n Example 2-9 | 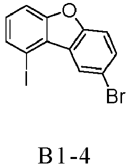 | 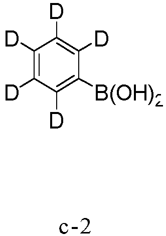 | 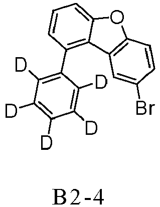 | whit e | 89% | 328 |

### Preparation Example 3-1: Preparation of Intermediate Compound F

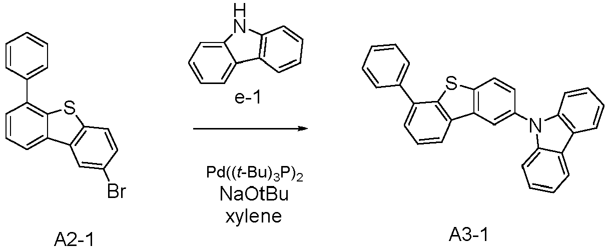

Compound A2-1 (15 g, 44.22 mmol) and Compound e-1 (9H-carbazole) (7.39 g, 44.22 mmol) were dissolved in xylene (150 mL) under a nitrogen atmosphere, to which NaOtBu (6.4 g, 66.32 mmol) was added and bis(tritert-butylphosphine)palladium(0) (0.7 g, 1.33 mmol) was added, and the mixture was heated and stirred for 12 hours. The temperature was lowered to room temperature, and the reaction mixture was filtered, washed with water and ethanol, and dried to prepare Compound A1-1 (13.9 g, yield: 74%, MS:[M+H]⁺=426).

### Preparation Examples 3-2 to 3-10: Preparation of Intermediate Compound F

Intermediate Compound F was prepared in the same manner as in Preparation Example 3-1, except that Intermediate Compound D and Intermediate Compound E shown in Table 3 below were used instead of Compound A2-1 and Compound e-1 (phenylboronic acid) in Preparation Example 3-1.

**[Table 3]**

| Category | Intermediate Compound D | Intermediat e Compound E | Intermediate Compound F | Color | Yiel d | MS : [M+H] ⁺ |
|---|---|---|---|---|---|---|
| Preparati on Example 3-1 | 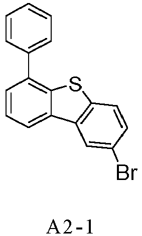 | 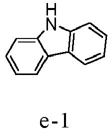 | 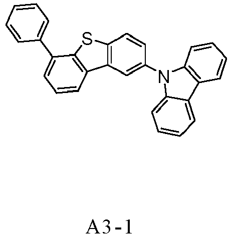 | yello w | 74% | 426 |
| Preparati on Example 3-2 | 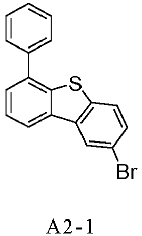 | 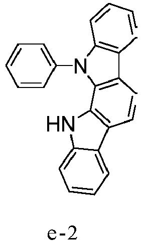 | 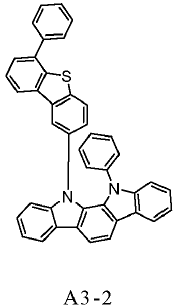 | yello w | 75% | 591 |
| Preparati on Example 3-3 | 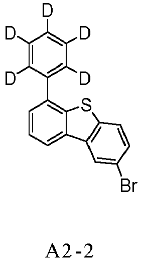 | 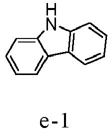 | 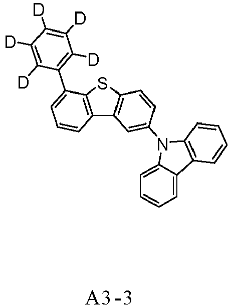 | yello w | 72% | 431 |
| Preparati on Example 3-4 | 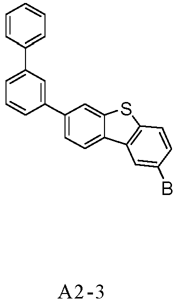 | 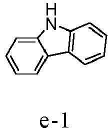 | 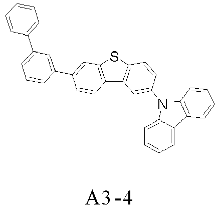 | yello w | 65% | 502 |
| Preparati on Example 3-5 | 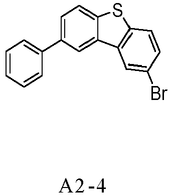 | 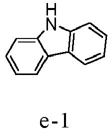 | 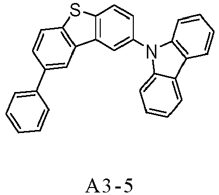 | yello w | 88% | 426 |
| Preparati on Example 3-6 | 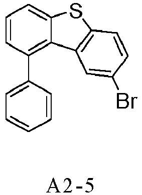 | 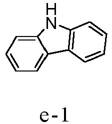 | 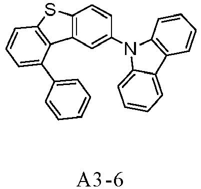 | yello w | 72% | 338 |
| Preparati on Example 3-7 | 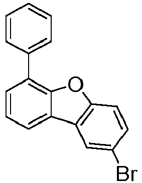 | 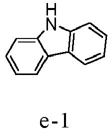 | 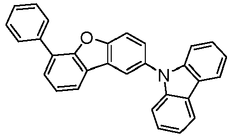 | yello w | 86% | 410 |
| | B2-1 | | B3-1 | | | |
| Preparati on Example 3-8 | 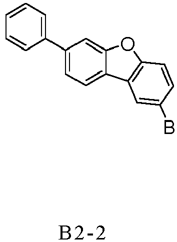 | 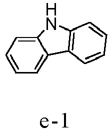 | 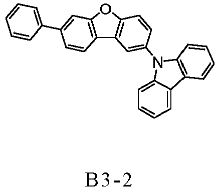 | yello w | 81% | 410 |
| Preparati on Example 3-9 | 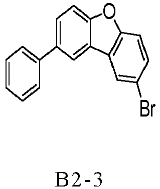 | 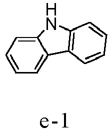 | 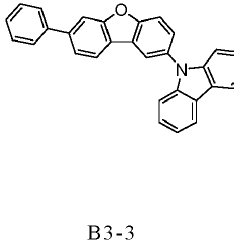 | yello w | 79% | 410 |
| Preparati on Example 3-10 | 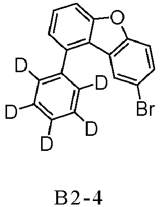 | 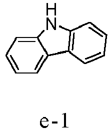 | 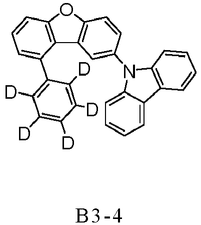 | yello w | 77% | 415 |

### Preparation Example 4-1: Preparation of Compound A1-4

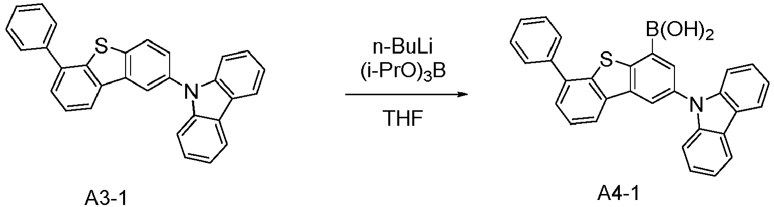

Compound A3-1 (14 g, 32.90 mmol) was dissolved in 150 mL of THF in a dried round bottom flask under a nitrogen atmosphere, to which 1.6M n-BuLi (26.73 mL, 42.77 mmol) was slowly added dropwise at -78°C, and the mixture was stirred at room temperature for 1 hour. Isopropylborate (i-PrO)3B (26.5mL, 115.1mmol) was added dropwise to the reaction mixture at -78°C, and then stirred at room temperature for 1 hour. After completion of the reaction, the mixture was extracted with an aqueous ammonium chloride solution at room temperature, and the organic layer was dried over MgSO₄, concentrated, and recrystallized from ethyl acetate to give Compound A4-1. (10 g, yield: 65%, MS:[M+H]⁺= 470)

### Preparation Examples 4-2 to 4-10: Preparation of Intermediate Compound G

Intermediate Compound G was prepared in the same manner as in Preparation Example 4-1, except that Intermediate Compound F shown in Table 4 below was used instead of Compound A3-1 in Preparation Example 4-1.

**[Table 4]**

| Category | Intermediate Compound F | Intermediate Compound G | Colo r | Yiel d | MS : [M+H] ⁺ |
|---|---|---|---|---|---|
| Preparatio n Example 4-1 | 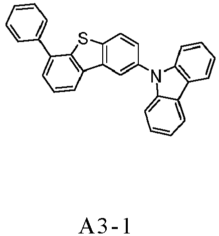 | 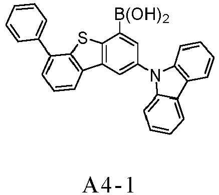 | whit e | 65% | 470 |
| Preparatio n Example 4-2 | 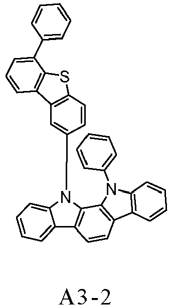 | 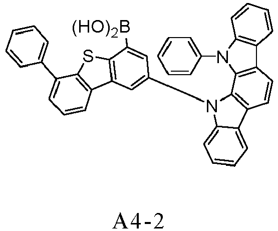 | whit e | 62% | 635 |
| Preparatio n Example 4-3 | 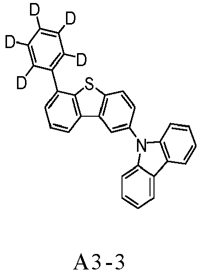 | 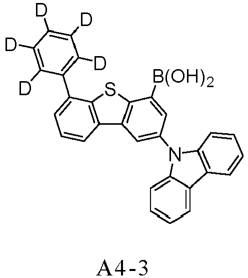 | whit e | 69% | 475 |
| Preparatio n Example 4-4 | 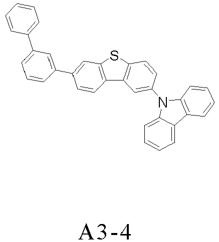 | 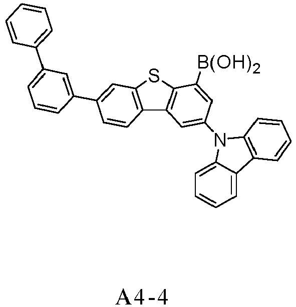 | whit e | 70% | 546 |
| Preparatio n Example 4-5 | 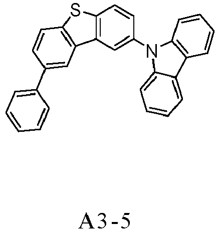 | 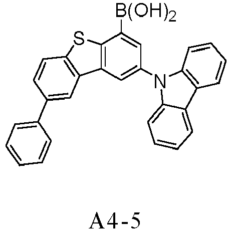 | whit e | 67% | 470 |
| Preparatio n Example 4-6 | 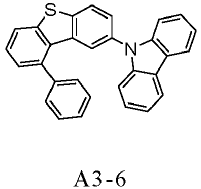 | 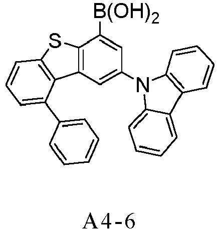 | whit e | 63% | 470 |
| Preparatio n Example 4-7 | 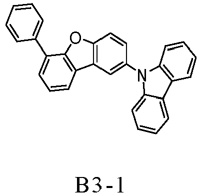 | 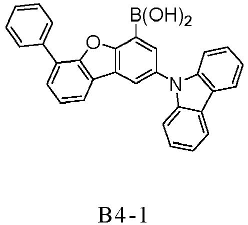 | whit e | 71% | 454 |
| Preparatio n Example 4-8 | 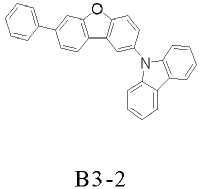 | 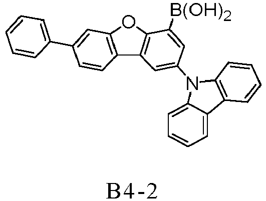 | whit e | 76% | 454 |
| Preparatio n Example 4-9 | 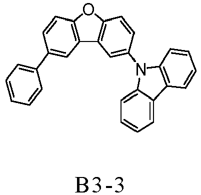 | 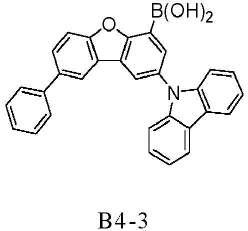 | whit e | 59% | 454 |
| Preparatio n Example 4-10 | 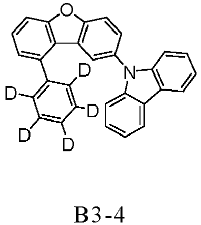 | 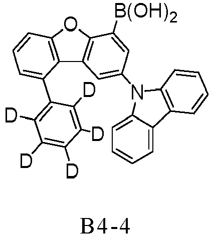 | whit e | 77% | 459 |

### <Example>

### Example 1: Preparation of Compound 1

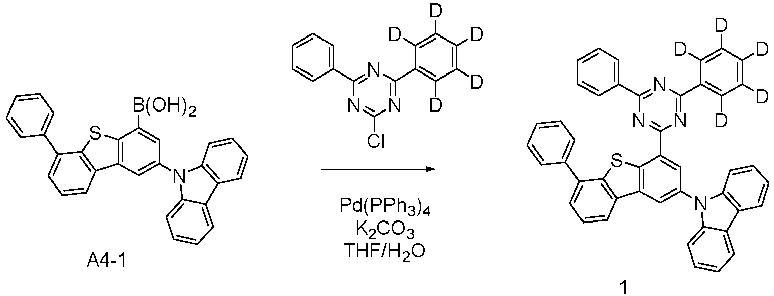

After Compound A4-1 (15 g, 31.96 mmol) and 2-chloro-4-phenyl-6-(phenyl-d5)-1,3,5-triazine (8.71 g, 31.96 mmol) were dissolved in THF (150 mL), potassium carbonate (13.3 g, 211.10 mmol) was added and tetrakis(triphenylphosphine)palladium (1.1 g, 0.96 mmol) was added, and the mixture was heated and stirred for 8 hours. The temperature was lowered to room temperature and the aqueous layer was separated and removed. The result was dried over anhydrous magnesium sulfate, filtered, concentrated under reduced pressure, recrystallized using ethyl acetate, filtered and dried to prepare Compound 1 (15.7g, yield: 74%, MS:[M+H]+=662).

### Example 2: Preparation of Compound 2

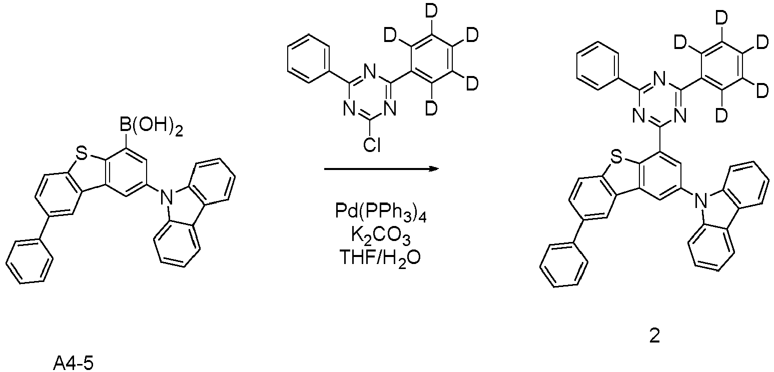

Compound 2 was prepared in the same manner as in the method for preparing Compound 1, except that Compound A4-5 was used instead of Compound A4-1. (15.0 g, yield: 71%; MS:[M+H]⁺=662)

### Example 3: Preparation of Compound 3

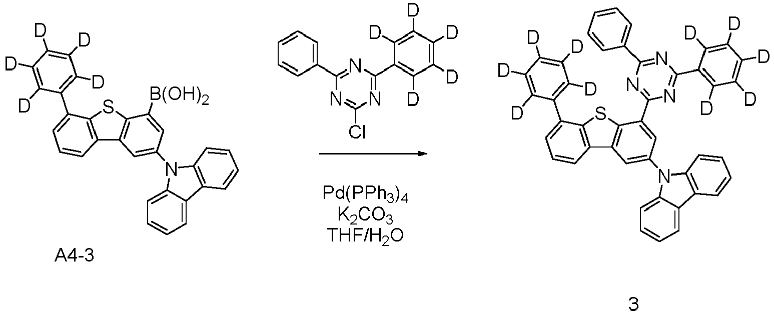

Compound 3 was prepared in the same manner as the method for preparing Compound 1, except that Compound A4-3 was used instead of Compound A4-1. (15.0 g, yield: 71%; MS:[M+H]⁺=667)

### Example 4: Preparation of Compound 4

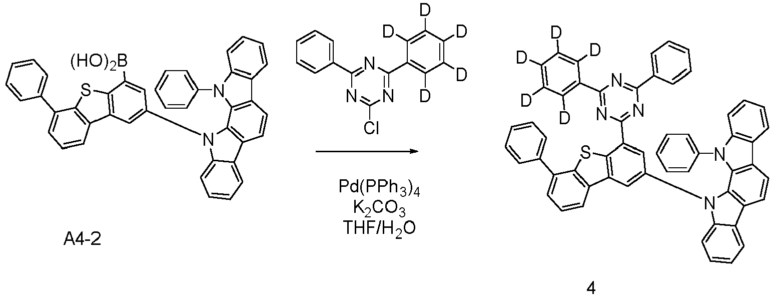

Compound 4 was prepared in the same manner as in the method for preparing Compound 1, except that Compound A4-2 was used instead of Compound A4-1. (16.0 g, yield: 82%; MS:[M+H]⁺=827)

### Example 5: Preparation of Compound 5

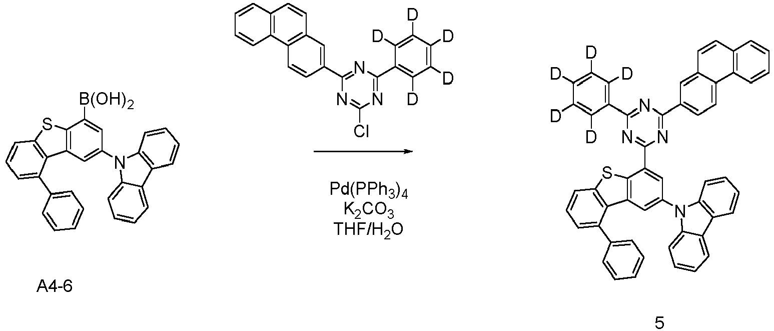

Compound 5 was prepared in the same manner as in the method for preparing Compound 1, except that Compounds A4-6 and 2-chloro-4-(phenanthren-2-yl)-6-(phenyl-d5)-1,3,5-triazine were used instead of Compounds A4-2 and 2-chloro-4-phenyl-6-(phenyl-d5)-1,3,5-triazine. (16.0 g, yield: 82%; MS:[M+H]⁺=827)

### Example 6: Preparation of Compound 6

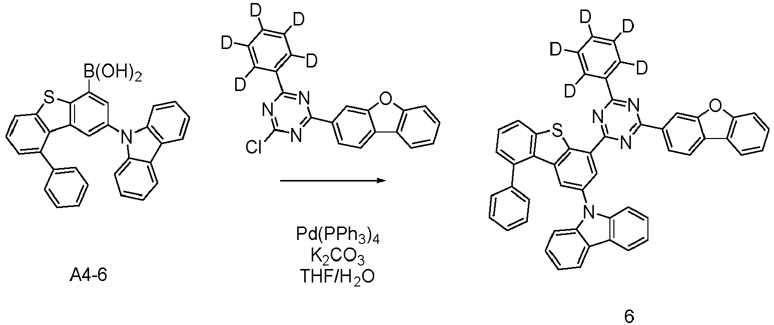

Compound 6 was prepared in the same manner as the method for preparing Compound 1, except that Compound A4-6 and 2-chloro-4-(dibenzo[b,d]furan-3-yl)-6-(phenyl-d5)-1,3,5-triazine were used instead of Compound A4-1 and 2-chloro-4-phenyl-6-(phenyl-d5)-1,3,5-triazine. (17.8 g, yield: 74%; MS:[M+H]⁺=752)

### Example 7: Preparation of Compound 7

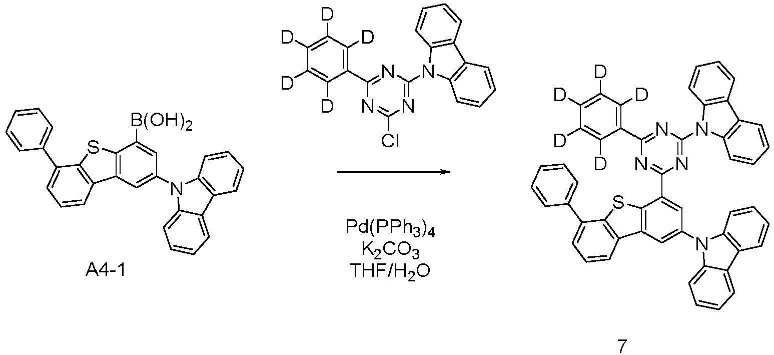

Compound 7 was prepared in the same manner as in the method for preparing Compound 1, except that 9-(4-chloro-6-(phenyl-d5)-1,3,5-triazin-2-yl)-9H-carbazole was used instead of 2-chloro-4-phenyl-6-(phenyl-d5)-1,3,5-triazine. (16.5 g, yield: 69%; MS:[M+H]⁺=751)

### Example 8: Preparation of Compound 8

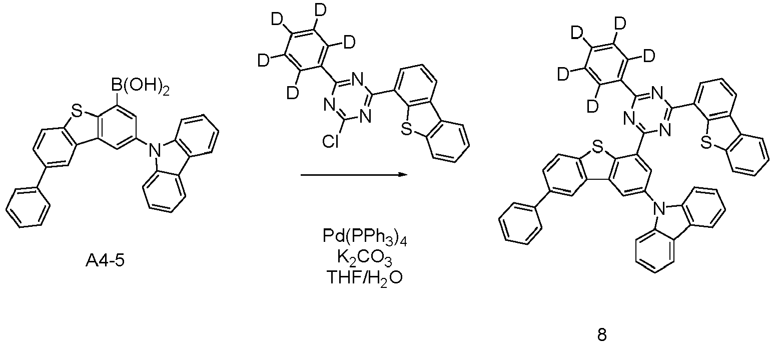

Compound 8 was prepared in the same manner as in the method for preparing Compound 1, except that Compound A4-5 and 2-chloro-4-(dibenzo[b,d]thiophen-4-yl)-6-(phenyl-d5)-1,3,5-triazine were used instead of Compounds A4-1 and 2-chloro-4-phenyl-6-(phenyl-d5)-1,3,5-triazine. (18.9 g, yield: 77%; MS:[M+H]⁺=768)

### Example 9: Preparation of Compound 9

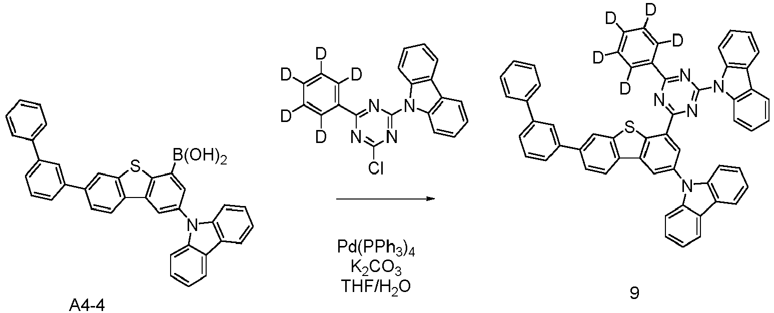

Compound 9 was prepared in the same manner as in the method for preparing Compound 1, except that Compound A4-4 and 9-(4-chloro-6-(phenyl-d5)-1,3,5-triazin-2-yl)-9H-carbazole were used instead of Compound A4-1 and 2-chloro-4-phenyl-6-(phenyl-d5)-1,3,5-triazine. (14.8 g, yield: 65%; MS:[M+H]⁺=827)

### Example 10: Preparation of Compound 10

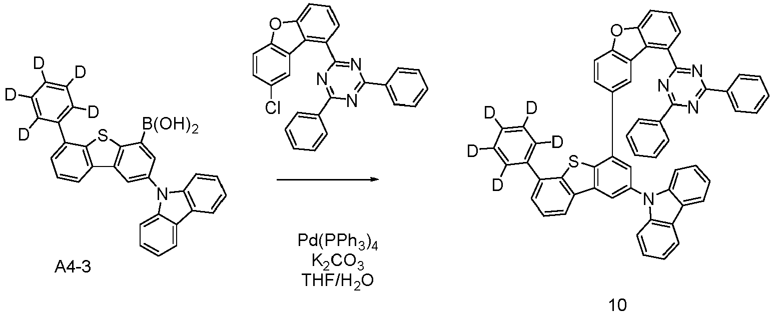

Compound 10 was prepared in the same manner as in the method for preparing Compound 1, except that Compound A4-3 and 2-(8-chlorodibenzo[b,d]furan-1-yl)-4,6-diphenyl-1,3,5-triazine were used instead of Compound A4-1 and 2-chloro-4-phenyl-6-(phenyl-d5)-1,3,5-triazine. (18.3 g, yield 70%; MS:[M+H]⁺=828)

### Example 11: Preparation of compound 11

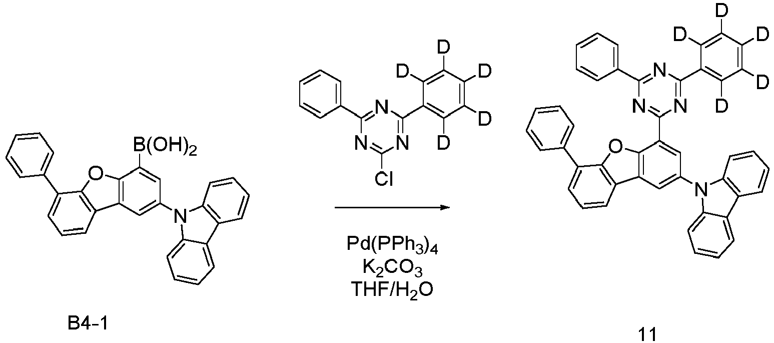

Compound 11 was prepared in the same manner as in the method for preparing Compound 1, except that Compound B4-1 was used instead of Compound A4-1. (16.2 g, yield: 76%; MS:[M+H]⁺=646)

### Example 12: Preparation of Compound 12

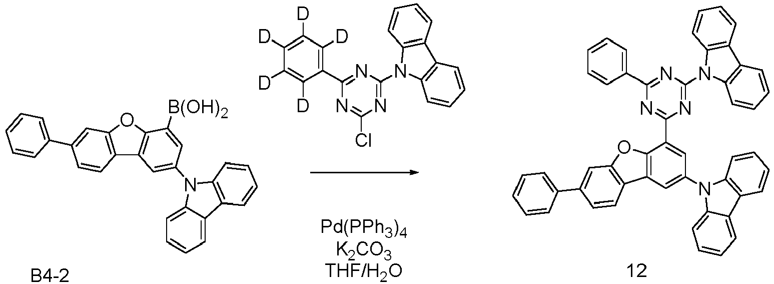

Compound 12 was prepared in the same manner as in the method for preparing Compound 1, except that Compounds B4-2 and 9-(4-chloro-6-(phenyl-d5)-1,3,5-triazin-2-yl)-9H-carbazole were used instead of Compound A4-1 and 2-chloro-4-phenyl-6-(phenyl-d5)-1,3,5-triazine. (19.3 g, yield: 80%; MS:[M+H]⁺=730)

### Example 13: Preparation of Compound 13

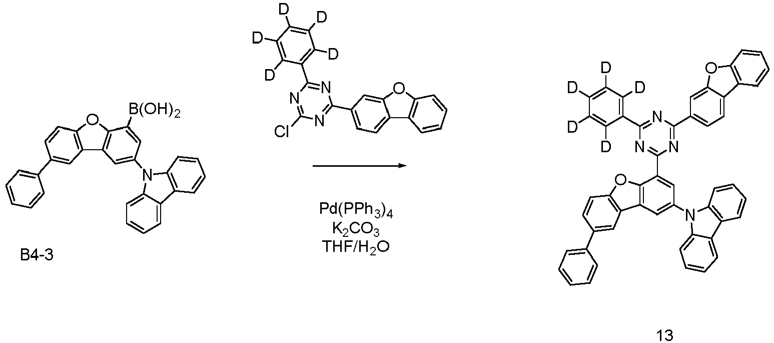

Compound 13 was prepared in the same manner as in the method for preparing Compound 1, except that Compound B4-3 and 2-chloro-4-(dibenzo[b,d]furan-3-yl)-6-(phenyl-d5)-1,3,5-triazine were used instead of Compound A4-1 and 2-chloro-4-phenyl-6-(phenyl-d5)-1,3,5-triazine. (16.6 g, yield: 68%; MS:[M+H]+=736)

### Example 14: Preparation of compound 14

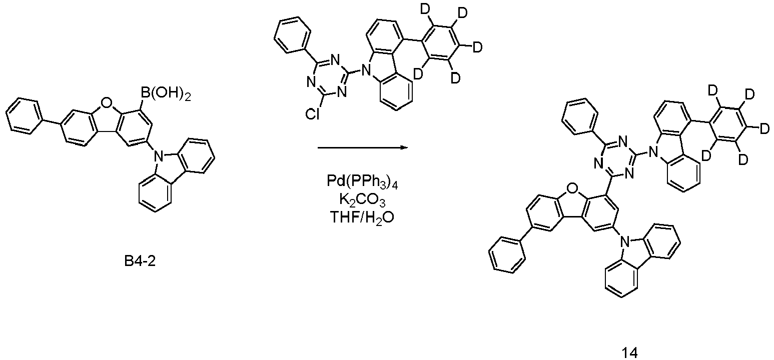

Compound 14 was prepared in the same manner as in the method for preparing Compound 1, except that Compounds B4-2 and 9-(4-chloro-6-phenyl-1,3,5-triazin-2-yl)-4-(phenyl-d5)-9H-carbazole were used instead of Compound A4-1 and 2-chloro-4-phenyl-6-(phenyl-d5)-1,3,5-triazine. (19.3 g, yield: 72%; MS:[M+H]⁺=811)

### Example 15: Preparation of Compound 15

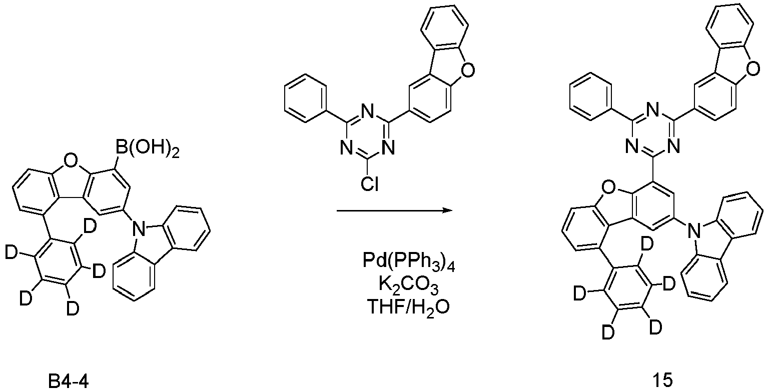

Compound 15 was prepared in the same manner as in the method for preparing Compound 1, except that Compound B4-4 and 2-chloro-4-(dibenzo[b,d]furan-3-yl)-6-(phenyl-d5)-1,3,5-triazine were used instead of Compound A4-4 and 2-chloro-4-phenyl-6-(phenyl-d5)-1,3,5-triazine. (18.8 g, yield: 78%; MS:[M+H]⁺=736)

### [Experimental Example 1]

### Experimental Example 1

A glass substrate on which ITO (indium tin oxide) was coated as a thin film to a thickness of 130 nm was put into distilled water in which a detergent was dissolved, and ultrasonically cleaned. In this case, a product manufactured by Fischer Co. was used as the detergent, and as the distilled water, distilled water filtered twice using a filter manufactured by Millipore Co. was used. After the ITO was cleaned for 30 minutes, ultrasonic cleaning was repeated twice using distilled water for 10 minutes. After the cleaning with distilled water was completed, the substrate was ultrasonically cleaned with solvents of isopropyl alcohol, acetone, and methanol, dried, and then transferred to a plasma cleaner. In addition, the substrate was cleaned for 5 minutes using oxygen plasma and then transferred to a vacuum depositor.

On the ITO transparent electrode thus prepared, the following compound HI-1 was thermally vacuum-deposited to a thickness of 5mn to form a hole injection layer. The following compound HT-1 was thermally vacuum-deposited on the hole injection layer to a thickness of 25 nm to form a hole transport layer, and the following compound HT-2 was vacuum-deposited on the HT-1 deposited layer to a thickness of 5 nm to form an electron blocking layer. Then, the compound 1 previously prepared and the following compound Dp-25 were co-deposited in a weight ratio of 88:12 on the HT-2 deposited layer to form a light emitting layer with a thickness of 40 nm. The following compound ET-1 was vacuum-deposited on the light emitting layer to a thickness of 25nm to form an electron transport layer, and the following compound ET-2 and LiQ were vacuum-deposited (thickness of 10 nm) in a weight ratio of 98:2 thereon to form an electron injection layer. Aluminum was deposited on the electron injection layer to a thickness of 100 nm to form a cathode.

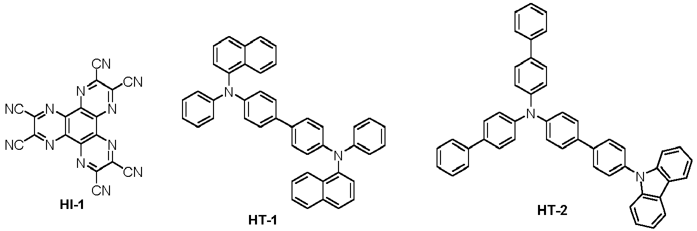

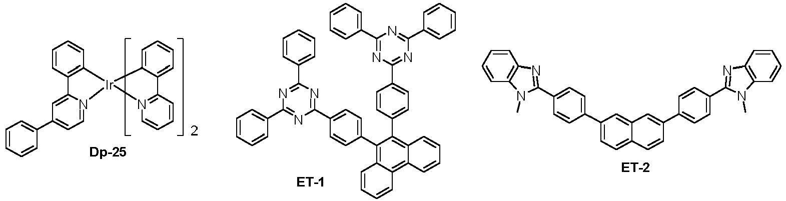

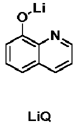

In the above-mentioned process, the vapor deposition rate of the organic material was maintained at 0.04 nm/sec to 0.07 nm/sec, the deposition rate of aluminum was maintained at 0.2 nm/sec, and the degree of vacuum during the deposition was maintained at 1×10⁻⁷ torr to 5×10⁻⁸ torr.

### Experimental Examples 2 to 21 and Comparative Experimental Examples 1 to 9

An organic light emitting device was manufactured in the same manner as in Experimental Example 1, except that the compounds shown in Table 1 below were used instead of Compound 1 in Experimental Example 1.

For reference, in Experimental Examples 16 to 21 and Comparative Experimental Examples 7 to 9, an organic light emitting device was manufactured by using the compounds shown in Table 5 below in the weight ratio of 1:1 instead of Compound 1 in Experimental Example 1. For example, in Example 16, Compound 1 and Compound H-2 were used in a weight ratio of 1:1 instead of Compound 1 in Experimental Example 1.

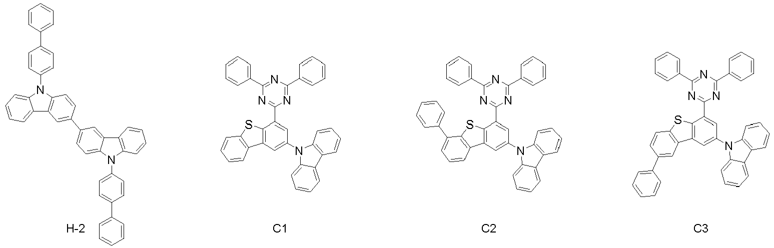

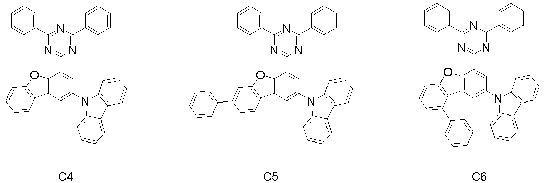

The voltage, efficiency, luminous color, and lifetime were measured by applying a current density of 10 mA/cm² for the organic light emitting devices of Experimental Examples and Comparative Experimental Examples, and the results are shown in Table 1 below. In this case, T95 means the time required for the luminance to be reduced to 95% when the initial luminance at the current density of 20 mA/cm2 is taken as 100%.

**[Table 5]**

| Category | Light emitting layer compound | Voltage (V) (@10mA/cm ²) | Efficiency (cd/A) (@10mA/cm² ) | Luminous color | T₉₅ (@20mA/cm² ) |
|---|---|---|---|---|---|
| Experimental Example 1 | Compound 1 | 3.01 | 65.9 | green | 75 |
| Experimental Example 2 | Compound 2 | 3.00 | 65.8 | green | 79 |
| Experimental Example 3 | Compound 3 | 3.06 | 66.6 | green | 69 |
| Experimental Example 4 | Compound 4 | 2.97 | 66.1 | green | 67 |
| Experimental Example 5 | Compound 5 | 2.89 | 64.0 | green | 66 |
| Experimental Example 6 | Compound 6 | 2.84 | 66.1 | green | 79 |
| Experimental Example 7 | Compound 7 | 3.11 | 67.2 | green | 68 |
| Experimental Example 8 | Compound 8 | 2.98 | 67.9 | green | 63 |
| Experimental Example 9 | Compound 9 | 2.92 | 66.9 | green | 65 |
| Experimental Example 10 | Compound 10 | 3.05 | 68.0 | green | 58 |
| Experimental Example 11 | Compound 11 | 3.03 | 65.9 | green | 63 |
| Experimental Example 12 | Compound 12 | 3.04 | 63.5 | green | 62 |
| Experimental Example 13 | Compound 13 | 2.98 | 64.9 | green | 62 |
| Experimental Example 14 | Compound 14 | 2.94 | 63.1 | green | 66 |
| Experimental Example 15 | Compound 15 | 3.01 | 66.2 | green | 59 |
| Comparative Experimental Example 1 | Compound C1 | 3.01 | 60.0 | green | 50 |
| Comparative Experimental Example 2 | Compound C2 | 3.07 | 62.5 | green | 60 |
| Comparative Experimental Example 3 | Compound C3 | 3.11 | 59.5 | green | 62 |
| Comparative Experimental Example 4 | Compound C4 | 3.06 | 59.2 | green | 51 |
| Comparative Experimental Example 5 | Compound C5 | 3.12 | 61.2 | green | 53 |
| Comparative Experimental Example 6 | Compound C6 | 3.09 | 60.9 | green | 55 |
| Experimental Example 16 | Compound 1, Compound H-2 | 3.29 | 72.1 | green | 160 |
| Experimental Example 17 | Compound 2, Compound H-2 | 3.20 | 74.8 | green | 169 |
| Experimental Example 18 | Compound 6, Compound H-2 | 3.28 | 74.0 | green | 161 |
| Experimental Example 19 | Compound 10, Compound H-2 | 3.32 | 74.7 | green | 165 |
| Experimental Example 20 | Compound 11, Compound H-2 | 3.48 | 74.9 | green | 169 |
| Experimental Example 21 | Compound 15, Compound H-2 | 3.26 | 74.1 | green | 171 |
| Experimental Example 7 | Compound C1, Compound H-2 | 3.14 | 58.9 | green | 138 |
| Comparative Experimental Example 8 | Compound C2, Compound H-2 | 3.50 | 65.0 | green | 154 |
| Comparative Experimental Example 9 | Compound C5, Compound H-2 | 3.48 | 65.9 | green | 141 |

Experimental Examples 1 to 15 and Comparative Experimental Examples 1 to 6 are examples of devices in which a single host was used for the light emitting layer. Compounds C1 to C3 used in Comparative Experimental Examples 1 to 3 are compounds in which Ar₁ of dibenzothiophene is hydrogen or all of Ar₁, Ar₂ and Ar₃ are substituted with only an unsubstituted aryl group. From Table 5, the examples of devices of Experimental Examples 1 to 3 are compounds in which all of Ar₁, Ar₂, and Ar₃ are aryl groups, or are substituted with deuterium. It can be confirmed that the lifetime characteristics are superior by about 21% to 40% compared to Comparative Experimental Examples 1 to 3.

Compounds C4 to C6 used in Comparative Experimental Examples 4 to 6 are compounds in which Ar₁ of dibenzofuran is hydrogen or all of Ar₁, Ar₂ and Ar₃ are substituted with only an unsubstituted aryl group. The example of device of Experimental Example 11 in Table 5 is a compound in which all of Ar₁, Ar₂, and Ar₃ are aryl groups and are substituted with deuterium. It can be confirmed that the lifetime characteristics are superior by about 11% to 21% compared to Comparative Experimental Examples 1 to 3.

The compounds used in Experimental Examples 6 to 9 and Experimental Examples 12-15 are compounds in which Ar₁ and Ar₂ are substituted with deuterium or a heteroaryl group. It can be confirmed that the lifetime characteristics are superior by about 11% to 26% compared to Comparative Experimental Examples 1 to 6 in which Ar₁ and Ar₂ are substituted with only an aryl group.

Experimental Examples 16 to 21 are examples of devices in which two types of hosts are used for the light emitting layer. Even when two types of hosts are used for the light emitting layer, it can be confirmed that the devices of Experimental Examples 16 to 21 using the compound of the present disclosure have superior current efficiency and lifetime characteristics compared to the devices of Comparative Experimental Examples 7 to 9.

### <Description of Symbols>

| | |
|---|---|
| 1: substrate | 2: anode |
| 3: light emitting layer | 4: cathode |
| 5: hole injection layer | |
| 6: hole transport layer | |
| 7: light emitting layer | |
| 8: electron transport layer | |

## Claims

1. A compound represented by the following Chemical Formula 1: 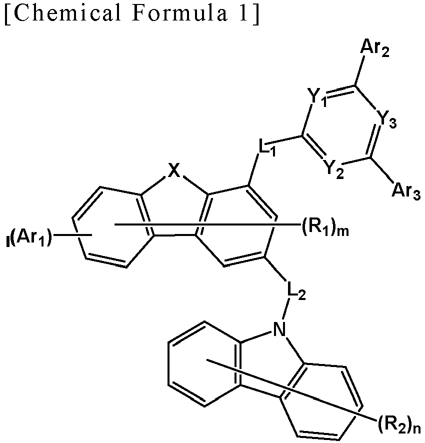 wherein, in Chemical Formula 1,
X is O or S,
Y₁, Y₂ and Y₃ are each independently N,
L₁ and L₂ are each independently a direct bond or any one selected from the group consisting of: 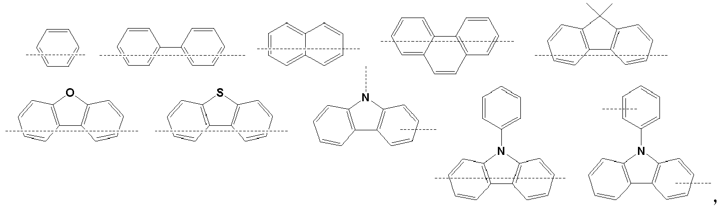
Ar₁ is a substituted or unsubstituted C₆₋₆₀ aryl,
Ar₂ and Ar₃ are each independently a substituted or unsubstituted C₆₋₆₀ aryl; or a substituted or unsubstituted C₅₋₆₀ heteroaryl containing at least one heteroatom selected from the group consisting of N, O and S,
provided that when all of Ar₁, Ar₂ and Ar₃ are a substituted or unsubstituted C₆₋₆₀ aryl, any one of Ar₁, Ar₂ and Ar₃ is substituted with 4 or more deuteriums,
each R₁ is independently hydrogen; deuterium; halogen; or a substituted or unsubstituted C₁₋₆₀ alkyl,
each R₂ is independently hydrogen; deuterium; halogen; cyano; a substituted or unsubstituted C₁₋₆₀ alkyl; a substituted or unsubstituted C₁₋₆₀ alkoxy; a substituted or unsubstituted C₃₋₆₀ cycloalkyl; a substituted or unsubstituted C₁₋₆₀ haloalkyl; a substituted or unsubstituted C₁₋₆₀ haloalkoxy; a substituted or unsubstituted C₆₋₆₀ arylamine; a substituted or unsubstituted C₁₋₆₀ alkylamine; a C₁₋₆₀ trifluoroalkyl; a C₁₋₆₀ trifluoroalkoxy; a substituted or unsubstituted C₆₋₆₀ aryl; or a substituted or unsubstituted C₅₋₆₀ heteroaryl containing at least one of N, O and S, which can bond with the carbon atom of carbazole to form a condensed ring,
m is an integer from 0 to 3,
n is an integer from 0 to 6, and
l is 1 or 2.

2. The compound of claim 1,
wherein the compound represented by the following Chemical Formula 1 is any one selected from the group consisting of compounds represented by the following Chemical Formulae 2 to 8: 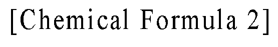 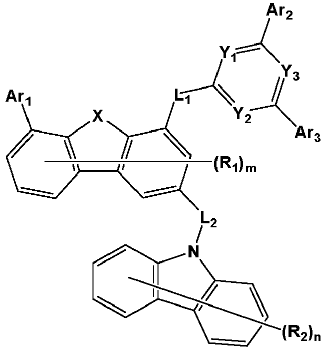 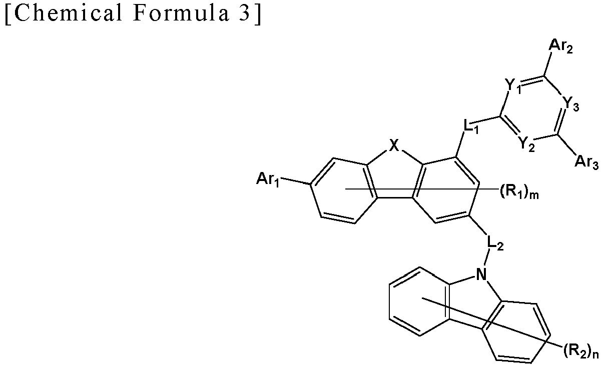 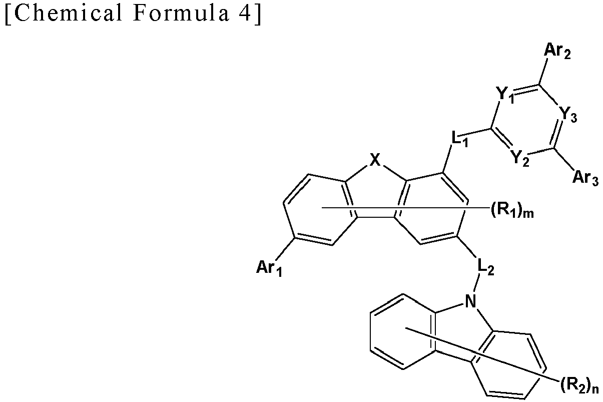 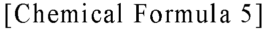 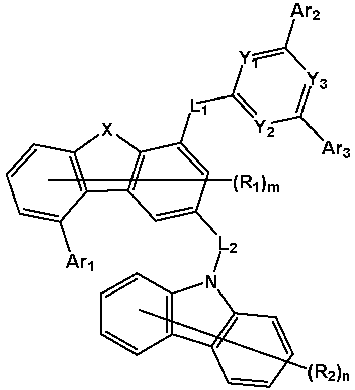 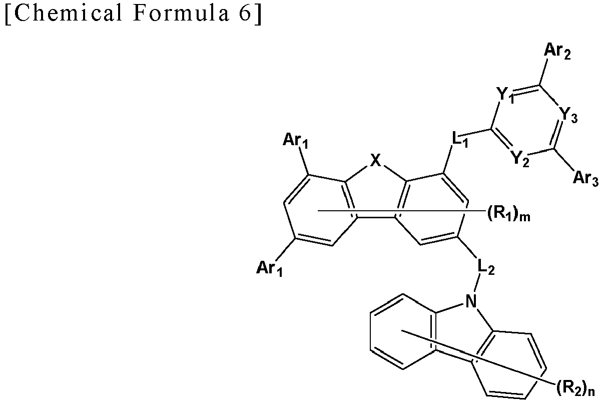 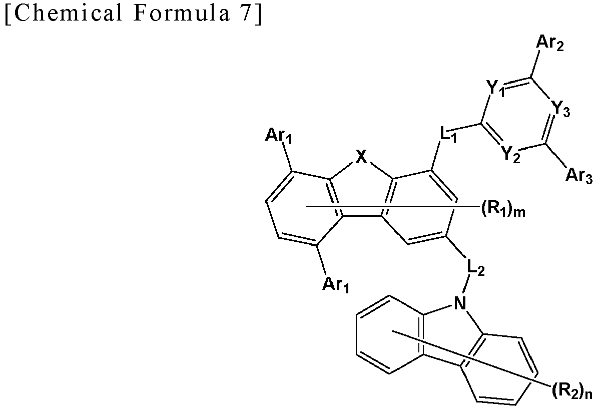 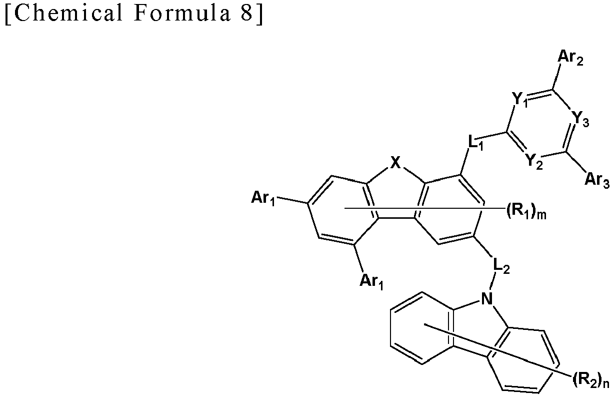 wherein, in Chemical Formulas 2 to 8,
X, Y₁, Y₂, Y₃, L₁, L₂, Ar₁, Ar₂, Ar₃, R₁, R₂, m and n are as defined in claim 1.

3. The compound of claim 1,
wherein Ar₁ is any one selected from the group consisting of: 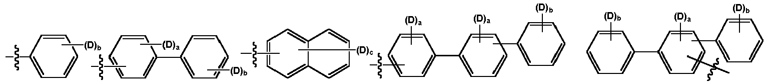 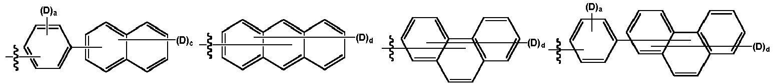 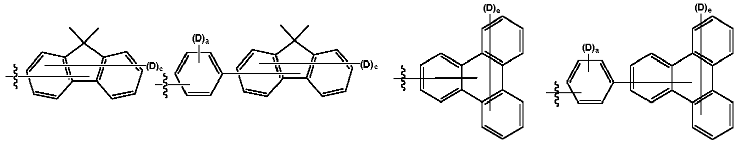 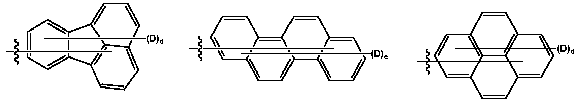 in the above formulas,
a is an integer from 0 to 4,
b is an integer from 0 to 5,
c is an integer from 0 to 7,
d is an integer from 0 to 9, and
e is an integer from 0 to 11.

4. The compound of claim 1,
wherein Ar₂ and Ar₃ are each independently any one selected from the group consisting of: 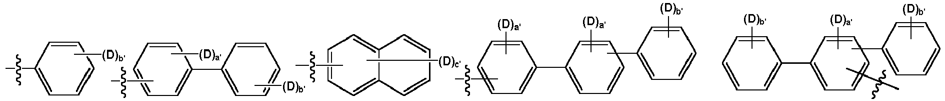 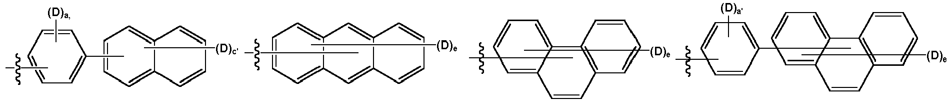 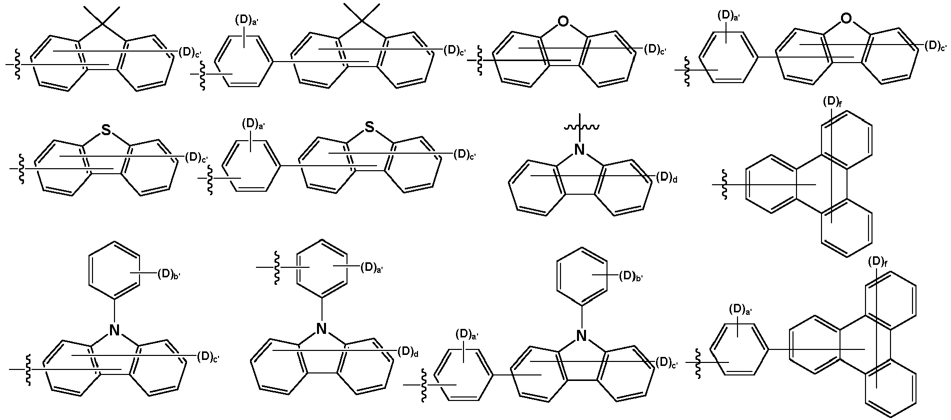 in the above formulas,
a' is an integer from 0 to 4,
b' is an integer from 0 to 5,
c' is an integer from 0 to 7,
d' is an integer from 0 to 8,
e' is an integer from 0 to 9, and
f' is an integer from 0 to 11.

5. The compound of claim 1,
wherein each R₁ is independently hydrogen or deuterium.

6. The compound of claim 1,
wherein each R₂ is independently hydrogen; deuterium; halogen; cyano; methoxy; trifluoromethyl; trifluoromethoxy; phenyl; pyridinyl; isoquinolinyl; or any one selected from the group consisting of, 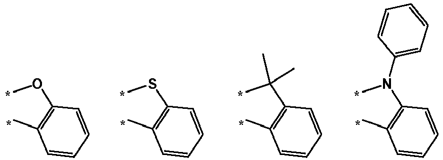
in the above formulas, two * are each connected to an adjacent carbon of the carbazole in Chemical Formula 1, and
the phenyl is substituted or unsubstituted with any one selected from the group consisting of halogen, cyano, methoxy, trifluoromethyl and trifluoromethoxy.

7. The compound of claim 1,
wherein the compound represented by Chemical Formula 1 is any one selected from the group consisting of:

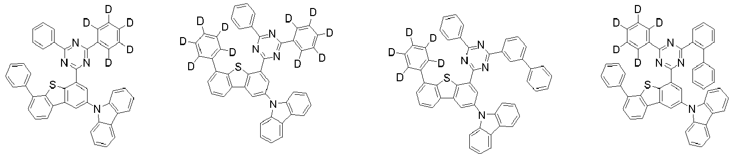

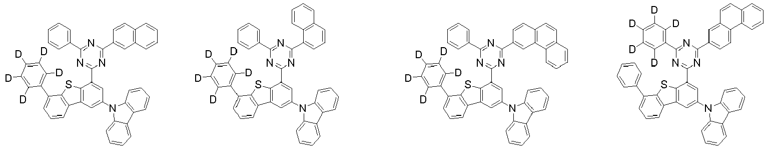

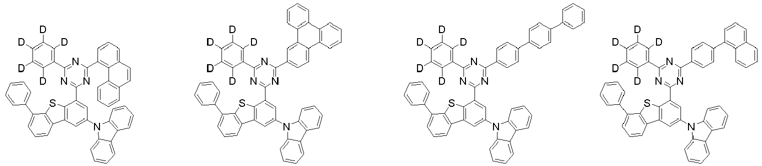

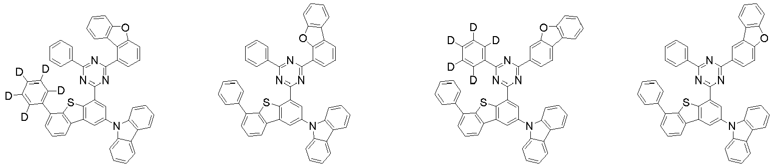

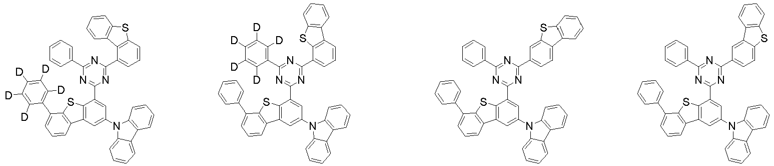

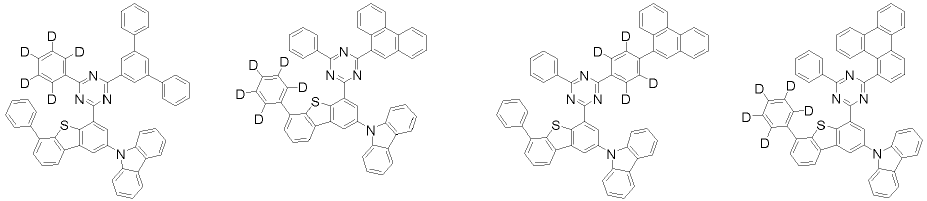

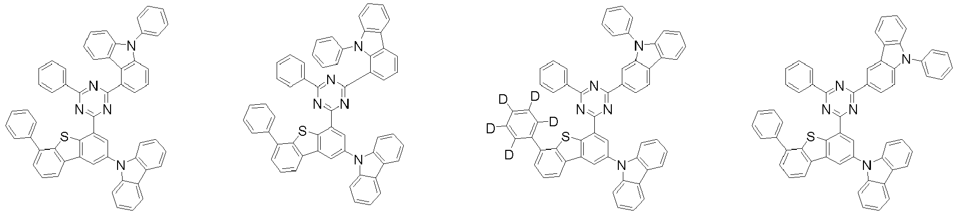

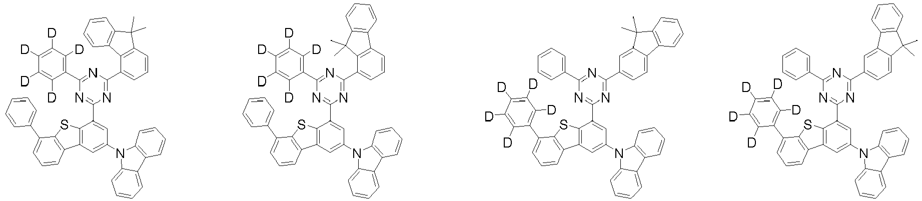

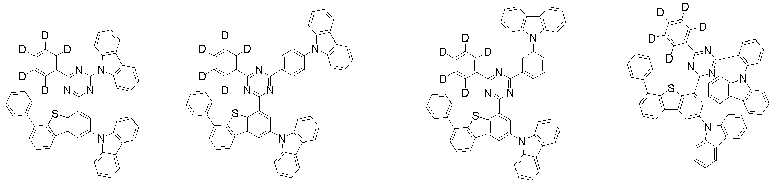

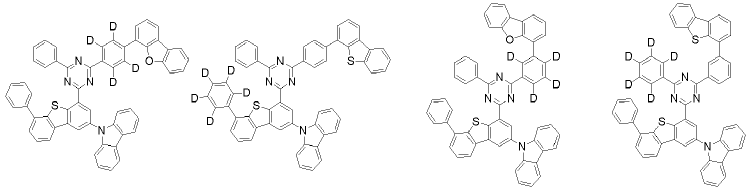

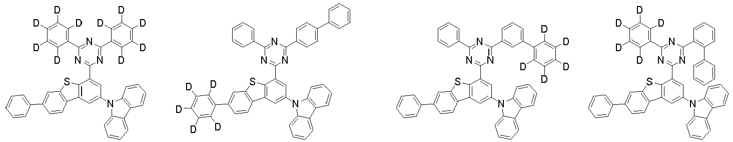

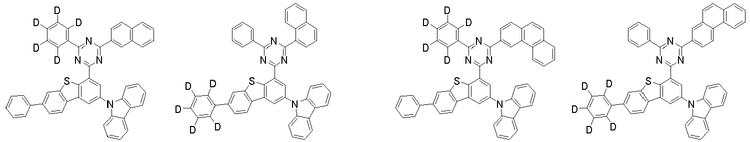

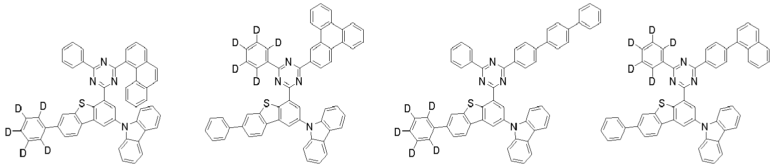

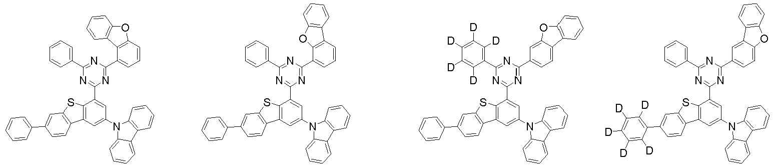

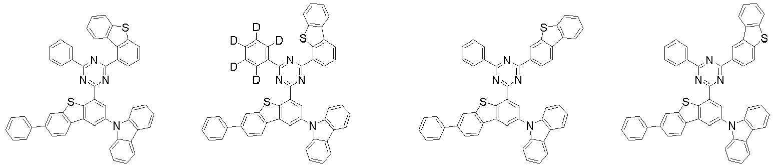

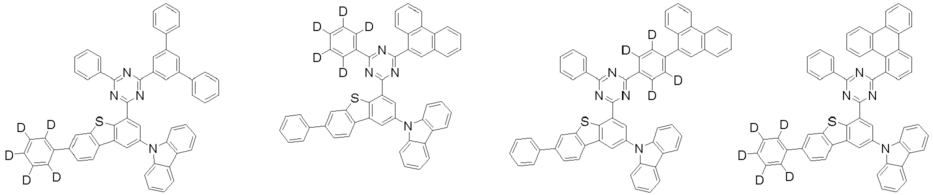

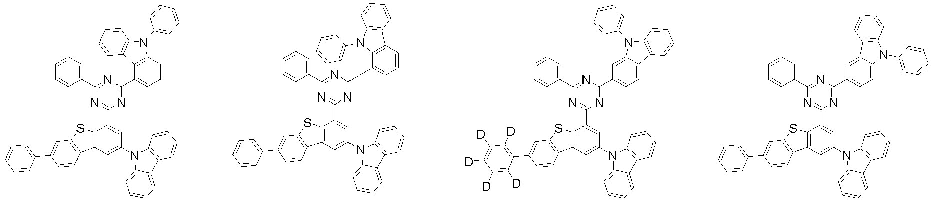

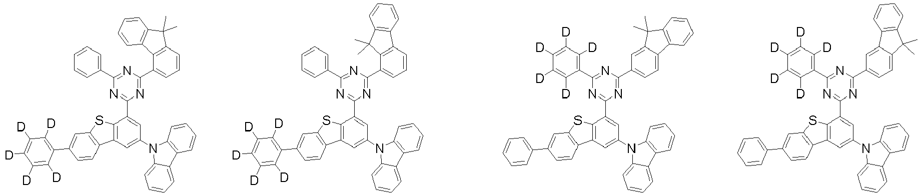

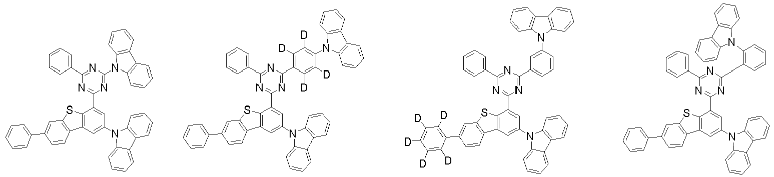

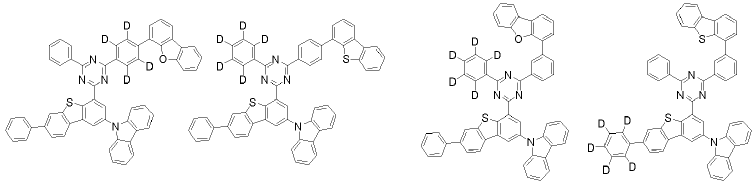

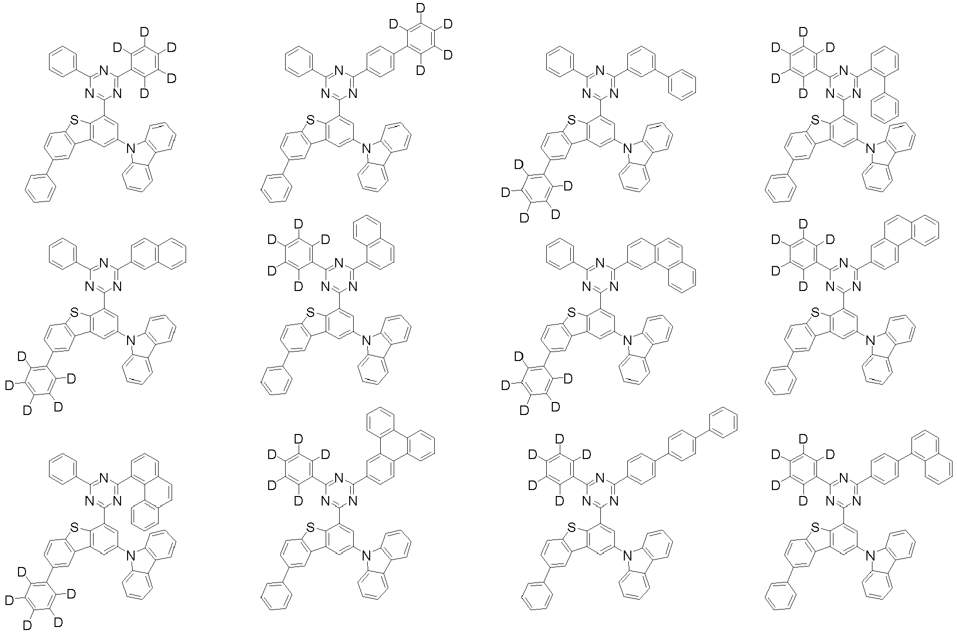

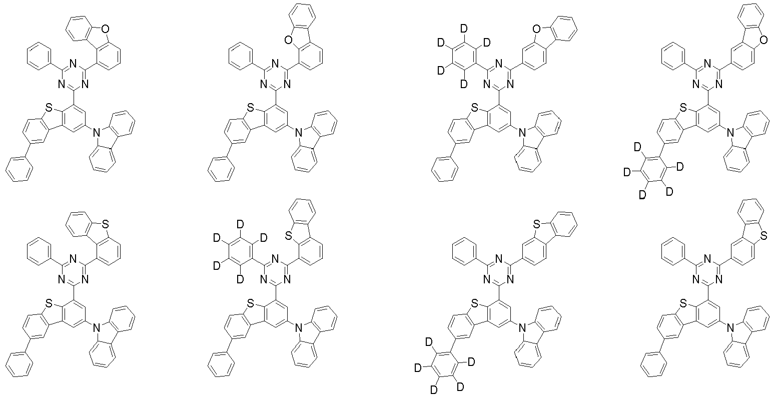

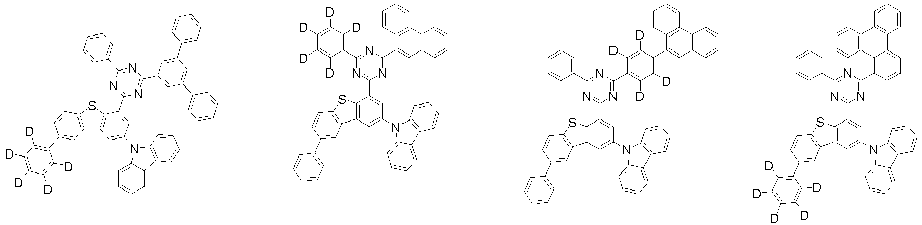

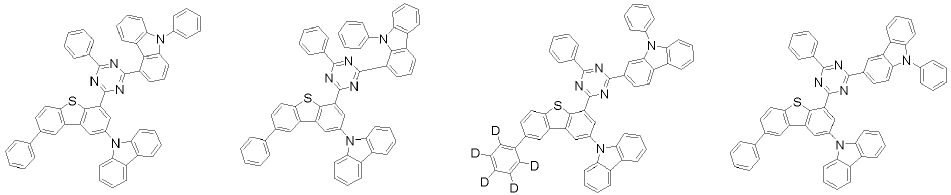

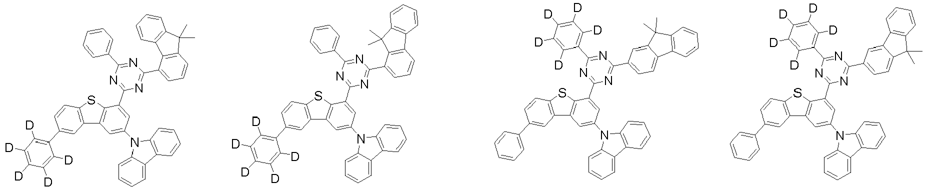

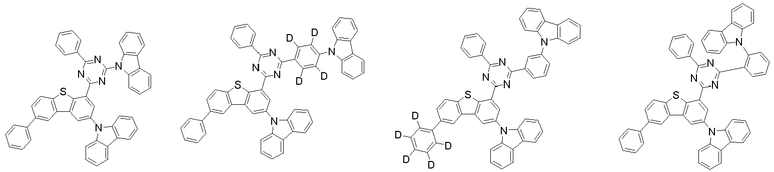

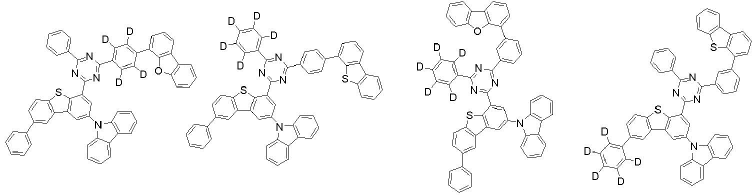

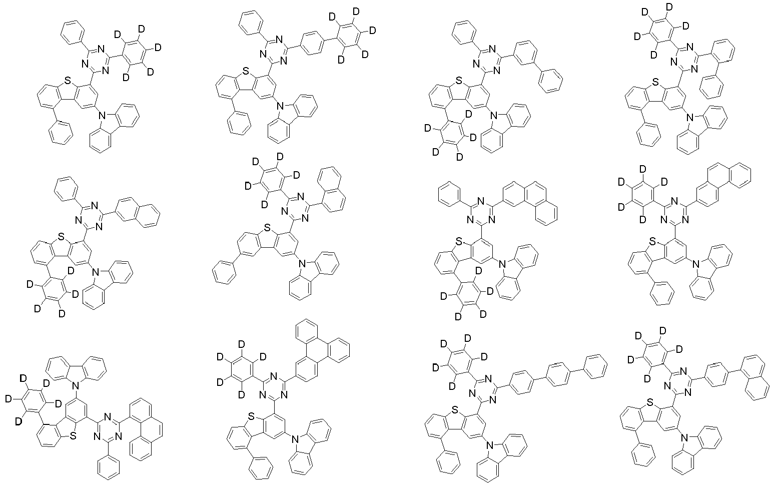

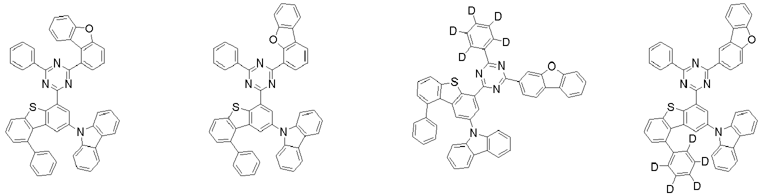

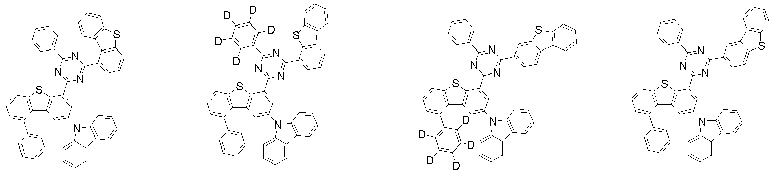

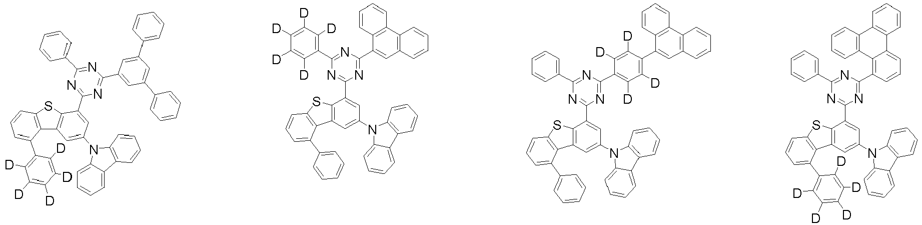

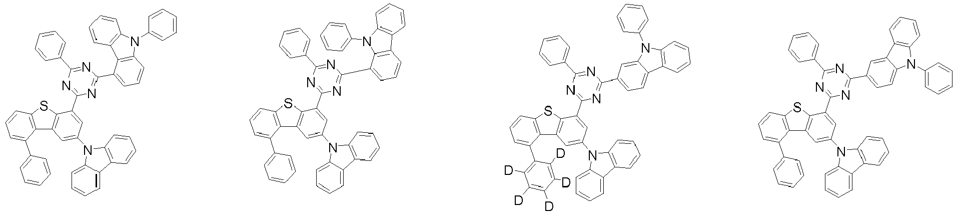

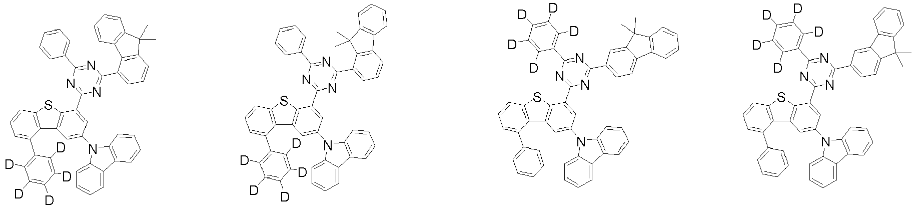

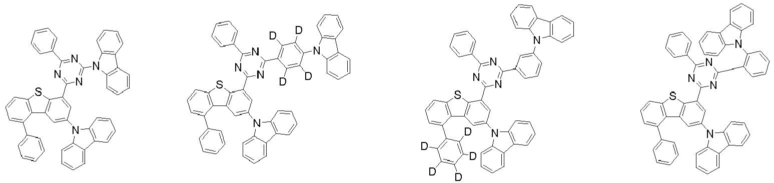

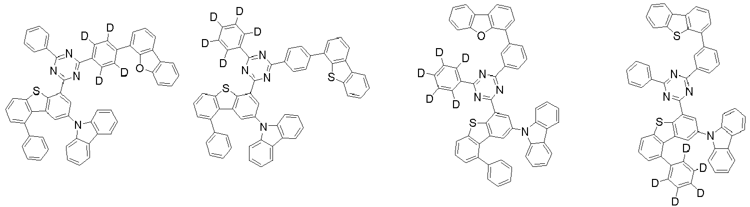

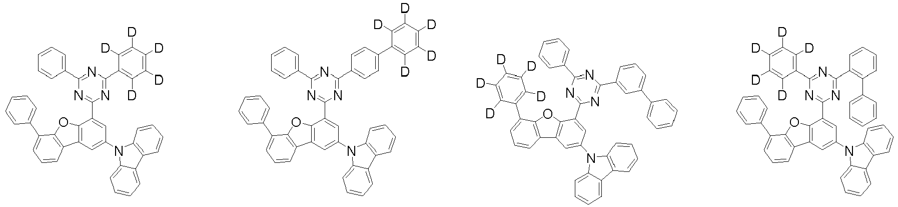

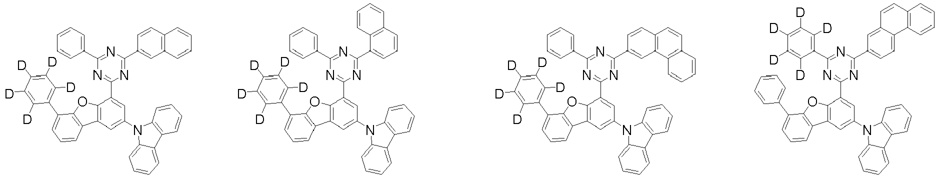

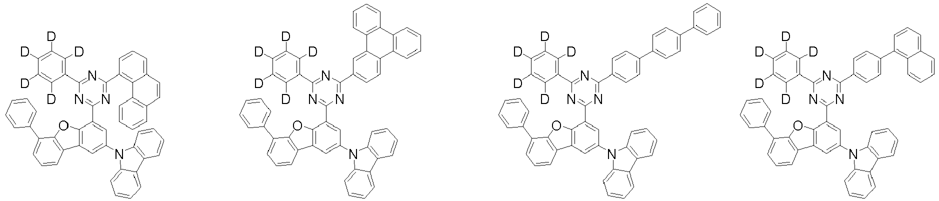

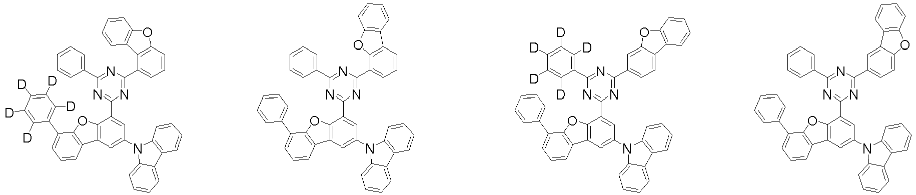

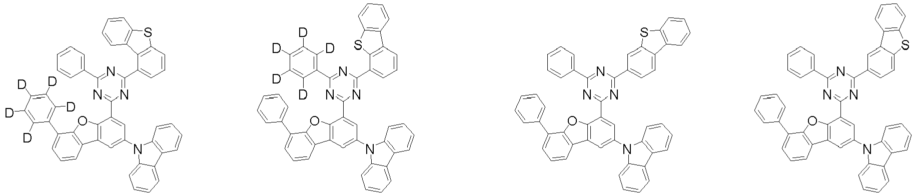

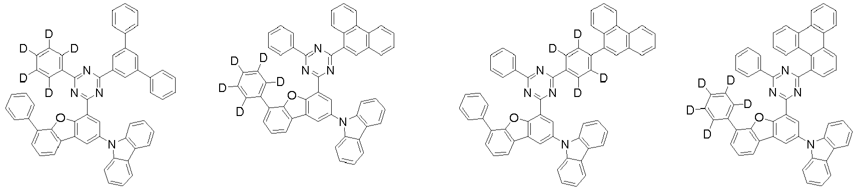

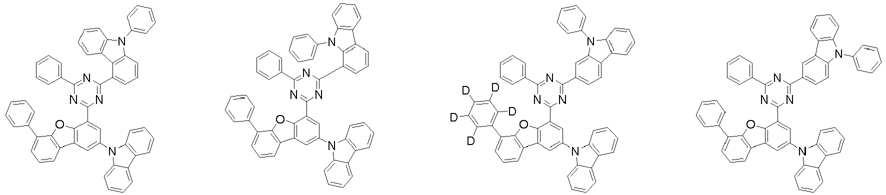

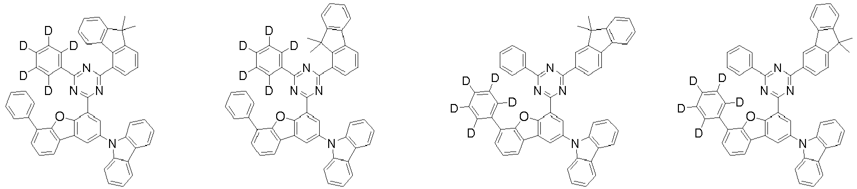

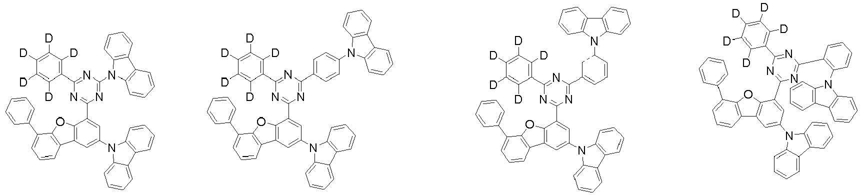

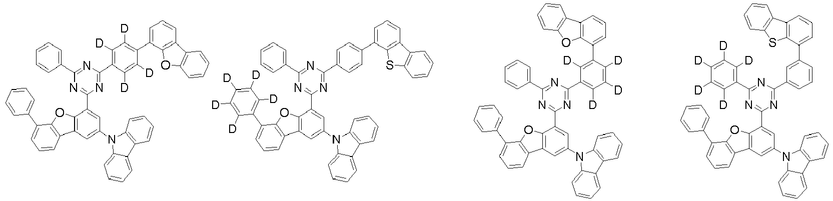

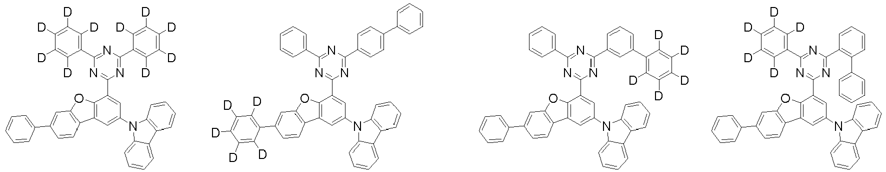

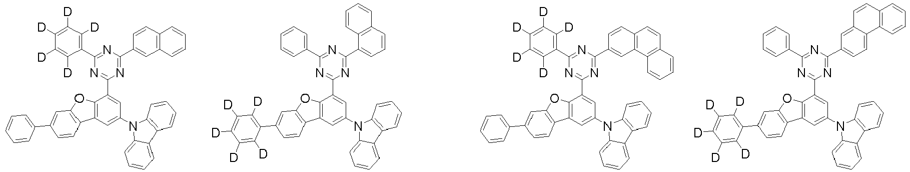

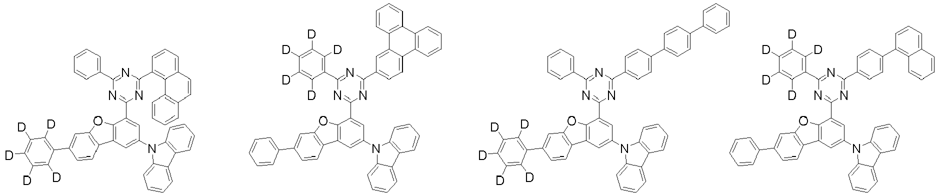

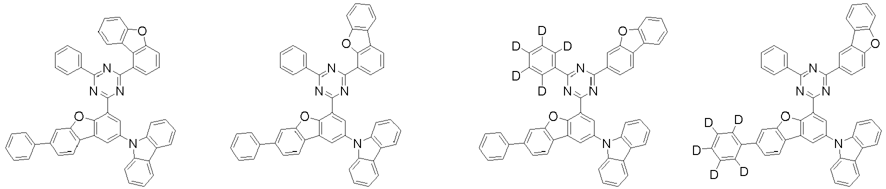

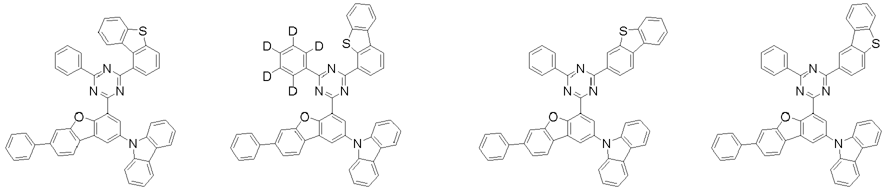

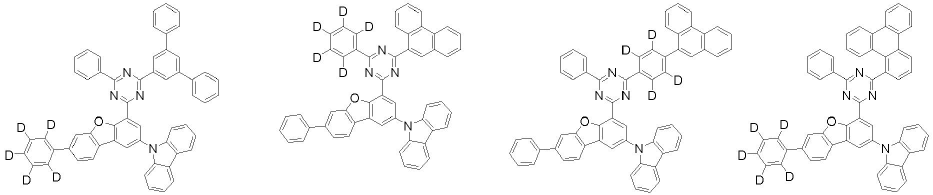

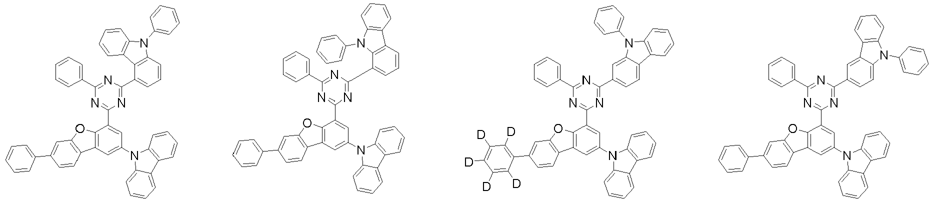

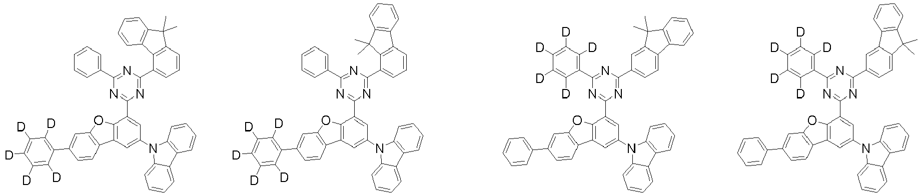

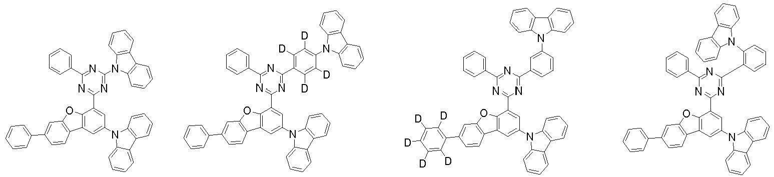

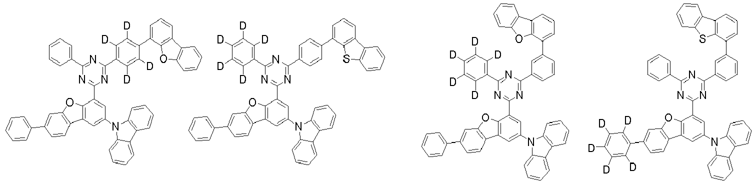

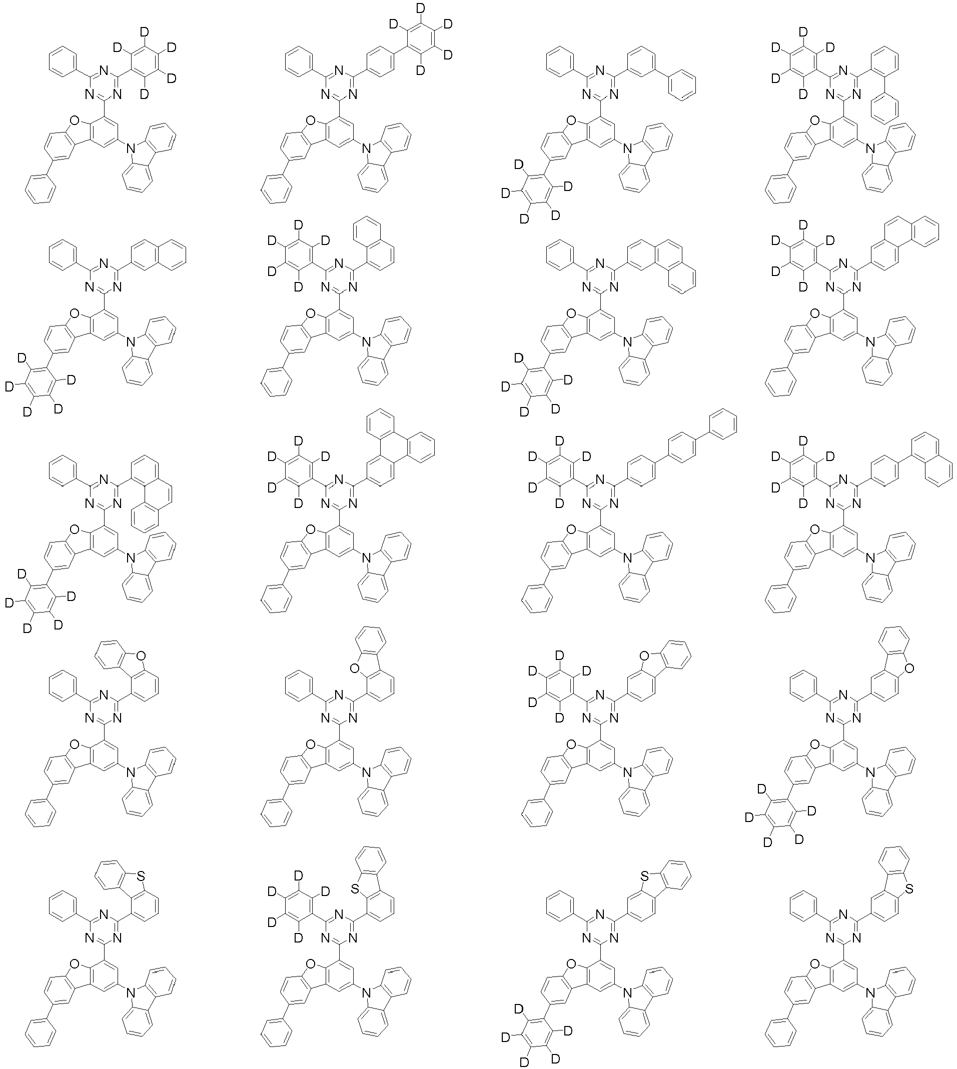

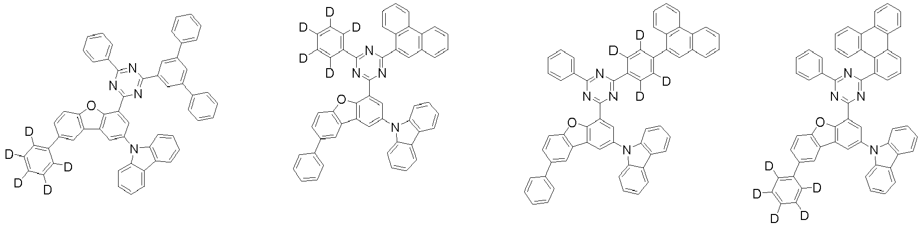

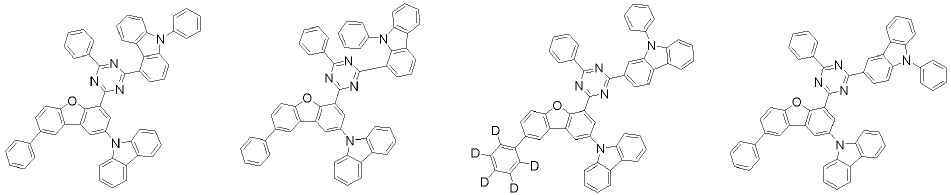

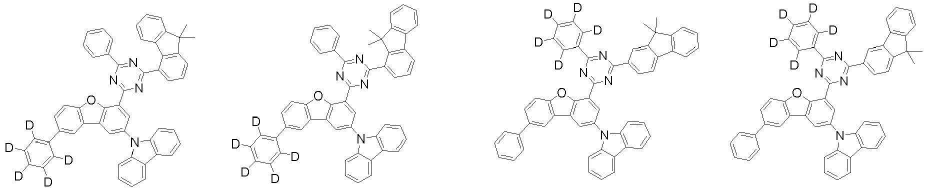

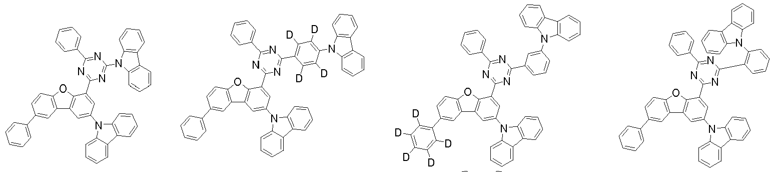

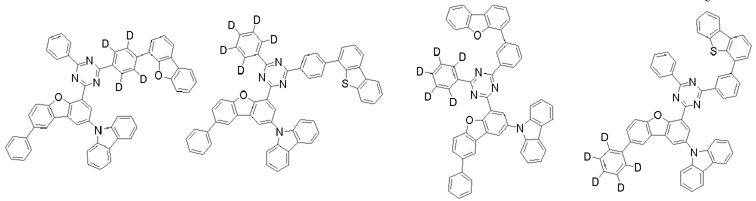

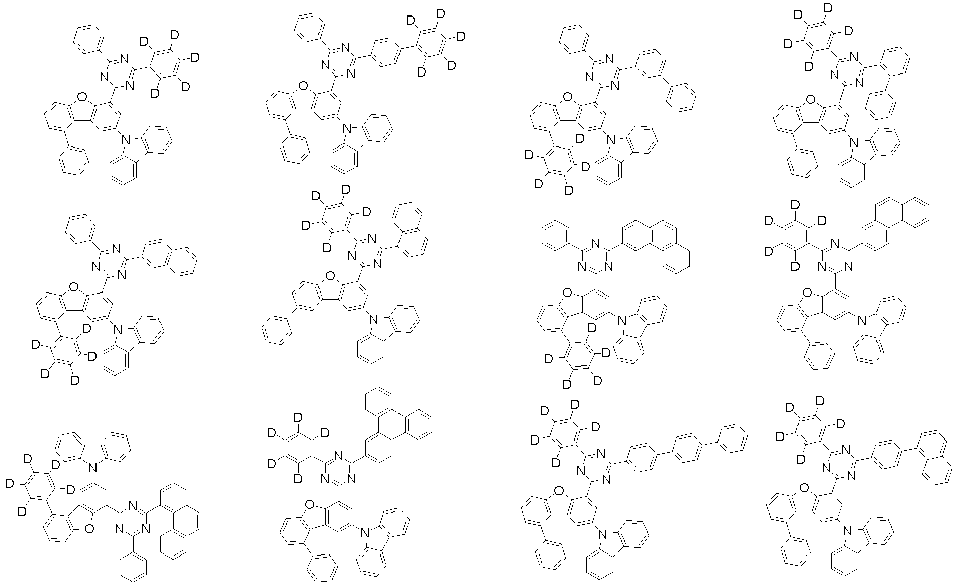

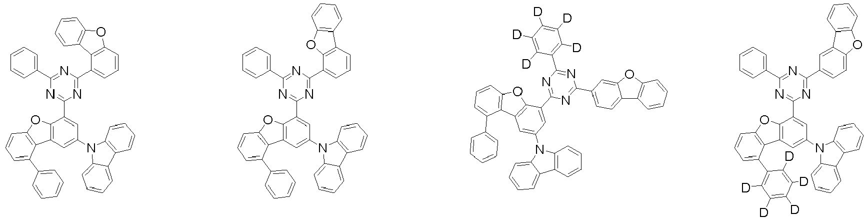

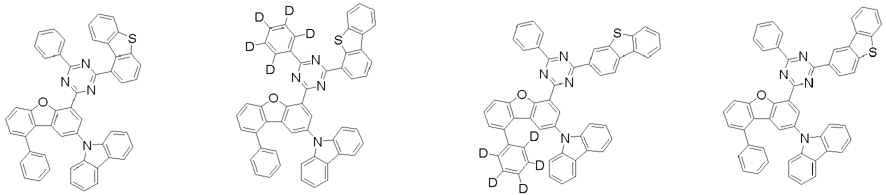

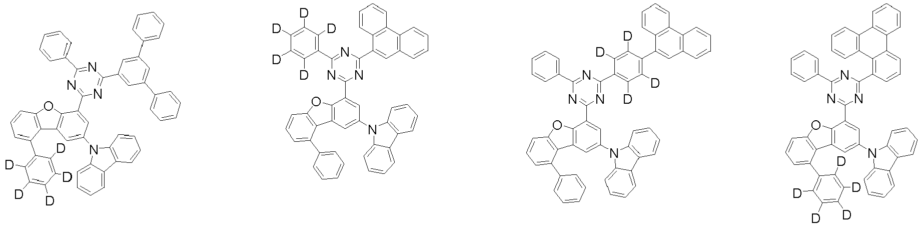

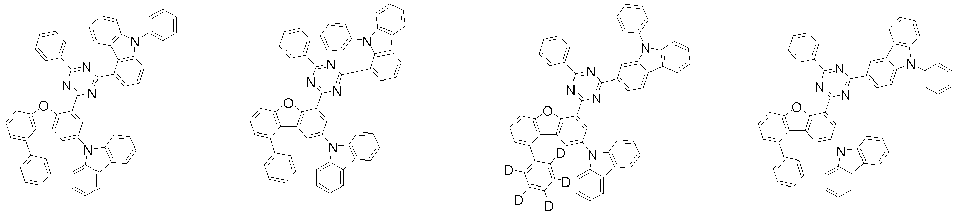

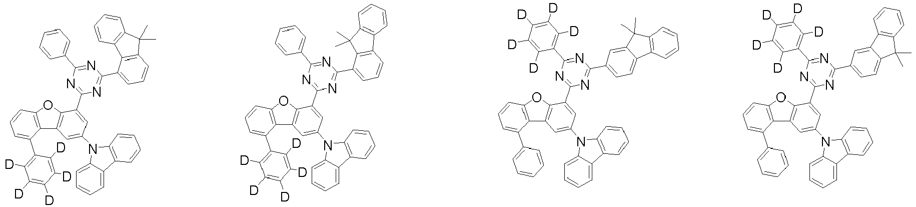

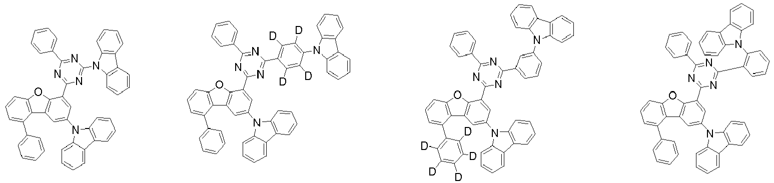

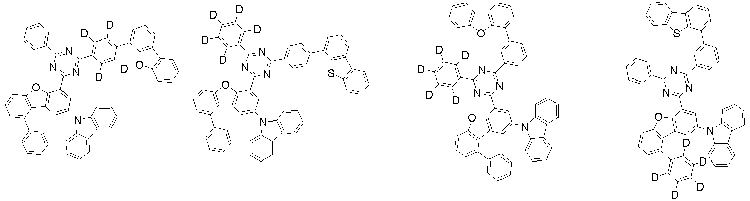

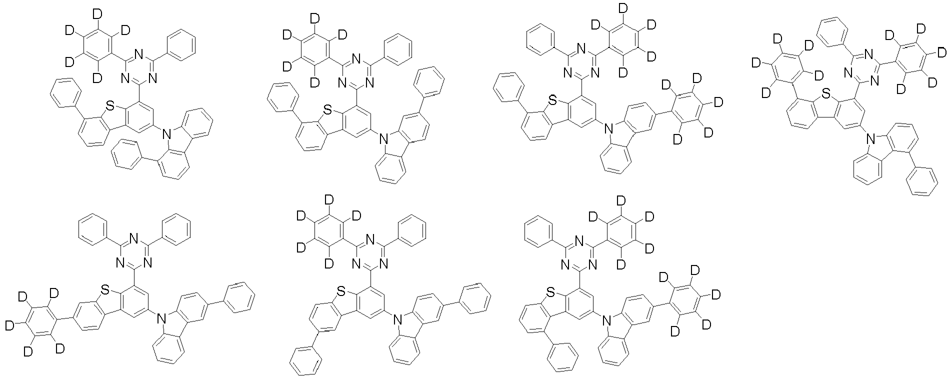

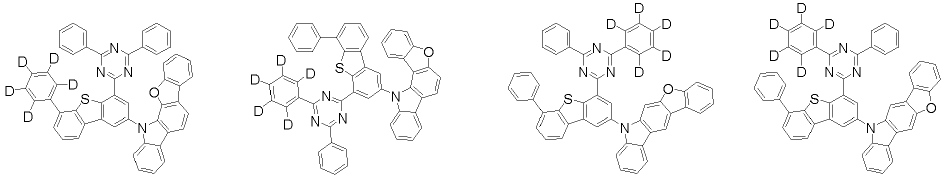

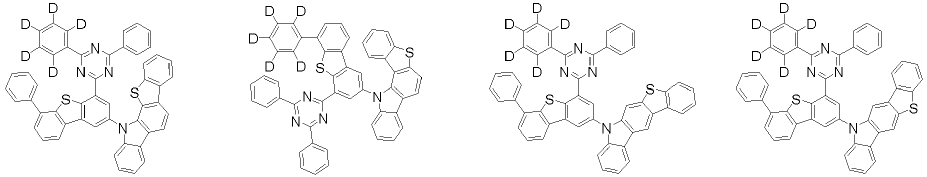

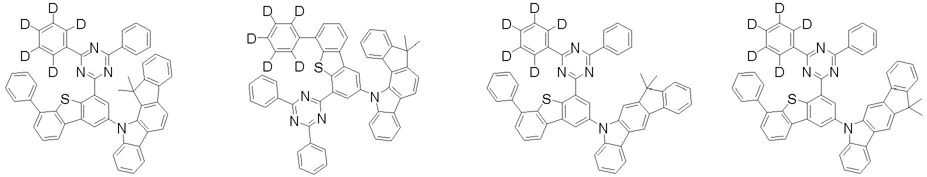

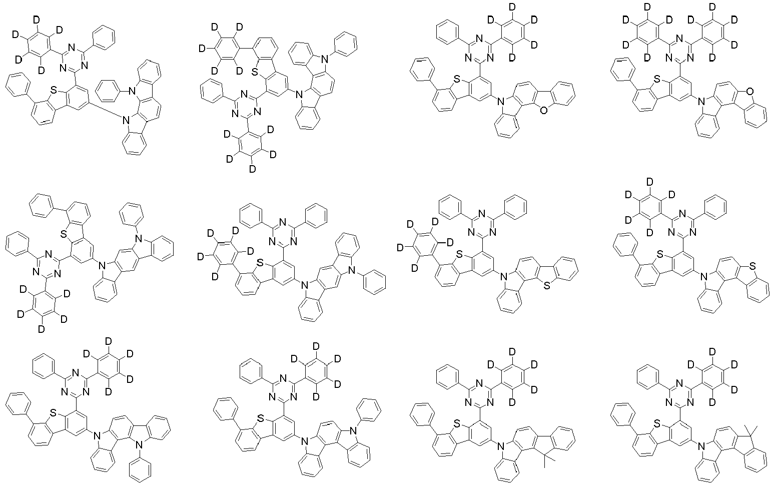

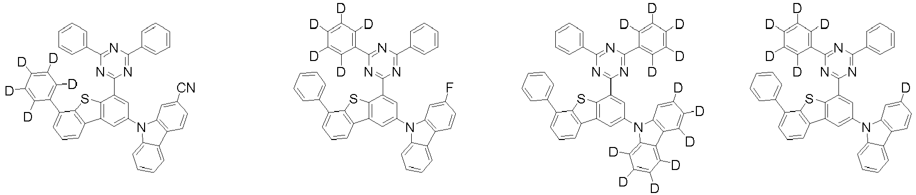

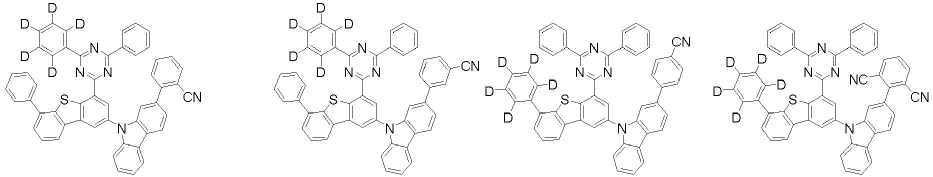

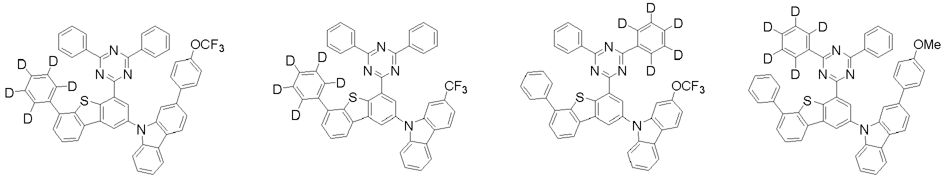

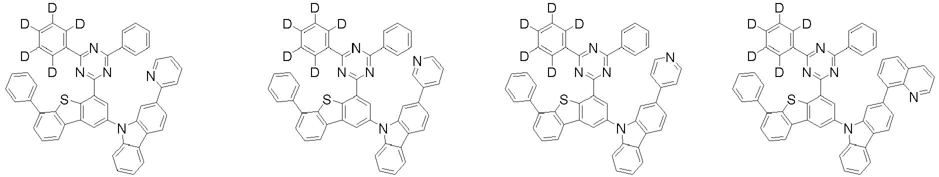

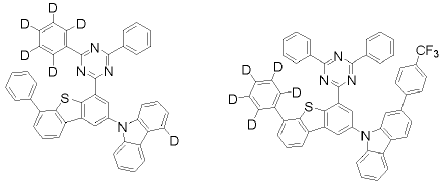

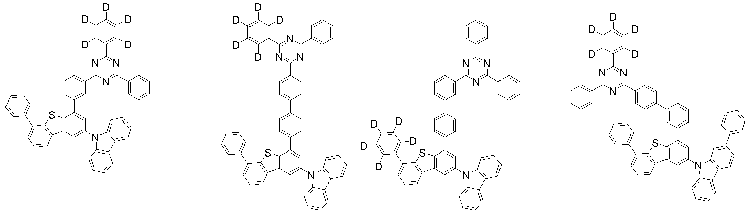

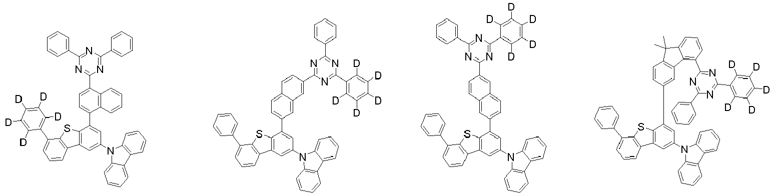

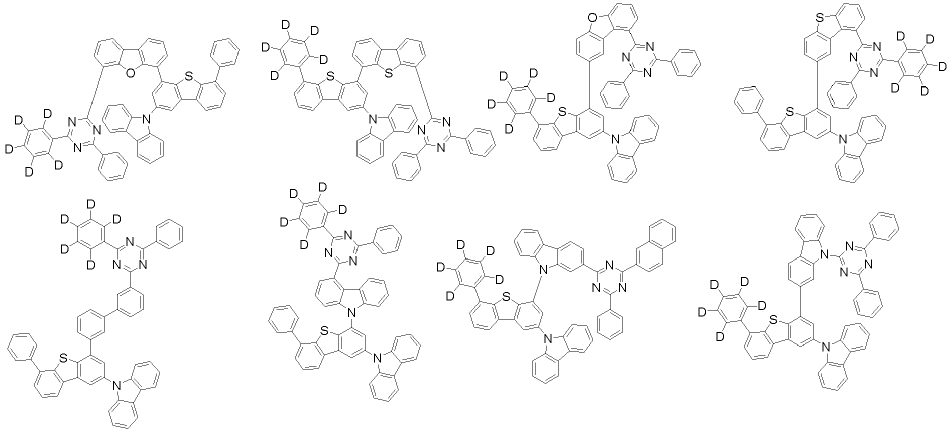

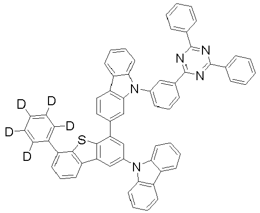

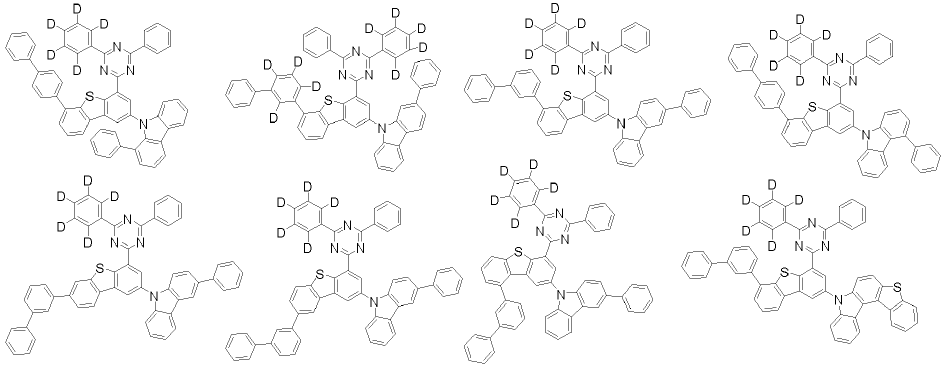

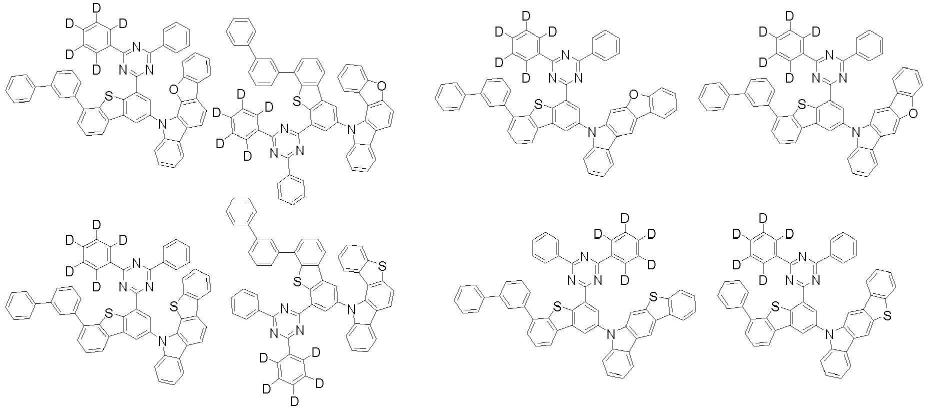

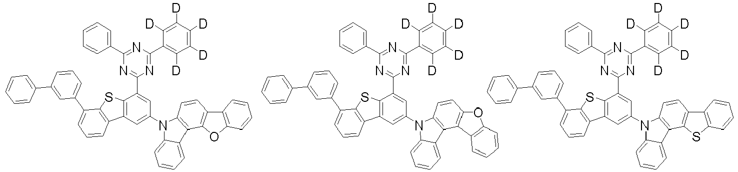

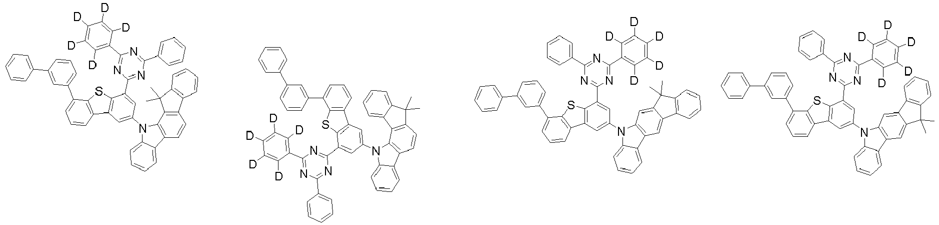

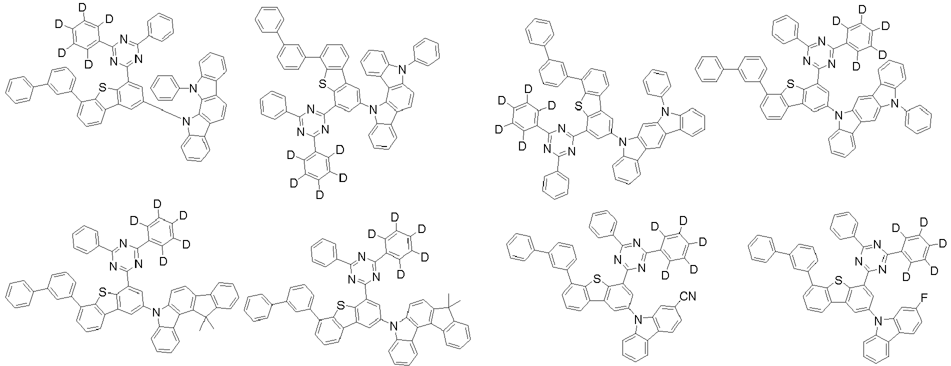

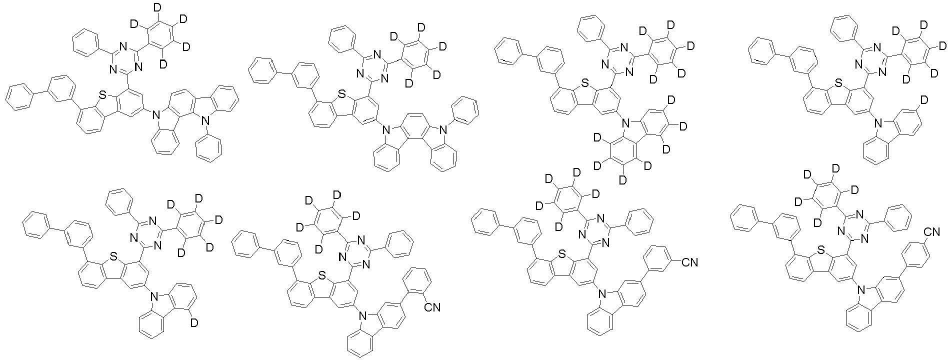

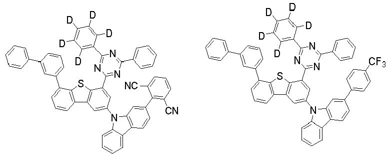

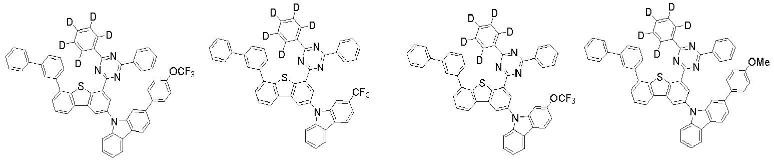

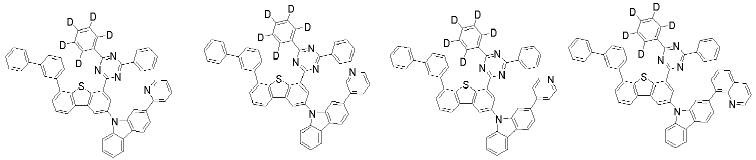

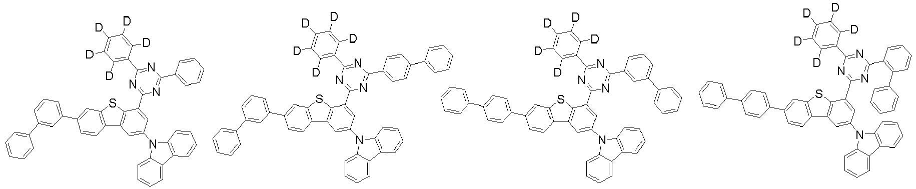

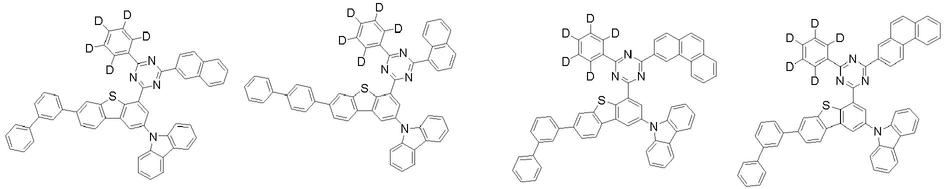

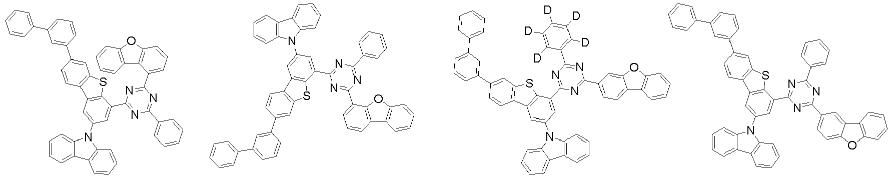

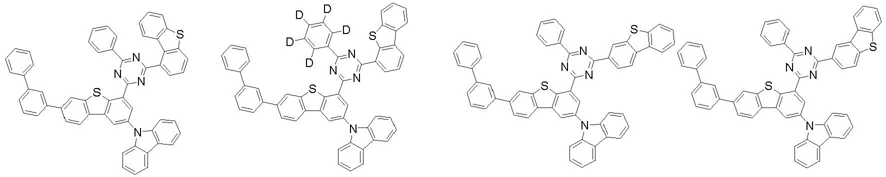

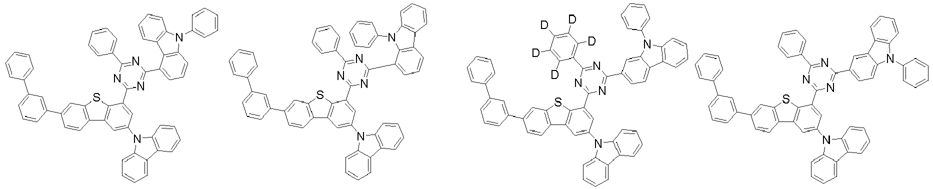

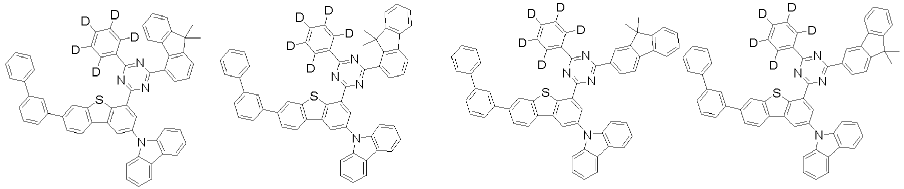

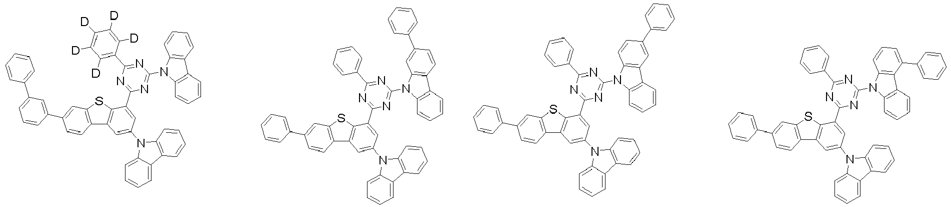

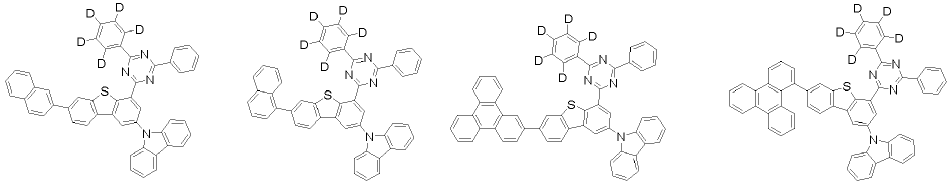

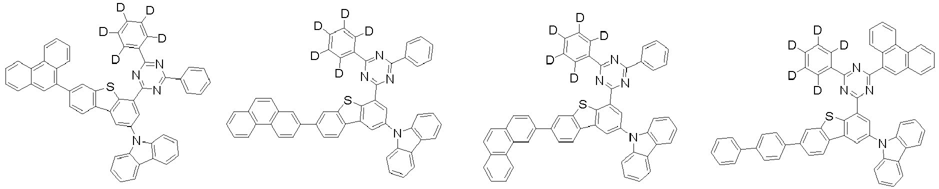

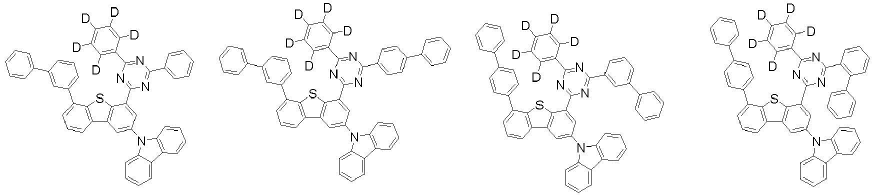

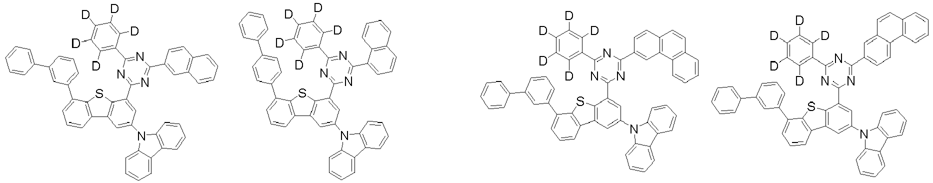

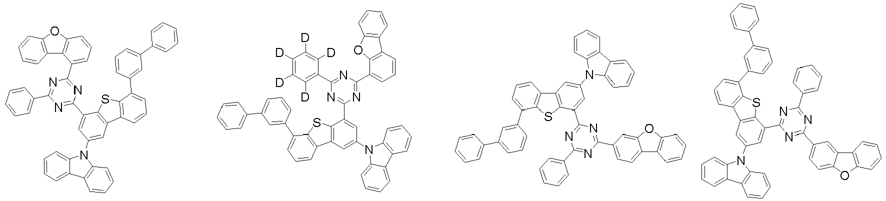

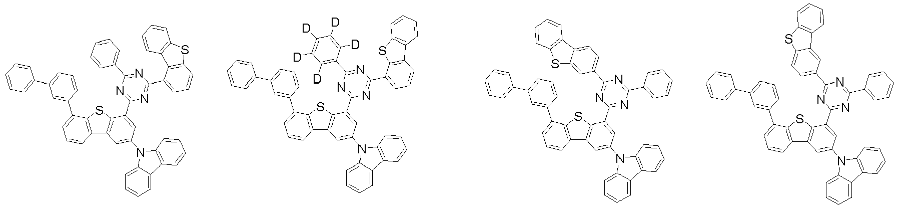

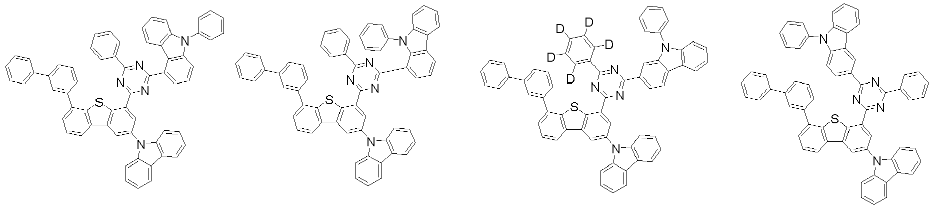

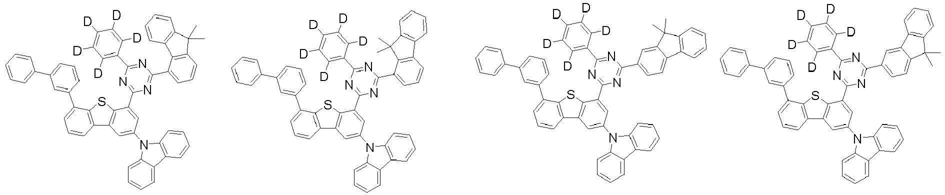

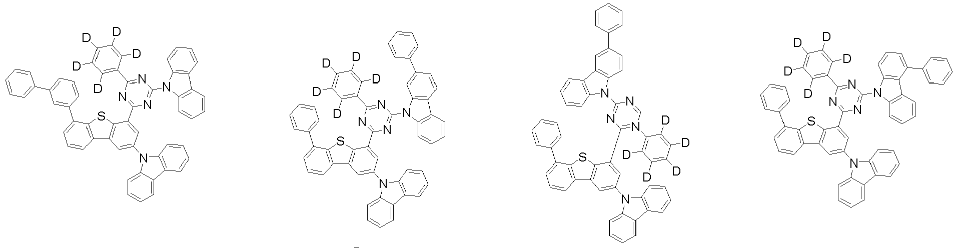

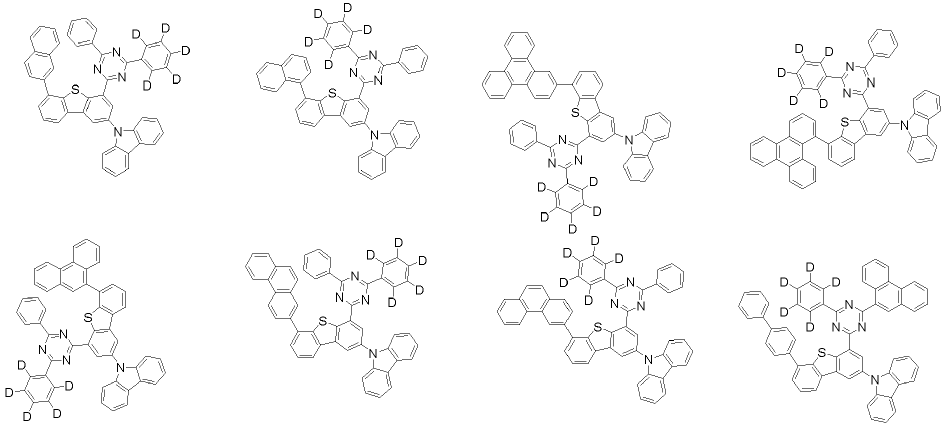

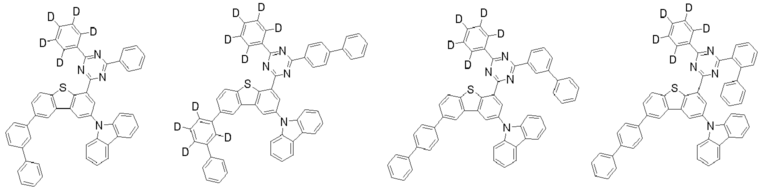

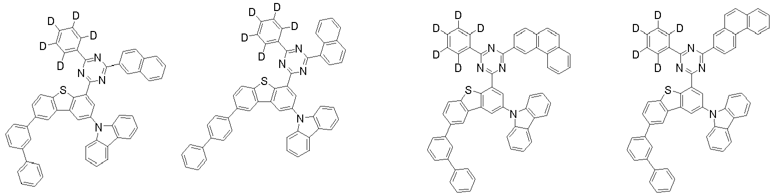

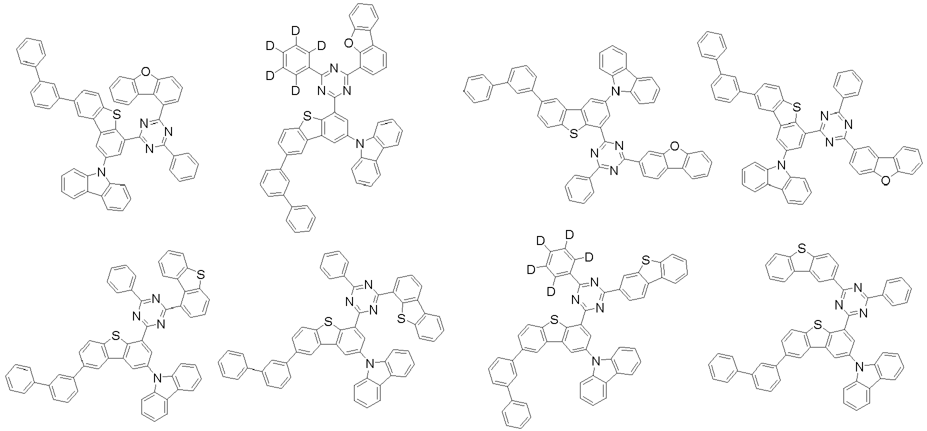

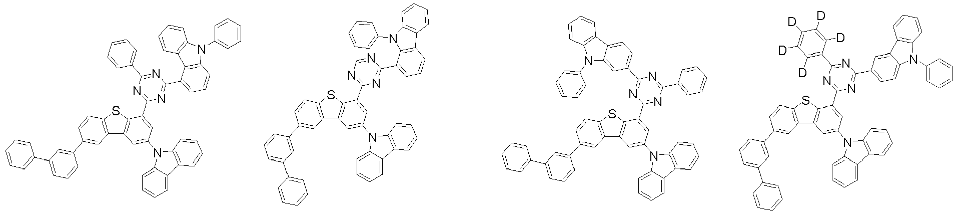

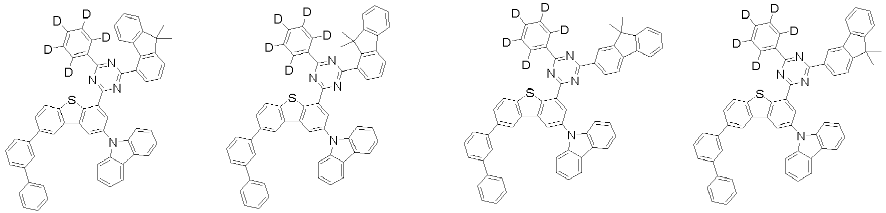

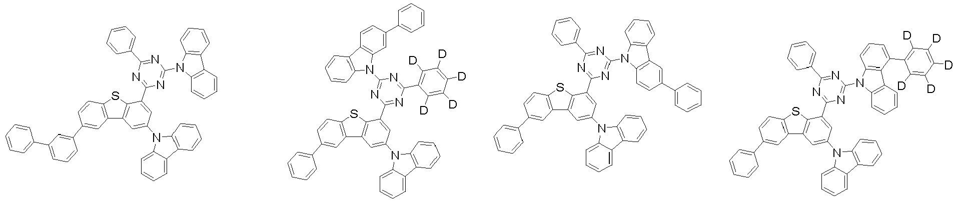

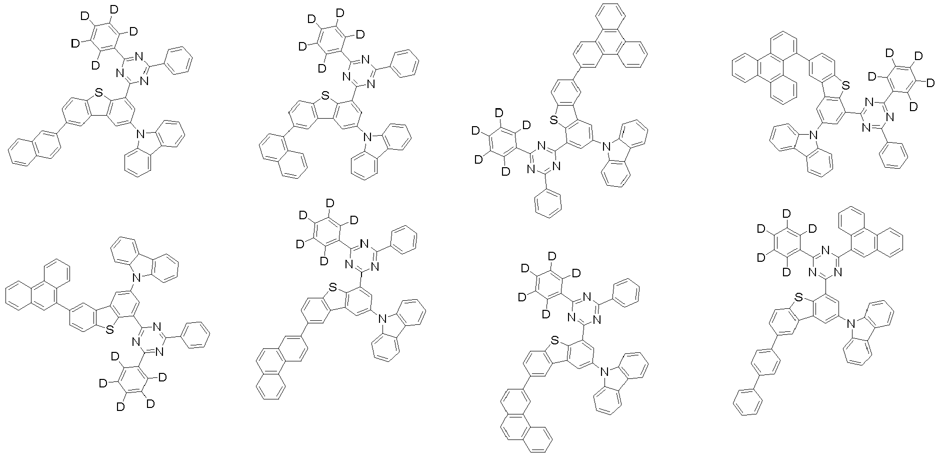

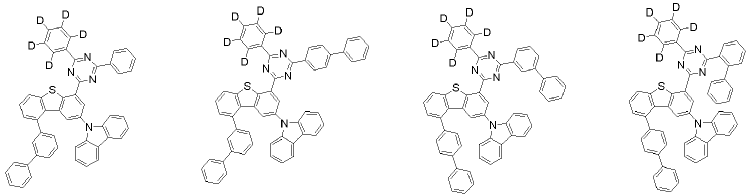

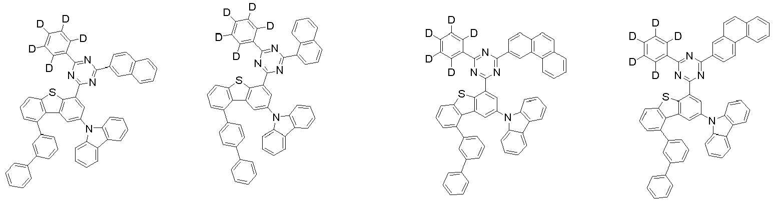

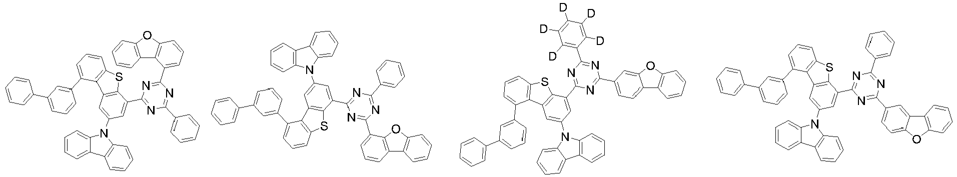

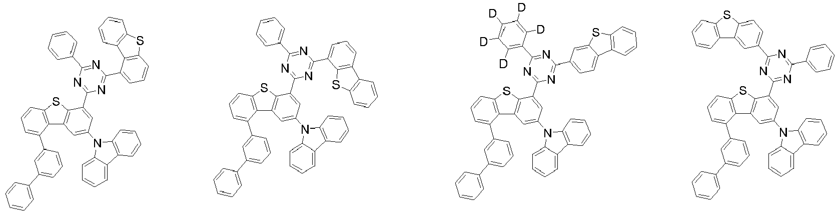

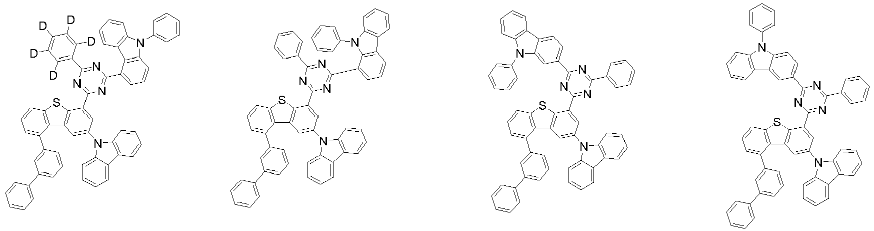

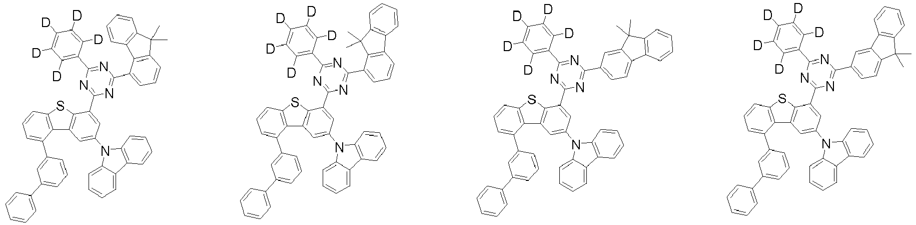

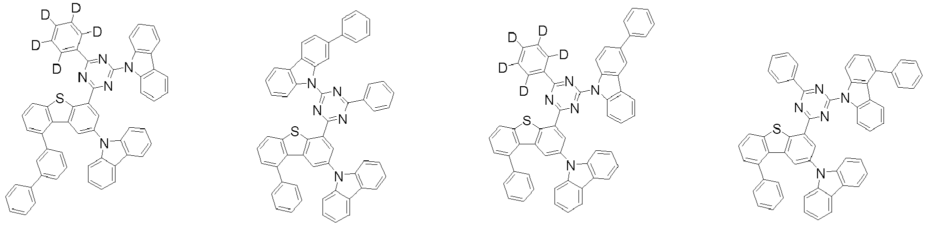

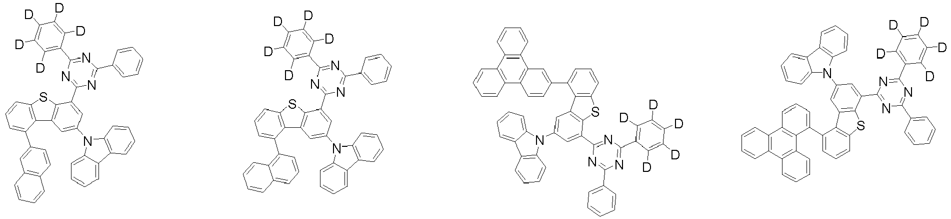

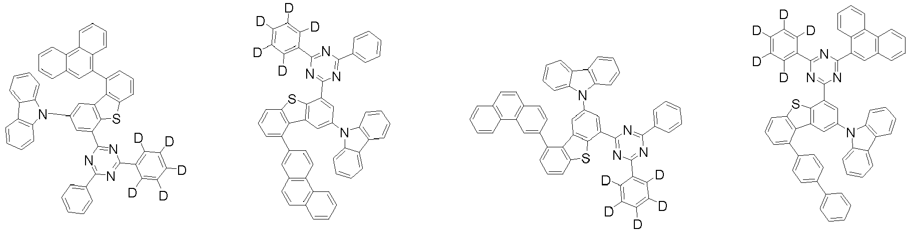

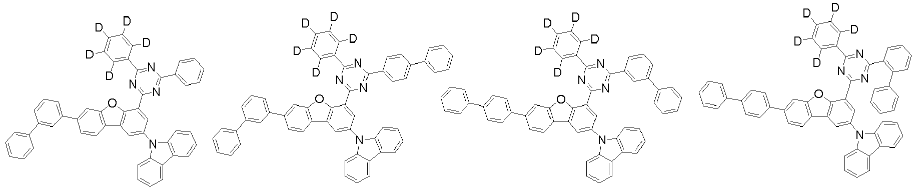

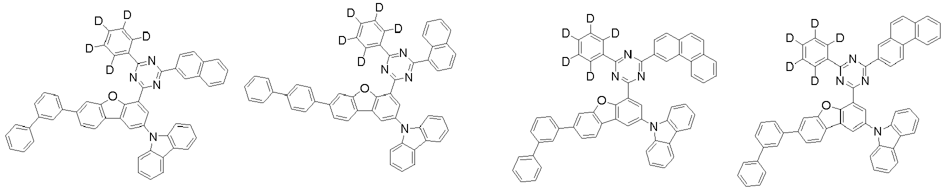

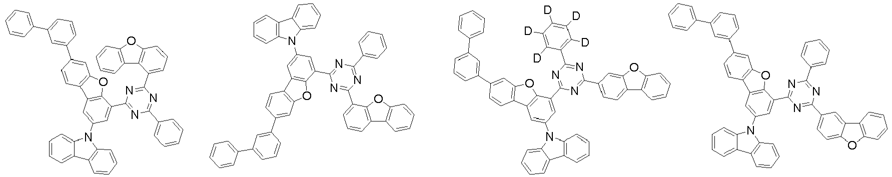

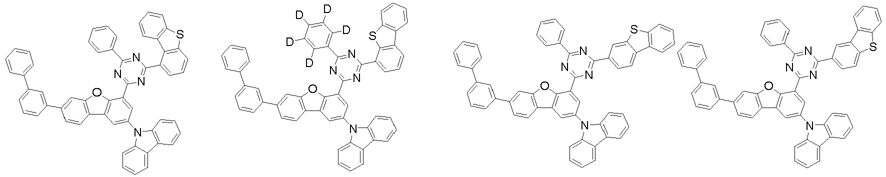

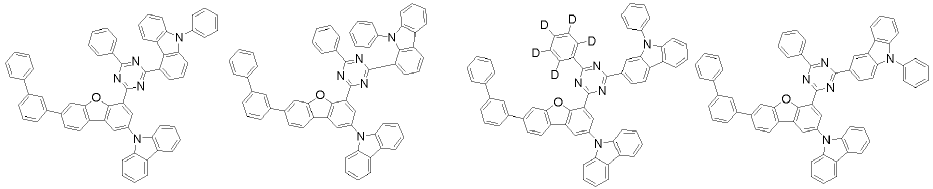

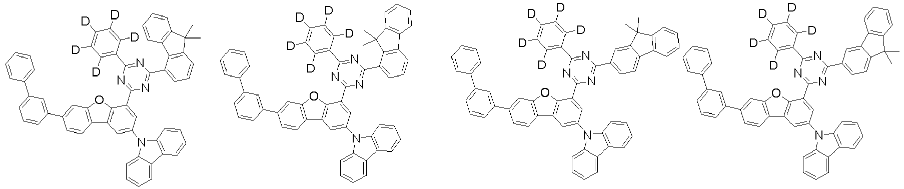

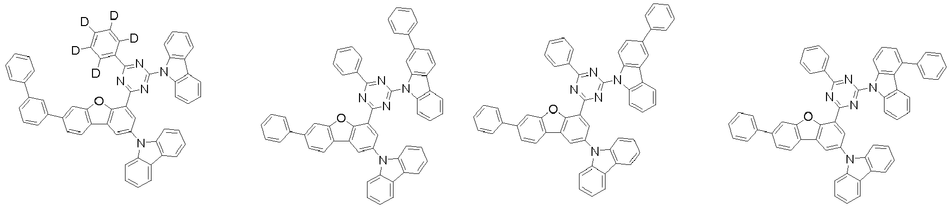

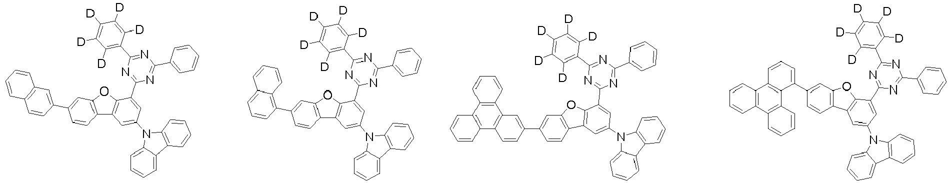

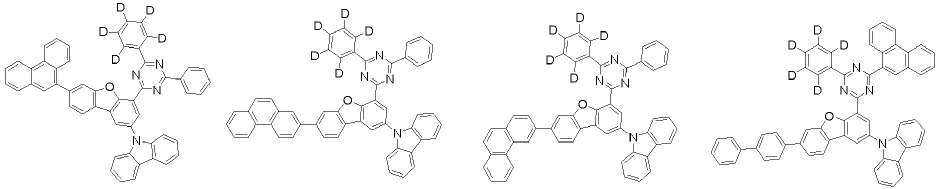

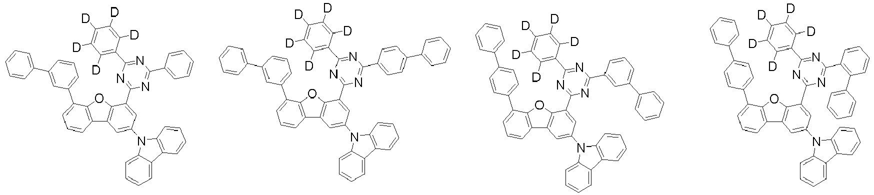

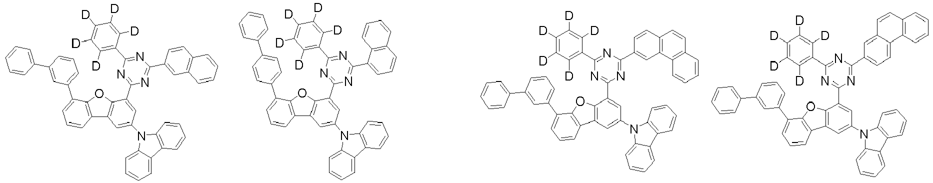

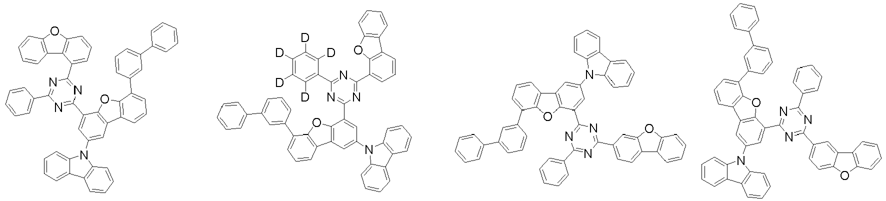

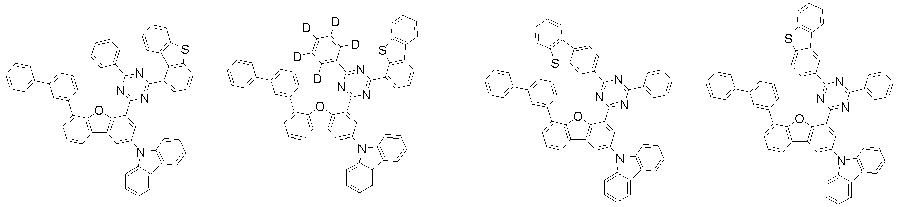

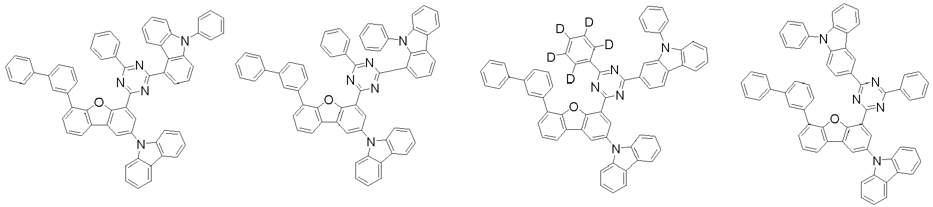

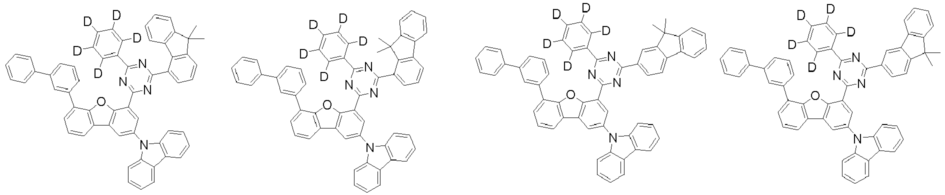

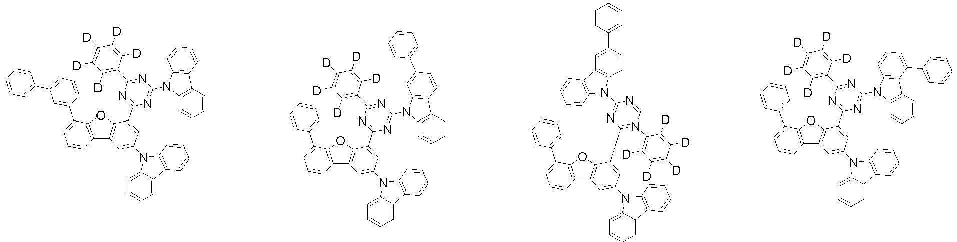

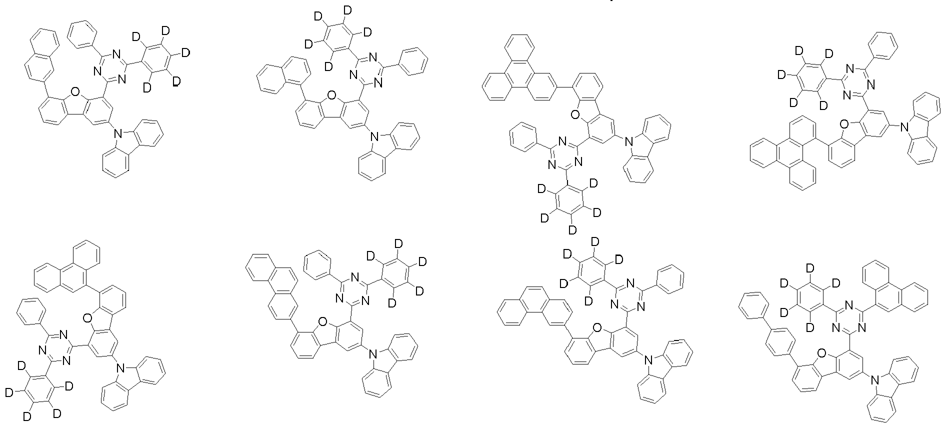

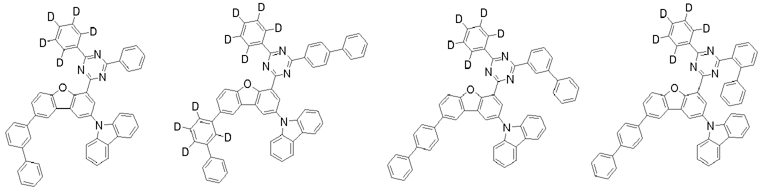

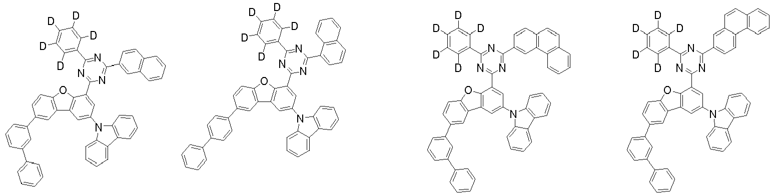

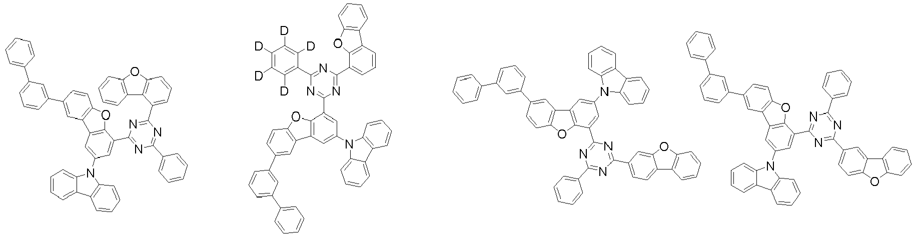

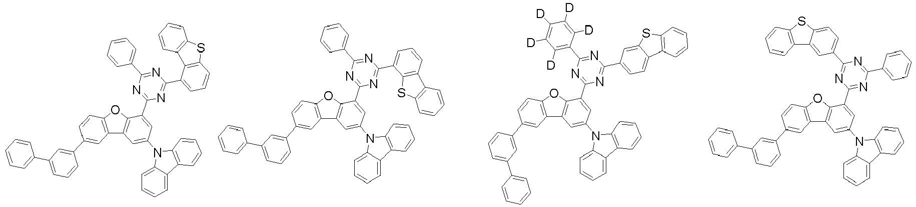

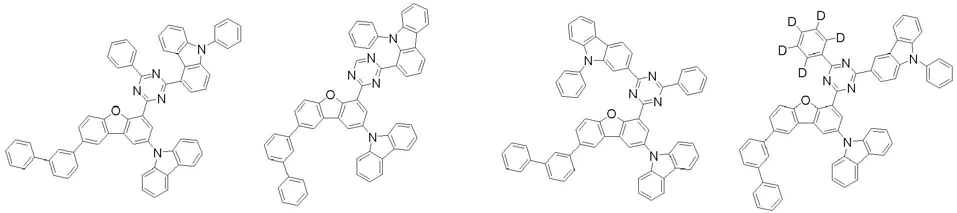

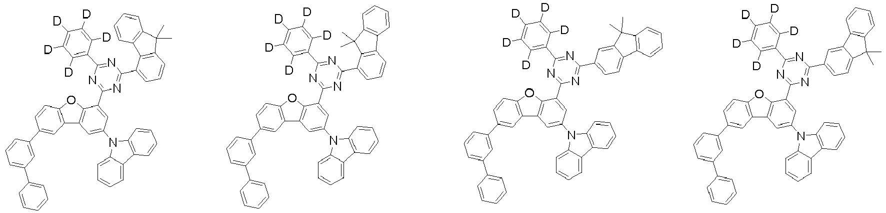

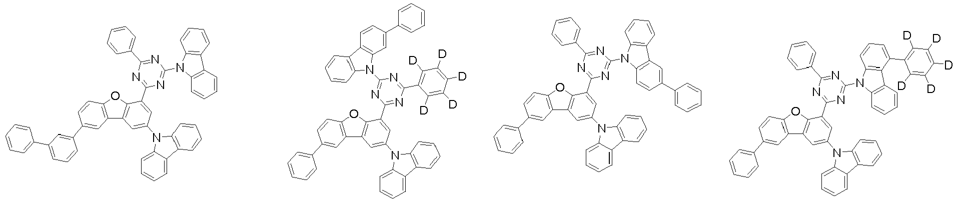

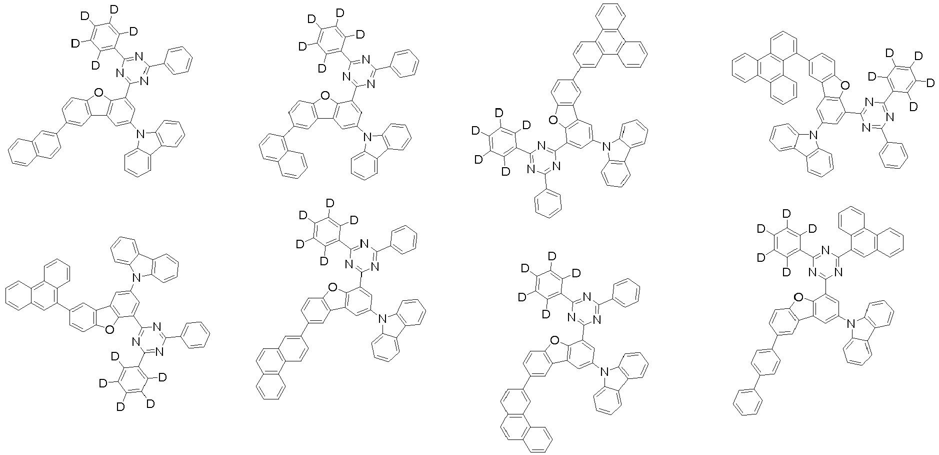

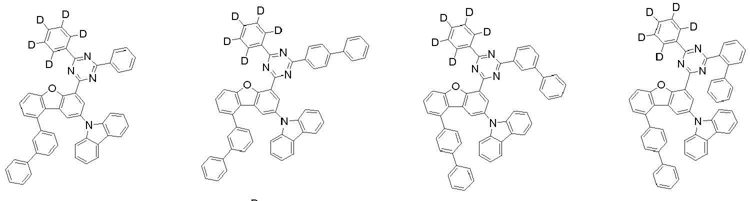

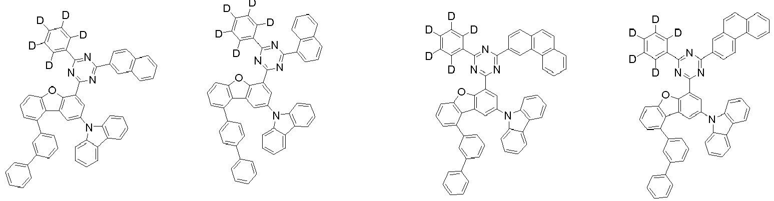

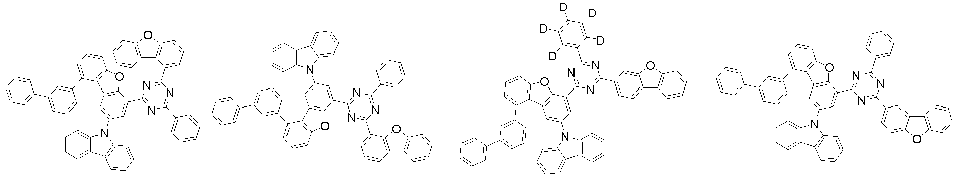

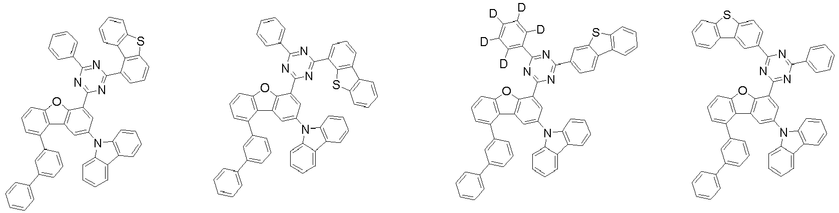

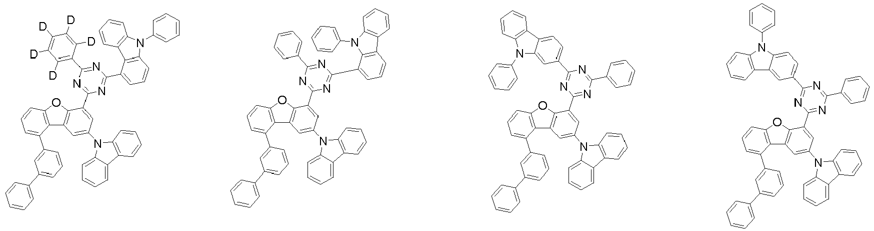

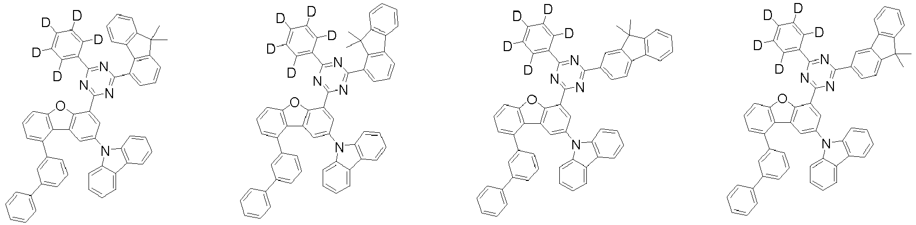

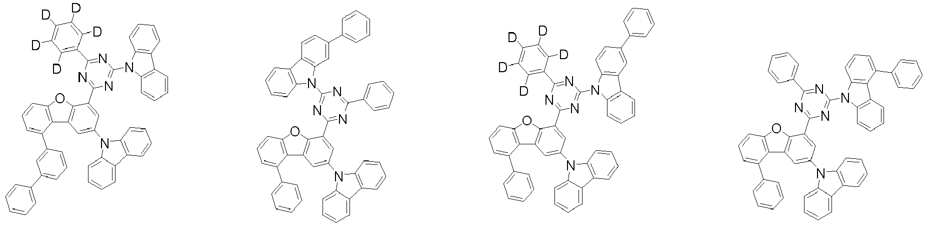

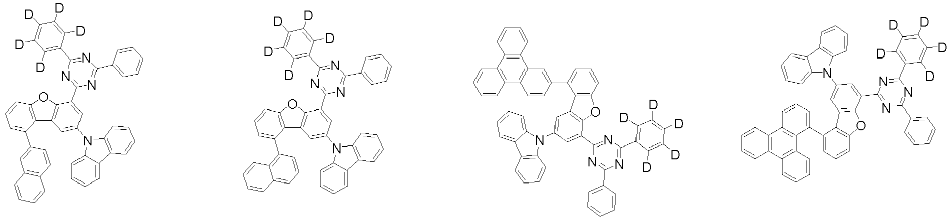

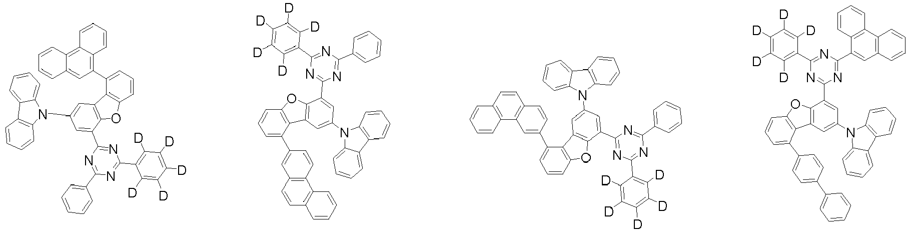

8. An organic light emitting device including: a first electrode; a second electrode that is provided opposite to the first electrode; and one or more organic material layers that are provided between the first electrode and the second electrode, wherein one or more layers of the organic material layers include the compound of claim 1.

## Patentansprüche

1. Verbindung, dargestellt durch die folgende Chemische Formel 1: 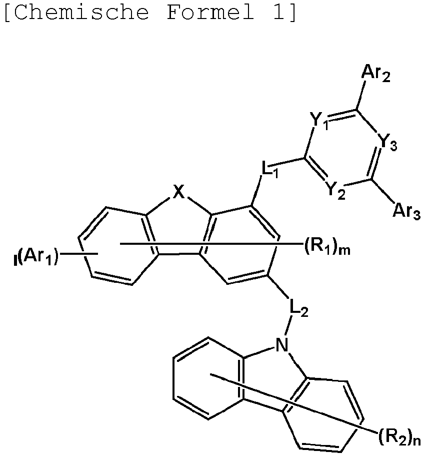 worin in der chemischen Formel 1
X O oder S ist,
Y₁, Y₂ und Y₃ jeweils unabhängig N sind,
L₁ und L₂ jeweils unabhängig eine direkte Bindung oder irgendeines sind, das ausgewählt ist aus der Gruppe bestehend aus: 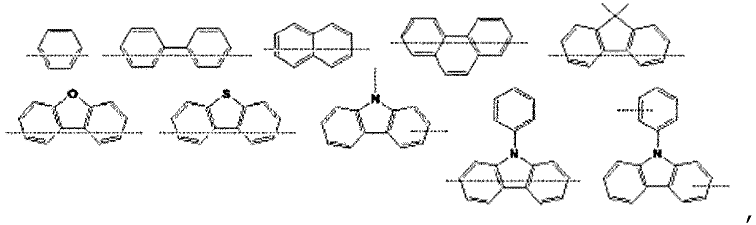
Ar₁ substituiertes oder unsubstituiertes C₆₋₆₀-Aryl ist;
Ar₂ und Ar₃ jeweils unabhängig ein substituiertes oder unsubstituiertes C₆₋₆₀-Aryl; oder ein substituiertes oder unsubstituiertes C₅₋₆₀-Heteroaryl, enthaltend zumindest ein Heteroatom, das ausgewählt ist aus der Gruppe bestehend aus N, O und S, sind,
mit der Maßgabe, dass, wenn alle von Ar₁, Ar₂ und Ar₃ ein substituiertes oder unsubstituiertes C₆₋₆₀-Aryl sind, irgendeines von Ar₁, Ar₂ und Ar₃ mit 4 oder mehr Deuteriumatomen substituiert ist,
jedes R₁ unabhängig Wasserstoff; Deuterium; Halogen; oder ein substituiertes oder unsubstituiertes C₁₋₆₀-Alkyl ist,
jedes R₂ unabhängig Wasserstoff, Deuterium; Halogen; Cyano; ein substituiertes oder unsubstituiertes C₁₋₆₀-Alkyl; ein substituiertes oder unsubstituiertes C₁₋₆₀-Alkoxy; ein substituiertes oder unsubstituiertes C₃₋₆₀-Cycloalkyl; ein substituiertes oder unsubstituiertes C₁₋₆₀-Haloalkyl; ein substituiertes oder unsubstituiertes C₁₋₆₀-Haloalkoxy; ein substituiertes oder unsubstituiertes C₆₋₆₀-Arylamin; ein substituiertes oder unsubstituiertes C₁₋₆₀-Alkylamin; ein C₁₋₆₀-Trifluoralkyl; ein C₁₋₆₀-Trifluoralkoxy; ein substituiertes oder unsubstituiertes C₆₋₆₀-Aryl; oder ein substituiertes oder unsubstituiertes C₅₋₆₀-Heteroaryl, enthaltend zumindest eins von N, O und S, welches an das Kohlenstoffatom des Carbazols binden kann, um einen kondensierten Ring zu bilden, ist
m eine ganze Zahl von 0 bis 3 ist,
n eine ganze Zahl von 0 bis 6 ist, und
1 1 oder 2 ist.

2. Verbindung gemäß Anspruch 1,
worin die durch die folgende Chemische Formel 1 dargestellte Verbindung irgendeine ist, die ausgewählt ist aus der Gruppe bestehend aus Verbindungen, die durch die folgenden Chemischen Formeln 2 bis 8 dargestellt werden: 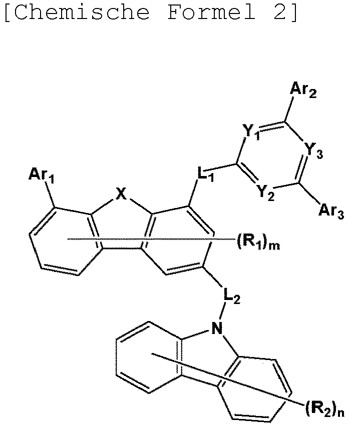 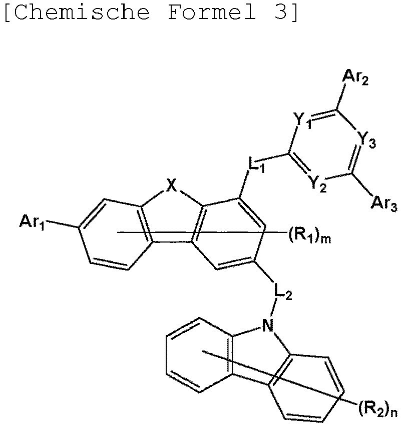 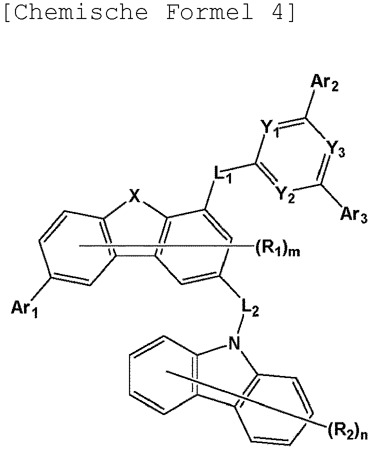 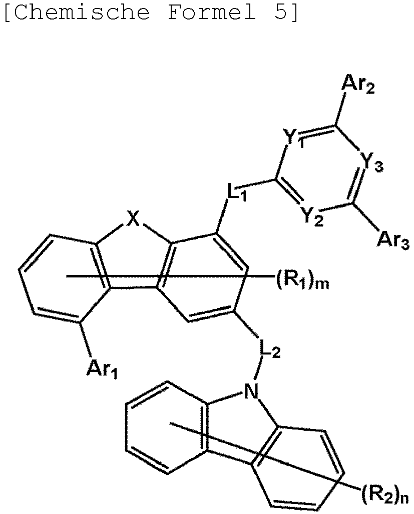 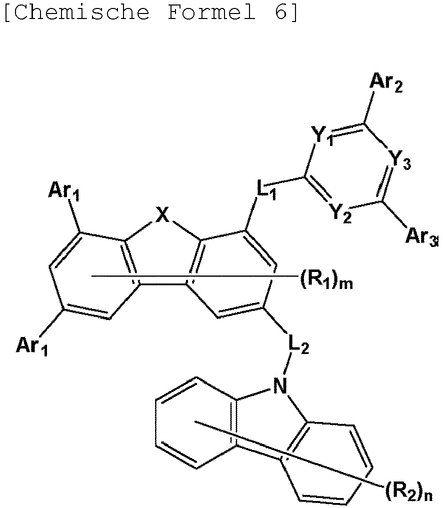 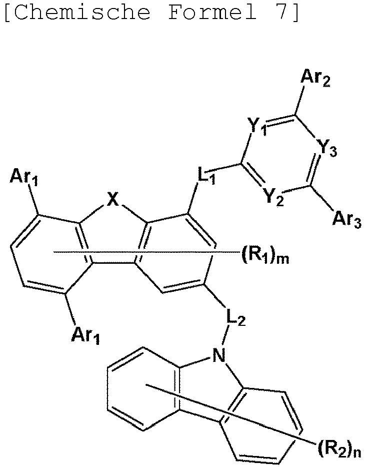 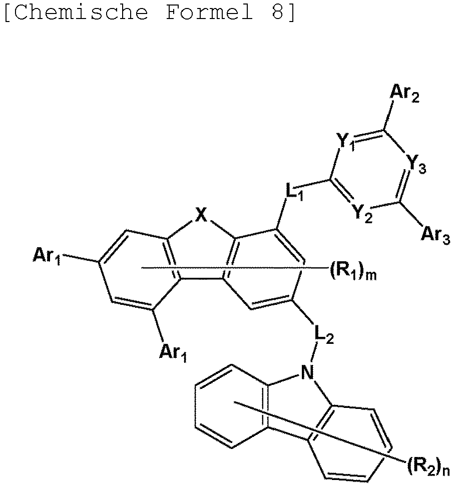 worin in den chemischen Formeln 2 bis 8
X, Y₁, Y₂, Y₃, L₁, L₂, Ar₁, Ar₂, Ar₃, R₁, R₂, m und n wie in Anspruch 1 definiert sind.

3. Verbindung gemäß Anspruch 1,
worin Ar₁ irgendeines ist, das ausgewählt ist aus der Gruppe bestehend aus: 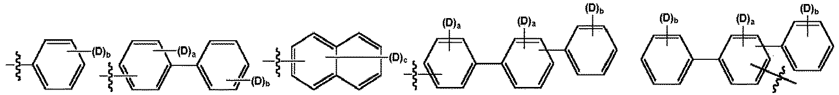 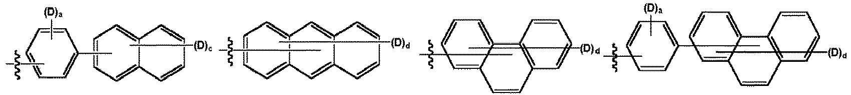 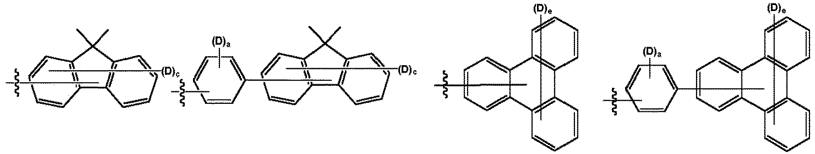 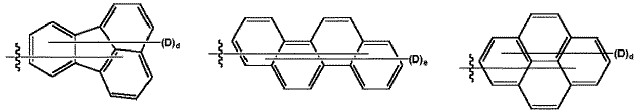 wobei in den vorstehenden Formeln
a eine ganze Zahl von 0 bis 4 ist,
b eine ganze Zahl von 0 bis 5 ist,
c eine ganze Zahl von 0 bis 7 ist,
d eine ganze Zahl von 0 bis 9 ist und
e eine ganze Zahl von 0 bis 11 ist.

4. Verbindung gemäß Anspruch 1,
worin Ar₂ und Ar₃ jeweils unabhängig eines sind, das ausgewählt ist aus der Gruppe bestehend aus: 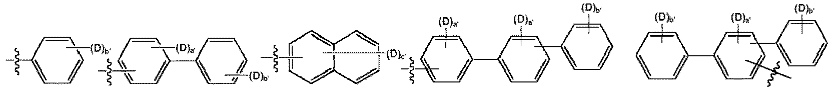 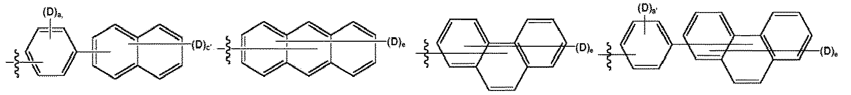 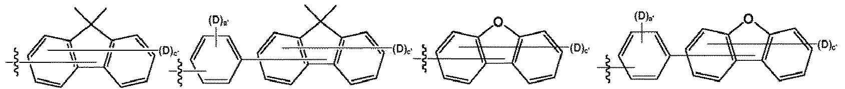 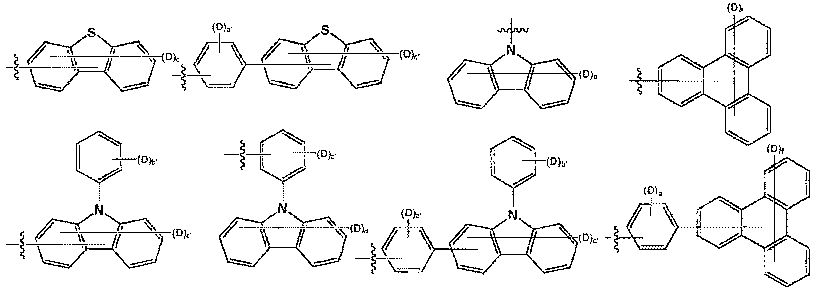 wobei in den vorstehenden Formeln
a' eine ganze Zahl von 0 bis 4 ist,
b' eine ganze Zahl von 0 bis 5 ist,
c' eine ganze Zahl von 0 bis 7 ist,
d' eine ganze Zahl von 0 bis 8 ist,
e' eine ganze Zahl von 0 bis 9 ist, und
f' eine ganze Zahl von 0 bis 11 ist.

5. Verbindung gemäß Anspruch 1,
worin jedes R₁ unabhängig Wasserstoff oder Deuterium ist.

6. Verbindung gemäß Anspruch 1,
worin jedes R₂ unabhängig Wasserstoff; Deuterium; Halogen; Cyano; Methoxy; Trifluormethyl; Trifluormethoxy; Phenyl; Pyridinyl; Isochinolinyl; oder irgendeines ist, das ausgewählt ist aus der Gruppe bestehend aus: 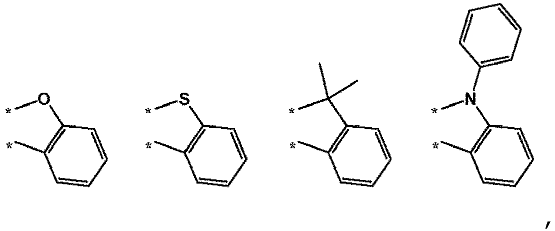 worin in den vorstehenden Formeln zwei * jeweils mit einem angrenzenden Kohlenstoffatom des Carbazols in der chemischen Formel 1 verbunden sind, und
das Phenyl substituiert oder unsubstituiert mit irgendeinem aus der Gruppe bestehend aus Halogen, Cyano, Methoxy, Trifluormethyl und Trifluormethoxy ist.

7. Verbindung gemäß Anspruch 1,
worin die durch die Chemische Formel 1 dargestellte Verbindung irgendeine ist, die ausgewählt ist aus der Gruppe bestehend aus:

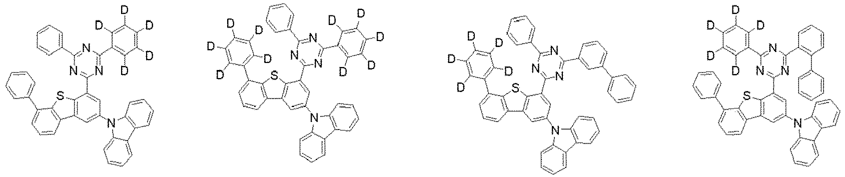

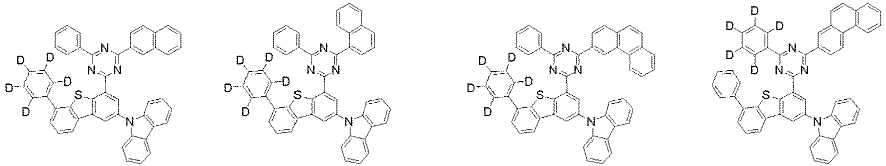

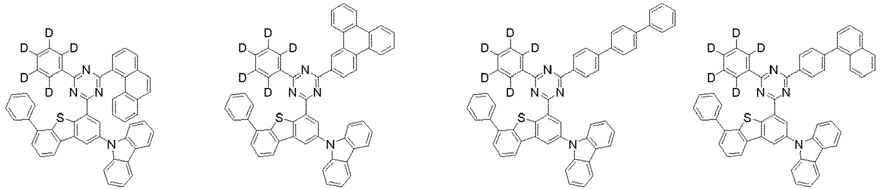

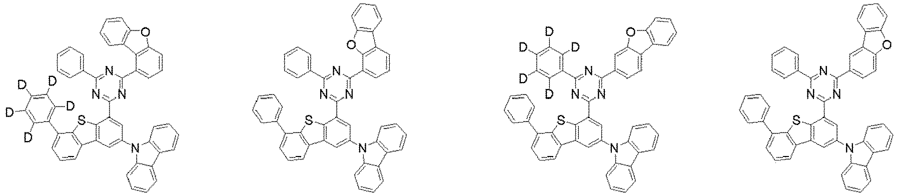

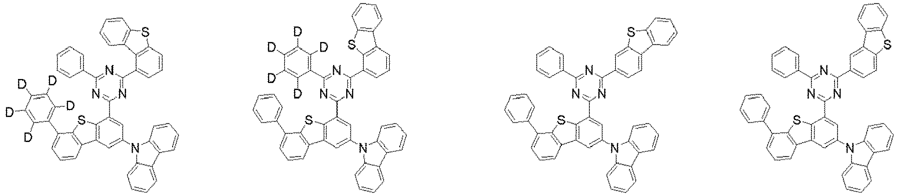

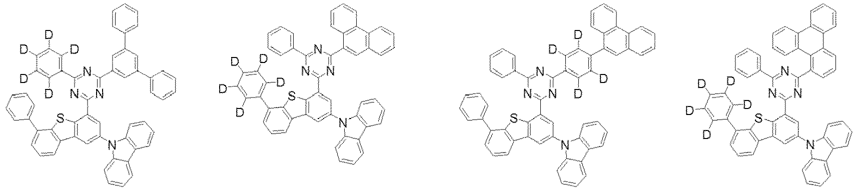

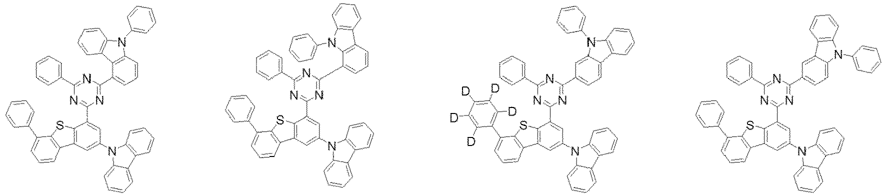

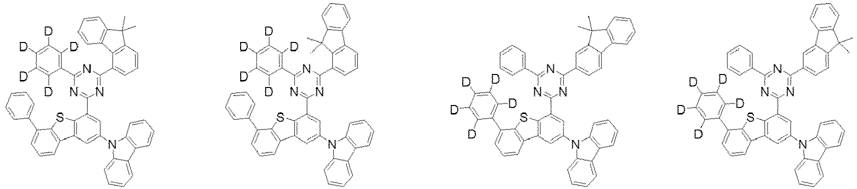

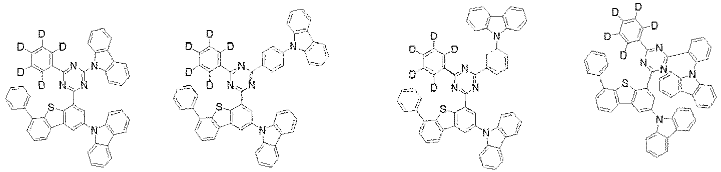

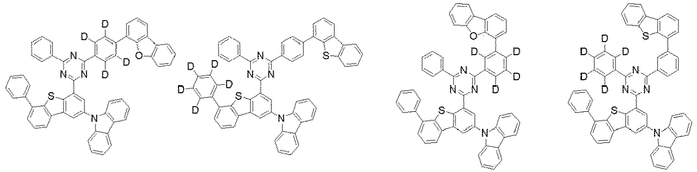

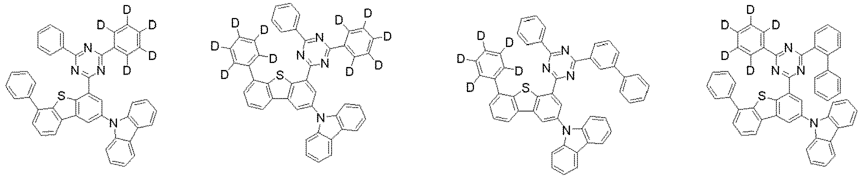

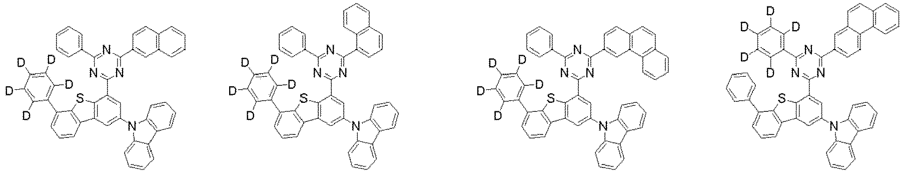

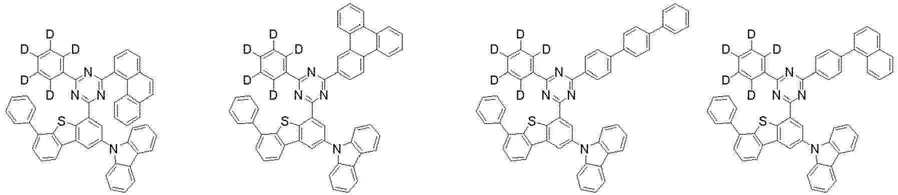

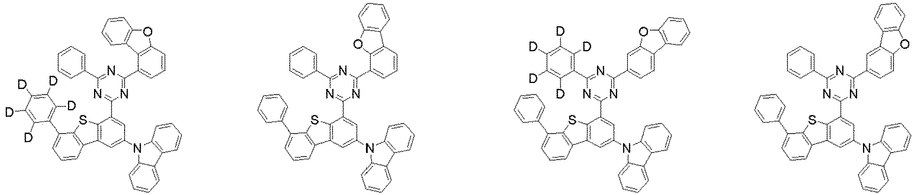

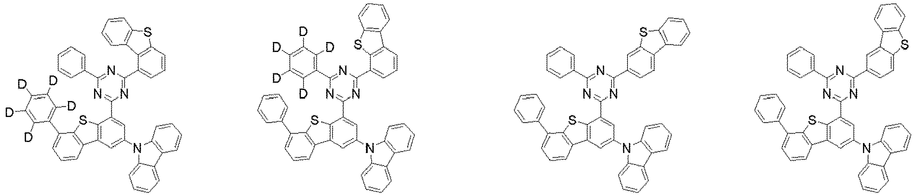

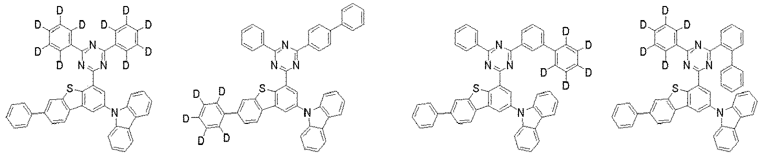

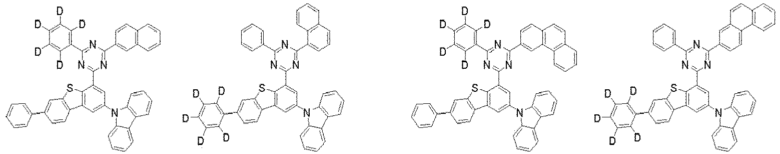

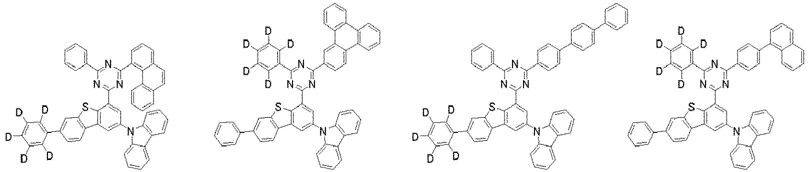

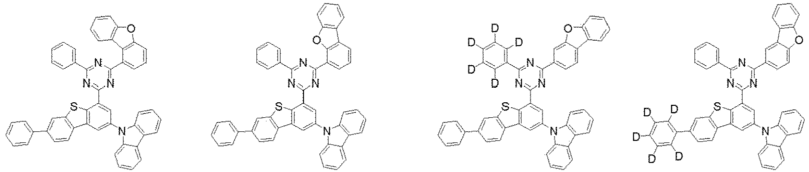

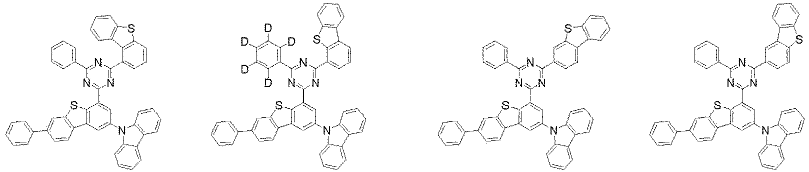

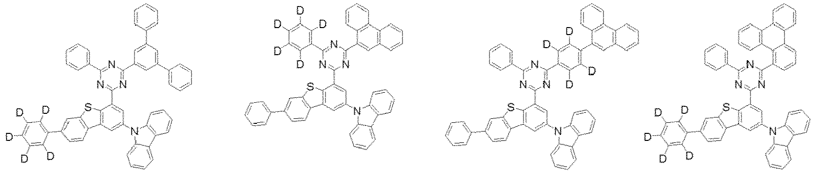

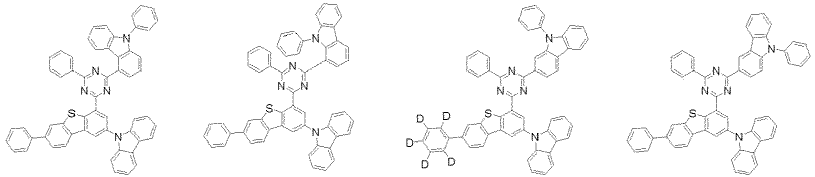

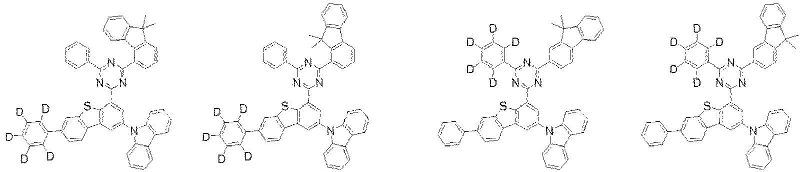

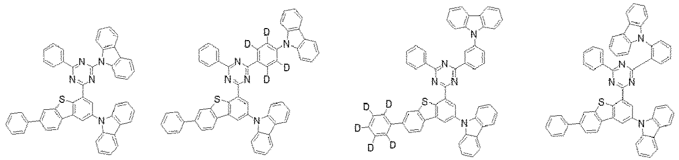

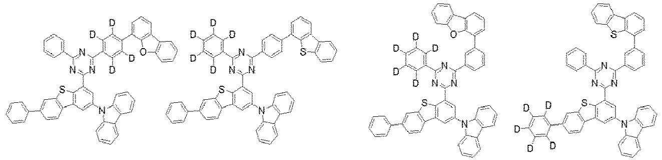

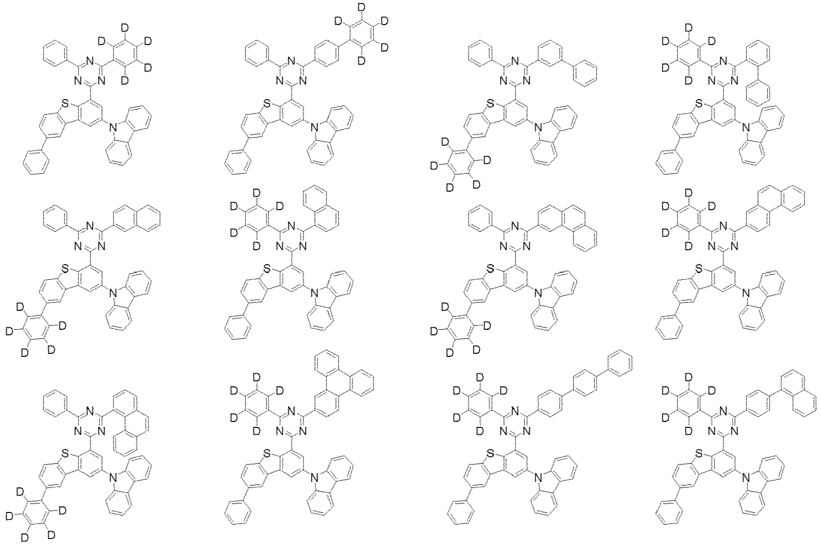

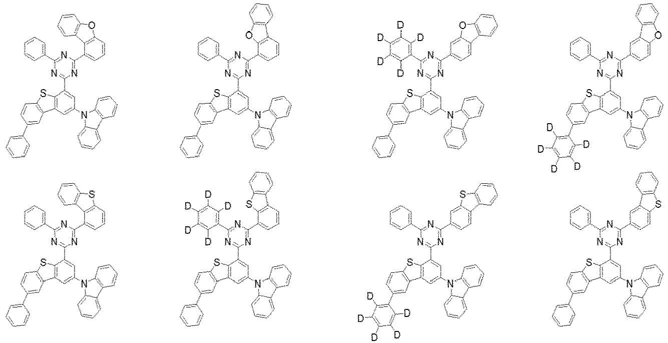

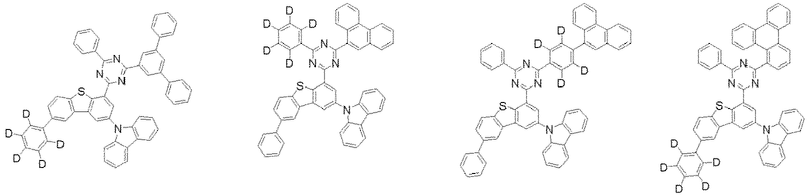

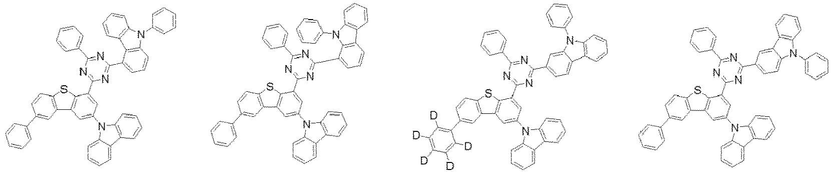

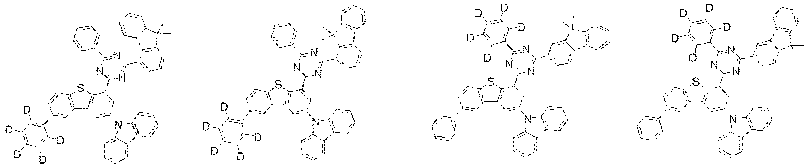

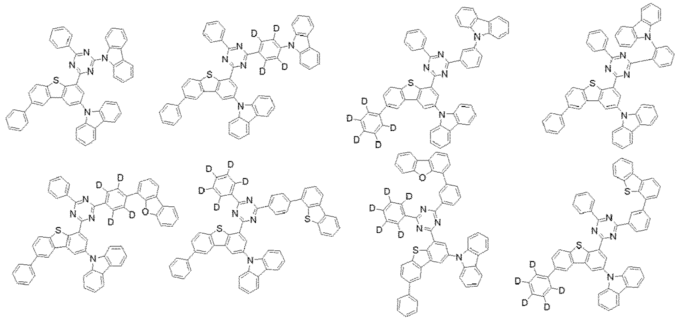

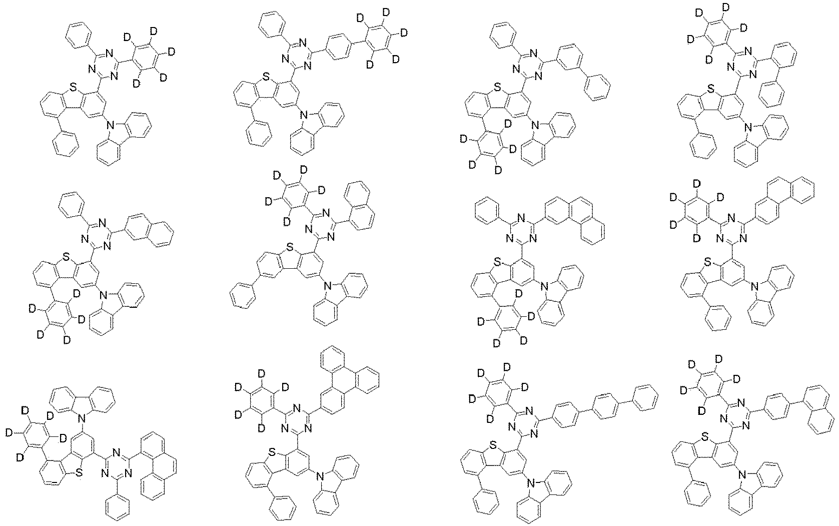

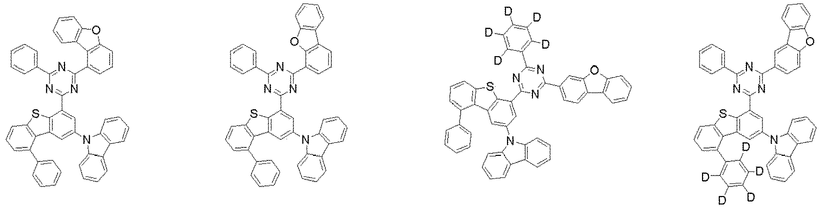

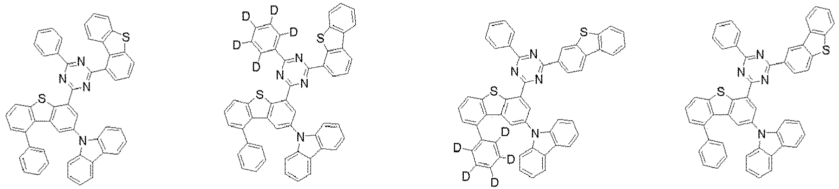

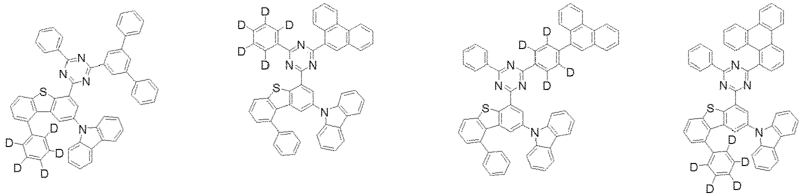

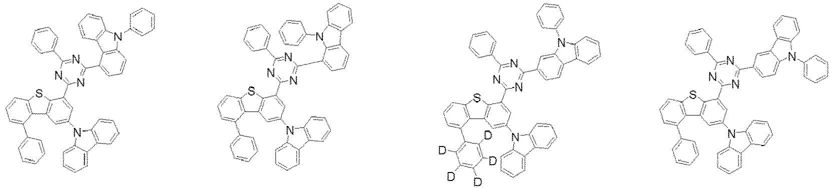

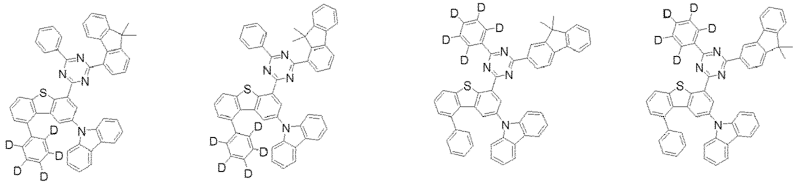

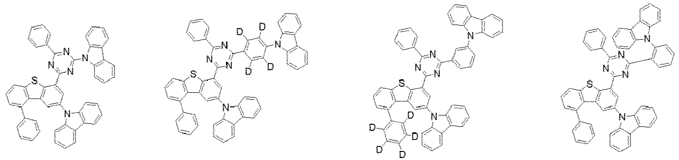

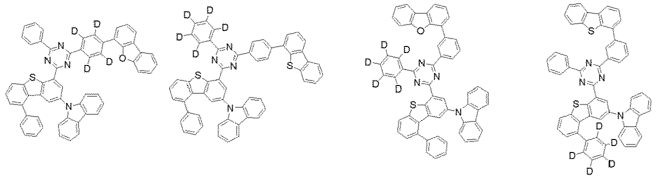

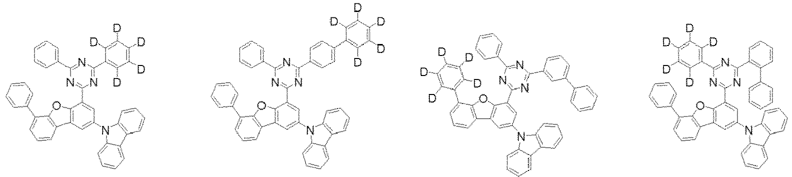

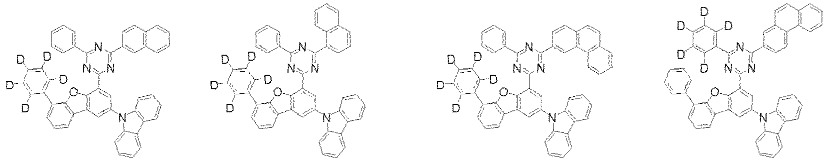

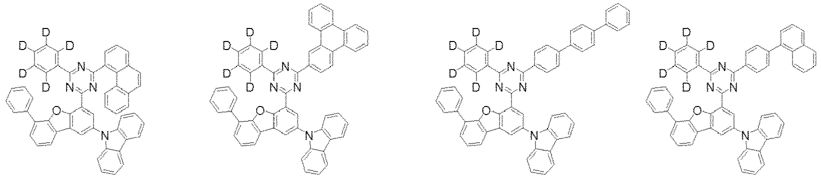

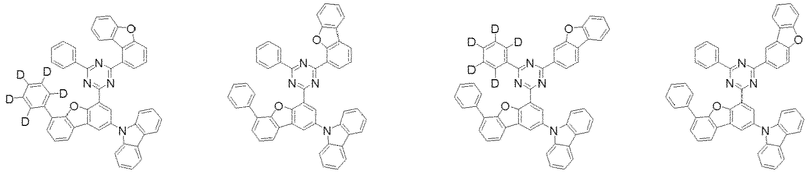

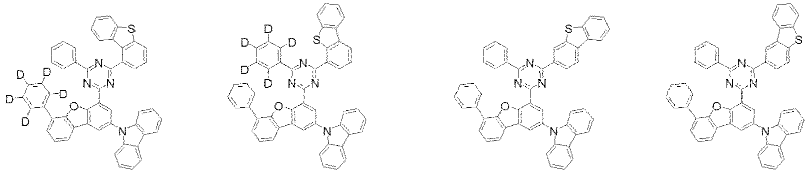

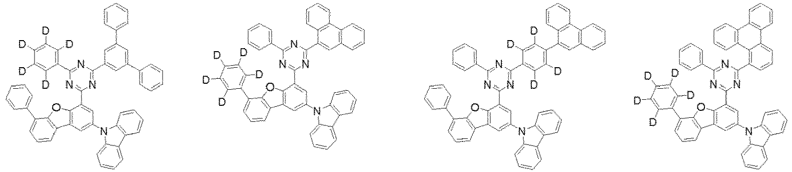

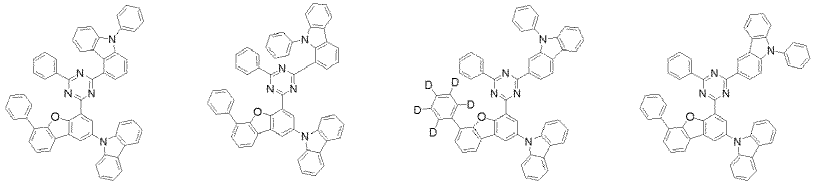

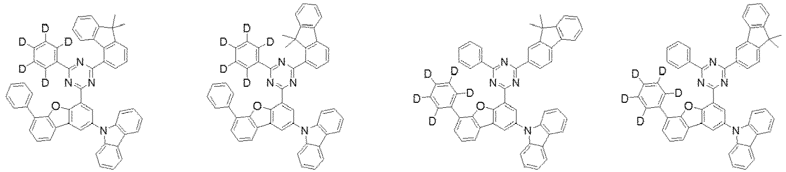

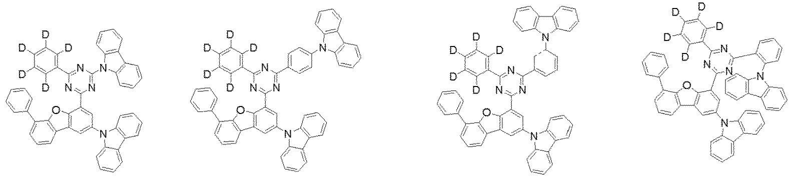

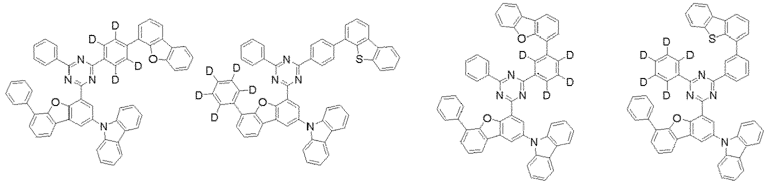

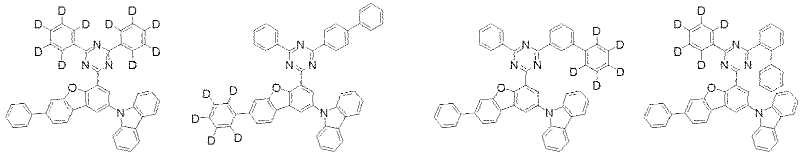

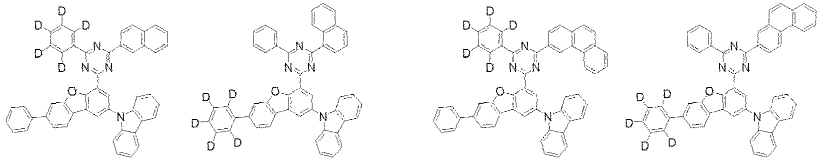

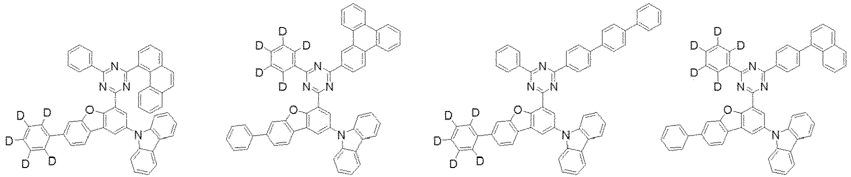

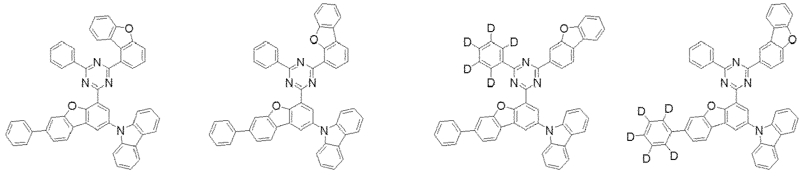

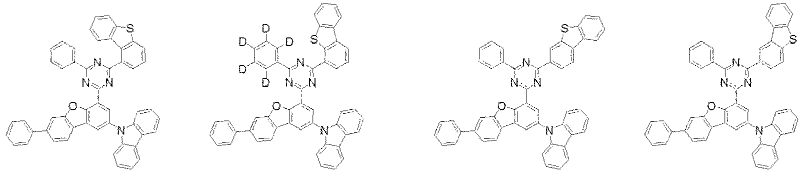

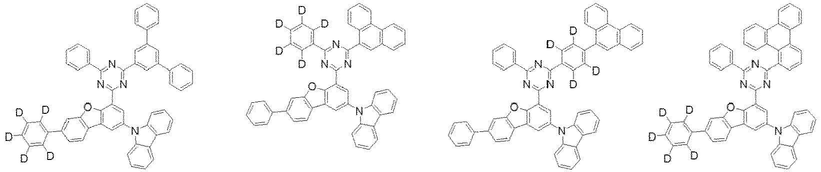

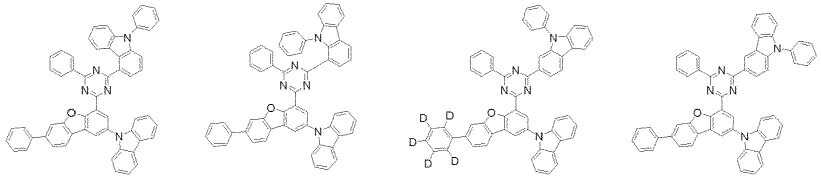

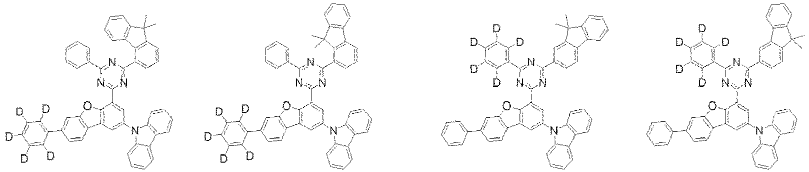

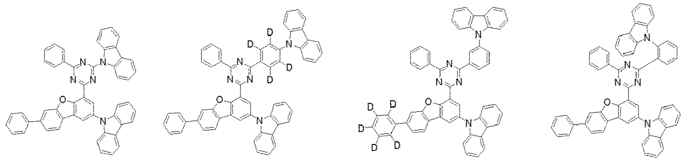

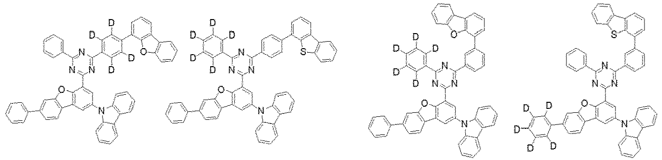

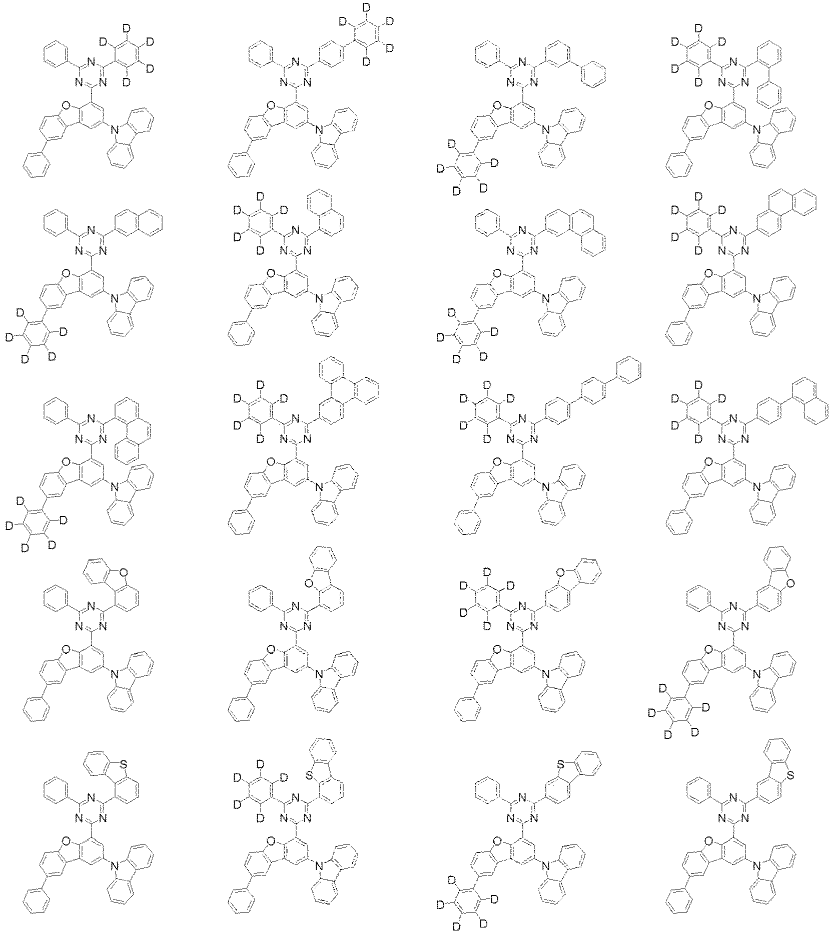

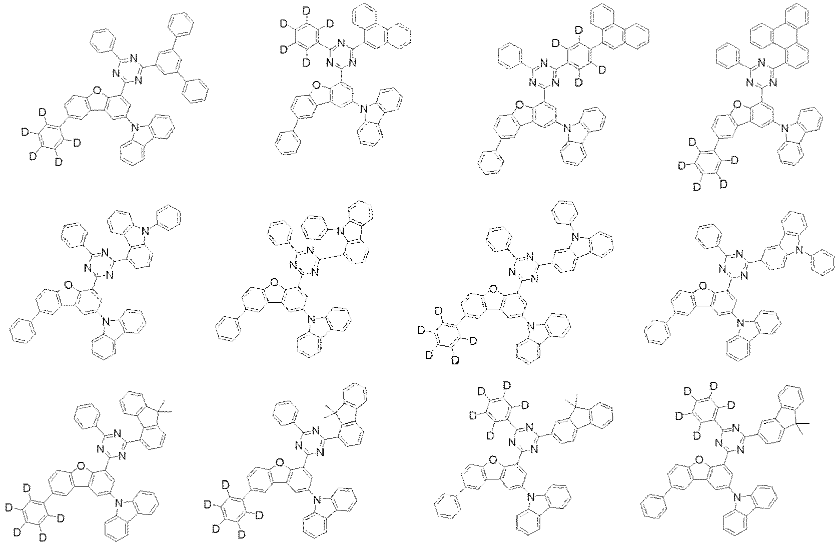

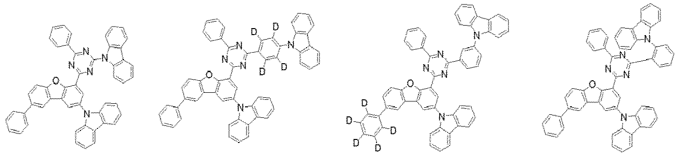

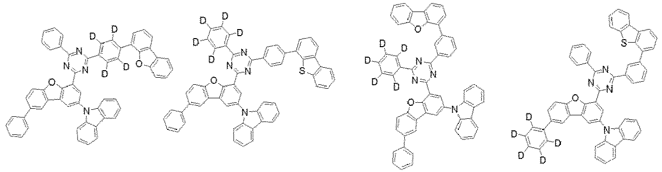

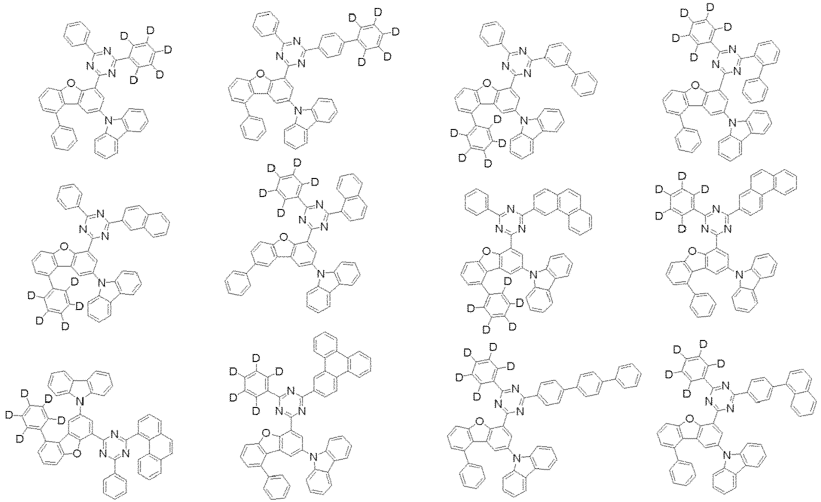

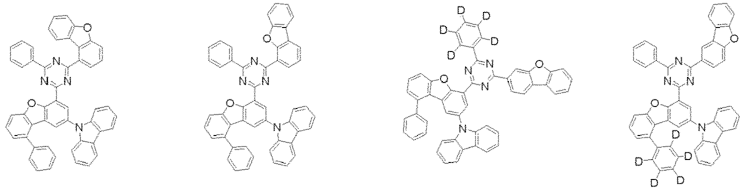

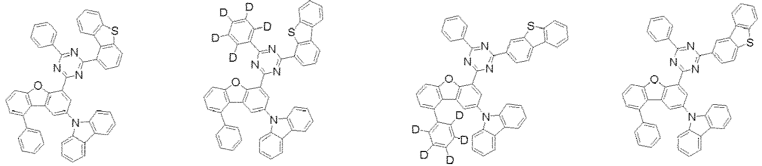

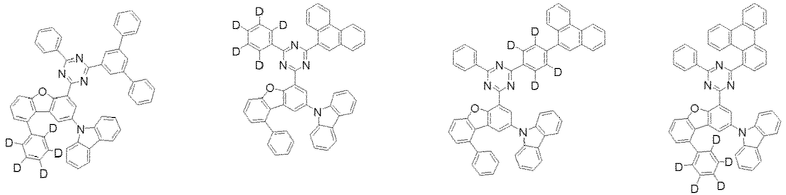

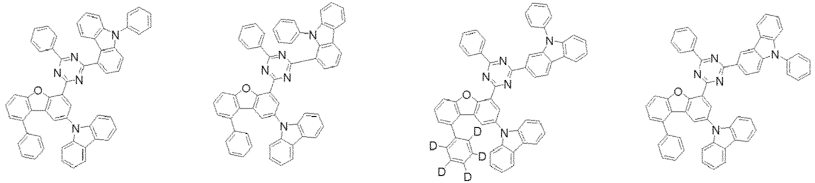

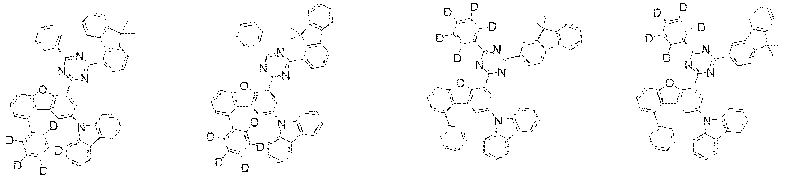

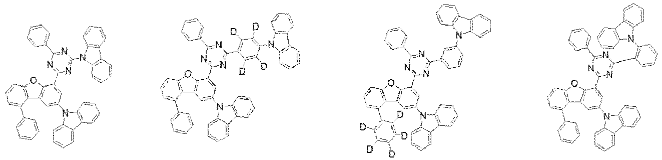

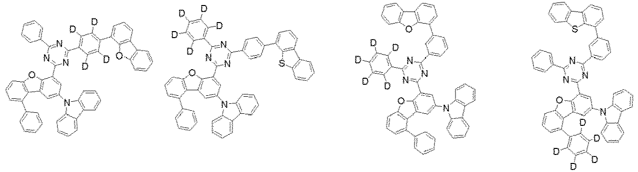

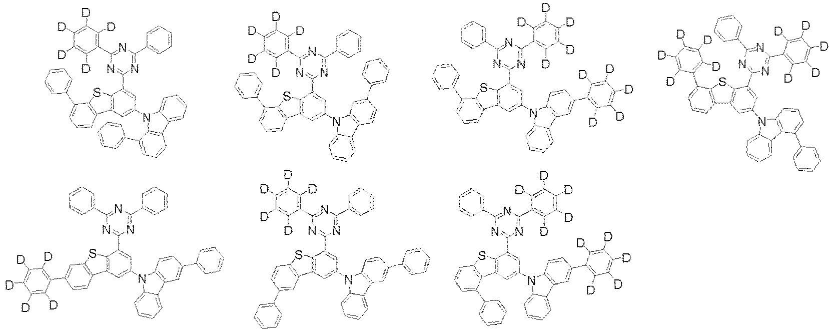

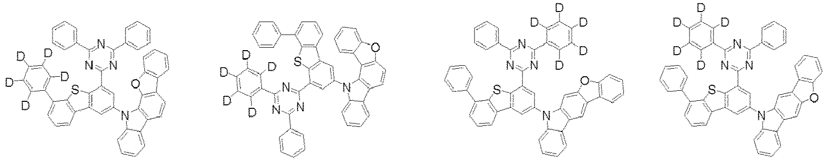

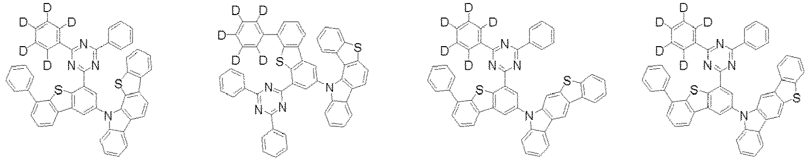

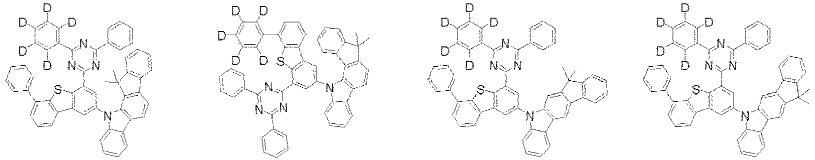

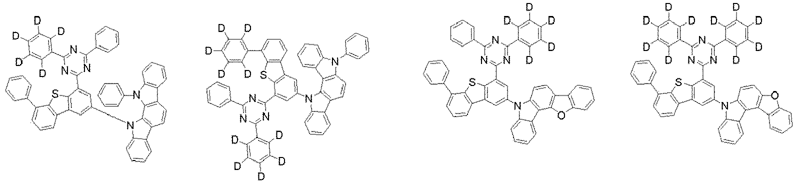

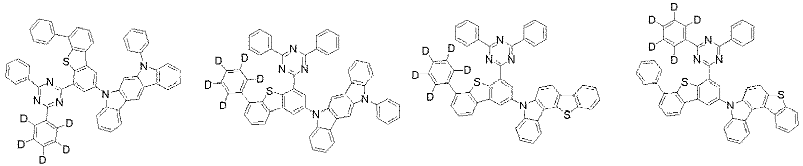

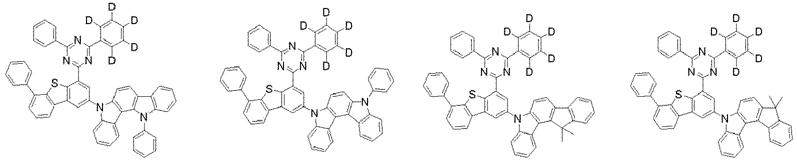

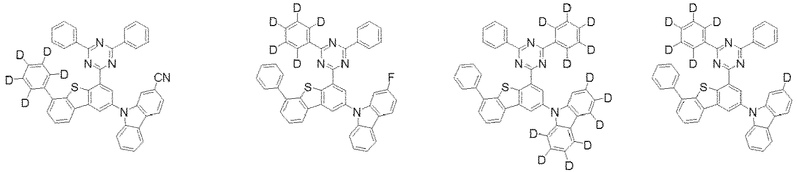

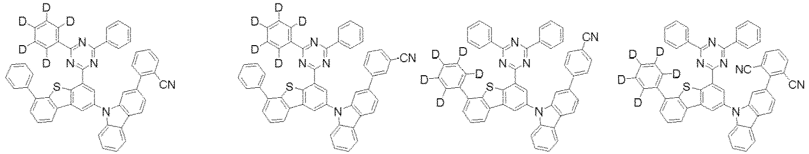

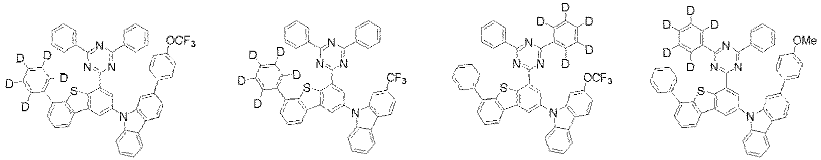

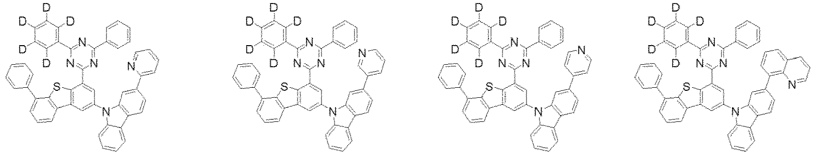

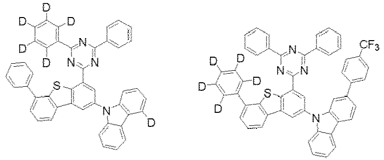

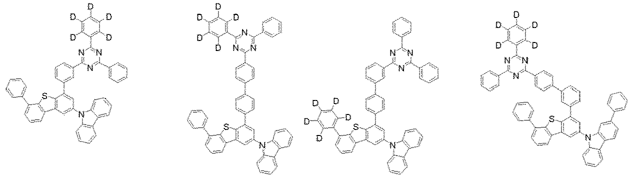

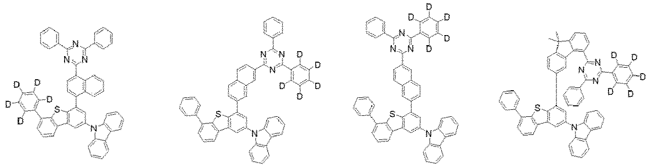

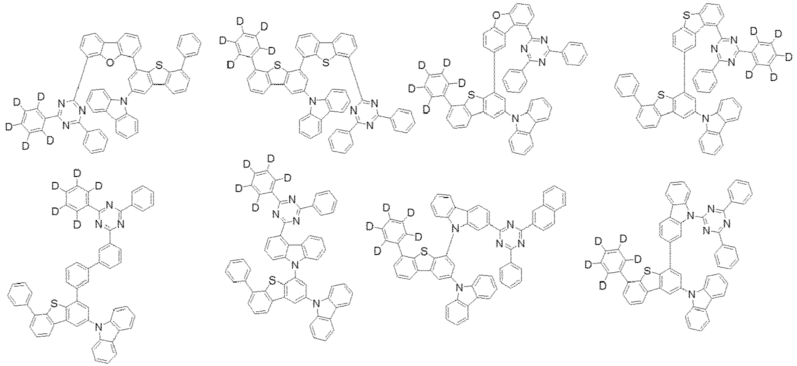

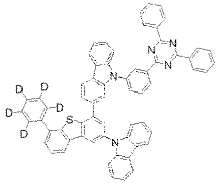

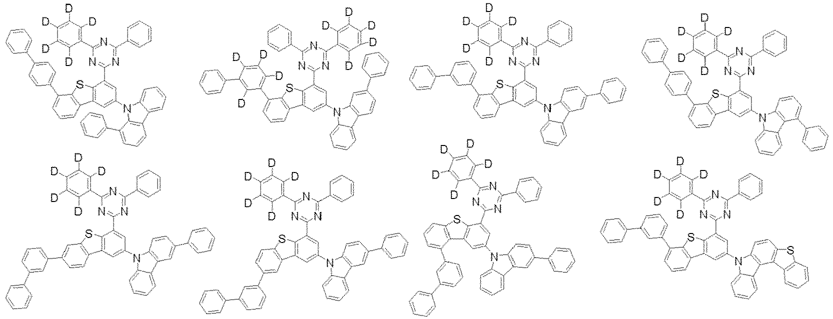

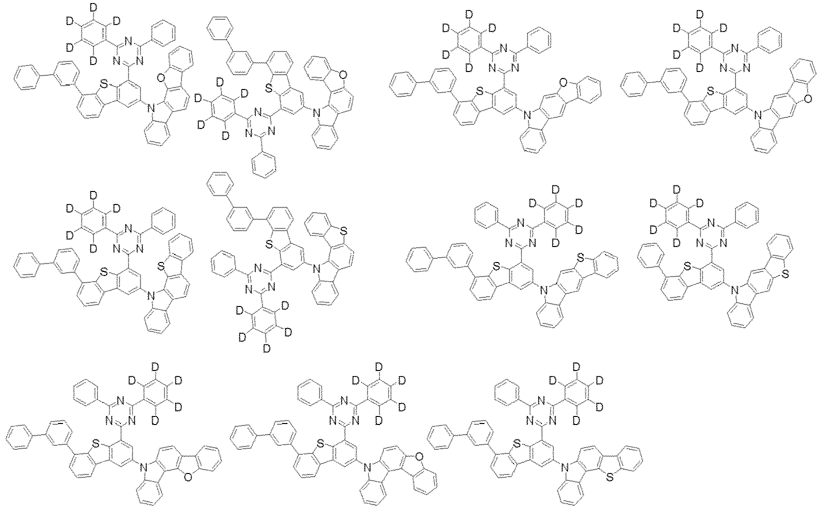

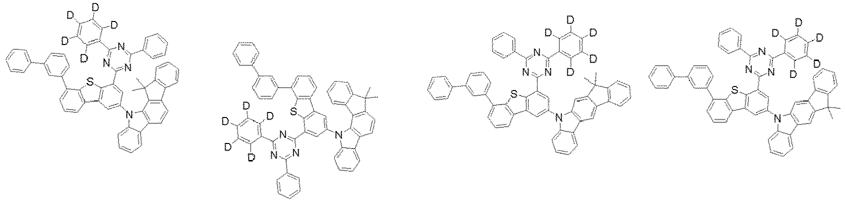

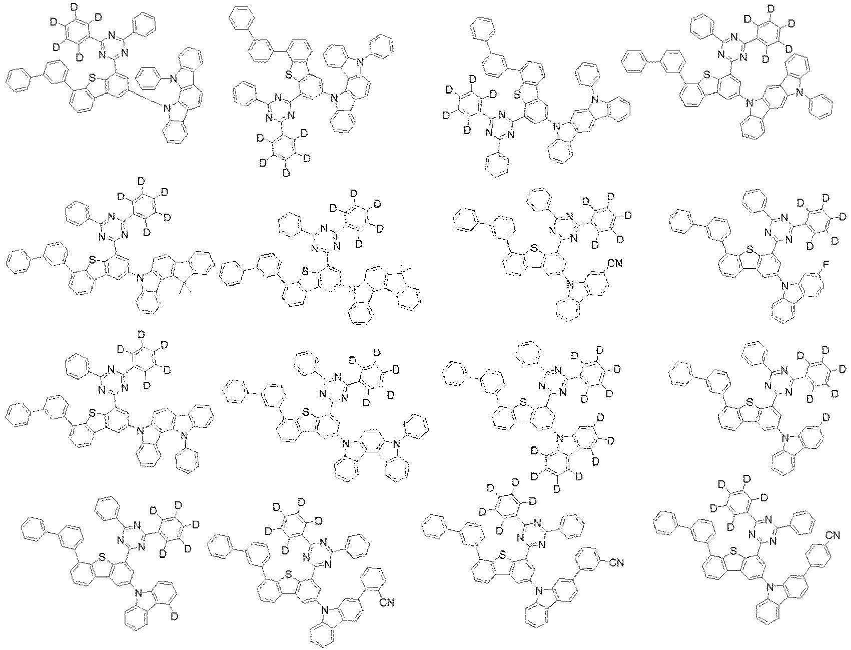

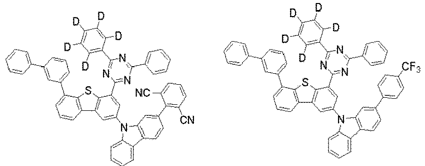

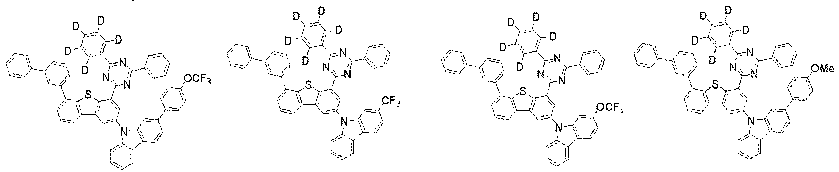

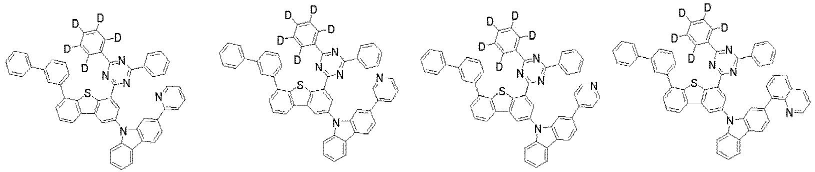

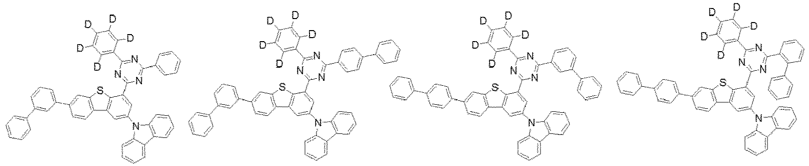

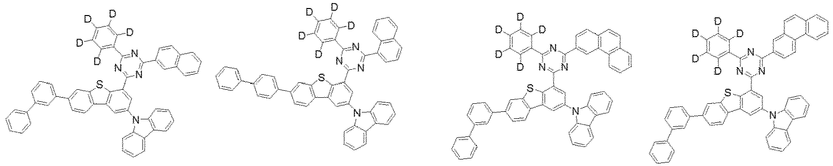

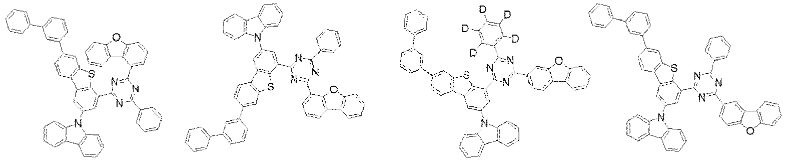

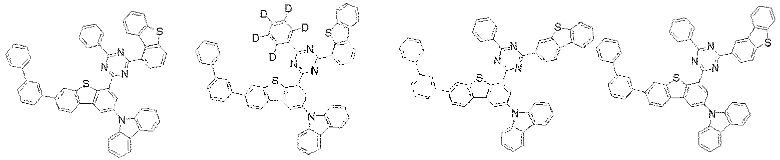

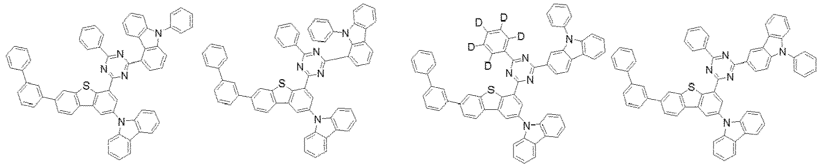

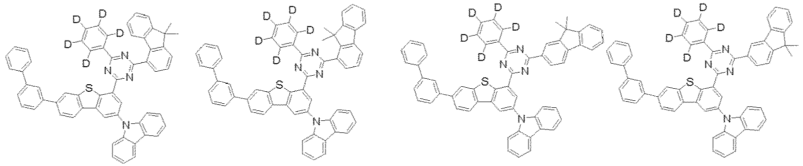

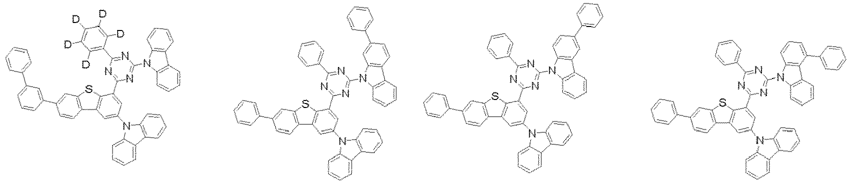

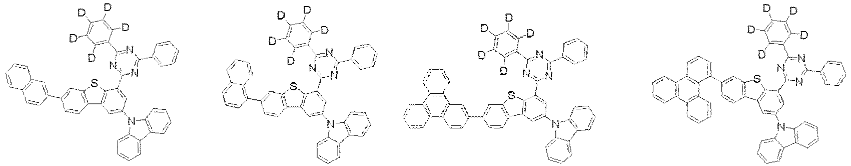

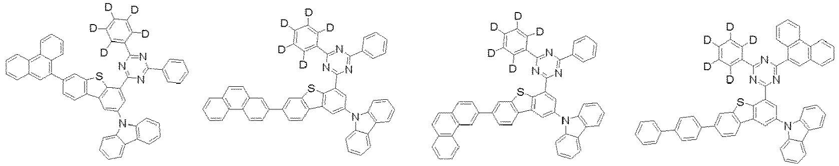

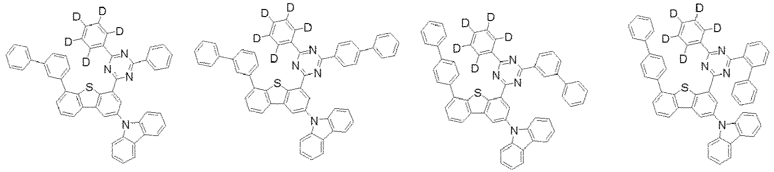

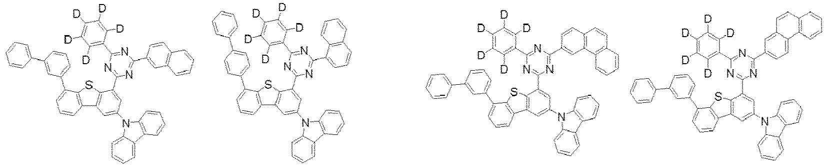

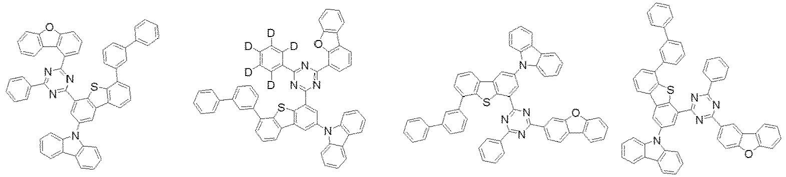

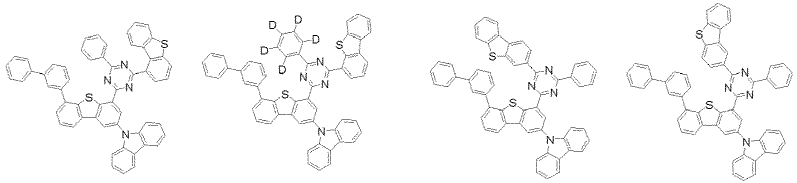

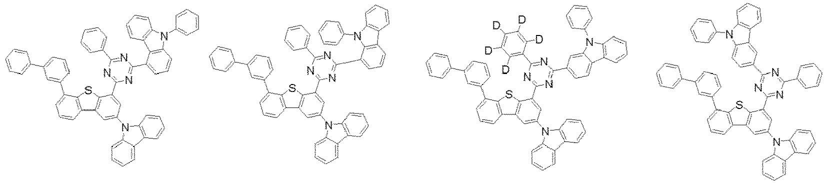

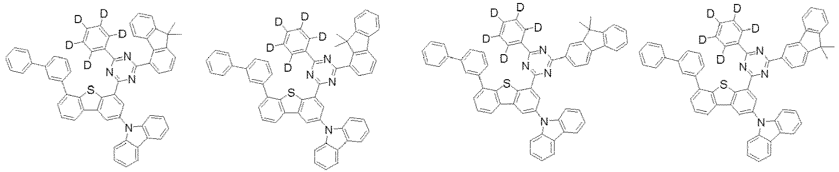

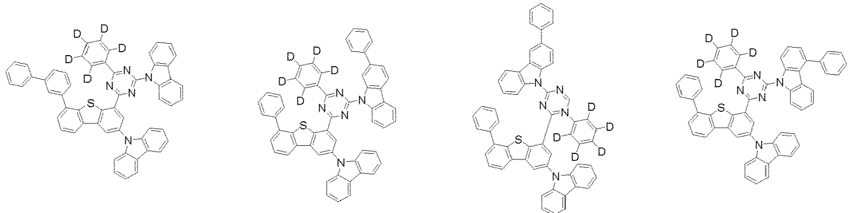

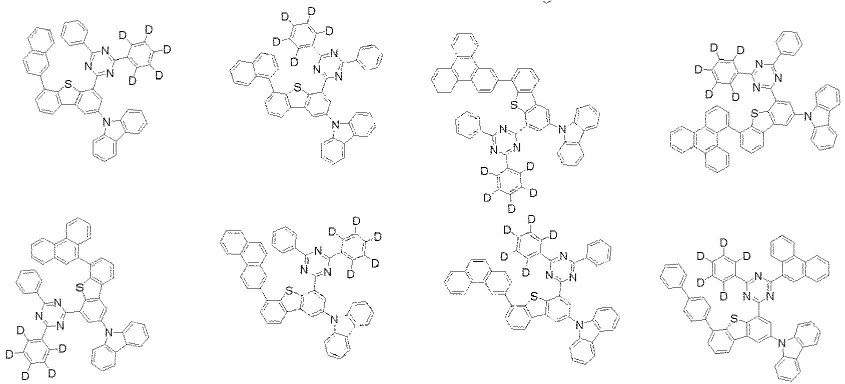

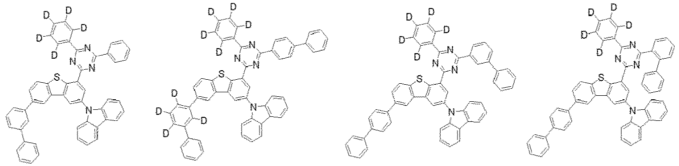

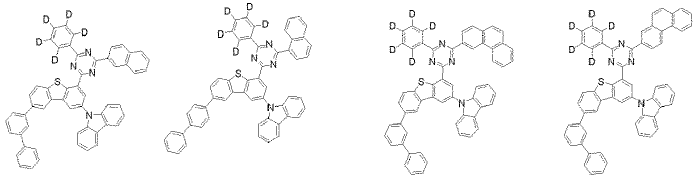

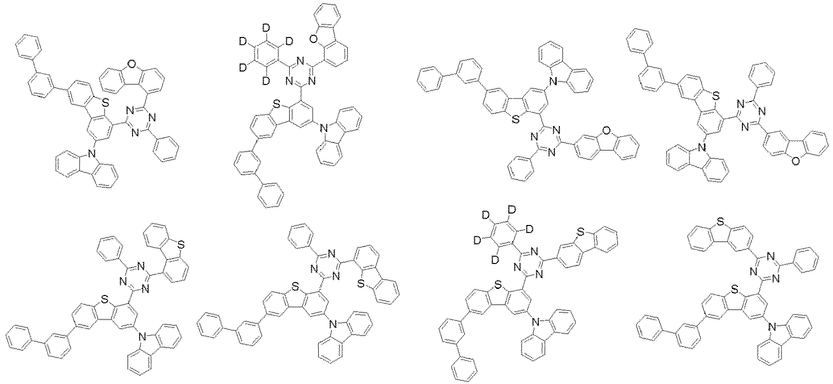

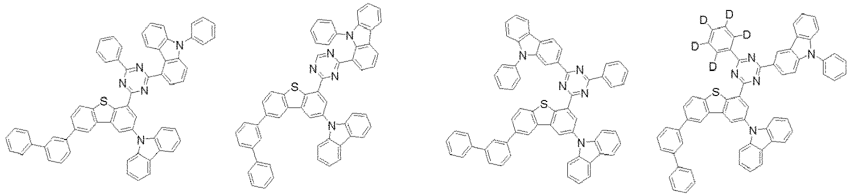

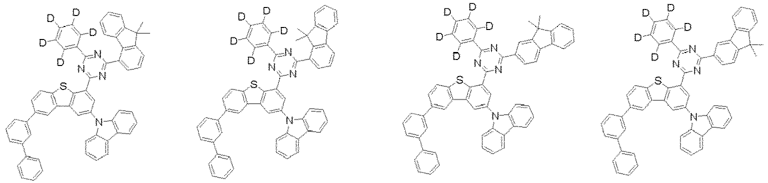

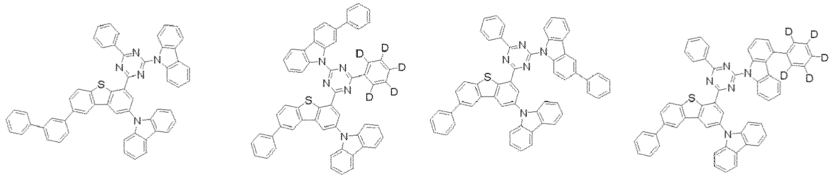

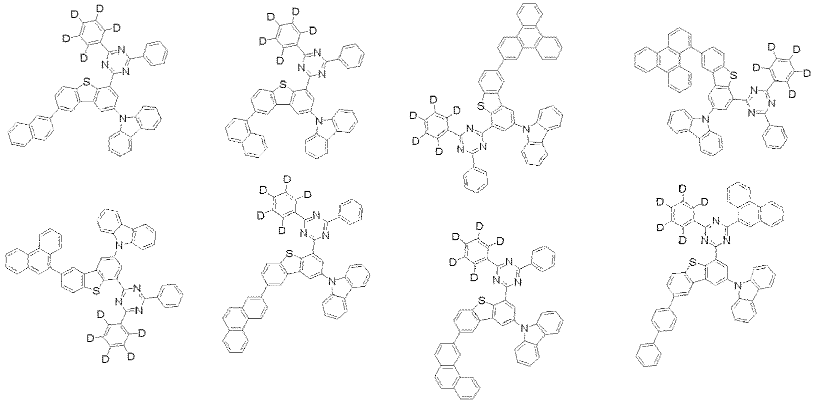

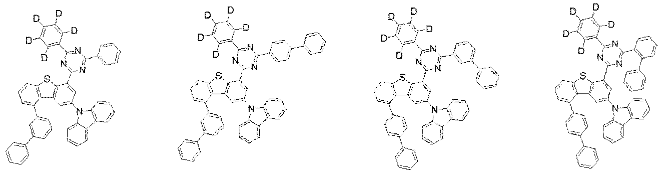

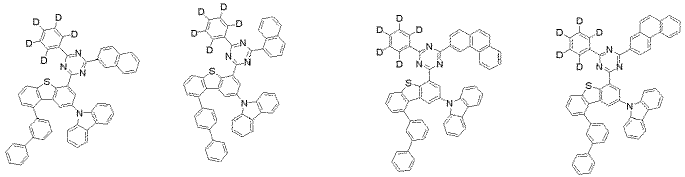

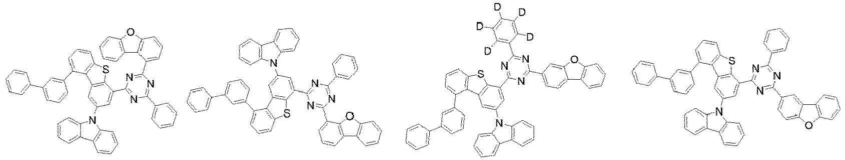

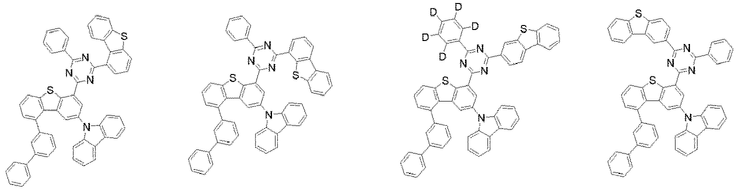

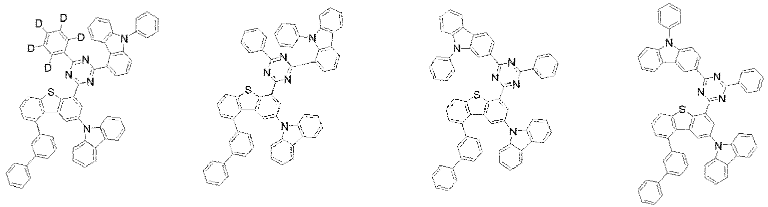

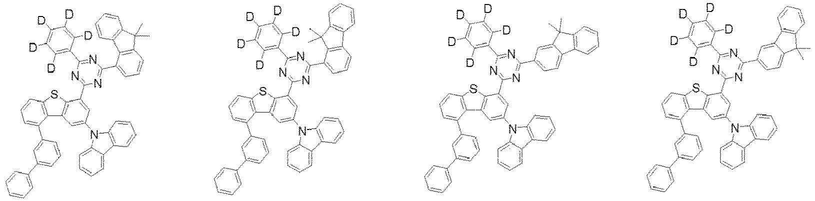

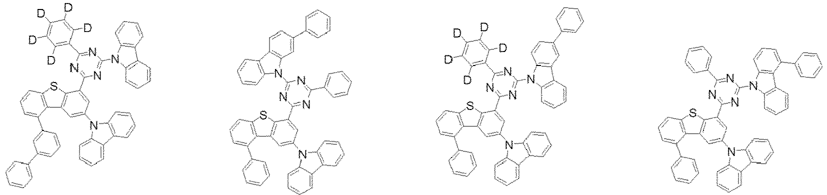

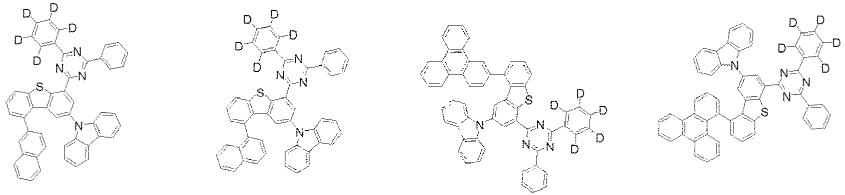

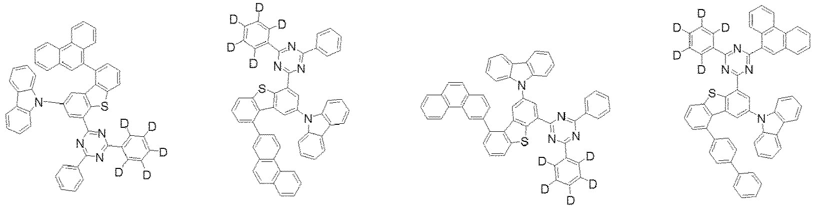

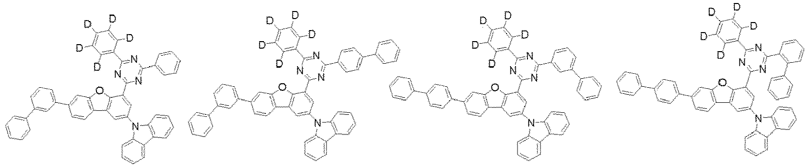

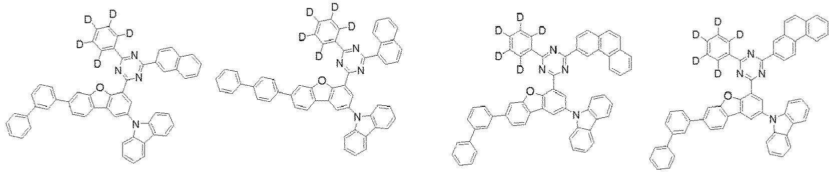

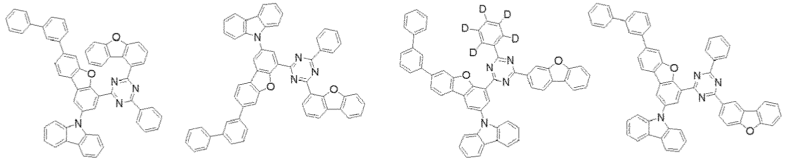

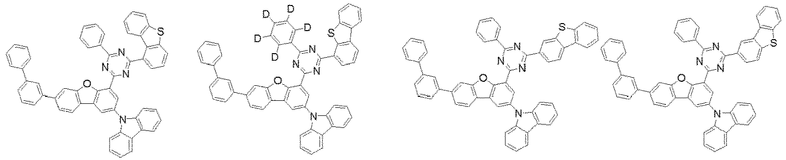

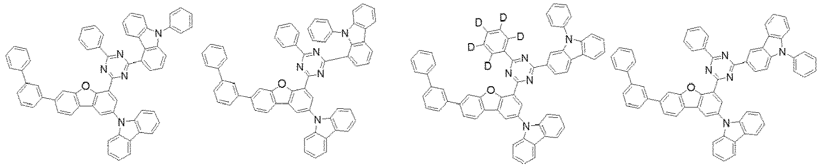

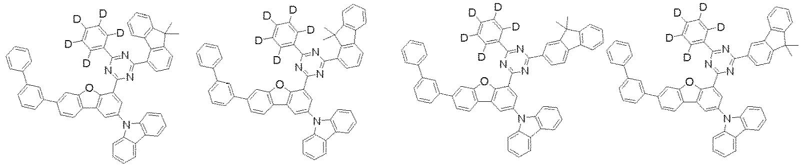

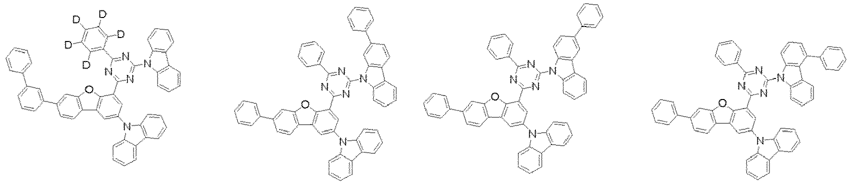

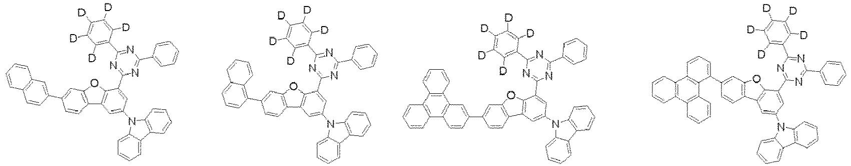

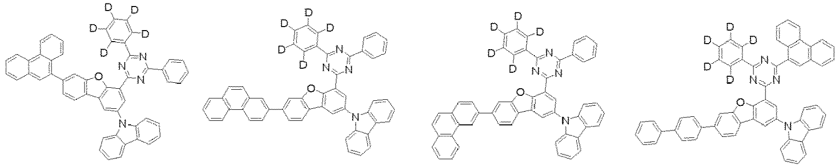

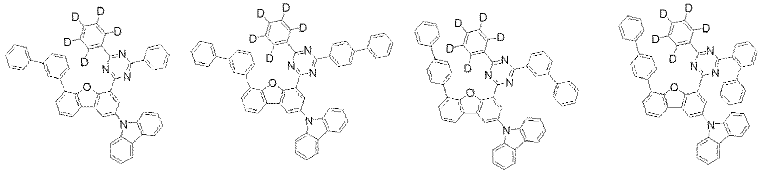

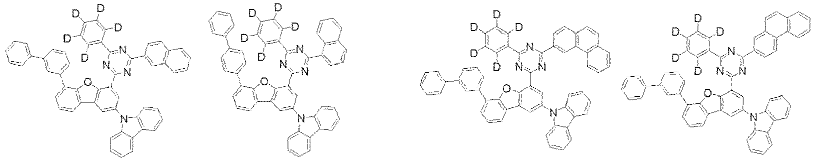

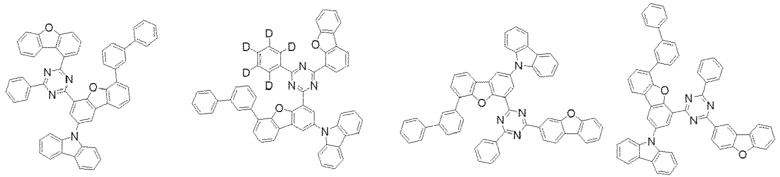

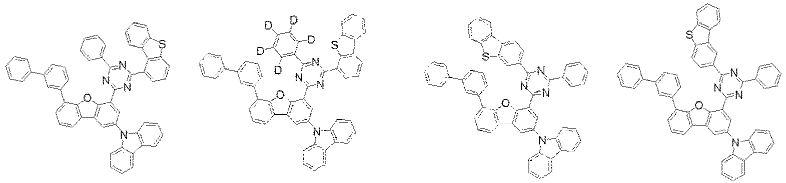

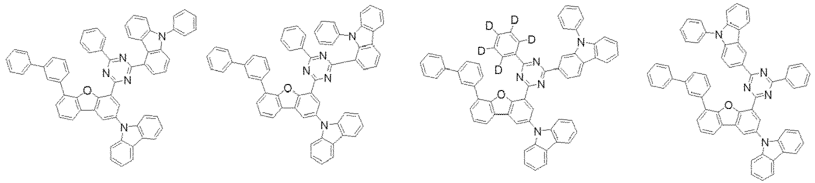

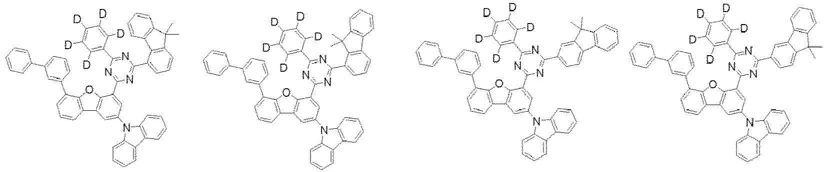

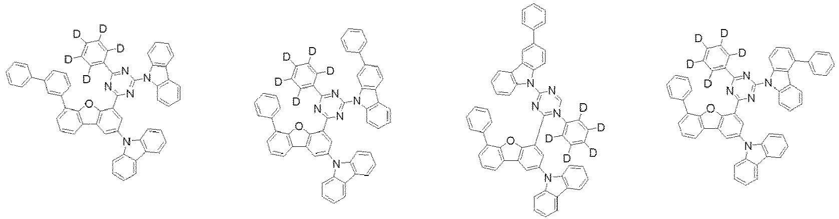

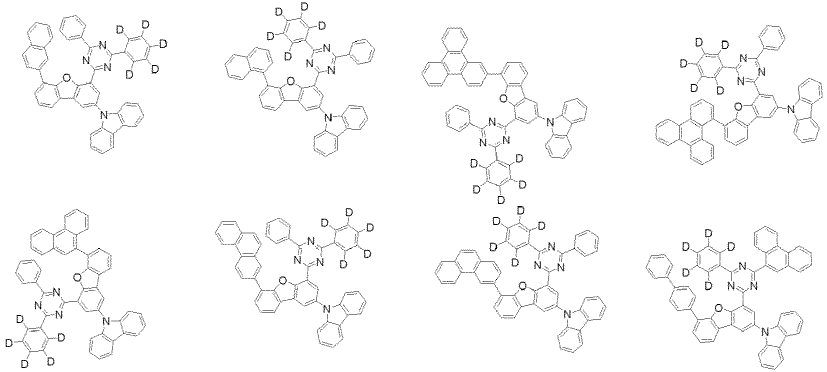

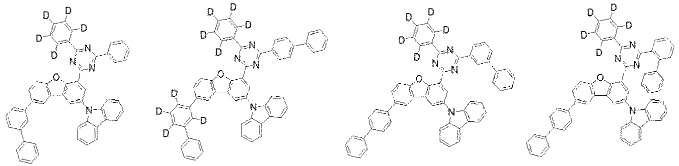

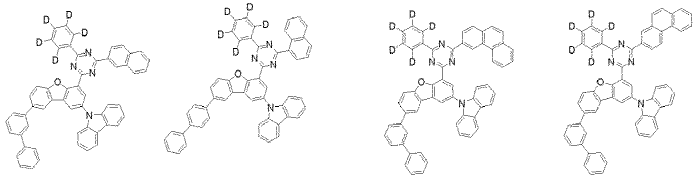

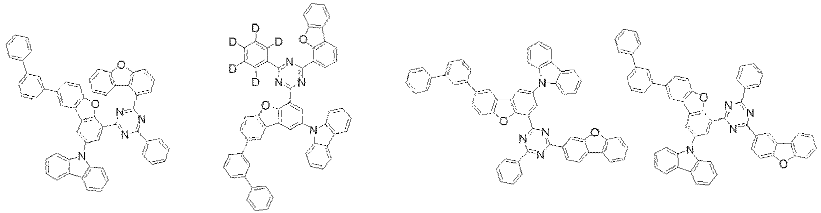

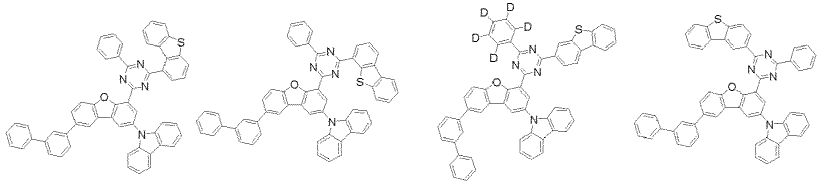

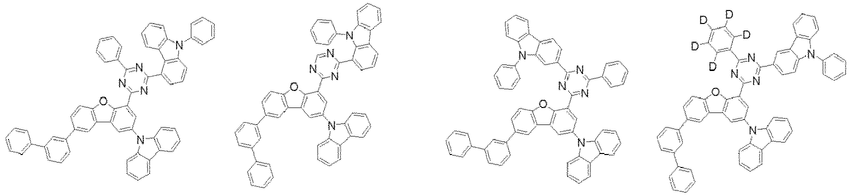

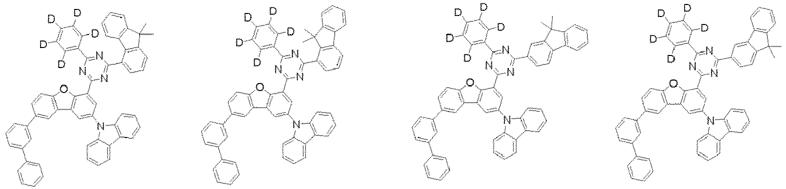

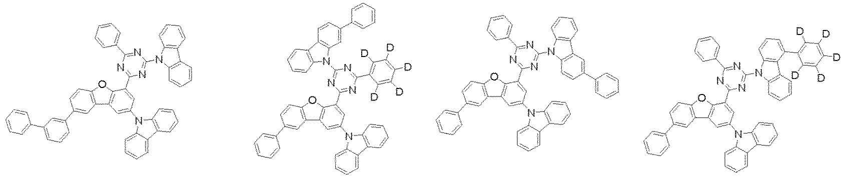

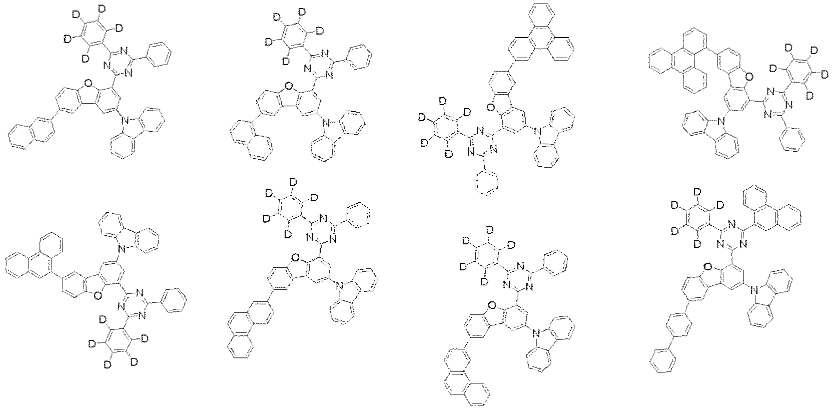

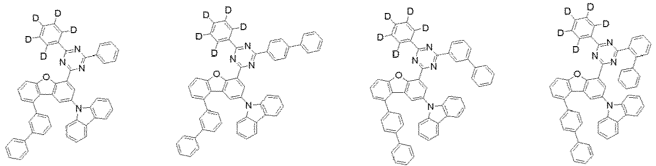

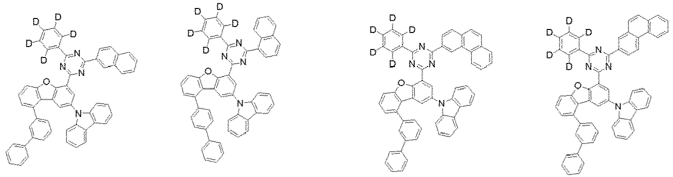

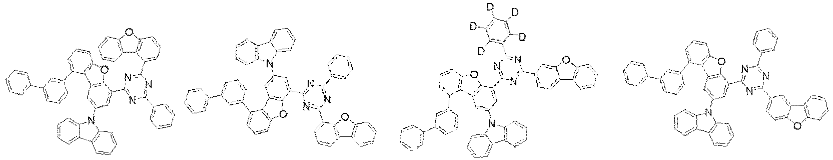

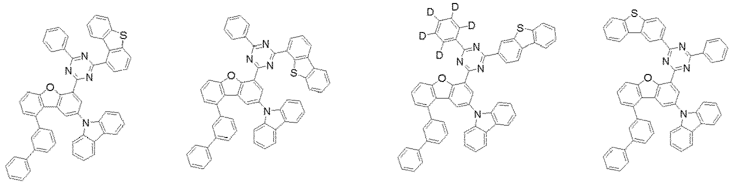

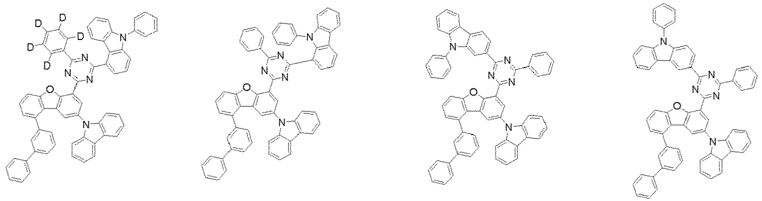

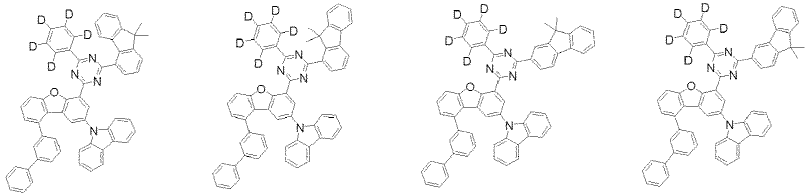

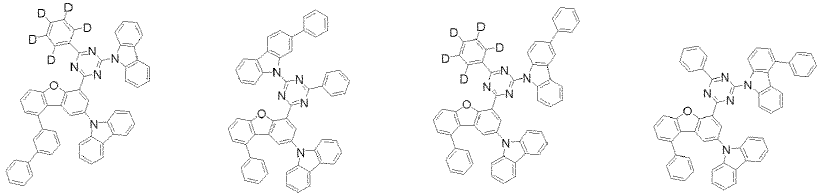

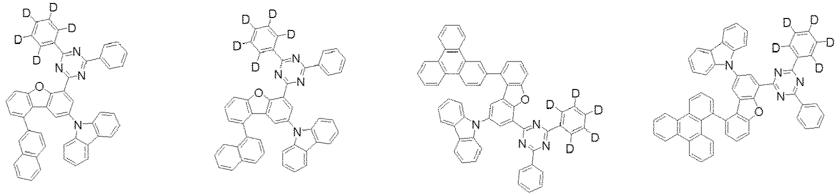

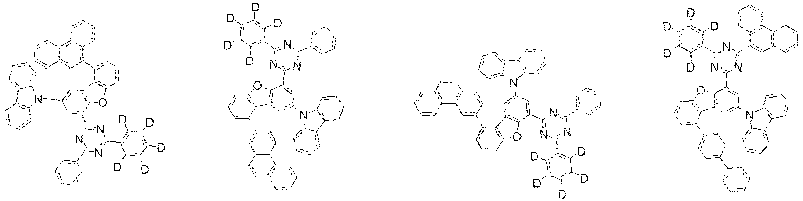

8. Organische lichtemittierende Vorrichtung, umfassend:
eine erste Elektrode; eine zweite Elektrode, die zur ersten Elektrode gegenüberliegend vorgesehen ist; und
eine oder mehr organische Materialschicht(en),
vorgesehen zwischen der ersten Elektrode und der zweiten Elektrode, worin eine oder mehr Schichten der organischen Materialschichten die Verbindung gemäß Anspruch 1 umfasst.

## Revendications

1. Composé représenté par la formule chimique 1 suivante : 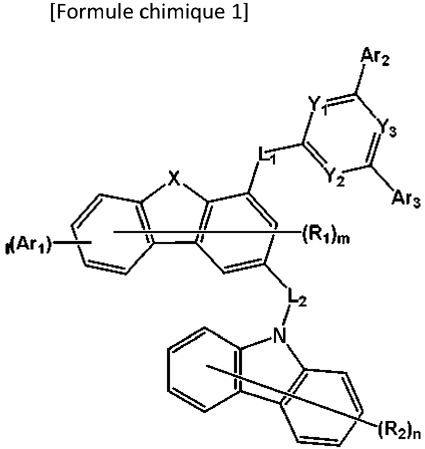 dans lequel, dans la formule chimique 1,
X est O ou S,
Y₁, Y₂ et Y₃ sont chacun indépendamment N,
L₁ et L₂ sont chacun indépendamment une liaison directe ou l'un quelconque sélectionné dans le groupe consistant en : 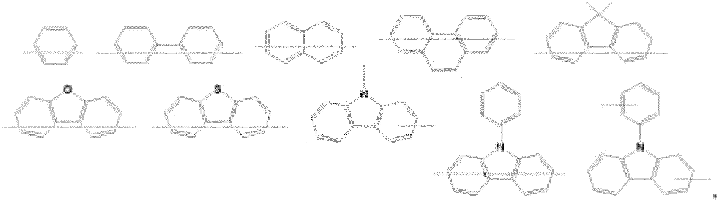
Ar₁ est un aryle en C₆₋₆₀ substitué ou non substitué,
Ar₂ et Ar₃ sont chacun indépendamment un aryle en C₆₋₆₀ substitué ou non substitué ; ou un hétéroaryle en C₅₋₆₀ substitué ou non substitué contenant au moins un hétéroatome sélectionné dans le groupe consistant en N, O et S,
à condition que quand tous parmi Ar₁, Ar₂ et Ar₃ sont un aryle en C₆₋₆₀ substitué ou non substitué, l'un quelconque parmi Ar₁, Ar₂ et Ar₃ soient substitué avec 4 deutériums ou plus,
chaque R₁ est indépendamment un hydrogène ; un deutérium ; un halogène ; ou un alkyle en C₁₋₆₀ substitué ou non substitué,
chaque R₂ est indépendamment un hydrogène ; un deutérium ; un halogène ; un cyano; un alkyle en C₁₋₆₀ substitué ou non substitué ; un alcoxy en C₁₋₆₀ substitué ou non substitué ; un cycloalkyle en C₃₋₆₀ substitué ou non substitué ; un haloalkyle en C₁₋₆₀ substitué ou non substitué ; un haloalcoxy en C₁₋₆₀ substitué ou non substitué ; une arylamine en C₆₋₆₀ substituée ou non substituée ; une alkylamine en C₁₋₆₀ substituée ou non substituée ; un trifluoroalkyle en C₁₋₆₀ ; un trifluoroalcoxy en C₁₋₆₀ ; un aryle en C₆₋₆₀ substitué ou non substitué ; ou un hétéroaryle en C₅₋₆₀ substitué ou non substitué contenant au moins l'un parmi N, O et S, qui peut se lier à l'atome de carbone du carbazole pour former un cycle condensé,
m est un nombre entier allant de 0 à 3,
n est un nombre entier allant de 0 à 6, et
l est 1 ou 2.

2. Composé selon la revendication 1,
dans lequel le composé représenté par la formule chimique 1 suivante est l'un quelconque sélectionné dans le groupe consistant en les composés représentés par les formules chimiques 2 à 8 suivantes : 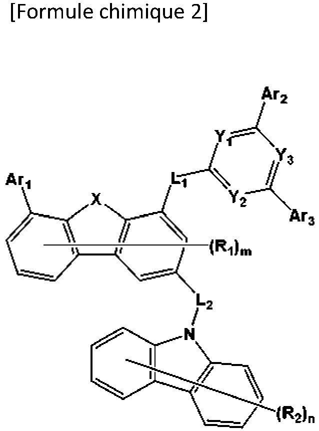 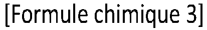 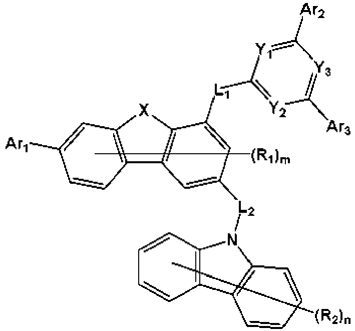 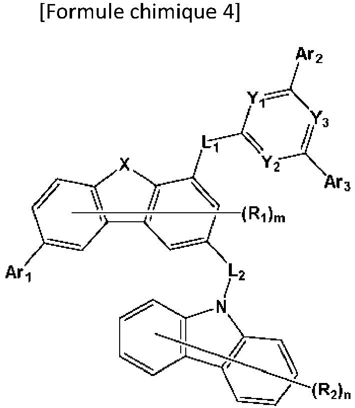 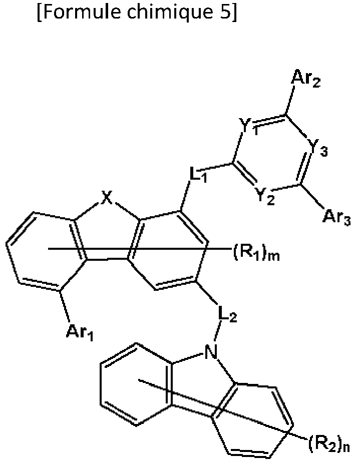 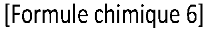 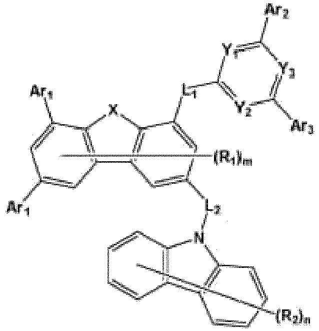 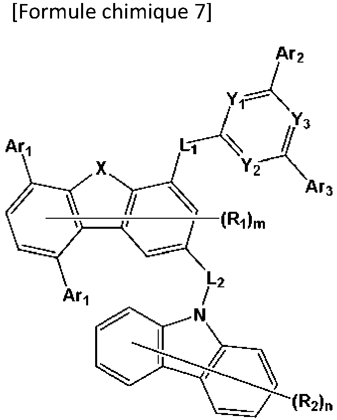 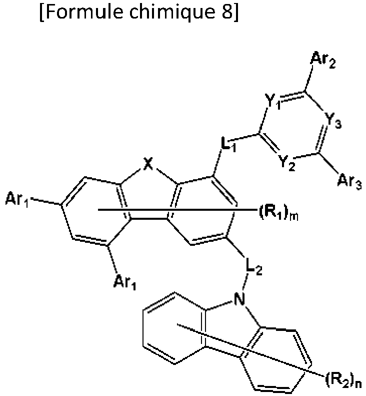 dans lequel, dans les formules chimiques 2 à 8,
X, Y₁, Y₂, Y₃, L₁, L₂, Ar₁, Ar₂, Ar₃, R₁, R₂, m et n sont tels que définis dans la revendication 1.

3. Composé selon la revendication 1,
dans lequel Ar₁ est l'un quelconque sélectionné dans le groupe consistant en : 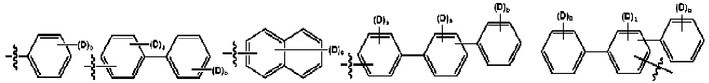 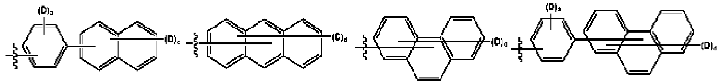 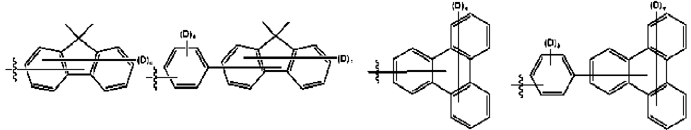 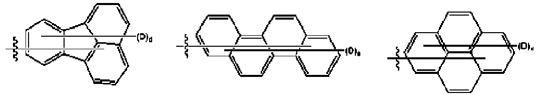
dans les formules ci-dessus,
a est un nombre entier allant de 0 à 4,
b est un nombre entier allant de 0 à 5,
c est un nombre entier allant de 0 à 7,
d est un nombre entier allant de 0 à 9, et
e est un nombre entier allant de 0 à 11.

4. Composé selon la revendication 1,
dans lequel Ar₂ et Ar₃ sont chacun indépendamment l'un quelconque sélectionné dans le groupe consistant en : 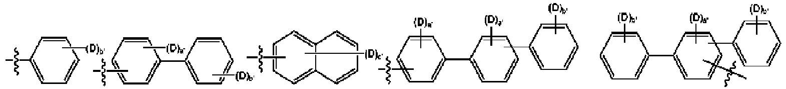 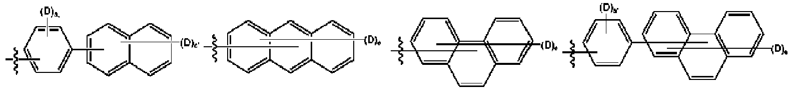 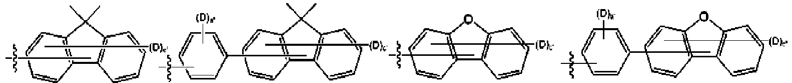 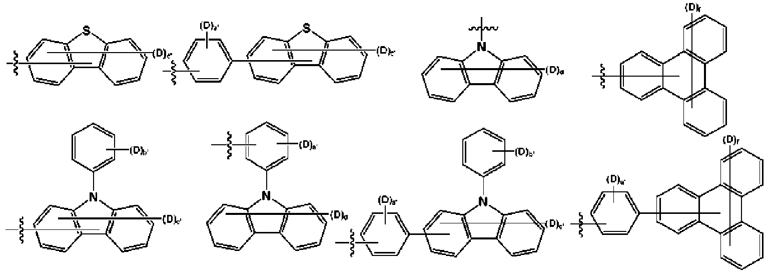 dans les formules ci-dessus,
a' est un nombre entier allant de 0 à 4,
b' est un nombre entier allant de 0 à 5,
c' est un nombre entier allant de 0 à 7,
d' est un nombre entier allant de 0 à 8,
e' est un nombre entier allant de 0 à 9, et
f' est un nombre entier allant de 0 à 11.

5. Composé selon la revendication 1,
dans lequel chaque R1 est indépendamment un hydrogène ou un deutérium.

6. Composé selon la revendication 1,
dans lequel chaque R₂ est indépendamment un hydrogène ; un deutérium ; un halogène ; un cyano ; un méthoxy ; un trifluorométhyle ; un trifluorométhoxy ; un phényle ; un pyridinyle ; un isoquinoléinyle ; ou l'un quelconque sélectionné dans le groupe consistant en, 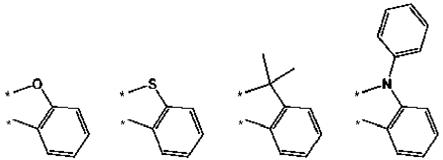
dans les formules ci-dessus, deux * sont chacun liés à un carbone adjacent du carbazole dans la formule chimique 1, et
le phényle est substitué ou non substitué avec l'un quelconque sélectionné dans le groupe consistant en un halogène, un cyano, un méthoxy, un trifluorométhyle et un trifluorométhoxy.

7. Composé selon la revendication 1,
dans lequel le composé représenté par la formule chimique 1 est l'un quelconque sélectionné dans le groupe consistant en :

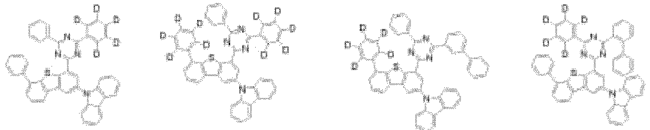

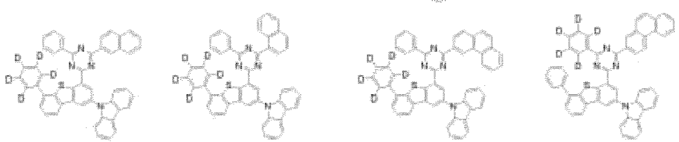

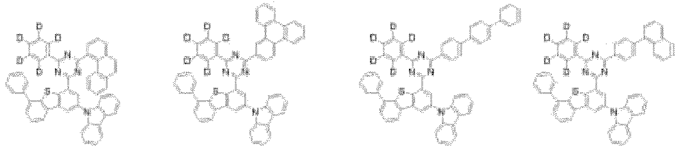

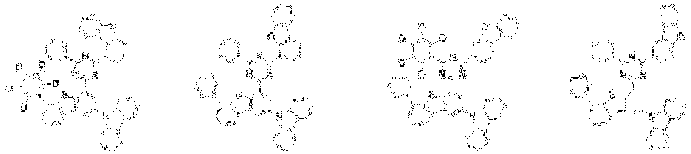

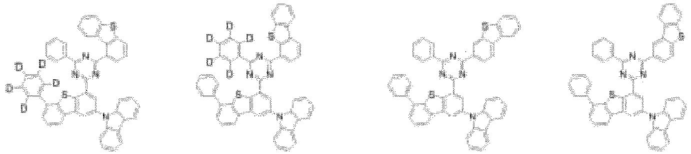

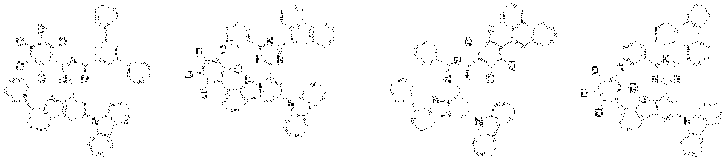

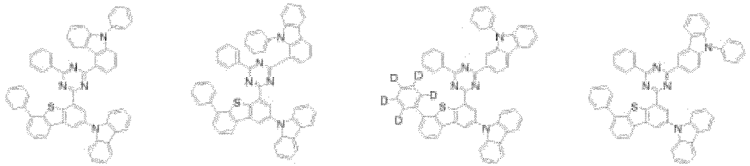

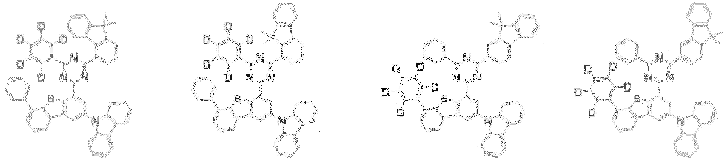

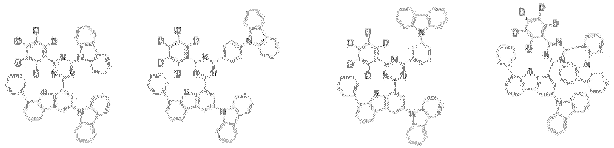

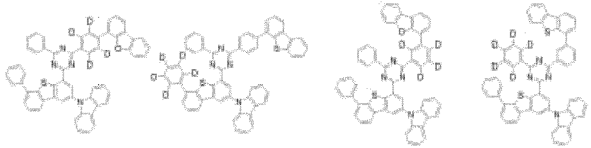

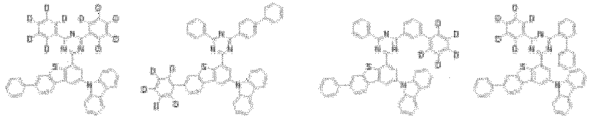

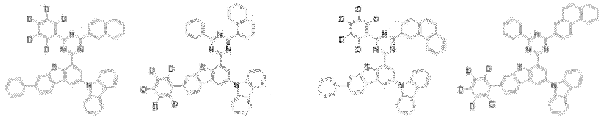

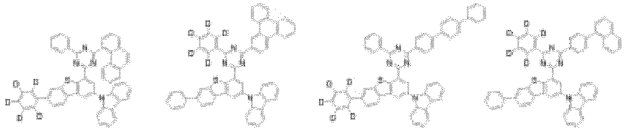

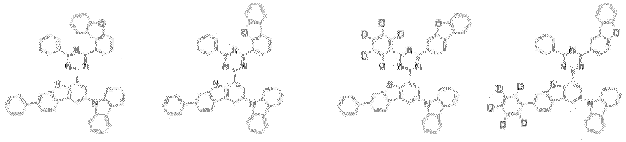

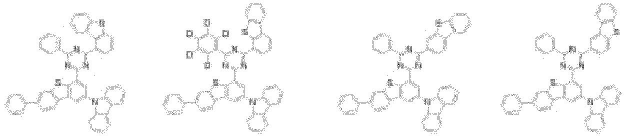

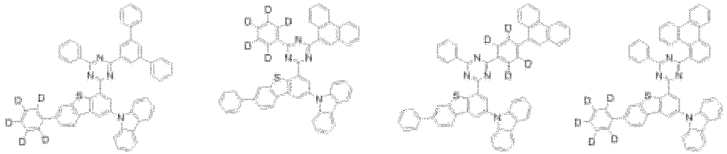

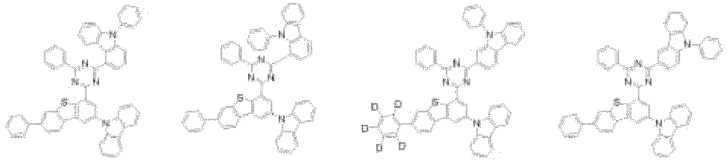

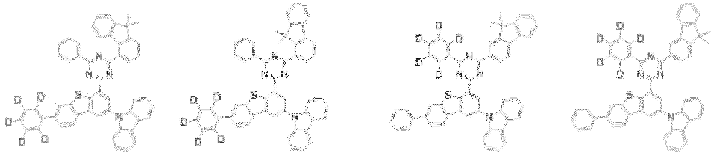

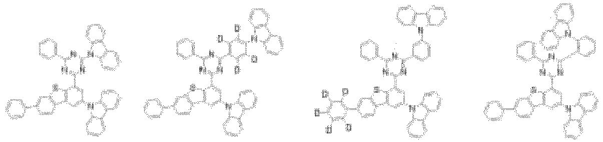

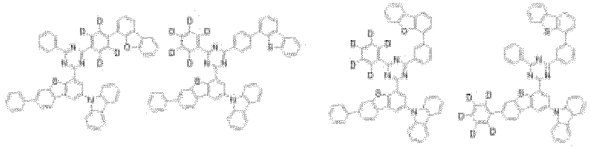

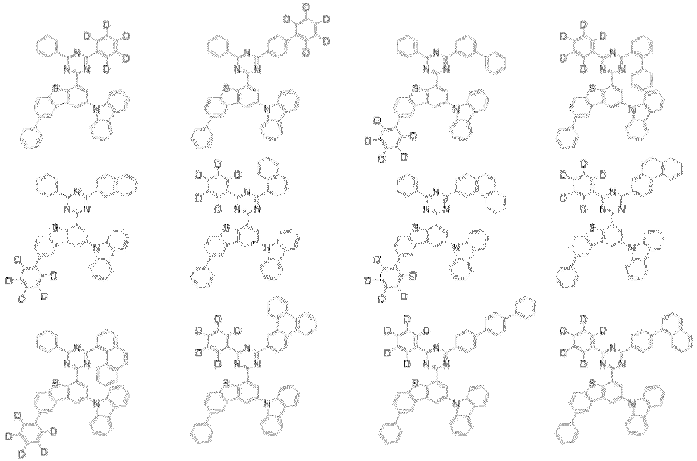

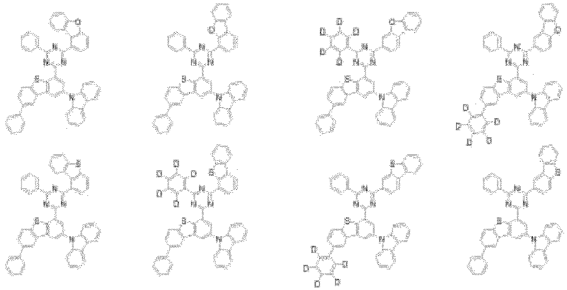

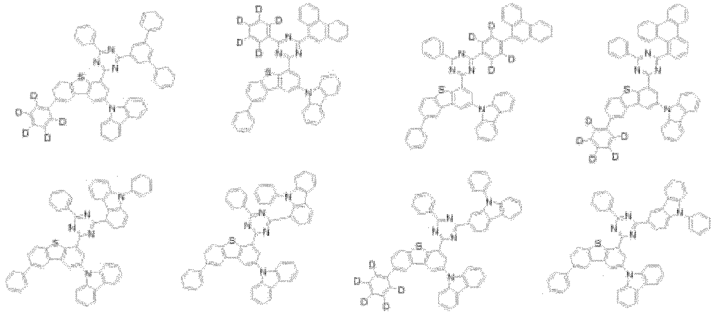

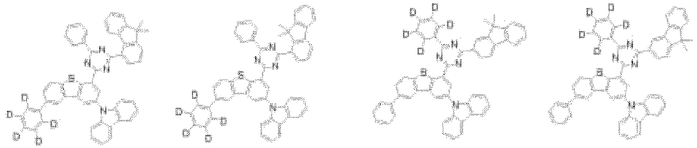

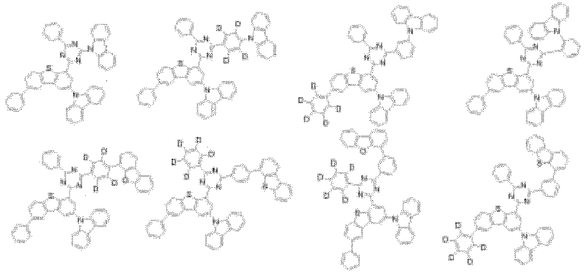

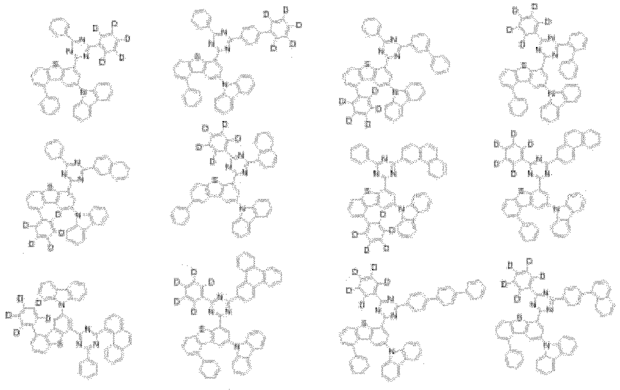

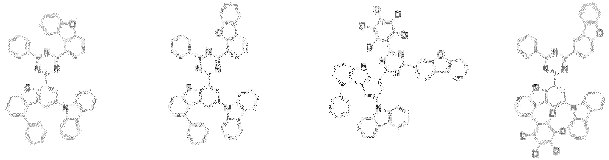

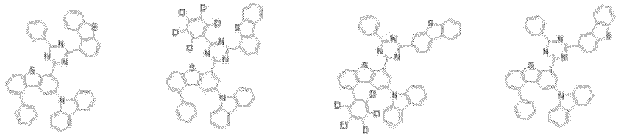

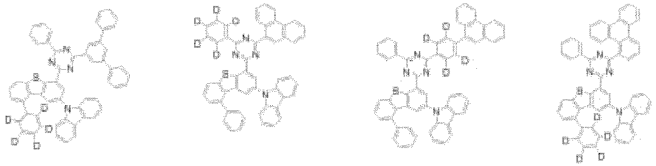

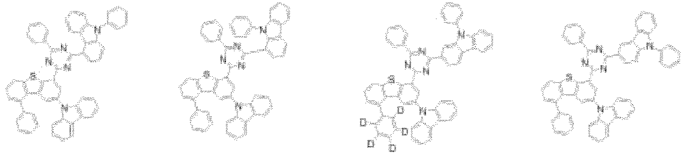

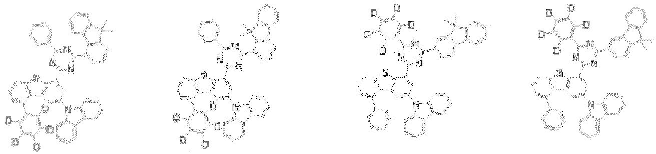

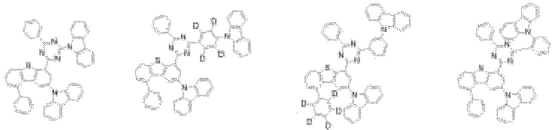

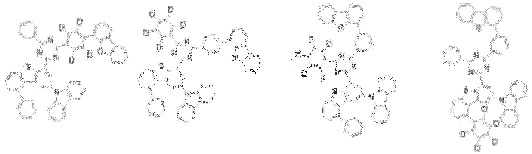

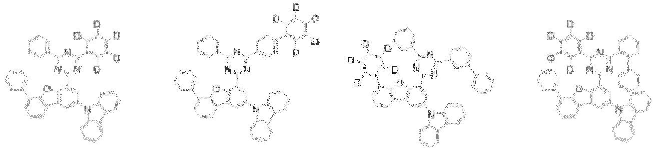

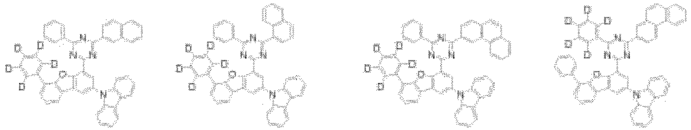

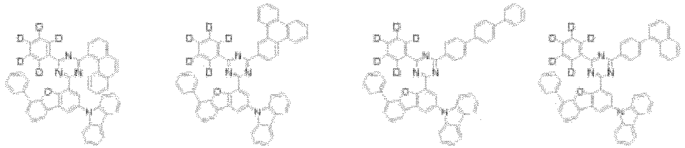

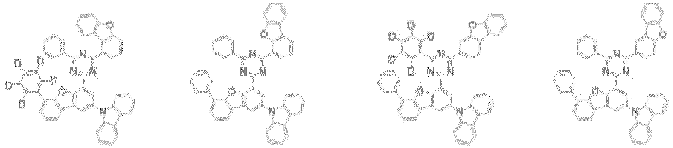

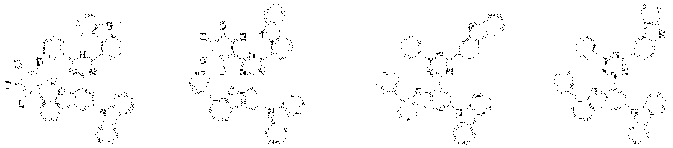

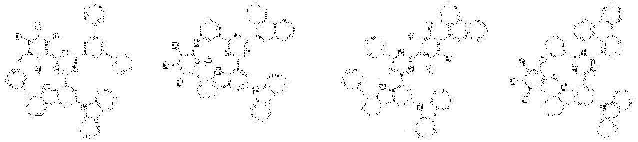

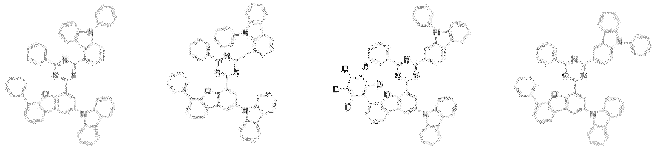

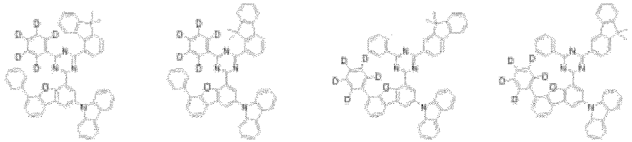

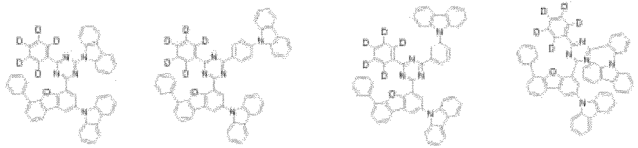

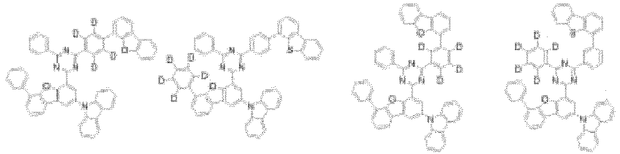

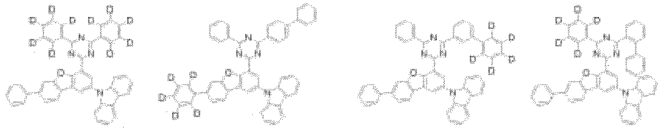

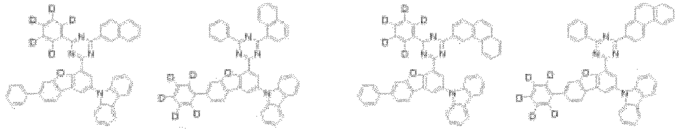

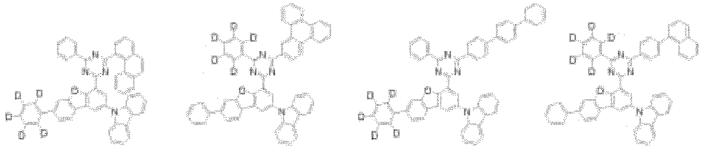

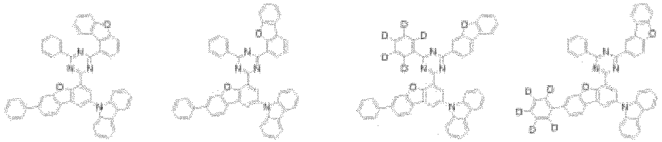

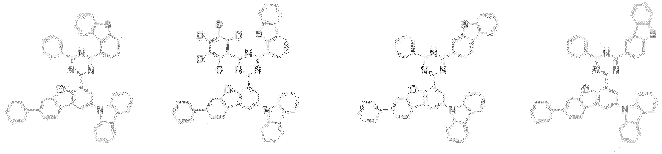

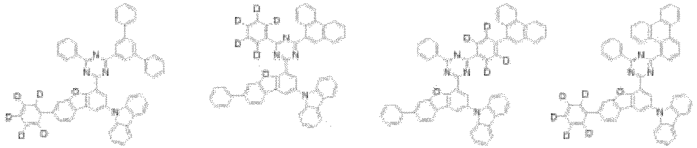

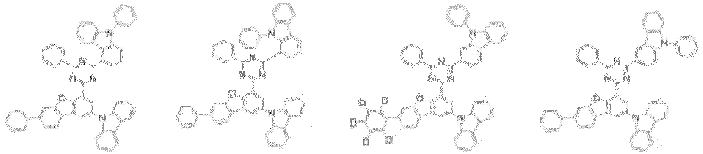

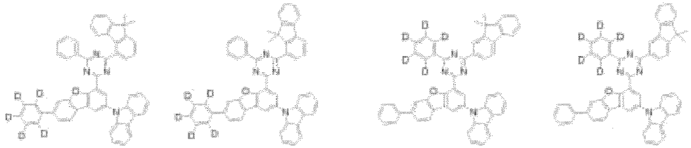

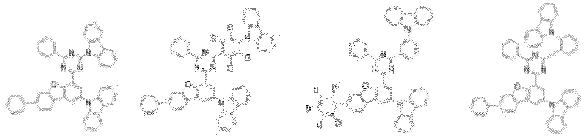

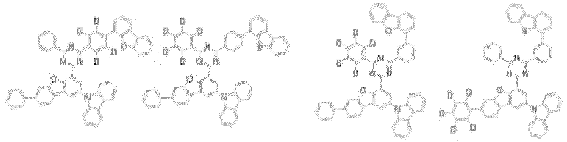

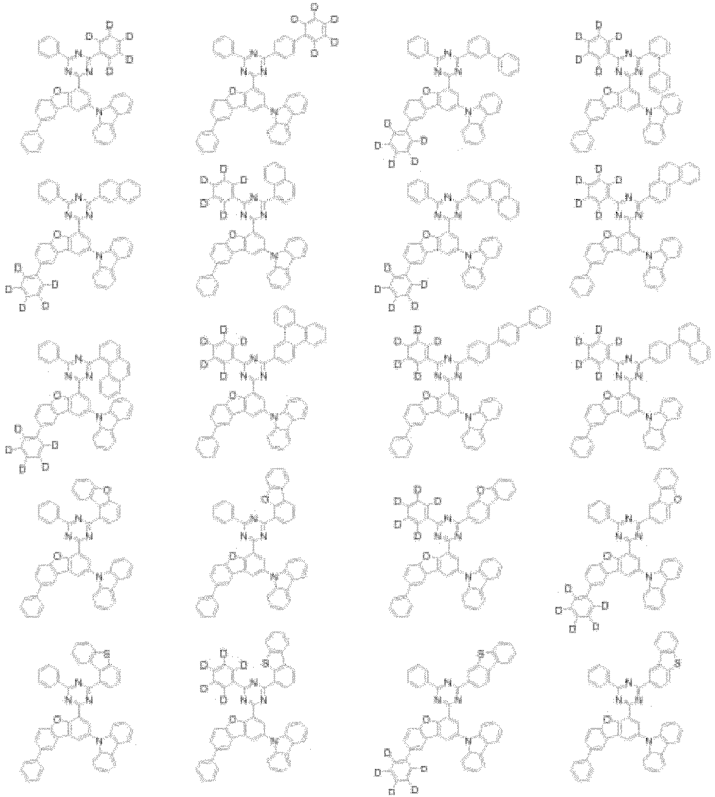

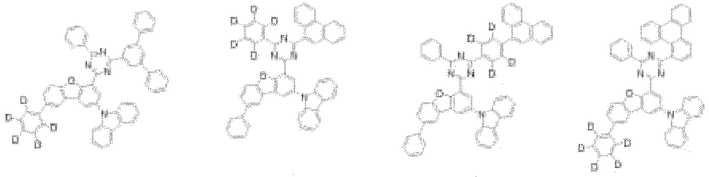

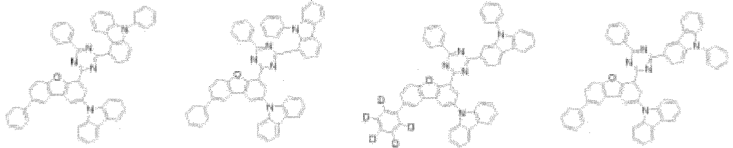

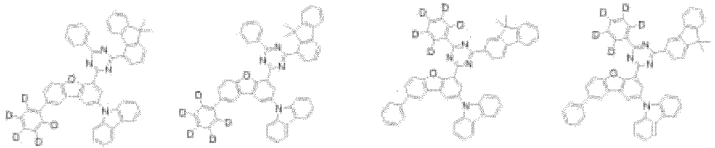

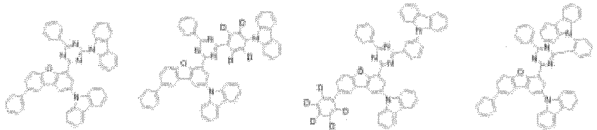

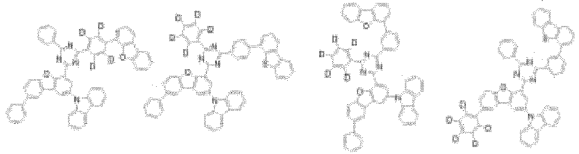

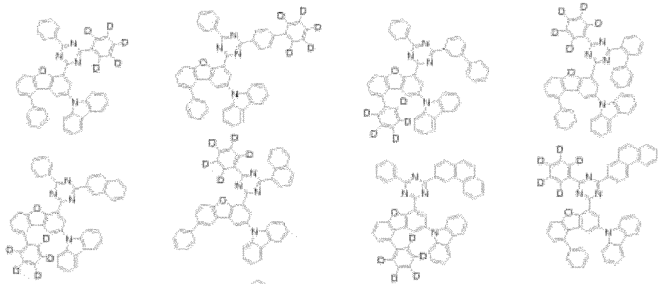

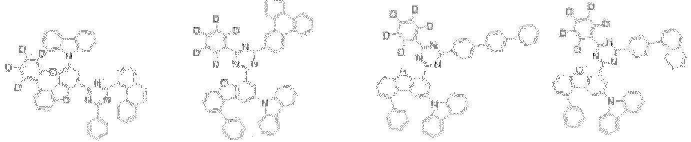

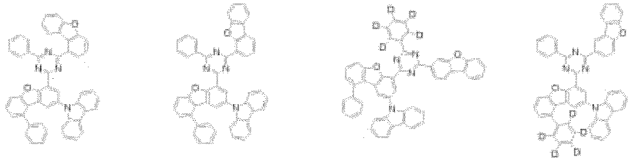

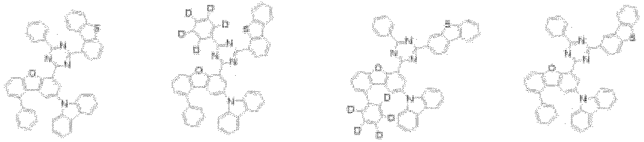

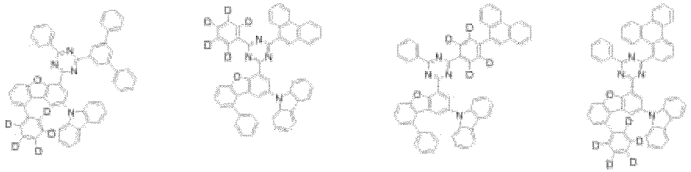

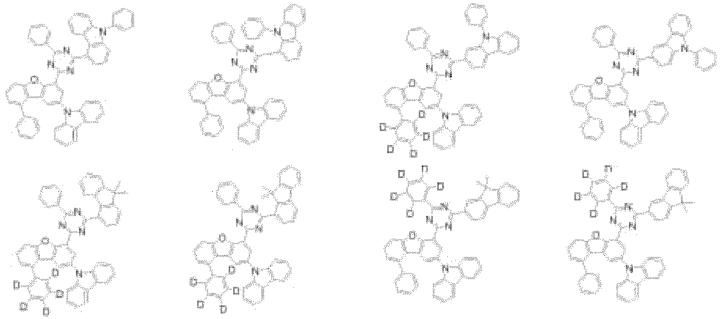

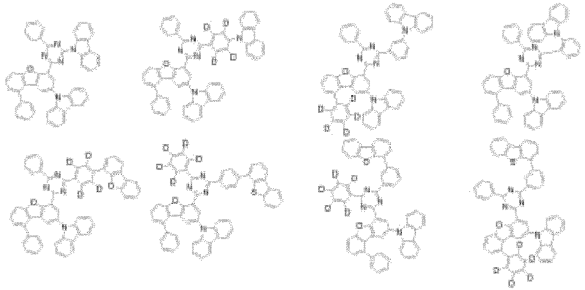

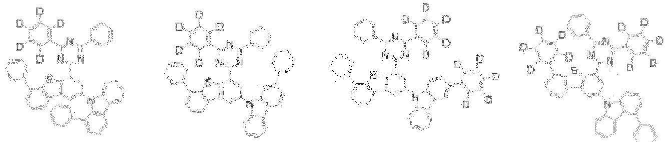

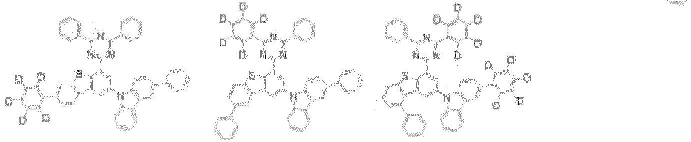

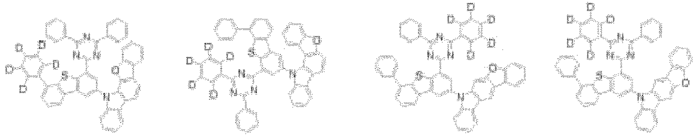

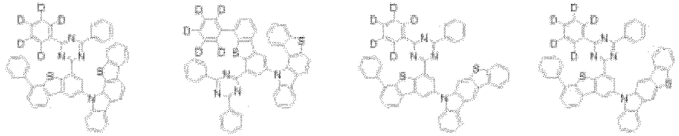

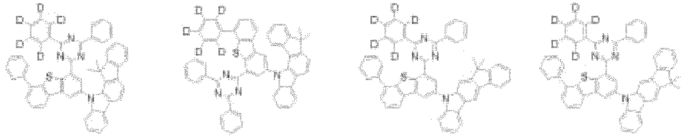

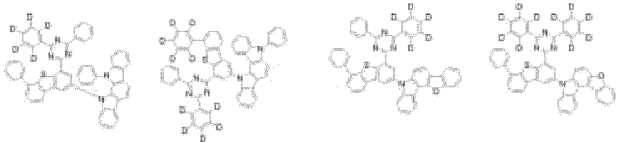

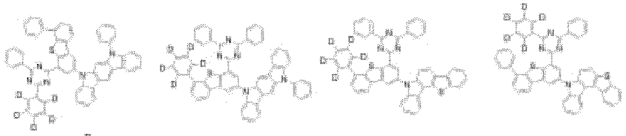

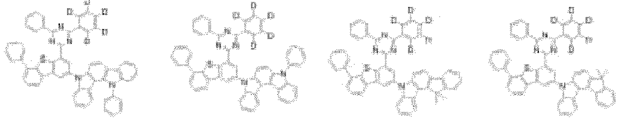

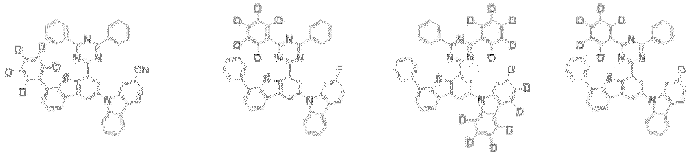

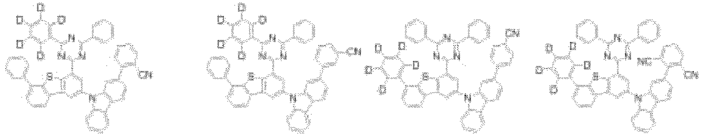

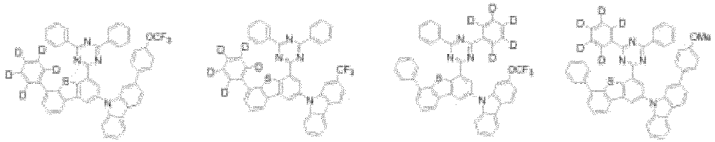

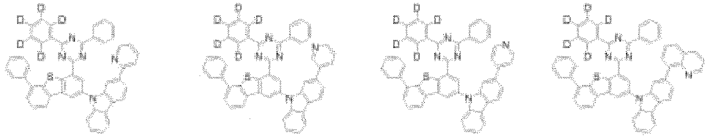

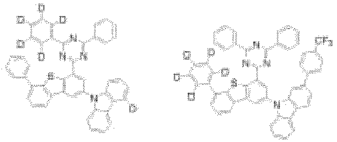

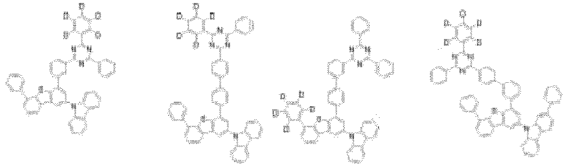

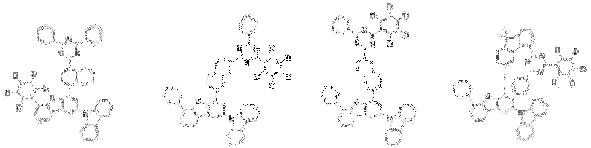

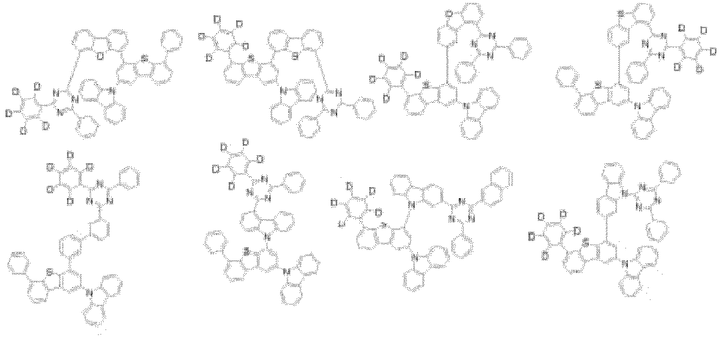

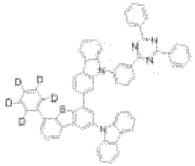

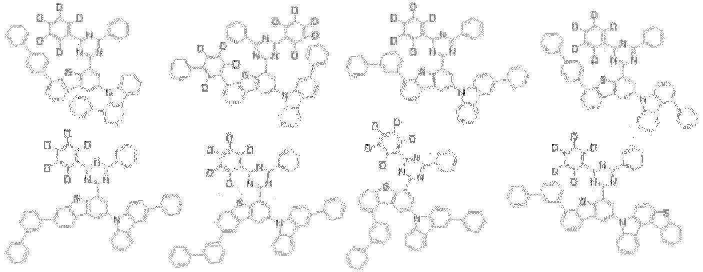

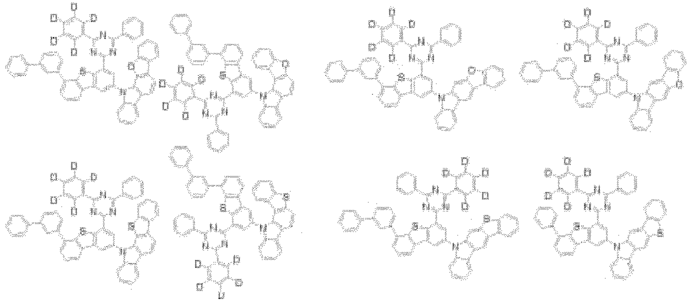

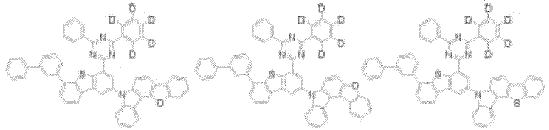

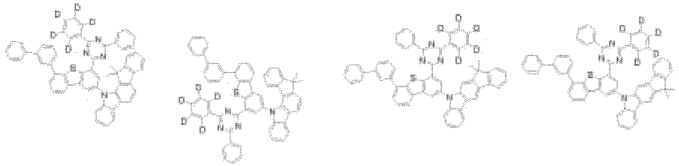

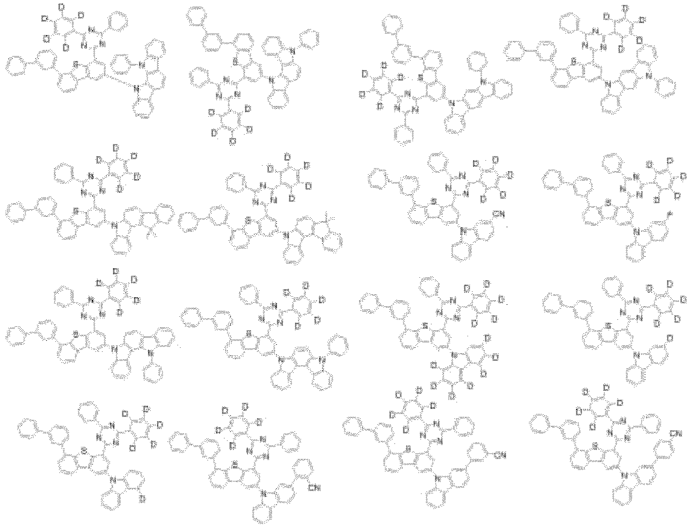

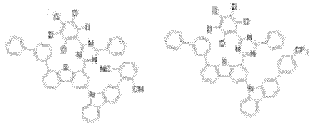

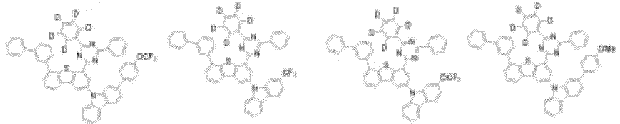

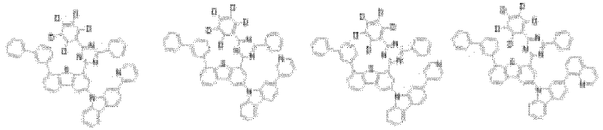

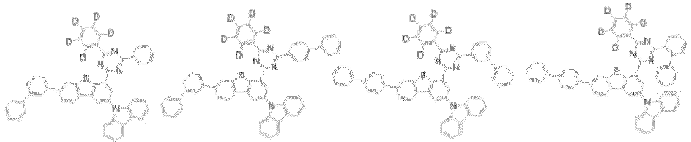

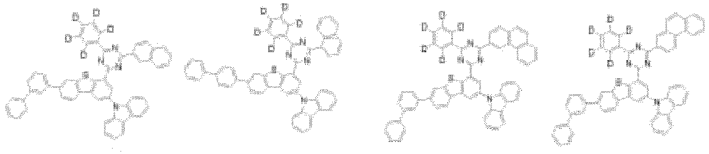

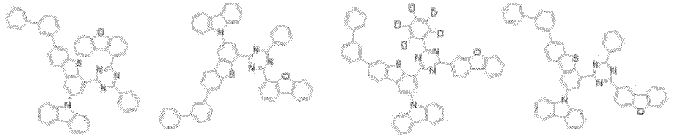

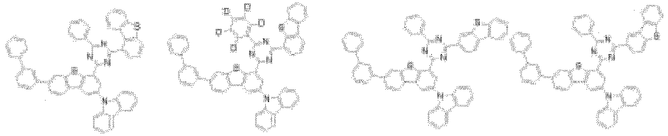

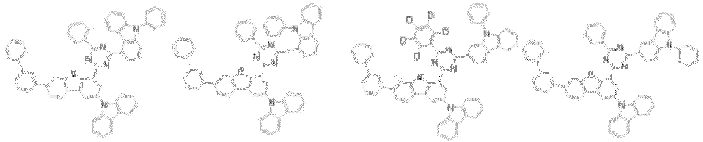

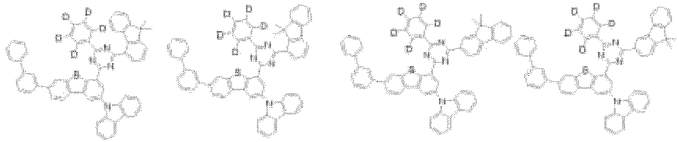

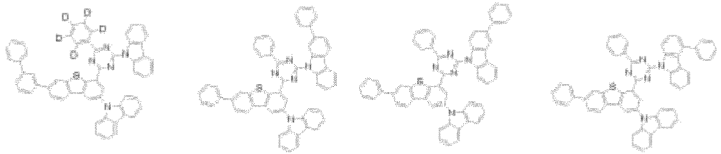

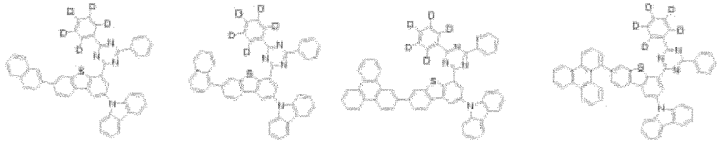

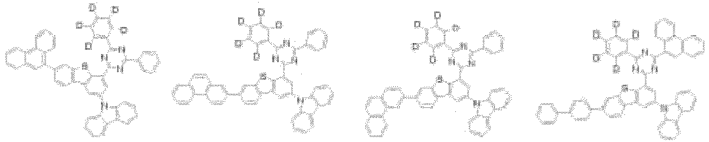

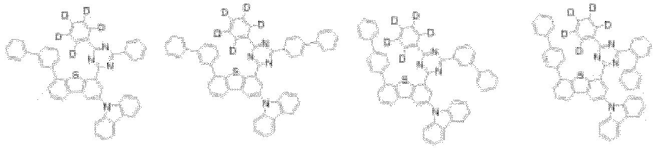

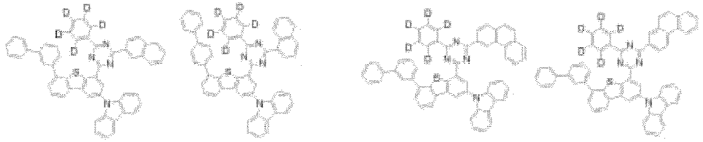

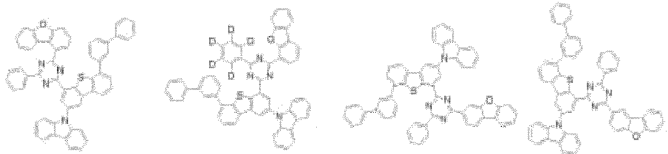

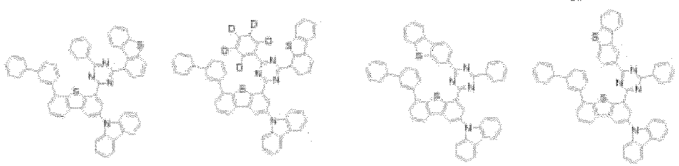

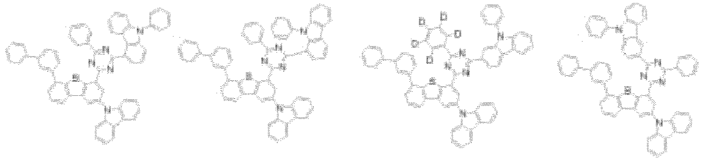

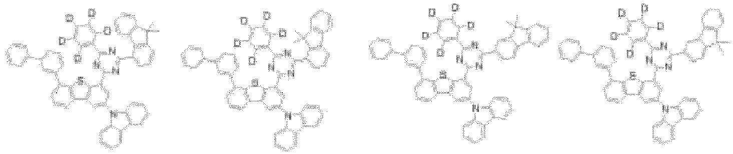

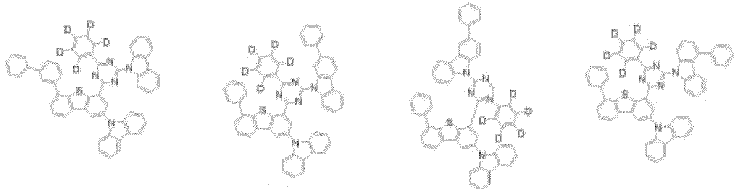

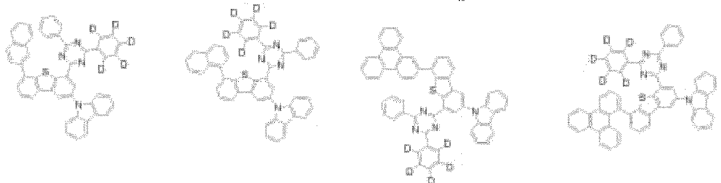

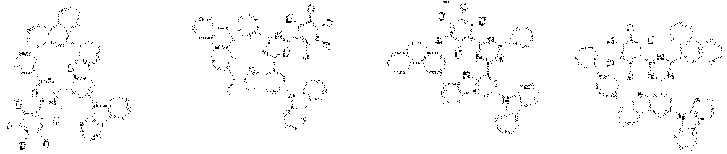

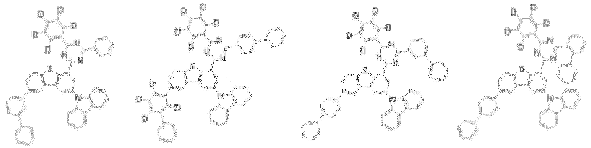

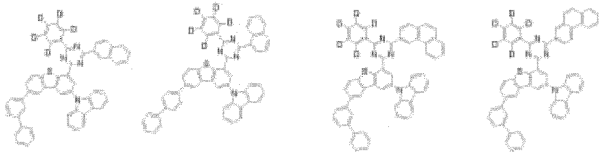

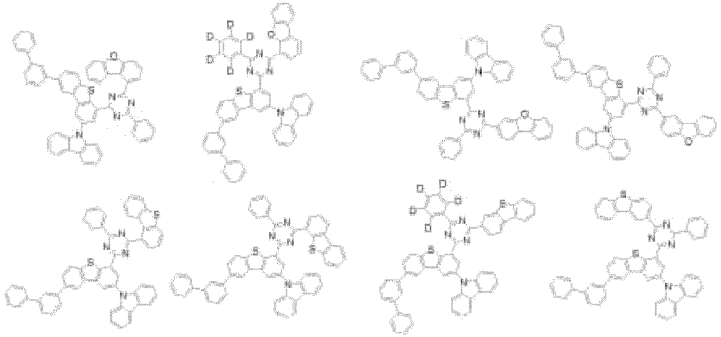

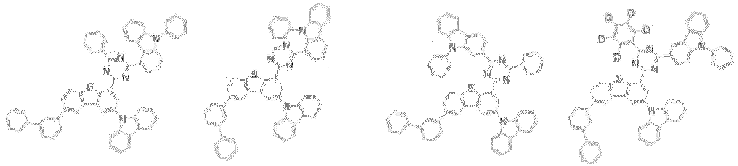

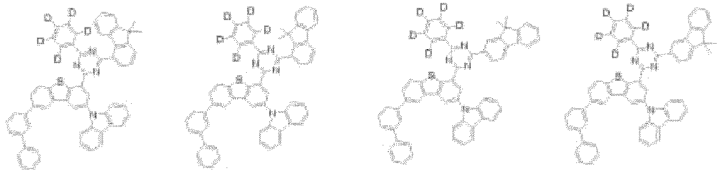

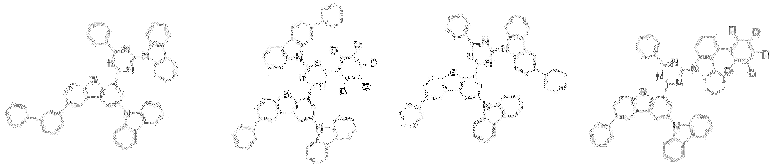

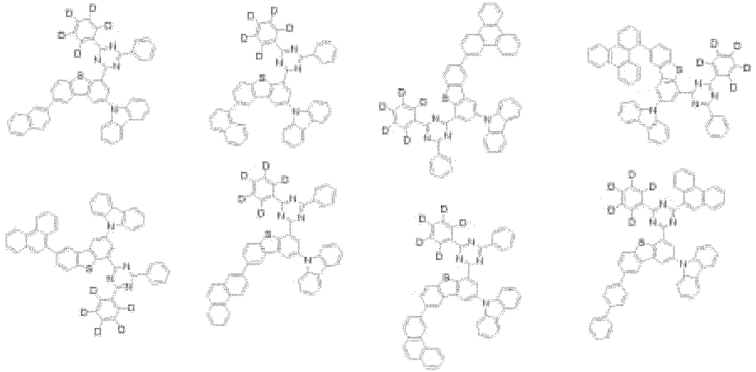

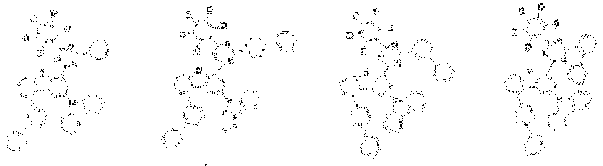

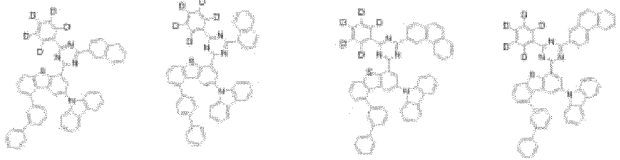

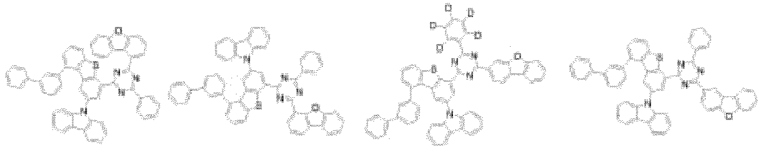

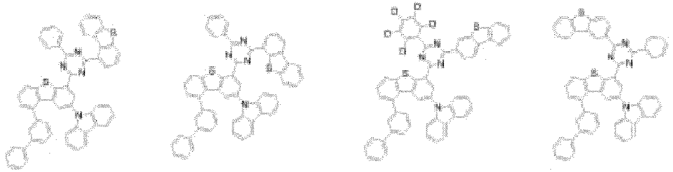

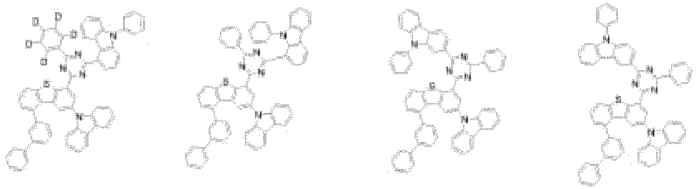

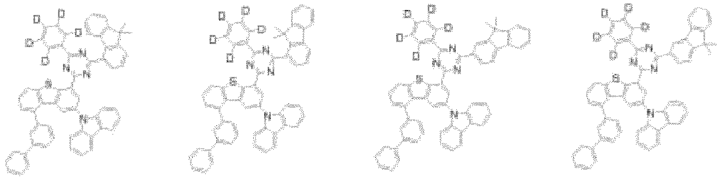

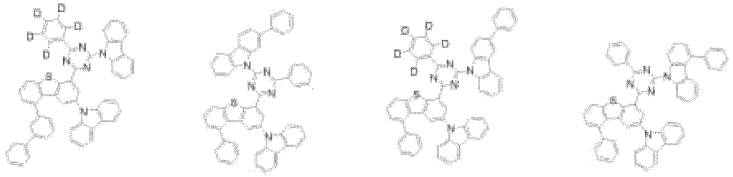

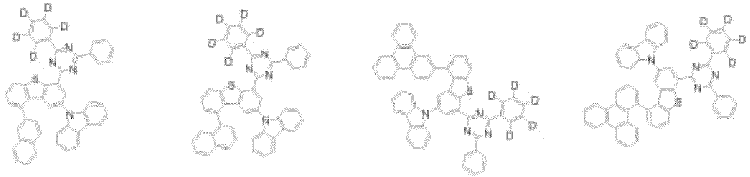

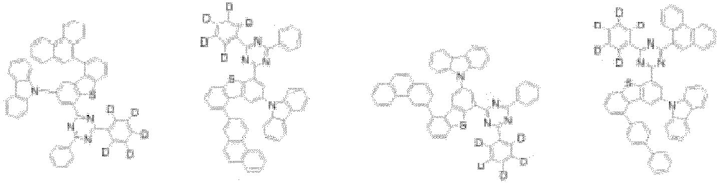

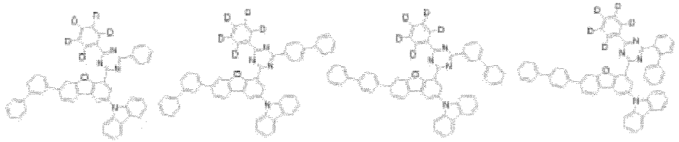

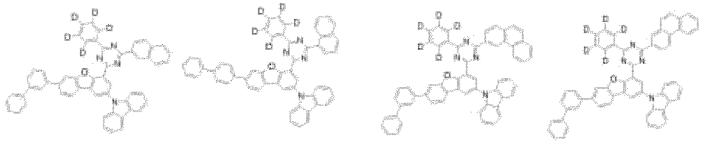

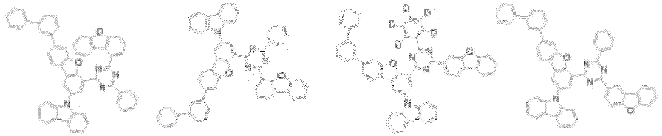

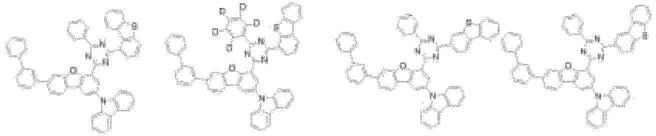

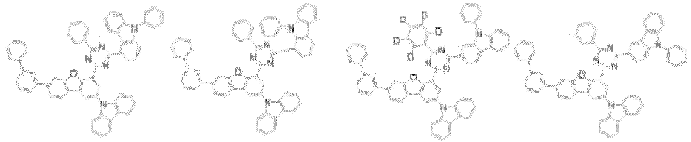

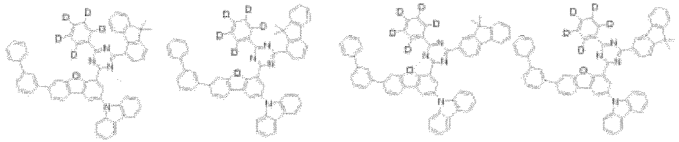

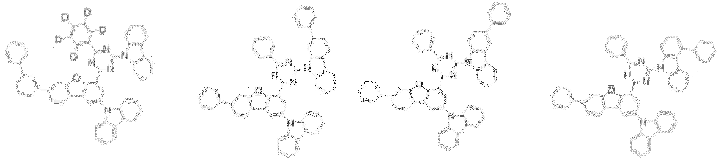

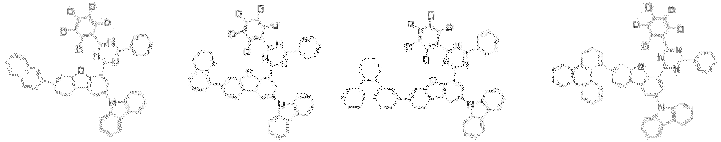

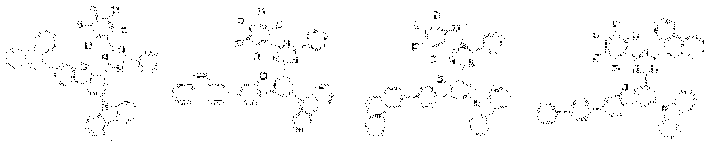

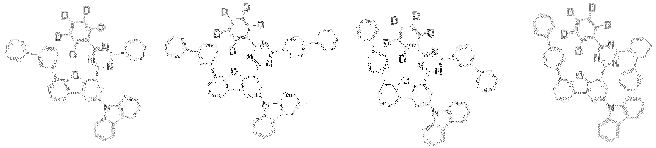

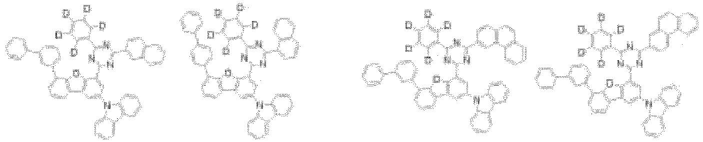

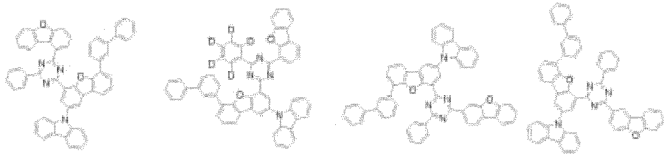

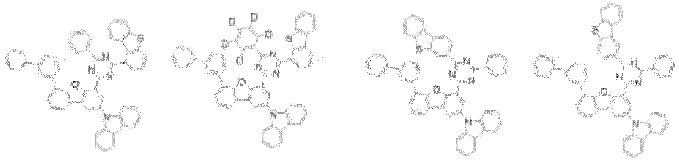

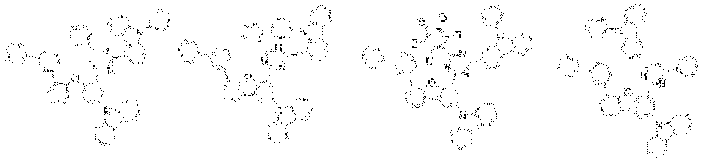

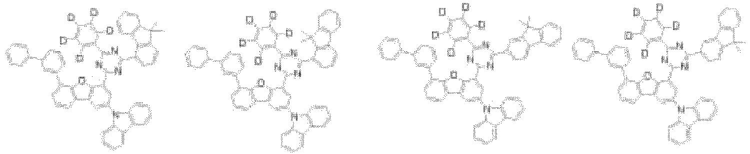

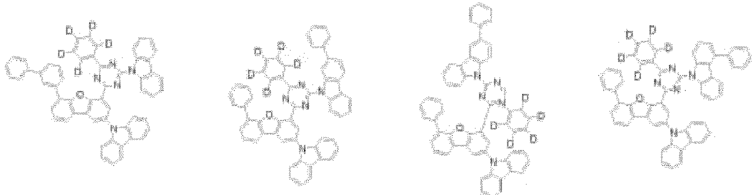

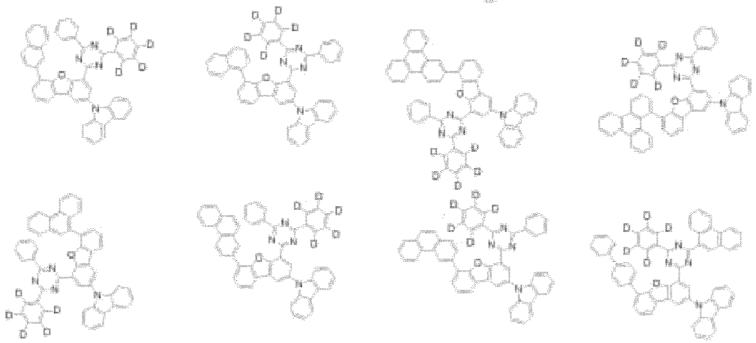

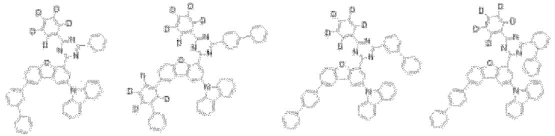

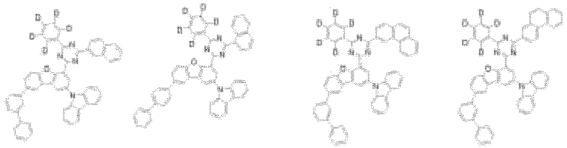

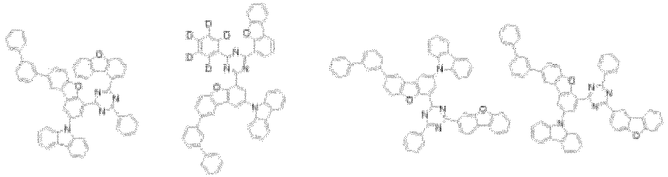

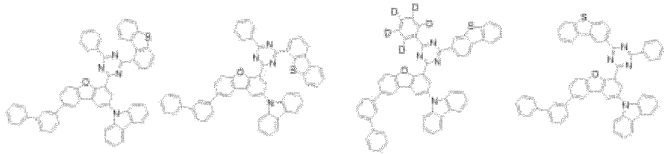

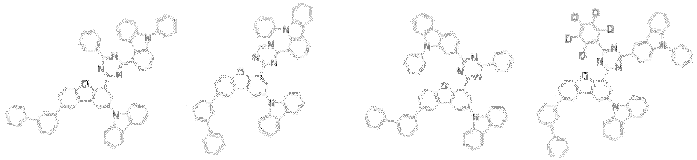

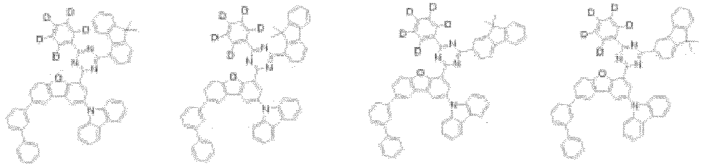

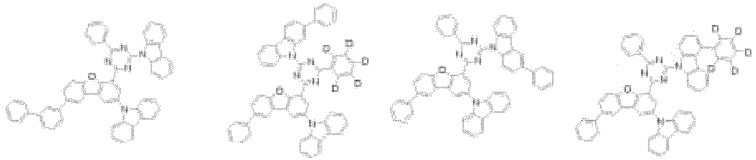

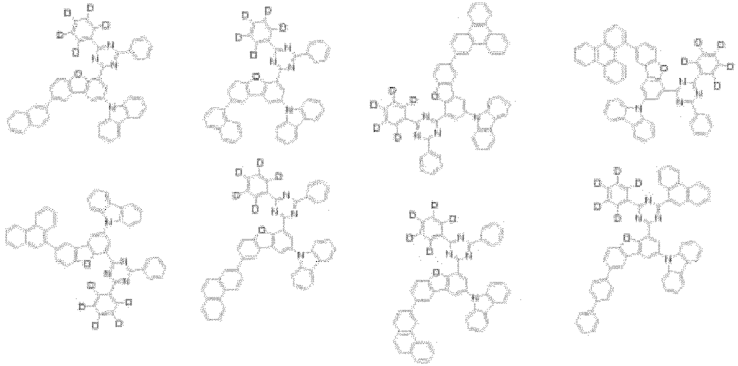

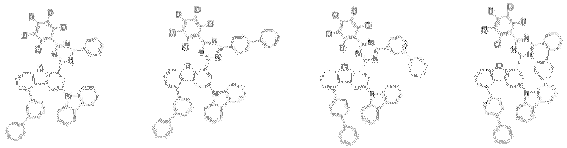

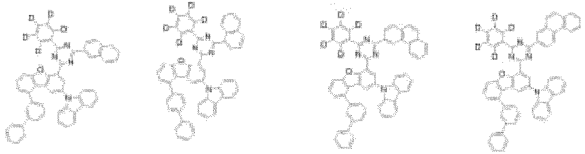

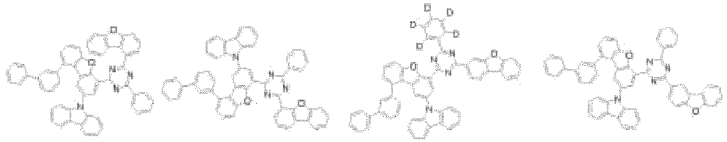

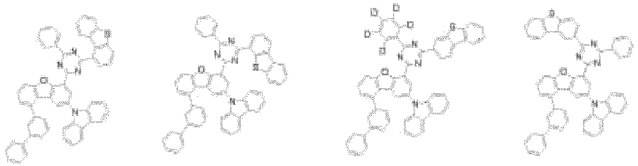

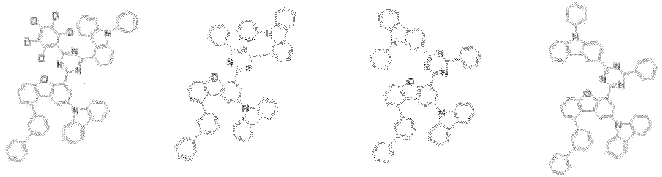

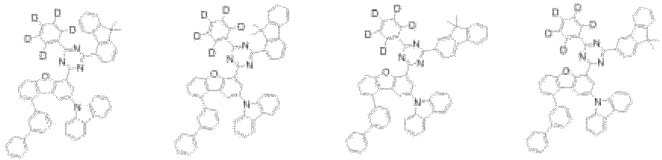

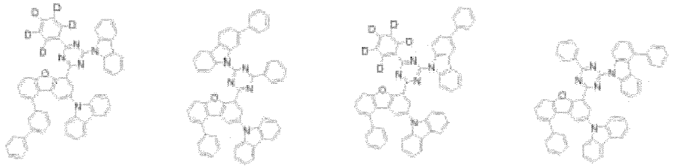

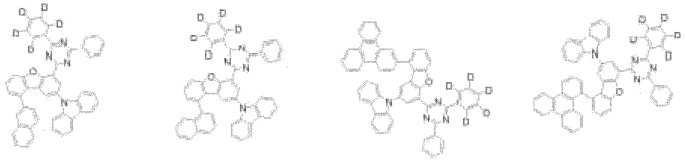

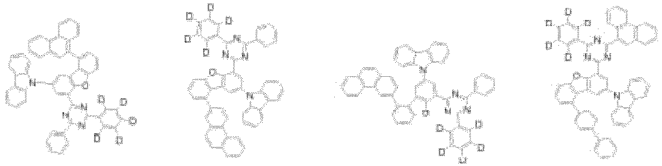

8. Dispositif électroluminescent organique incluant : une première électrode; une seconde électrode qui est fournie à l'opposé de la première électrode ; et une ou plusieurs couches de matériau organique qui sont fournies entre la première électrode et la seconde électrode, dans lequel une ou plusieurs couches des couches de matériau organique incluent le composé selon la revendication 1.
